# EUROPEAN PATENT SPECIFICATION

(11) **EP 2 399 611 B1**
(45) Date of publication and mention of the grant of the patent: **14.06.2017**
(21) Application number: 10181204.8
(22) Date of filing: 28.02.2005
(51) Int. Cl.: A61K 48/00, A61K 31/713, C12N 15/11, C07H 21/00, A61P 35/04, C12N 15/113

(54) **USE OF AN OLIGONUCLEOTIDE OR ITS ACTIVE DERIVATIVE FOR THE PREPARATION OF A PHARMACEUTICAL COMPOSITION FOR INHIBITING THE FORMATON OF METASATASES IN CANCER TREATMENT**
VERWENDUNG EINES OLIGONUKLEOTIDE ODER SEINER AKTIVEN ABLEITUNG FÜR DIE ZUBEREITUNG EINER PHARMAZEUTISCHE ZUSAMMENSETZUNG ZUR HEMMUNG DER METASTASENBILDUNG IN KREBSBEHANDLUNG
UTILISATION D'UN OLIGONUCLÉOTIDE OU D'UN DÉRIVÉ ACTIF POUR LA PRÉPARATION D'UNE COMPOSITION PHARMACEUTIQUE INHIBANT LA FORMATION DE MÉTASTASES DANS LE TRAITEMENT DU CANCER

(30) Priority: 01.04.2004 US 558135 P; 27.02.2004 EP 04004478
(43) Date of publication of application: 28.12.2011
(62) Divisional of application: 05715605.1
(73) Proprietor: Autotelic LLC, City of Industry, CA 91748 (US)
(72) Inventor: Schlingensiepen, Karl-Hermann, Dr., 93093 Donaustauf (DE); Schlingensiepen, Reimar, Dr., 93051 Regensburg (DE); Jachimczak, Piotr, Dr., 97074 Würzburg (DE); Hafner, Michael, Dr., 82549 Königsdorf (DE); Bischof, Astrid, Dr., 93049 Regensburg (DE); Bauer, Tamara, Dr. (geboren Egger), 3427 Utzenstorf (CH); Stauder, Gerhard, Dr., 82538 Geretsried (DE)
(74) Representative: V.O.

(56) References cited:
- EP-A1- 1 133 988
- WO-A-94/25588
- WO-A-99/63975
- SPEARMAN M ET AL: "Antisense oligodeoxyribonucleotide inhibition of TGF-beta1 gene expression and alterations in the growth and malignant properties of mouse fibrosarcoma cells", GENE, ELSEVIER BIOMEDICAL PRESS. AMSTERDAM, NL, vol. 149, 1994, pages 25-29, XP002089439, ISSN: 0378-1119
- HUANG FEI ET AL: "Transforming growth factor beta-1 (TGF-beta-1) is an autocrine positive regulator of colon carcinoma U9 cells in vivo as shown by transfection of a TGF-beta-1 antisense expression plasmid", CELL GROWTH AND DIFFERENTIATION, vol. 6, no. 12, 1995, pages 1635-1642, XP009049279, ISSN: 1044-9523
- JACHIMCZAK P ET AL: "TRANSFORMING GROWTH FACTOR-BETA-MEDIATED AUTOCRINE GROWTH REGULATION OF GLIOMAS AS DETECTED WITH PHOSPHOROTHIOATE ANTISENSE OLIGONUCLEOTIDES", INTERNATIONAL JOURNAL OF CANCER, NEW YORK, NY, US, vol. 65, no. 3, 26 January 1996 (1996-01-26), pages 332-337, XP000676566, ISSN: 0020-7136
- PICON ANTONIO ET AL: "A subset of metastatic human colon cancers expresses elevated levels of transforming growth factor beta1", CANCER EPIDEMIOLOGY BIOMARKERS AND PREVENTION, vol. 7, no. 6, June 1998 (1998-06), pages 497-504, XP009049280, ISSN: 1055-9965

## Description

This invention is related to effective medicaments in cancer therapy.

The formation of cancer on the one hand is combined with the unwanted growth of tissue and on the other hand is combined with the formation of metastases. The research in this field has disclosed a lot of mechanisms but still there is no therapy without severe side effects inhibiting metastases or inhibiting tumor progression in solid tumors respectively such as prostate cancer, bladder carcinoma, colon cancer, endometrial cancer, hepatocellular carcinoma, melanoma, leukemia, lymphoma, non-small cell lung cancer (NSCLC) or ovarian cancer. Further cancers in this field are mesothelioma, myeloma multiple, osteosarcoma, renal cancer, esophageal cancer or soft tissue cancer.

Tumor derived transforming growth factor beta (TGF-beta) is discussed to play a pivotal role for the malignant progression by inducing metastasis, angiogenesis and tumor cell proliferation. Furthermore, it seems to play a central role in the escape mechanism from the immune system in tumor cells.

But the role of TGF beta in the literature is diversely discussed. On the one hand there are experiments indicating that TGF-beta inhibits tumor growth, on the other hand there are experiments that point out that TGF-beta induces cell proliferation, which makes its role in the tumor therapy ambivalent.

An additional point is that TGF-beta has a lot of different subclasses, TGF-beta 1, TGF-beta 2 and TGF-beta 3 whose specific roles in tumor progression are differently discussed, often summarized as TGF-beta and thus sometimes mixed up. The specific role of each TGF-beta subclass, namely TGF-beta 1, TGF-beta 2 and TGF-beta 3 are not so far sufficiently investigated.

EP 1008 649 and EP 0695354 teaches that both, TGF-beta 1 and TGF-beta 2 antisense oligonucleotides can be used for manufacturing of a pharmaceutical composition for the treatment of breast tumor esophageal, gastric carcinomas and skin carcinogenesis. Whereas clinical studies seem to show, that in glioma the TGF-beta seems to play a key role and therefore the TGF-beta 2 antisense oligonucleotides are preferred targets for the treatment of e.g. glioma and breast cancer the Situation is completely different in other tumors.

In prostate cancer for example (Wikström, P., Scand J Urol Nephrol 34 S. 85-94), as well as in some other cancers, there are hints, that TGF-beta levels are increased, but it is not clear if this system can be manipulated for therapeutical purposes.

It is known from interleukin 10 (IL-10) that it plays a central role in the regulation of the immune response. Since it was shown, that some interleukin 10 antisense oligonucleotides can enhance cell-mediated immune response it was important to find potent inhibitors of IL-10 in human to modulate the immune response in a way, that escape mechanisms of tumor cells are compensated, tumor growth is inhibited and the formation of metastases is reduced.

Therefore it was a task of this invention to find appropriate therapeutics to inhibit the formation of metastases in pancreatic cancer.

Tumors such as bladder carcinoma, colon cancer, endometrial cancer, hepatocellular carcinoma, leukemia, lymphoma, melanoma, non-small cell lung cancer (NSCLC), ovarian cancer, pancreatic cancer and prostate cancer have poor prognosis and so far no successful therapy is found. This is also the case for tumors such as mesothelioma, myeloma multiple, osteosarcomna, renal cancer, esophageal cancer or soft tissue cancer. Therefore it was the task of this invention to find new therapeutics, that have the property specifically inhibiting the formation of metastases in pancreatic cancer and the use of these therapeutics for the treatment of the respective cancer.

The mechanism of antisense oligonucleotides seems to work by immunomodulating effects as well as by direct effects, which could be proofed in experimental studies. This is superior to state of the art inhibition of TGF-beta by e.g. antibodies, since we could show, that cell migration is more effectively inhibited by TGF-beta antisense oligonucleotides than it was possible with TGF-beta antibodies.

The pharmaceutical compositions of this invention have less side effects, show more efficacy, have more bioavailability, show more safety and/or improved chemical stability.

We surprisingly discovered, that antisense oligonucleotides of SEQ ID No. 30 that inhibit the formation of TGF-beta 1, TGF-beta 2, TGF-beta 3 inhibit the formation of metastases in pancreatic tumor cell lines and in tumors.

The antisense oligonucleotide of TGF-beta2 of SEQ ID No. 30 inhibits the tumor proliferation as described above for antisense oligonucleotides of TGF-beta 1, TGF-beta3 and/or interleukin.

A preferred embodiment of the invention relates to the use of at least one oligonucleotide or its active derivative for the preparation of a pharmaceutical composition for inhibiting the formation of metastases in pancreatic cancer.

In a further preferred embodiment, the oligonucleotide is an antisense oligonucleotide inhibiting the synthesis of proteins involved in the formation of metastases.

The oligonucleotide is identified in the sequence listing under SEQ ID NO.30.

Another preferred embodiment of the invention relates to the use of oligonucleotides or their active derivatives for the preparation of a pharmaceutical composition for the treatment of pancreatic cancer.

In a further preferred embodiment, the oligonucleotide is an antisense oligonucleotide inhibiting the production of transforming growth factor beta 1 (TGF-beta 1), TGF-beta 3 or is inhibiting the production of transforming growth factor TGF-beta2.

A preferred embodiment of the invention relates to the use of the antisense oligonucleotide identified in the sequence listing under SEQ ID NO.30 for the preparation of a pharmaceutical composition for the treatment of metastasis in pancreatic cancer.

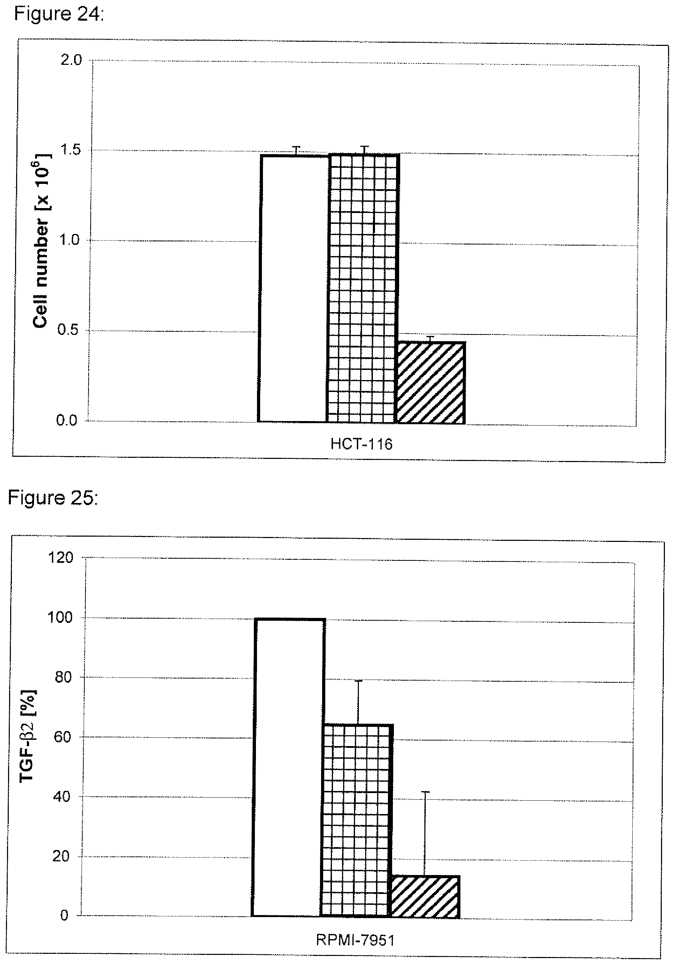

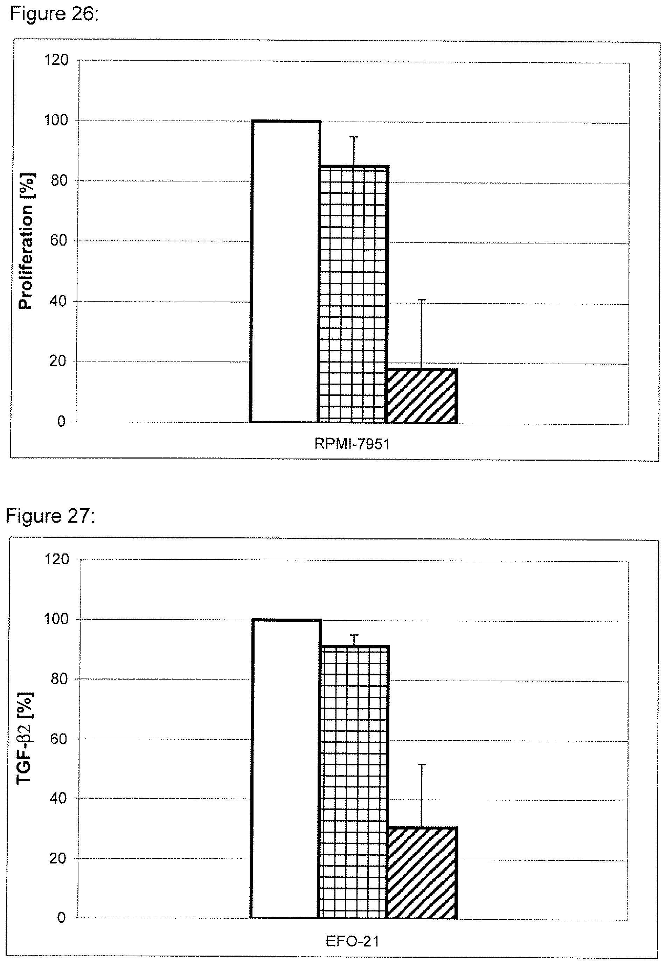

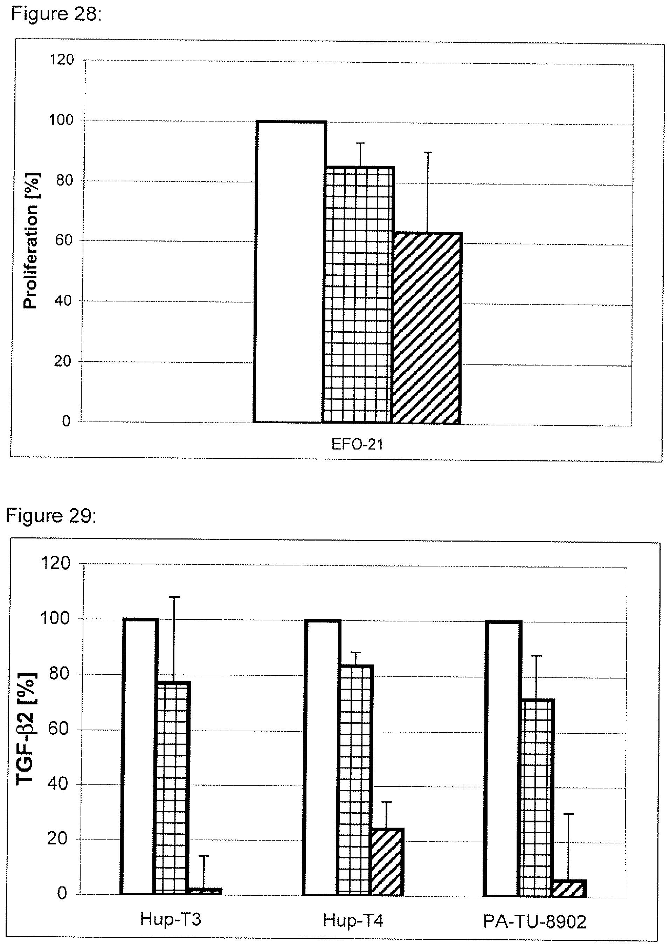

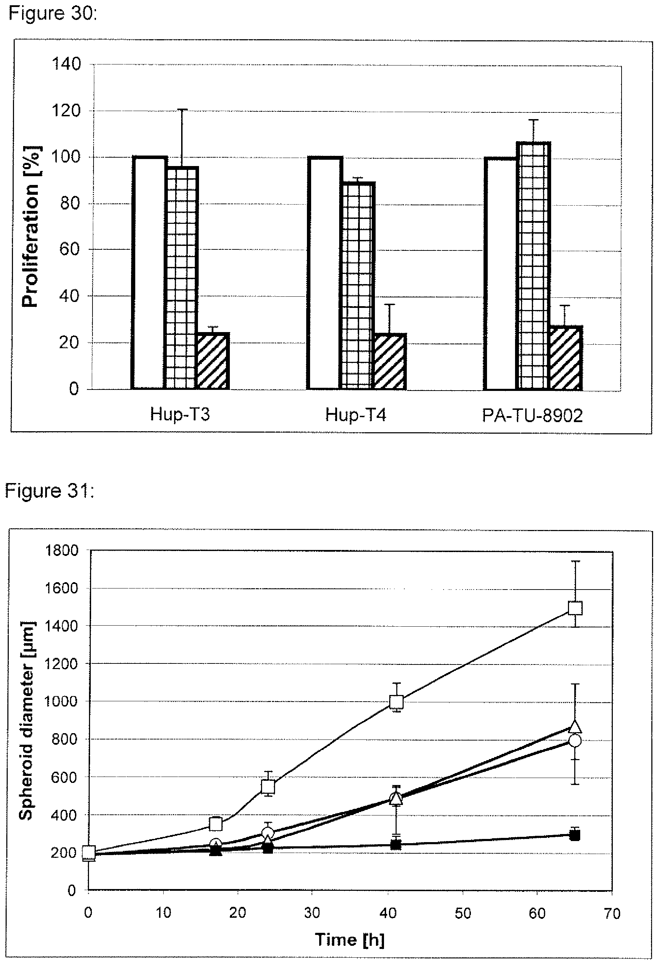

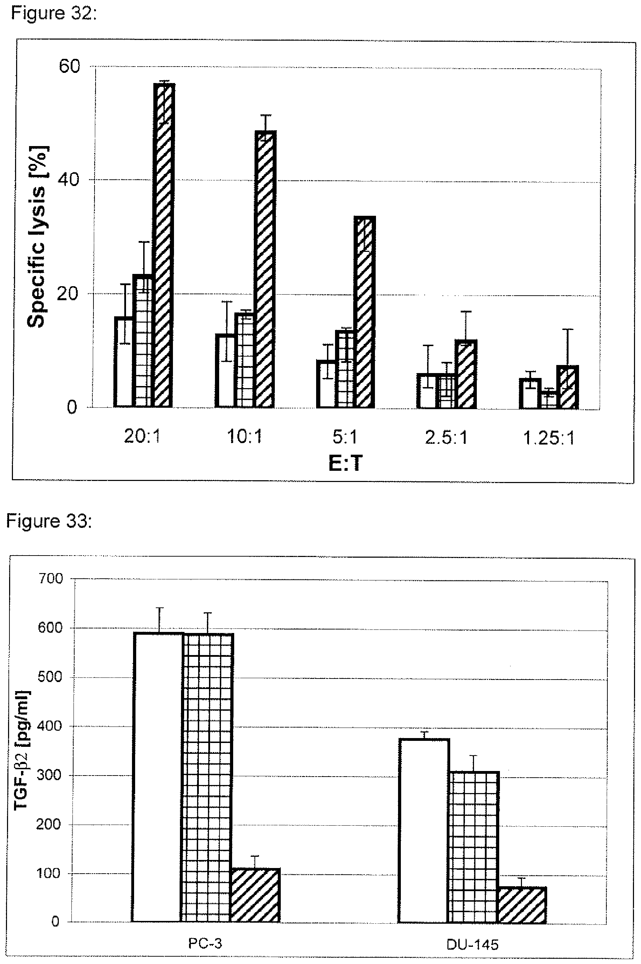

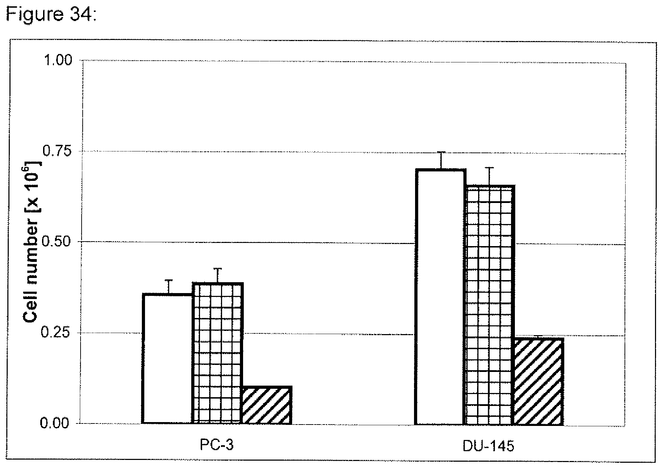

A preferred embodiment of the invention relates to the use of at least one oligonucleotide or its active derivatives of SEQ ID No. 30 for the treatment of pancreas carcinoma. The Figures present details of the technique, wherein Figures 24 to 34 refer to the present invention.
Figure 1:

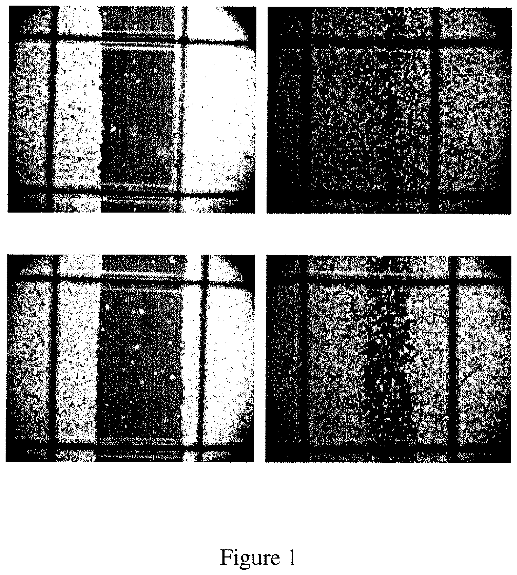

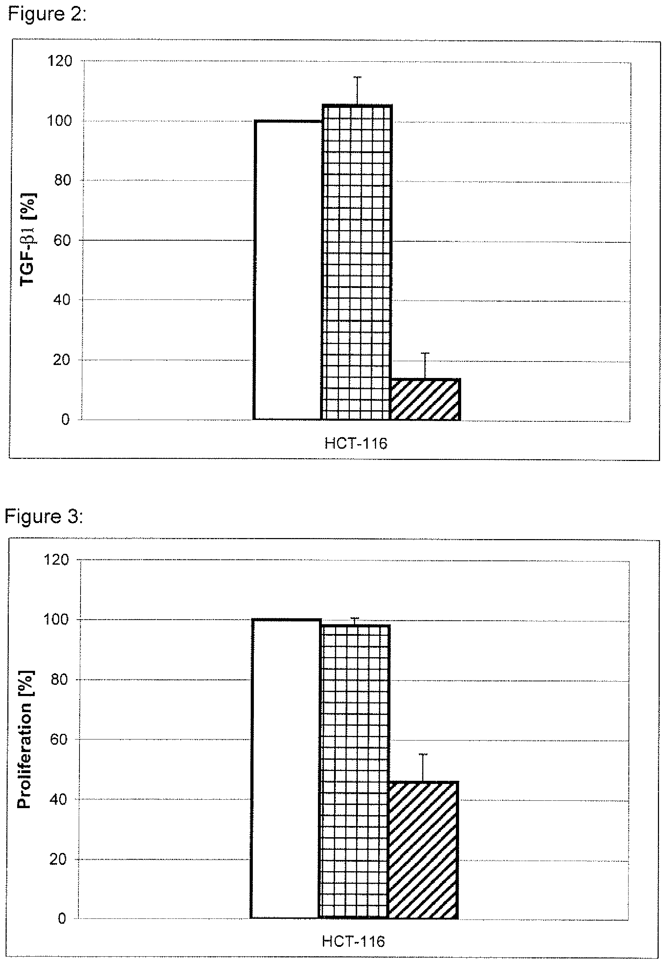

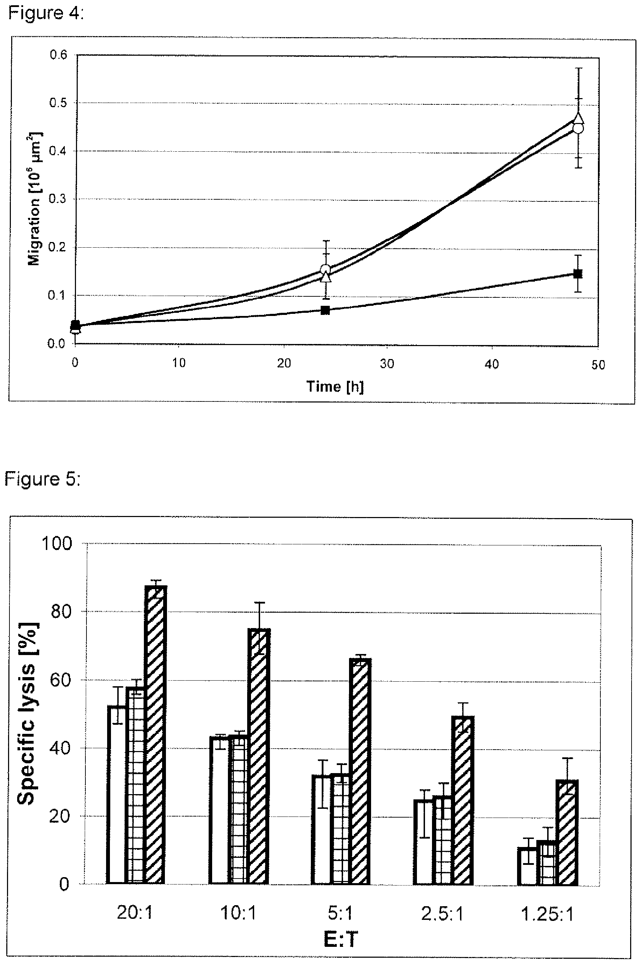

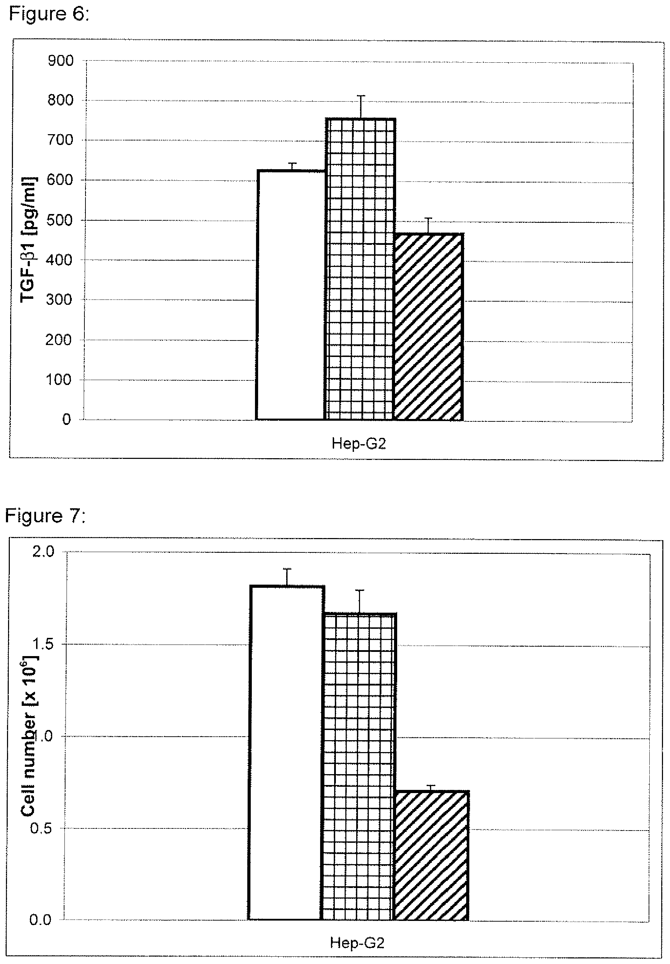

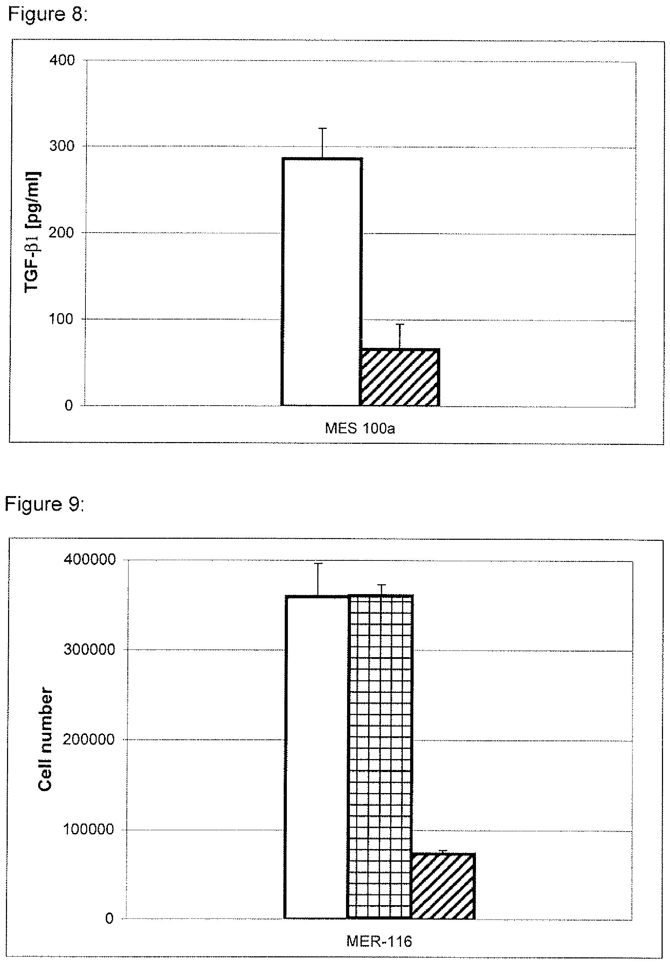

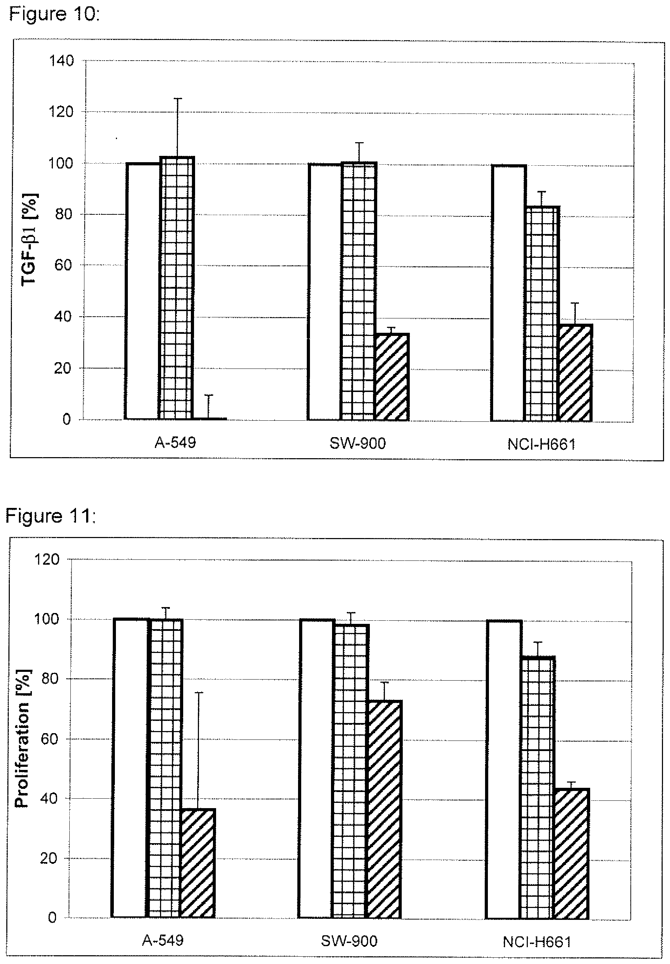

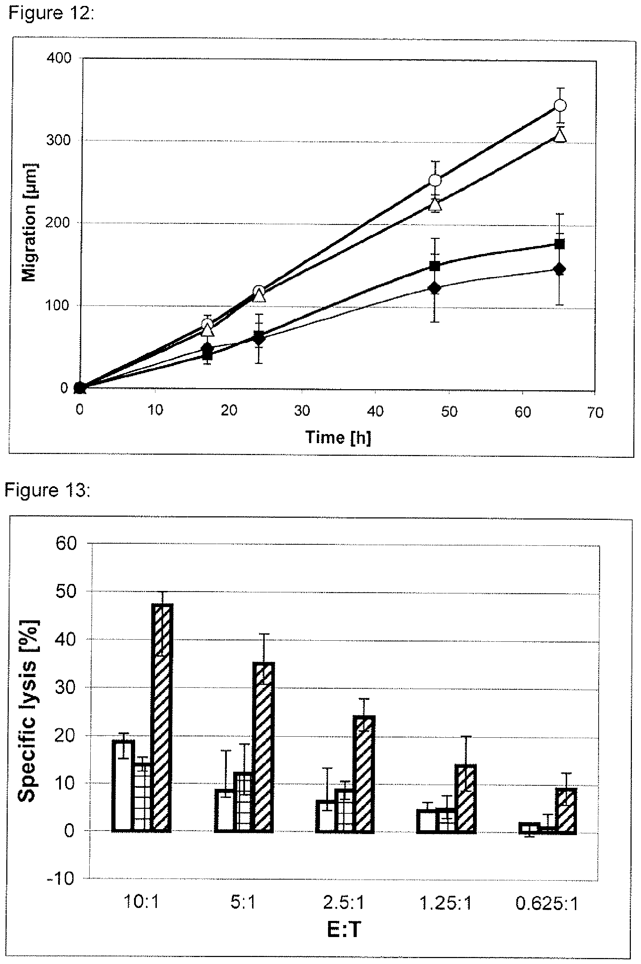

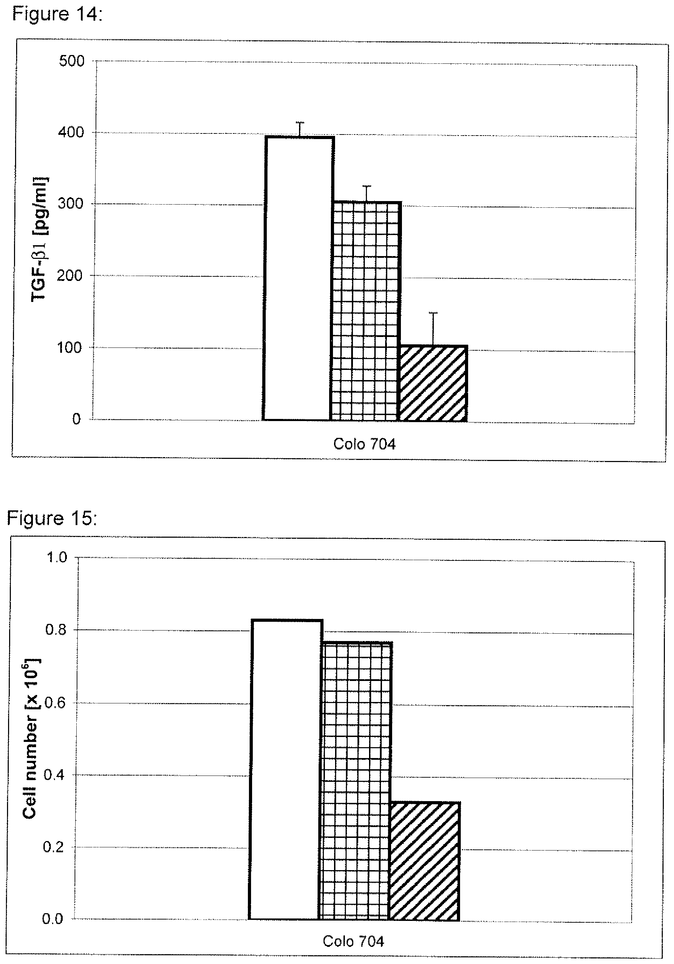

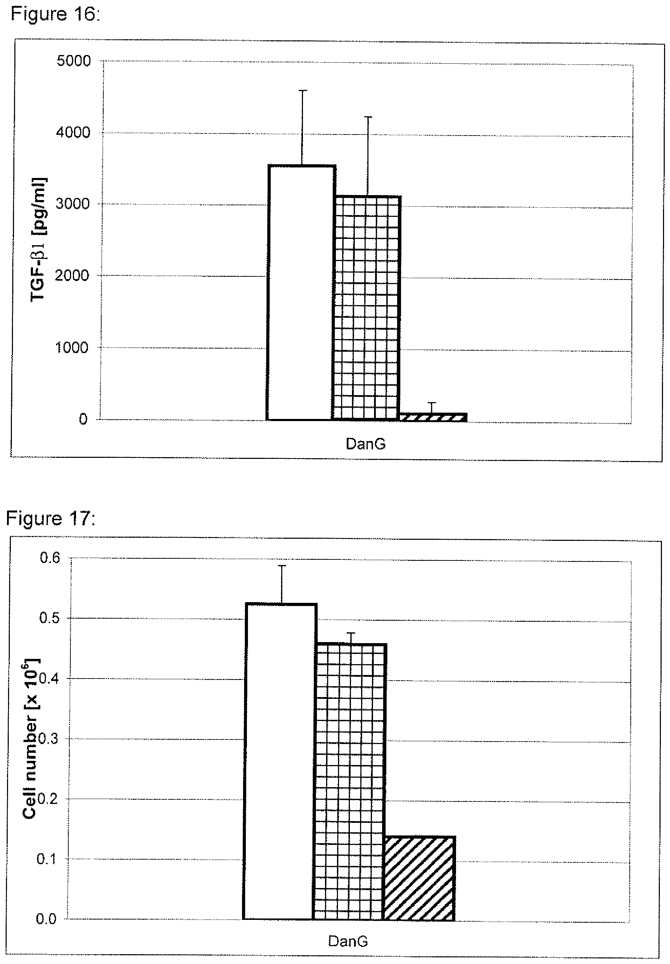

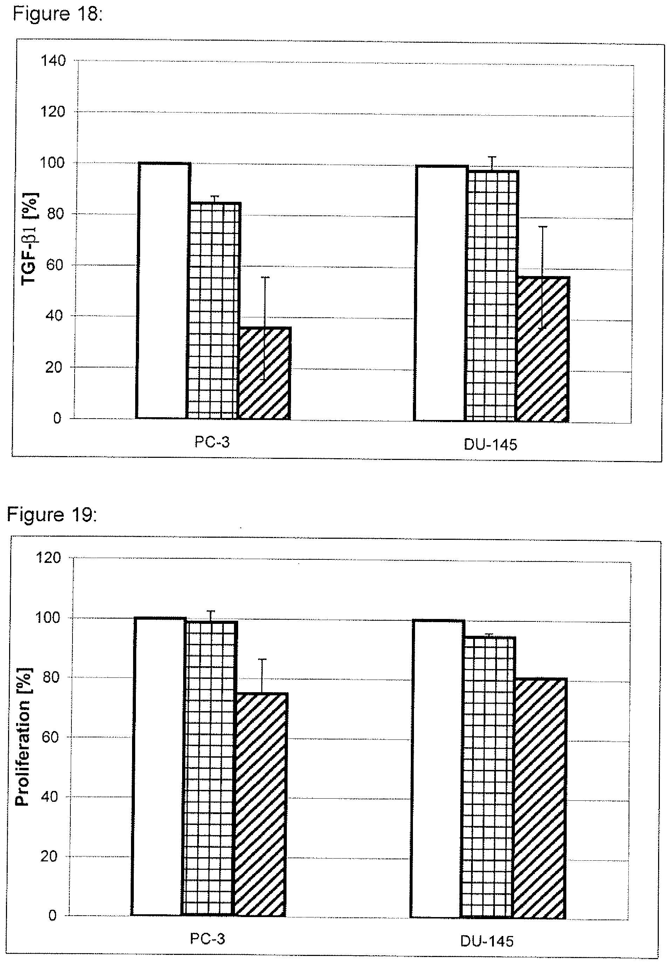

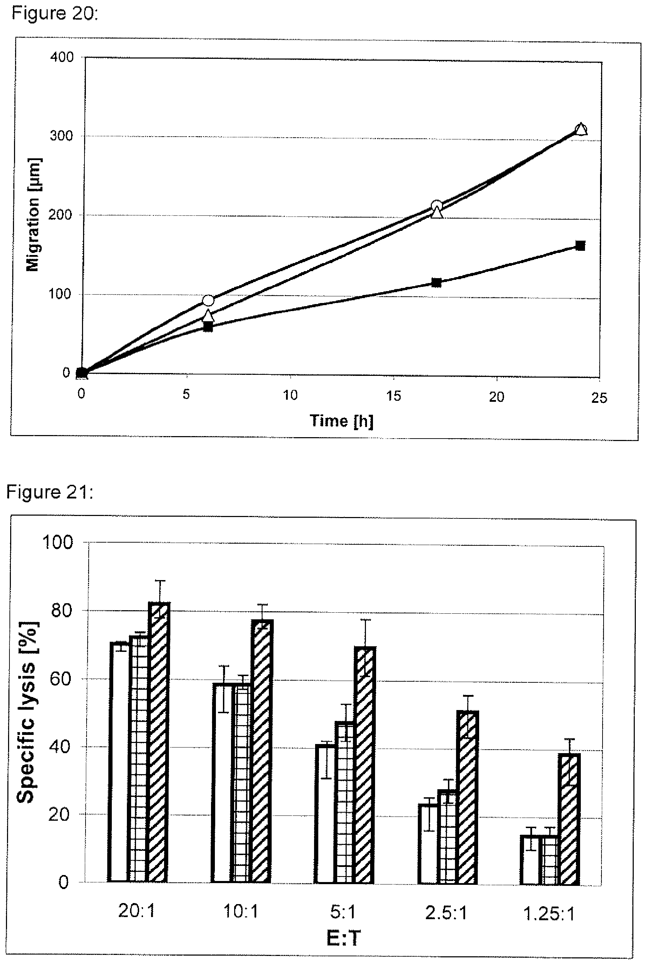

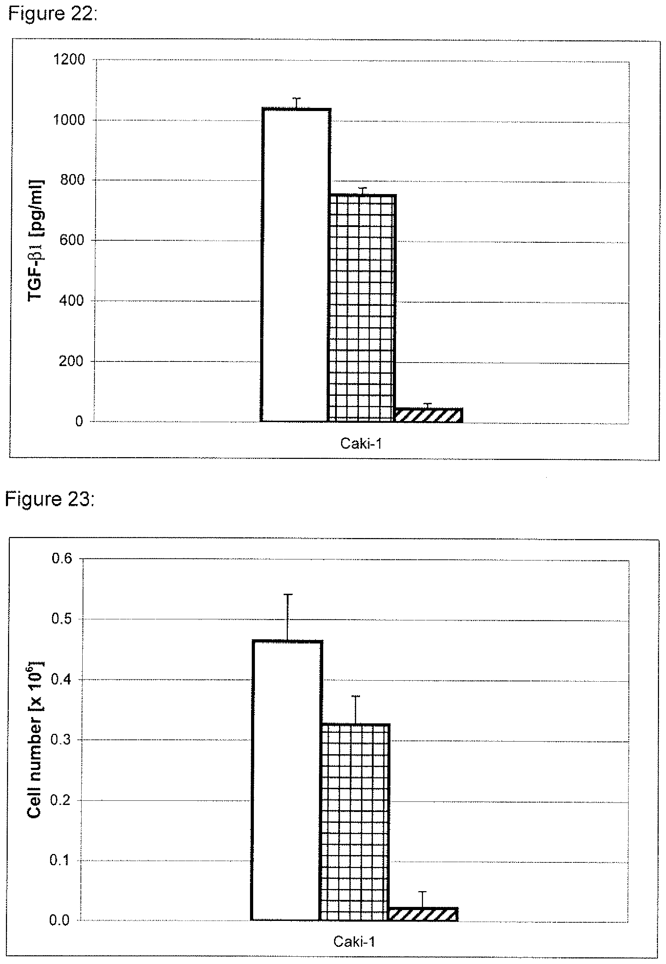

   Figure 1 shows the inhibition of prostate cancer cell Line PC-3. The upper two Squares indicate the migration of the control group incubated only with Lipofectin. The two Squares below clearly show reduced migration of cells incubated with Lipofectin and the antisense oligonucleotide identified in the Sequence listing with Seq. Id. No. 14. The two Squares left hand show the starting conditions. The two Squares at the right hand show the migration after 24 hours, which was clearly inhibited. This indicates a reduced formation of metastases.
Figure 2:
   PTO with Seq. Id. No. 14 inhibits TGF-beta1 secretion of HCT-116 CRC cells according to example 8. TGF-beta1 concentration in the supernatant of untreated cells (open bar) is set to 100%. TGF-beta1 concentration in supernatants of Lipofectin-treated cells (checkered bar) and PTO with Seq. Id. No. 14/Lipofectin-treated cells (diagonal striped bar) are demonstrated in % of the untreated control. Indicated are means and SD of three independent experiments.
Figure 3:
   PTO with Seq. Id. No. 14 inhibits proliferation of HCT-116 CRC cells according to example 8. Data of a tetrazolium-based proliferation assay (EZ4U assay) from untreated cells (open bar) are set to 100%. Data of Lipofectin-treated cells (checkered bar) and PTO with Seq. Id. No. 14/Lipofectin-treated cells (diagonal striped bar) are demonstrated in % of the untreated control. Indicated are means and SD of two independent experiments.
Figure 4:
   PTO with Seq. Id. No. 14 inhibits migration of HCT-116 CRC spheroids according to example 8. Areas of untreated spheroids (open cycles) at 0, 24, 48 h are represented in µm2. Areas of Lipofectin-treated spheroids are demonstrated as open triangles. Areas of PTO with Seq. Id. No. 14/Lipofectin-treated spheroids are demonstrated as closed squares. Indicated are means ± SD of at least duplicates.
Figure 5:
   PTO with Seq. Id. No. 14 enhances cell-mediated cytotoxicity of PBMC cultured in HCT-116 CRC cell supernatant according to example 18. Cell-mediated cytotoxicity on K562 target cells of PBMC cultured in supernatants of untreated HCT-116 cells (open bars) is determined by CARE-LASS assay at indicated effector:target cell ratios (E:T). Respectively, cell-mediated cytotoxicity on K562 target cells of PBMC cultured in supernatants of Lipofectin-treated HCT-116 cells is demonstrated in checkered bars and cell-mediated cytotoxicity on K562 target cells of PBMC cultured in supernatants of PTO with Seq. Id. No. 14/Lipofectin-treated HCT-116 cells is demonstrated in diagonal striped bars. Indicated are median, maxima and minima of quadruplicates.
Figure 6:
   PTO with Seq. Id. No. 14 inhibits TGF-beta1 secretion of Hep-G2 HCC cells according to example 9. TGF-beta1 concentration in the supernatant of untreated cells (open bar) is indicated in pg/ml. Respectively, TGF-beta1 concentration in supernatants of Lipofectin-treated cells is demonstrated as checkered bar and TGF-beta1 concentration in supernatants of PTO with Seq. Id. No. 14/Lipofectin-treated cells is demonstrated as diagonal striped bar. Indicated are means and SD of triplicates.
Figure 7:
   PTO with Seq. Id. No. 14 inhibits proliferation of Hep-G2 HCC cells according to example 9. Cell number of untreated cells determined by electronic cell counting is indicated as open bar. Respectively, cell number of Lipofectin-treated cells is demonstrated as checkered bar and cell number of PTO with Seq. Id. No. 14/Lipofectin-treated cells is demonstrated as diagonal striped bar. Indicated are means and SD of triplicates.
Figure 8:
   PTO with Seq. Id. No. 14 inhibits TGF-beta1 secretion of MES 100a melanoma cells according to example 10. TGF-beta1 concentration in the supernatant of untreated cells (open bar) is indicated in pg/ml. Respectively, TGF-beta1 concentration in supernatants of PTO with Seq. Id. No. 14-treated cells is demonstrated as diagonal striped bar. Indicated are means and SD of triplicates.
Figure 9:
   PTO with Seq. Id. No. 14 inhibits proliferation of MER-116 melanoma cells according to example 10. Cell number of untreated cells determined by electronic cell counting is indicated as open bar. Respectively, cell number of Lipofectin-treated cells is demonstrated as checkered bar and cell number of PTO with Seq. Id. No. 14/Lipofectin-treated cells is demonstrated as diagonal striped bar. Indicated are means and SD of triplicates.
Figure 10:
   PTO with Seq. Id. No. 14 inhibits TGF-beta1 secretion of A-549, SW-900, and NCI-H661 NSCLC cells according to example 11. TGF-beta1 concentration in the supernatants of untreated cells (open bars) are set to 100%. TGF-beta1 concentration in supernatants of Lipofectin-treated cells (checkered bars) and PTO with Seq. Id. No. 14/Lipofectin-treated cells (diagonal striped bars) are demonstrated in % of the untreated control. Indicated are means and SD of three independent experiments for each cell line.
Figure 11:
   PTO with Seq. Id. No. 14 inhibits proliferation of A-549, SW-900, and NCI-H661 NSCLC cells according to example 11. Data of a tetrazolium-based proliferation assay (EZ4U assay) from untreated cells (open bar) are set to 100%. Data of Lipofectin-treated cells (checkered bar) and PTO with Seq. Id. No. 14/Lipofectin-treated cells (diagonal striped bar) are demonstrated in % of the untreated control. Indicated are means and SD of at least two independent experiments for each cell line.
Figure 12:
   PTO with Seq. Id. No. 14 inhibits migration of SW-900 NSCLC cells according to example 11. Migration of untreated cells (open cycles) determined by scratch assay at 0, 17, 24, 48, and 65 h are represented in µm. Respectively, migration of Lipofectin-treated cells are demonstrated as open triangles and migration of PTO with Seq. Id. No. 14/Lipofectin-treated cells are demonstrated as closed squares (200 nM) or closed diamonds (400 nM). Indicated are means ± SD of three independent experiments.
Figure 13:
   PTO with Seq. Id. No. 14 enhances cell-mediated cytotoxicity of PBMC cultured in A-549 NSCLC cell supernatant according to example 18. Cell-mediated cytotoxicity on NCI-H661 target cells of PBMC cultured in supernatants of untreated A-549 cells (open bars) is determined by CARE-LASS assay at indicated effector:target cell ratios (E:T). Respectively, cell-mediated cytotoxicity on NCI-H661 target cells of PBMC cultured in supernatants of Lipofectin-treated A-549 cells is demonstrated in checkered bars and cell-mediated cytotoxicity on NCI-H661 target cells of PBMC cultured in supernatants of PTO with Seq. Id. No. 14/Lipofectin-treated A-549 cells is demonstrated in diagonal striped bars. Indicated are median, maxima and minima of quadruplicates.
Figure 14:
   PTO with Seq. Id. No. 14 inhibits TGF-betaI secretion of Colo 704 ovarian cancer cells according to example 12. TGF-betaI concentration in the supernatant of untreated cells (open bar) is indicated in pg/ml. Respectively, TGF-betaI concentration in supernatants of Lipofectin-treated cells is demonstrated as checkered bar and TGF-beta1 concentration in supernatants of PTO with Seq. Id. No. 14/Lipofectin-treated cells is demonstrated as diagonal striped bar. Indicated are means and SD of duplicates.
Figure 15:
   PTO with Seq. Id. No. 14 inhibits proliferation of Colo 704 ovarian cancer cells according to example 12. Cell number of untreated cells determined by counting in a Fuchs-Rosenthal hemacytometer is indicated as open bar. Respectively, cell number of Lipofectin-treated cells is demonstrated as checkered bar and cell number of PTO with Seq. Id. No. 14/Lipofectin-treated cells is demonstrated as diagonal striped bar. Indicated are data of single countings.
Figure 16:
   PTO with Seq. Id. No. 14 inhibits TGF-beta1 secretion of DanG pancreatic cancer cells according to example 13. TGF-beta1 concentration in the supernatant of untreated cells (open bar) is indicated in pg/ml. Respectively, TGF-beta1 concentration in supernatants of Lipofectin-treated cells is demonstrated as checkered bar and TGF-beta1 concentration in supernatants of PTO with Seq. Id. No. 14/Lipofectin-treated cells is demonstrated as diagonal striped bar. Indicated are means and SD of triplicates.
Figure 17:
   PTO with Seq. Id. No. 14 inhibits proliferation of DanG pancreatic cancer cells according to example 13. Cell number of untreated cells determined by electronic cell counting is indicated as open bar. Respectively, cell number of Lipofectin-treated cells is demonstrated as checkered bar and cell number of PTO with Seq. Id. No. 14/Lipofectin-treated cells is demonstrated as diagonal striped bar. Indicated are means and SD of triplicates.
Figure 18:
   PTO with Seq. Id. No. 14 inhibits TGF-beta1 secretion of PC-3 and DU-145 prostate cancer cells according to example 14. TGF-beta1 concentration in the supernatants of untreated cells (open bars) are set to 100%. TGF-beta1 concentration in supernatants of Lipofectin-treated cells (checkered bars) and PTO with Seq. Id. No. 14/Lipofectin-treated cells (diagonal striped bars) are demonstrated in % of the untreated control. Indicated are means and SD of three independent experiments for each cell line.
Figure 19:
   PTO with Seq. Id. No. 14 inhibits proliferation of PC-3 and DU-145 prostate cancer cells according to example 14. Data of a tetrazolium-based proliferation assay (EZ4U assay) from untreated cells (open bar) are set to 100%. Data of Lipofectin-treated cells (checkered bar) and PTO with Seq. Id. No. 14/Lipofectin-treated cells (diagonal striped bar) are demonstrated in % of the untreated control. Indicated are means and SD of at least two independent experiments for each cell line.
Figure 20:
   PTO with Seq. Id. No. 14 inhibits migration of PC-3 prostate cancer cells according to example 14. Migration of untreated cells (open cycles) determined by scratch assay at 0, 6, 17, and 24 h are represented in µm. Respectively, migration of Lipofectin-treated cells are demonstrated as open triangles and migration of PTO with Seq. Id. No. 14/Lipofectin-treated cells are demonstrated as closed squares. Indicated are means of three independent experiments.
Figure 21:
   PTO with Seq. Id. No. 14 enhances cell-mediated cytotoxicity of PBMC cultured in PC-3 prostate cancer cell supernatant according to example 18. Cell-mediated cytotoxicity on K562 target cells of PBMC cultured in supernatants of untreated PC-3 cells (open bars) is determined by CARE-LASS assay at indicated effector:target cell ratios (E:T). Respectively, cell-mediated cytotoxicity on K562 target cells of PBMC cultured in supernatants of Lipofectin-treated PC-3 cells is demonstrated in checkered bars and cell-mediated cytotoxicity on K562 target cells of PBMC cultured in supernatants of PTO with Seq. Id. No. 14/Lipofectin-treated PC-3 cells is demonstrated in diagonal striped bars. Indicated are median, maxima and minima of quadruplicates.
Figure 22:
   PTO with Seq. Id. No. 14 inhibits TGF-beta1 secretion of Caki-1 renal cancer cells according to example 15. TGF-beta1 concentration in the supernatant of untreated cells (open bar) is indicated in pg/ml. Respectively, TGF-beta1 concentration in supernatants of Lipofectin-treated cells is demonstrated as checkered bar and TGF-beta1 concentration in supernatants of PTO with Seq. Id. No. 14/Lipofectin-treated cells is demonstrated as diagonal striped bar. Indicated are means and SD of triplicates.
Figure 23:
   PTO with Seq. Id. No. 14 inhibits proliferation of Caki-1 renal cancer cells according to example 15. Cell number of untreated cells determined by electronic cell counting is indicated as open bar. Respectively, cell number of Lipofectin-treated cells is demonstrated as checkered bar and cell number of PTO with Seq. Id. No. 14/Lipofectin-treated cells is demonstrated as diagonal striped bar. Indicated are means and SD of triplicates.
Figure 24:
   PTO with Seq. Id. No. 30 inhibits proliferation of HCT-116 CRC cells according to example 8. Cell number of untreated cells determined by electronic cell counting is indicated as open bar. Respectively, cell number of Lipofectin-treated cells is demonstrated as checkered bar and cell number of PTO with Seq. Id. No. 30/Lipofectin-treated cells is demonstrated as diagonal striped bar. Indicated are means and SD of triplicates.
Figure 25:
   PTO with Seq. Id. No. 30 inhibits TGF-beta2 secretion of RPMI-7951 melanoma cells according to example 10. TGF-beta2 concentration in the supernatant of untreated cells (open bar) is set to 100%. TGF-beta2 concentration in supernatants of Lipofectin-treated cells (checkered bar) and PTO with Seq. Id. No. 30/Lipofectin-treated cells (diagonal striped bar) are demonstrated in % of the untreated control. Indicated are means and SD of four independent experiments.
Figure 26:
   PTO with Seq. Id. No. 30 inhibits proliferation of RPMI-7951 melanoma cells according to example 10. Cell number of untreated cells (open bar) determined by electronic cell counting is set to 100%. Cell number of Lipofectin-treated cells (checkered bar) and PTO with Seq. Id. No. 30/Lipofectin-treated cells (diagonal striped bar) are demonstrated in % of the untreated control. Indicated are means and SD of four independent experiments.
Figure 27:
   PTO with Seq. Id. No. 30 inhibits TGF-beta2 secretion of EFO-21 ovarian cancer cells according to example 12. TGF-beta2 concentration in the supernatant of untreated cells (open bar) is set to 100%. TGF-beta2 concentration in supernatants of Lipofectin-treated cells (checkered bar) and PTO with Seq. Id. No. 30/Lipofectin-treated cells (diagonal striped bar) are demonstrated in % of the untreated control. Indicated are means and SD of three independent experiments.
Figure 28:
   PTO with Seq. Id. No. 30 inhibits proliferation of EFO-21 ovarian cancer cells according to example 12. Cell number of untreated cells (open bar) determined by electronic cell counting is set to 100%. Cell number of Lipofectin-treated cells (checkered bar) and PTO with Seq. Id. No. 30/Lipofectin-treated cells (diagonal striped bar) are demonstrated in % of the untreated control. Indicated are means and SD of three independent experiments.
Figure 29:
   PTO with Seq. Id. No. 30 inhibits TGF-beta2 secretion of Hup-T3, Hup-T4, and PA-TU-8902 pancreatic cancer cells according to example 13. TGF-beta2 concentration in the supernatants of untreated cells (open bars) are set to 100%. TGF-beta2 concentration in supernatants of Lipofectin-treated cells (checkered bars) and PTO with Seq. Id. No. 30/Lipofectin-treated cells (diagonal striped bars) are demonstrated in % of the untreated control. Indicated are means and SD of three independent experiments for each cell line.
Figure 30:
   PTO with Seq. Id. No. 30 inhibits proliferation of Hup-T3, Hup-T4, and PA-TU-8902 pancreatic cancer cells according to example 13. Data of a tetrazolium-based proliferation assay (EZ4U assay) or electronic cell counting from untreated cells (open bar) are set to 100%. Data of Lipofectin-treated cells (checkered bar) and PTO with Seq. Id. No. 30/Lipofectin-treated cells (diagonal striped bar) are demonstrated in % of the untreated control. Indicated are means and SD of three independent experiments for each cell line.
Figure 31:
   PTO with Seq. Id. No. 30 inhibits migration of PA-TU-8902 pancreatic cancer spheroids according to example 13. Diameter of untreated spheroids (open cycles) at 0, 17, 24, 41, and 65 h are represented in µm. Diameter of PTO with Seq. Id. No. 30-treated spheroids are demonstrated as closed squares. Diameter of rh TGF-beta2-treated spheroids are demonstrated as open squares. Diameter of anti TGF-beta2 antibody-treated spheroids are demonstrated as open triangles. Indicated are median, maxima and minima of at least quadruplicates.
Figure 32:
   PTO with Seq. Id. No. 30 enhances cell-mediated cytotoxicity of PBMC cultured in PA-TU-8902 pancreatic cancer cell supernatant according to example 18. Cell-mediated cytotoxicity on Hup-T3 target cells of PBMC cultured in supernatants of untreated PA-TU-8902 cells (open bars) is determined by CARE-LASS assay at indicated effector:target cell ratios (E:T). Respectively, cell-mediated cytotoxicity on Hup-T3 target cells of PBMC cultured in supernatants of Lipofectin-treated PA-TU-8902 cells is demonstrated in checkered bars and cell-mediated cytotoxicity on Hup-T3 target cells of PBMC cultured in supernatants of PTO with Seq. Id. No. 30/Lipofectin-treated PA-TU-8902 cells is demonstrated in diagonal striped bars. Indicated are median, maxima and minima of quadruplicates.
Figure 33:
   PTO with Seq. Id. No. 30 inhibits TGF-beta2 secretion of PC-3 and DU-145 prostate cancer cells according to example 14. TGF-beta2 concentration in the supernatants of untreated cells (open bar) are indicated in pg/ml. Respectively, TGF-beta1 concentrations in supernatants of Lipofectin-treated cells are demonstrated as checkered bar and TGF-beta2 concentrations in supernatants of PTO with Seq. Id. No. 30/Lipofectin-treated cells are demonstrated as diagonal striped bar. Indicated are means and SD of triplicates.
Figure 34:
   PTO with Seq. Id. No. 30 inhibits proliferation of PC-3 and DU-145 prostate cancer cells according to example 14. Cell numbers of untreated cells determined by electronic cell counting are indicated as open bar. Respectively, cell numbers of Lipofectin-treated cells are demonstrated as checkered bar and cell numbers of PTO with Seq. Id. No. 14/Lipofectin-treated cells are demonstrated as diagonal striped bar. Indicated are means and SD of triplicates.

In one embodiment the oligonucleotides or active derivatives of this invention are antisense oligonucleotides of SEQ ID No. 30 inhibiting the formation of metastases in pancreatic cancer. These oligonucleotides are used for the preparation of pharmaceutical compositions. These pharmaceutical compositions are used for the treatment of metastases in pancreatic cancer. Metastases in the context of this invention means that at least one cell separates or dissociates from a tumor tissue and is moving by e.g. the lymphatic system and/or the blood vessels to another part of the body of a human or an animal, where it settles down and forms new tumor tissue.

In another embodiment the oligonucleotides of SEQ ID No. 30 or their active derivatives are antisense oligonucleotides inhibiting the synthesis of proteins involved in the formation of metastases in pancreatic cancer.

The proteins inhibited in their synthesis are selected from the group of e.g. tumor growth factor beta 1 (TGF-beta 1), TGF-beta 2, and/or TGF-beta 3.

In the context of this invention tumor growth factor is used as a synonym to transforming growth factor. The term TGF-beta comprises in particular TGF-beta1, TGF-beta2 and/or TGF-beta3.

One embodiment of the invention is the use of at least one oligonucleotide of SEQ ID No. 30 or its active derivatives of TGF-beta 1, TGF-beta 2, TGF-beta 3 for the preparation of a pharmaceutical composition for inhibiting the formation of metastases in cancer treatment.

Active derivatives of this invention are modifications of the oligonucleotides as described below.

The at least one antisense oligonucleotide or its active derivative for the preparation of a pharmaceutical composition for inhibiting the formation of metastases in pancreatic cancer is listed under SEQ ID NO 30.

In the context of this invention the use of cancer is synonymous with carcinoma.

The oligonucleotide of SEQ ID No. 30 respectively their active derivatives are described in more detail later in this text.

In yet another embodiment the oligonucleotide of SEQ ID No. 30 or its active derivative for the preparation of a pharmaceutical composition for the treatment of the pancreatic cancer as described above is an antisense oligonucleotide inhibiting the production of tumor growth factor beta 1 respectively transforming growth factor (TGF-beta 1), and/or TGF-beta 3..

In another embodiment the oligonucleotide of SEQ ID No. 30 or its active derivative for the treatment of pancreatic cancer as described above and/or the preparation of a pharmaceutical composition for the treatment of the pancreatic cancer as described above is an antisense olgionculeotide of SEQ ID No. 30 inhibiting the production of transforming growth factor beta 2 (TGF-beta2).

This sequence has especially high affinity to the m-RNA of the respective gene, has less side effects, shows more efficacy, has more bioavailability, shows more safety and/or improved chemical stability. Another aspect of this invention is the use of TGF-beta2 antagonists for the treatment of pancreas cancer. Though some of these tumors are accompanied with high levels of TGF-beta1 surprisingly the inhibition of TGF-beta 2 reduces tumor cell proliferation significantly, which is an important factor for the treatment of these cancers.

TGF-beta2 (transforming growth factor 2) antagonists in the context of this invention comprises the antisense oligonucleotide of SEQ ID No. 30 that inhibit the function TGF-beta2, which means that any effect that is induced by TGF-beta is inhibited.

The terms oligonucleotide or nucleic acid refer to multiple nucleotides (i.e. molecules comprising a sugar (e.g. ribose or deoxyribose) linked to a phosphate group and to an variable organic base, which is either a substituted pyrimidine (e.g. cytosine (C), thymine (T) or uracil (U)) or a substituted purine (e.g. adenine (A) or guanine (G)) or a modification thereof. As used herein, the terms refer to oligoribonucleotides as well as oligodeoxyribonucleotides. The terms shall also include oligonucleosides (i.e. a oligonucleotide without the phosphate) and any other organic base containing polymer.

Whereas the oligonucleotides of this invention are single stranded, in some embodiments at least parts of the single-stranded nucleic acid is double-stranded. Double-stranded molecules may be more stable in vivo, while single-stranded molecules may have increased activity.

In one embodiment the antisense oligonucleotide is complementary to the whole m-RNA of the gene or any smaller part of the protein which shall be inhibited can be selected, e.g. TGF-beta 1 TGF-beta 2, and/or TGF-beta 3. In more preferred embodiments the antisense oligonucleotide of SEQ ID No. 30 has length from about 8 to about 30 nucleotides, even more preferred from 12 to 24 nucleotides.

In one embodiment the respective ends of this linear polymeric structure can be further joined to form a circular structure. However, open linear structures are generally preferred. Within the oligonucleotides structure, the phosphate groups are commonly referred to as forming the internucleoside backbone of the oligonucleotide. The normal linkage or backbone of RNA and DNA is a 3'to 5'phophodiester linkage.

Oligonucleotides or nucleic acids includes oligonucleotides having non-naturally-occurring portions with similar function. Such modified or substituted oligonucleotides are often preferred over native forms because of desirable properties such as, for example, enhanced cellular uptake, enhanced affinity for nucleic acid target (e.g. protein), altered intracellular localization and increased stability in the presence of nucleases. Modifications of the oligonucleotides as used herein comprise any chemical modifications of the sugar, the base moiety and/or the internucleoside linkage.

In one embodiment nucleic acids or oligonucleotides with a covalently modified base and/or sugar include for example nucleic acids having backbone sugars which are covalently attached to low molecular weight organic groups other than a hydroxyl group at the 3' and/or 2' position and other than a phosphate group at the 5' position. Thus modified nucleic acids may include a 2'-O-alkylated ribose group. In yet another embodiment modified nucleic acids include sugars such as arabinose instead of ribose. Thus the nucleic acids may be heterogeneous in backbone composition thereby containing any possible combination of polymer units linked together such as peptide-nucleic acids (which have amino acid backbone with nucleic acid bases). In some embodiments the nucleic acids are homogeneous in backbone composition.

The substituted purines and pyrimidines of the nucleic acids include standard purines and pyrimidines such as cytosine as well as base analogs such as C-S propyne substituted bases (Wagner et al., Nature Biotechnology 14:840-844, 1996). Purines and pyrimidines include but are not limited to adenine, cytosine, guanine, thymine, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The single nucleotides in each oligonucleotide or polynucleotide polymer may contain the same modifications, may contain combinations of these modifications, or may combine these modifications with phosphodiester linkages. Methods of rendering oligonucleotide or polynucleotide polymers nuclease resistant include, but are not limited to, covalently modifying the purine or pyrimidine bases. For example, bases may be methylated, hydroxymethylated, or otherwise substituted (e.g., glycosylated) such that the oligonucleotides or polynucleotides are rendered substantially acid and nuclease resistant.

It is understood to someone skilled in the art, that antisense oligonucleotides in which some nucleotides of the sequence are substituted by another nucleotide or even other spacers, the derivatives of the antisense oligonucleotides of this invention still inhibit the synthesis of proteins such as TGF-beta 2. In a preferred embodiment about 0,1% to about 50% of the nucleotides are substituted or from about 0,1% to about 10% or from about 0,1% to about 5%.

A spacer as mentioned above can be any chemic substance connecting the at least two parts of the antisense oligonucleotide substituting the space of at least one nucleic acid.

In yet another embodiment at least one T (Thymidine) is substituted by an U (Uracil).

In a preferred embodiment the spacer is another nucleotide or is a sugar such as glucose, ribose etc., an amino acid or one or several units of a polymer such as polypropylene.

In a preferred embodiment, at least one end-block on the oligonucleotide is a biotin, biotin analog, avidin, or avidin analog. These molecules have the ability to both block the degradation of the protected oligonucleotide or polynucleotide and provide means for high affinity attachment of the modified nucleic acids to the solid support. Avidin and biotin derivatives which can be used to prepare the reagents of this invention include streptavidin, succinylated avidin, monomeric avidin, biocytin (biotin-.epsilon.-N-lysine), biocytin hydrazide, amine or sulfhydryl derivatives of 2-iminobiotin and biotinyl-epsilon-aminocaproic acid hydrazide. Additional biotin derivatives, such as biotin-N-hydroxysuccinimide ester, biotinyl-epsilon-aminocaproic acid-N-hydroxysuccinimide ester, sulfosuccinimidyl 6-(biotin amido)hexanoate, N-hydroxysuccinimideiminobiotin, biotinbromoacetylhydrazide, p-diazobenzoyl biocytin and 3-(N-maleimidopropionyl)biocytin, can also be used as end-blocking groups on the polynucleotides of the present invention.

In another embodiment the ring structure of the ribose group of the nucleotides in the modified oligonucleotide or polynucleotide has an oxygen in the ring structure substituted with N-H, N-R (with R being an alkyl or aryl substituent), S and/or methylene.

In a preferred embodiment at least one sugar moiety of the oligonucleotide is a morpholino derivative and the active derivative then is a morpholino oligonucleotide.

In another embodiment two carbones of at least one sugar moiety are linked. The linkage may be with a methoxy group or thereelse. This linker fixes the the sugar moiety in a way that it remains in one specific conformation, which enables e.g. higher selectivity and higher stability of this locked nucleic acids. Locked nucleic acids are described eg. In J. Wengel, Acc. Chem. Res., 120, 5458-5463 (1999) or J. Wengel et al., nucleosides & nucleotides, 18(6&7), S. 1365-1370.

In yet another embodiment the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are bound directly or indirectly to aza nitrogen atoms of the amide portion of the backbone. Representative United States patents that teach the preparation of PNA compounds include, but are not limited to, U.S. Pat. Nos.: 5,539,082 ; 5,714,331; and 5,719,262 , each of which is herein incorporated by reference. Further teaching of PNA compounds can be found in Nielsen et al., Science, 1991, 254, 1497-1500.

Further modified oligonucleotide backbones include, for example, phosphorothioates, chiral phosphorothioates, phosphorodithioates, phosphotriesters, aminoalkylphosphotriesters, methyl and other alky phosphonates including 3'-alkylene phosphonates and chiral phosphonates, phosphinates, phosphoramidates including 3 '-aminophosphoamidate and aminoalkylphosphoramidates, thionophosphoramidates, thionoalkylphosphonates, thionoalkylphosphotriesters, and boranophosphates having norm 3'-5'linkages, 2'-5'linked analogs of these, and those having inverted polarity wherein the adjacent pairs of nucleoside units are linked 3'-5'to 5'-3' or 2'-5' to 5'-2'. Various salts, mixed salts, and free acid forms are also included.

The nucleic acids having backbone modifications useful according to the invention in some embodiments are S- or R-chiral antineoplastic nucleic acids. An "S chiral nucleic acid" as used herein is an nucleic acid wherein at least two nucleotides have a backbone modification forming a chiral center and wherein a plurality of the chiral centers have S chirality. An "R chiral nucleic acid" as used herein is an nucleic acid wherein at least two nucleotides have a backbone modification forming a chiral center and wherein a plurality of the chiral centers have R chirality. The backbone modification may be any type of modification that forms a chiral center. The modifications include but are not limited to phosphorothioate, methylphosphonate, methylphosphorothioate, phosphorodithioate, p-ethoxy, 2'-0-Me and combinations thereof.

In a further embodiment at least one nucleotide of an oligonucleotide is modified as described in one of the modifications above.

In yet another embodiment both of these modifications of the oligonucleotide are combined.

In preferred embodiments the 1 to about 12 or 1 to about 8 or 1 to about 4 or 1 to about 2 oligonucleotides and/or nucleotide linkages at the 3' and/or 5' end of the oligonucleotide are modified as described above.

In yet another embodiment the oligonucleotides of this invention hybridizing with the same target (e.g. TGF-beta 2) comprising the oligonucleotides SEQ ID No. 30 but additionally have sequences with about 1 to about 20 nucleotides more preferred from1 to 15, 1 to 10, 1 to 5, 1 to 3 or 1 to 2 nucleotides on at least one of the 2', 3' and/or 5' end are still within the scope of this invention.

Nucleotides can be added at either end of these sequences following the sequence of the antisense complementary to the m-RNA of the target molecule to create new antisense oligonucleotides hybridising with the m-RNA. At any end of the oligonucleotide nucleotides with a length from about 1 to about 20 nucleotidesare added. As mentioned above it is understood to someone skilled in the art, that if some of the nucleotides are substituted or spacers are included instead of a nucleotide, this oligonucleotide derivative will still hybridize with the m-RNA of the target molecule.

Targets of oligonucleotide of SEQ ID No. 30 are known to persons skilled in the art. The targets are selected from the group of m-RNA of TGF-beta 1, TGF-beta 2 and/or TGF-beta 3. The sequence of the m-RNA of TGF-beta-1, and TGF beta-2 is given in example 6. The sequences of antisense complementary to the m-RNA of TGF-beta1, TGF-beta2, TGF-beta3 and IL-10 and a splice variation of the antisense complementary to the TGF-beta are given in example 16.

For the use in the instant invention, the nucleic acids can be synthesized de novo using any of a number of procedures well known in the art. Such compounds are referred to as 'synthetic nucleic acids.' For example, the b-cyanoethyl phosphoramidite method (Beaucage, S. L., and Caruthers, M. H., Tet. Let. 22:1859, 1981); nucleoside H-phosphonate method (Garegg et al., Tet. Let. 27:4051-4054, 1986 ; Froehler et al., Nucl. Acid. Res. 14:5399-5407, 1986, Garegg et a!, Tet. Let. 27:4055-4058, 1986, Gaffney et al., Tet. Let. 29:2619-2622, 1988). These chemistries can be performed by a variety of automated oligonucleotide synthesizers available in the market.

In yet another embodiment nucleic acids are produced on a large scale in plasmids, (see, e.g., Sambrook, et al., Molecular Cloning: A Laboratory Manual, Cold Spring Harbor Laboratory Press, New York, 1989) and separated into smaller pieces or administered as a whole.

In yet another embodiment nucleic acids are prepared from existing nucleic acid sequences (e.g., genomic or cDNA) using techniques known to someone skilled in the art, such as employing restriction enzymes, exonucleases or endonucleases. Nucleic acids prepared in this manner are isolated nucleic acids. The term nucleic acid encompasses both synthetic and isolated antineoplastic nucleic acids.

In another embodiment nucleic acids with modified backbone, such as those having phosphorothioates bonds are synthesized using automated techniques employing, for example, phosphoramidate or H-phosphonate chemistries. Aryl-and alkyl-phosphonates can be made, e.g., as described in U.S. Pat. No. 4,469,863 . Alkylphosphotriesters, in which the charged oxygen moiety is alkylated as described in U.S. Pat. No. 5,023,243 and European Patent No. 092,574, can be prepared by automated solid phase synthesis using commercially available reagents.

Methods for making other nucleic acid backbone modifications and substitutions have been described (Uhlmann, E. and Peyman, A., Chem. Rev. 90:544, 1990; Goodchild, J., Bioconjugate Chem. 1:165, 1990).

Descriptions for the synthesis of locked nucleic acids are known to someone skilled in the art, for further details see e.g. Wengel, J., Chem. Res., 120, S. 5458-5463 (1999) or Koch, T., J. Physics Condese Matter 15, S. 1861-1871 (2003).

In one embodiment phosphorothioates are synthesized using automated techniques employing either phosphoramidate or H-phosphonate chemistries. Aryl- and alkyl-phosphonates can be made, e.g., as described in U.S. Pat. No. 4,469,863 ; and alkylphosphotriesters (in which the charged oxygen moiety is alkylated as described in U.S. Pat. No. 5,023,243 and European Patent No. 092,574) can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165, 1990).

Other sources of nucleic acids useful according to the invention include standard viral and bacterial vectors, many of which are commercially available. In its broadest sense, a "vector" is any nucleic acid material which is ordinarily used to deliver and facilitate the transfer of nucleic acids to cells. The vector as used herein may be an empty vector or a vector carrying a gene which can be expressed. In the case when the vector is carrying a gene the vector generally transports the gene to the target cells with reduced degradation relative to the extent of degradation that would result in the absence of the vector. In this case the vector optionally includes gene expression sequences to enhance expression of the gene in target cells such as immune cells, but it is not required that the gene be expressed in the cell.

The oligonucleotide and oligonucleotide analogs within this invention is synonymous with active derivatives of this invention can be used in diagnostics, therapeutics and as research reagents and kits. For therapeutic use, the oligonucleotide or oligonucleotide analog is administered to an animal, especially a human, such as are suffering from an illness, more preferred suffering from a tumor and/or metastases in pancreatic cancer.

Presenting an oligonucleotide of SEQ ID No. 30 and its active derivatives in accordance with the present invention in pharmaceutically acceptable carriers is highly useful. This is especially true for treatment of diseases such as unwanted cancers and metastasis in pancreatic cancer.

In one embodiment the oligonucleotides of this invention are administered in aqueous isotonic water solutions is appropriate for intravenous application as well as intratumoral administration. Further pharmaceutical compositions of the present invention comprise oligonucleotides with one or more non-toxic, pharmaceutical acceptable carrier, excipients and/or adjuvants (collectively referred to herein as "carrier material"). The carrier materials are acceptable in the sense of being compatible with the other ingredients of the composition and are not deleterious to the recipient. The pharmaceutical compositions of the present invention can be adapted for administration by any suitable route by selection of appropriate carrier materials and a dosage of oligonucleotides effective for the treatment intended. For example, these compositions can be prepared in a from suitable for administration orally, intravascularyly, intraperitoneally, subcutaneously, intramusculary, rectally or intratumorally. Accordingly the carrier material employed can be a solid or a liquid, or both, and is preferably formulated with the compound as unit -dose composition, for example, a tablet, which can contain from about 1% to about 95%, by weight of oligonucleotides. the concentration of the oligonucleotides in a solution is dependent of the volume being administered. Such pharmaceutical compositions of the invention can be prepared by any of the well known techniques of pharmacy, consisting essentially of admixing the components.

The pharmaceutical composition of this invention is delivered solely or in mixtures. A mixture comprises one or several oligonucleotides of this invention. These at least two substances herein is also referred to as compounds.

In one embodiment the at least two compounds are mixed and pure or in a pharmaceutical acceptable carrier. In yet another embodiment the at least two compounds of the pharmaceutical composition are separate and pure or are separate and in a pharmaceutical acceptable carrier. In one embodiment the at least two components are in the same pharmaceutical acceptable carrier, in yet another embodiment the at least two components are in different pharmaceutical acceptable carriers.

### Forms of administration

Administering the pharmaceutical compositions of the present invention may be accomplished by any means known to the person skilled in the art. Routes of administration include but are not limited to oral, intranasal, intratracheal, ocular, pulmonal, vaginal, rectal, parenteral (e.g. intramuscular, intradermal, intravenous, intratumoral or subcutaneous or direct injection), topical, transdermal.

In one embodiment of a pharmaceutical composition for the treatment of unwanted cancers or carcinomas, the pharmaceutical composition is delivered by means of a biodegradable, polymeric implant or implanted catheters.

The term "pharmaceutical composition" implicates the liquids or substances of this composition are pure and/or combined with pharmaceutical acceptable carriers.

The term "pharmaceutically-acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Such carriers enable the compounds of the invention to be formulated as tablets, coated tablets, granules, powders, pills, dragees, (micro)capsules, liquids, gels, syrups, slurries, suspensions, emulsions and the like, for oral ingestion by a subject to be treated.

The pharmaceutical compositions may also include granules, powders, tablets, coated tablets, (micro)capsules, suppositories, syrups, emulsions, suspensions, creams, drops, coated onto microscopic gold particles or preparations with protracted release of active compounds, in whose preparation excipients and additives and/or auxiliaries such as disintegrants, binders, coating agents, swelling agents, lubricants, flavorings, sweeteners or solubilizers are customarily used as described above.

For a brief review of present methods for drug delivery, see Langer, Science 249:1527-1533, 1990.

For oral administration, the pharmaceutical composition is delivered alone without any pharmaceutical carriers or formulated readily by combining the compound(s) with pharmaceutically acceptable carriers.

In one embodiment pharmaceutical compositions for oral use is obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatine, gum tragacanth, methyl cellulose, hydroxypropylmethyl-cellulose, sodium carboxymethylcellulose, and/or polyvinylpyrrolidone (PVP).

In yet another embodiment disintegrating agents are added, such as the crosslinked polyvinyl pyrrolidone, agar, or alginic acid or a salt thereof such as sodium alginate. Optionally the oral formulations may also be formulated in saline or buffers for neutralizing internal acid conditions.

In yet another embodiment dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures.

In yet another embodiment dyestuffs or pigments are added to the tablets or dragee coatings for identification or to characterize different combinations of active compound doses.

In another embodiment pharmaceutical preparations which can be used orally include push-fit capsules made of gelatine, as well as soft, sealed capsules made of gelatine and a plasticizer, such as glycerol or sorbitol. In one embodiment the push-fit capsules contains the active ingredient in a mixture with filler such as lactose, binders such as starches, and/or lubricants such as talc or magnesium stearate and, optionally, stabilizers. In another embodiment of the soft capsules, the active compounds are dissolved or suspended in suitable liquids, such as fatty oils, liquid paraffin, or liquid polyethylene glycols. In addition, stabilizers may be added.

In yet another embodiment microspheres formulated for oral administration are used, well know to someone skilled in the art.

The formulations for oral administration are in dosages suitable for such administration.

In yet another embodiment for buccal administration, the compositions may take the form of tablets or lozenges formulated in conventional manner.

### Inhalation

In yet another embodiment for the administration by inhalation, the compounds for use according to the present invention may be conveniently delivered in the form of an aerosol spray, from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatine for use in an inhaler or insufflator may be formulated containing a powder mix of the compound and a suitable powder base such as lactose or starch.

Suitable pharmaceutical carriers are, for example, aqueous or saline solutions for inhalation, microencapsulated, encochleated, contained in liposomes, nebulized, aerosols.

In yet another embodiment the pharmaceutical acceptable carriers of the compounds for parenteral, intrathecal, intraventricular or intratumoral administration include sterile aqueous solutions which may also contain buffers, diluents and other suitable additives such as, but not limited to, penetration enhancers, carrier compounds and other pharmaceutically acceptable carriers or excipients.

In yet another embodiment for the systemic delivery of the compounds they are in pharmaceutical carriers for parenteral administration by injection (e.g., by bolus injection or continuous infusion). Formulations for injection may be presented in unit dosage form, e.g., in ampoules or in multi-dose containers, with an added preservative. The pharmaceutical compositions take such forms as suspensions, solutions or emulsions in oily or aqueous vehicles, and contain formulatory agents such as suspending, stabilizing and/or dispersing agents.

In one embodiment pharmaceutical carriers for parenteral administration include aqueous solutions of the active compounds in water-soluble form.

In yet another embodiment a suspensions of at least one oligonucleote is prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions comprise substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In yet another embodiment the active compounds may are in powder form for constitution with a suitable vehicle, e.g., sterile pyrogen-free water, before use or dried onto a sharp object to be scratched into the skin.

In yet another embodiment the compounds are formulated in rectal or vaginal compositions such as suppositories or retention enemas, e.g., containing conventional suppository bases such as cocoa butter or other glycerides.

In yet another embodiment the compounds are formulated as a depot preparation. In one embodiment such long acting formulations are formulated with suitable polymeric or hydrophobic materials (for example as an emulsion in an acceptable oil) or ion exchange resins, or as sparingly soluble derivatives, for example, as a sparingly soluble salt.

In other embodiments delivery systems include time-release, delayed release or sustained release delivery systems. Such systems can avoid repeated administrations of the compounds, increasing convenience to the subject and the physician. Many types of release delivery systems are available and known to those of ordinary skill in the art.

In one embodiment the delivery system includes polymer base systems such as poly(lactideglycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109 .

In another embodiment the delivery systems include non-polymer systems that are e.g. lipids including sterols such as cholesterol, cholesterol esters and fatty acids or neutral fats such as mono-di-and tri-glycerides; hydrogel release systems; sylastic systems; peptide based systems; wax coatings; compressed tablets using conventional binders and excipients; partially fused implants; and the like.

Specific examples include, but are not limited to: (a) erosional systems in which an agent of the invention is contained in a form within a matrix such as those described in U.S. Pat. No. 4,452,775 , 4,675,189 , and 5,736,152, and (b) diffusional systems in which an active component permeates at a controlled rate from a polymer such as described in U.S. Pat. No. 3,854,480, 5,133,974 and 5,407,686. In addition, pump-based hardware delivery systems can be used, some of which are adapted for implantation.

In still other embodiments, the antagonist and antineoplastic agent are formulated with GELFOAM, a commercial product consisting of modified collagen fibers that degrade slowly.

In one embodiment the pharmaceutical compositions also comprise suitable solid or gel phase carriers or excipients. Examples of such carriers or excipients include but are not limited to calcium carbonate, calcium phosphate, various sugars, starches, cellulose derivatives, gelatine, and polymers such as polyethylene glycols.

In one embodiment the oligonucleotides of this invention are administered neat or in the form of a pharmaceutically acceptable salt. The salts have to be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

In one embodiment suitable buffering agents include but are not limited to: acetic acid and a salt (1-2% w/v); citric acid and a salt (1-3% w/v); boric acid and a salt (0.5-2.5% w/v); and phosphoric acid and a salt (0.8-2% w/v).

Suitable preservatives include benzalkonium chloride (0.003-0.03% w/v); chlorobutanol (0.3-0.9% w/v); parabens (0.01-0.25% w/v) and thimerosal (0.004-0.02% w/v).

In one embodiment the pharmaceutical acceptable carrier for topical administration for the at least two compounds of a pharmaceutical composition according to this invention include transdermal patches, ointments, lotions, creams, gels, drops, suppositories, sprays, liquids and powders. Conventional pharmaceutical carriers, aqueous, powder or oily bases, thickeners and the like may be necessary or desirable. In yet another embodiment coated condoms, gloves and the like are useful.

In yet another embodiment the pharmaceutical compositions also include penetration enhancers in order to enhance the alimentary delivery. Penetration enhancers may be classified as belonging to one of five broad categories, i.e., fatty acids, bile salts, chelating agents, surfactants and non-surfactants (Lee et al., Critical Reviews in Therapeutic Drug Carrier Systems, 1991, 8, 91-192 ; Muranishi, Critical Reviews in Therapeutic Drug Carrier Systems, 1990, 7, 1-33). One or more penetration enhancers from one or more of these broad categories may be included.

Various fatty acids and their derivatives which act as penetration enhancers include, for example, oleic acid, lauric acid, capric acid, myristic acid, palmitic acid, stearic acid, linoleic acid, linolenic acid, dicaprate, tricaprate, recinleate, monoolein (a.k.a. 1-monooleoyl-rac-glycerol), dilaurin, caprylic acid, arichidonic acid, glyceryl 1-monocaprate, 1-dodecylazacycloheptan-2-one, acylcarnitines, acylcholines, mono- and di-glycerides and physiologically acceptable salts thereof (i.e., oleate, laurate, caprate, myristate, palmitate, stearate, linoleate, etc.) (Lee et al., Critical Reviews in Therapeutic Drug Carrier Systems, 1991, 8:2, 91-192 ; Muranishi, Critical Reviews in Therapeutic Drug Carrier Systems, 1990, 7:1, 1-33 ; El-Hariri et al., J. Pharm. Pharmacol., 1992, 44, 651-654). Examples of some presently preferred fatty acids are sodium caprate and sodium laurate, used singly or in combination at concentrations of 0.5 to 5%.

The physiological roles of bile include the facilitation of dispersion and absorption of lipids and fat-soluble vitamins (Brunton, Chapter 38 In: Goodman & Gilman's The Pharmacological Basis of Therapeutics, 9th Ed., Hardman et al., eds., McGraw-HilI, New York, N.Y., 1996, pages 934-935). Various natural bile salts, and their synthetic derivatives, act as penetration enhancers. Thus, the term "bile salt" includes any of the naturally occurring components of bile as well as any of their synthetic derivatives. A presently preferred bile salt is chenodeoxycholic acid (CDCA) (Sigma Chemical Company, St. Louis, Mo.), generally used at concentrations of 0.5 to 2%.

Complex formulations comprising one or more penetration enhancers may be used. For example, bile salts may be used in combination with fatty acids to make complex formulations. Preferred combinations include CDCA combined with sodium caprate or sodium laurate (generally 0.5 to 5%).

In one embodiment additionally chelating agents are used they include, but are not limited to, disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of betadiketones (enamines)(Lee et al., Critical Reviews in Therapeutic Drug Carrier Systems, 1991, 8:2, 92-192 ; Muranishi, Critical Reviews in Therapeutic Drug Carrier Systems, 1990, 7:1, 1-33 ; Buur et al., J. Control Rel., 1990, 14, 43-51). Cheating agents have the added advantage of also serving as DNase inhibitors. In yet another embodiment additionally surfactants are used. Surfactants include, for example, sodium lauryl sulfate, polyoxyethylene-9-lauryl ether and polyoxyethylene-20-cetyl ether (Lee et al., Critical Reviews in Therapeutic Drug Carrier Systems, 1991, 8:2, 92-191); and perfluorochemical emulsions, such as FC-43 (Takahashi et al., J. Pharm. Pharmacol., 1988, 40, 252-257).

Non-surfactants include, for example, unsaturated cyclic ureas, 1-alkyl- and 1-alkenylazacyclo-alkanone derivatives (Lee et al., Critical Reviews in Therapeutic Drug Carrier Systems, 1991, 8:2, 92-191); and non-steroidal anti-inflammatory agents such as diclofenac sodium, indomethacin and phenylbutazone (Yamashita et al., J. Pharm. Pharmacol., 1987, 39, 621-626).

In one embodiment the pharmaceutical compositions of the present invention additionally contain other adjunct components conventionally found in pharmaceutical compositions, at their art-established usage levels. Thus, for example, the compositions may contain additional compatible pharmaceutically-active materials such as, e.g., antipruritics, astringents, local anesthetics or anti-inflammatory agents, or may contain additional materials useful in physically formulating various dosage forms of the composition of present invention, such as dyes, flavoring agents, preservatives, antioxidants, opacifiers, thickening agents and stabilizers. However, such materials, when added, should not unduly interfere with the biological activities of the components of the compositions of the invention.

In the embodiments of oligonucleotides for the preparation of a pharmaceutical composition for inhibiting the formation of metastases in treatment of pancreas carcinomaat least one antisense oligonucleotide of SEQ ID No. 30 is applied in effective amounts. In other embodiments the active derivatives are applied in effective amounts. In general, the term "effective amount" of an antisense oligonucleotides,or active derivative refers to the amount necessary or sufficient to realize a desired biologic effect. Specifically, the effective amount is that amount that reduces the rate or inhibits altogether the formation of cancers or carcinomas respectively inhibits the formation of metastases. For instance, when the subject bears a pancreatic cancer, an effective amount is that amount which decreases or eliminates the pancreatic cancer. Additionally, an effective amount may be that amount which prevents an increase or causes a decrease in new pancreatic cancer and/or reduces the formation of metastases in pancreatic cancer.

The effective amount varies depending upon whether the pharmaceutical composition is used in single or multiple dosages and whether only one or several antisense oligonucleotides are within one pharmaceutical composition.

Dosages given in this writing are for adults. It is quite clear to someone skilled in the art, that these dosages have to be adapted if the human being is a child, a person stressed by a further illness or other circumstances. In the same way the effective amount must be adapted if an animal is treated which is also within the scope of this invention.

The effective dosage is dependent also on the method and means of delivery, which can be localized or systemic.

In one embodiment subject doses of the oligonucleotides described herein typically range from about 0.1 µg to about 10 mg per administration, which depending on the application could be given hourly, daily, weekly, or monthly and any other amount of time therebetween. In yet another embodiment the doses range from about 10 µg to about 5 mg per administration or from about 100 µg to about 1 mg, with 1-10 administrations being spaced hours, days or weeks apart. In some embodiments, however, doses may be used in a range even 2 to 100 fold higher or lower than the typical doses described above.

In one embodiment of this invention the at least one oligonucleotide of SEQ ID No. 30 of a pharmaceutical composition according to this invention is the antisense oligonucleotide inhibiting the production of TGF-beta 1, and/or TGF-beta 3 administered in a dose range from about 1 µg/kg/day to about 100 mg/kg/day or from about 10 µg/kg/day to about 10 mg/kg/day or from about 100 µg/kg/day to about 1 mg/kg/day.

### EXAMPLES

If not referred to in another way, the sequences used in the assays were used as phosphorothioates. AP12009 is an antisense oligonucleotide complementary to m-RNA of TGF-beta2 and AP11014 is an antisense oligonucleotide complementary to m-RNA of TGF-beta1. AP12009 is an antisense oligonucleotide of SEQ ID No. 30 according to the present invention. The antisense oligonucleotide of AP11014 (SEQ ID No. 14) is not comprised by the present invention. Antisense oligonucleotides disclosed in examples 19-24 are illustrative examples.

### Example 1

Cell culture conditions for test systems

The cell lines under test were:
Colon cancer: HCT-116Melanoma: MER 191 a, MER 116, MES 100a
NSCLC: SW 900, NCI-H661
Ovarian cancer: EFO-21, Colo 704
Pancreatic cancer: PATU-8902, Hup-T3, Hup-T4
Prostate cancer: PC-3, DU-145

Cell lines were obtained from American Type Culture Collection (ATCC) respectively from the German Collection of Microorganisms and Cell Cultures. All cells were cultured as described by the provider.

Further cell lines under test:
Hepatocellular cancer: HepG2
Melanoma RPMI-7951, SK-Mel3
NSCLC: A-549
Renal cancer Caki-1
Cell lines were further obtained from the Cell Line Service (CLS).

### Example 2

Cell-Mediated Cytotoxicity Assay:
For generation of tumor cell supernatants cells were cultured in culture medium supplemented with 1% FCS and 3% Panexin (Pan Biosystems) to reduce the TGF-β amount in the medium. Cells were treated with Lipofectin respectively the test substance with Lipofectin on two consecutive days or left untreated. Culture supernatants were taken 3 days after the last Lipofectin treatment. TGF-beta produced by the tumor cells was activated by 1 N HCl (1:20 dilution) for 10 min at RT. For neutralisation NaOH was added. Human peripheral blood monocytes (PBMC) isolated from healthy donors were incubated with the tumor cell supernatants in the presence of IL-2 (10 ng/ml) for the generation of lymphokine activated killer cells. To block activated TGF-beta 1 a TGF-beta1 specific antibody (1 µg/ml, R&D Systems) was added. After 3 days, cytotoxic activity of LAK cells was determined in a 4h CARE-LASS assay against the respective cancer cell line as target. For TGF-beta2 the same protocol was applied. Instead of the TGF-beta1 specific antibody a TGF-beta 2 specific antibody was added. In experiments for measuring TGF-beta1 or TGF-beta2 beneath the measurement of LAK cells the cytotoxic activity of PBMC was determined in 4h CARE-LASS assay

### Example 3

### Proliferation Assay with Lipofectin^{®}

About 75.000 cells per millilitre of the respective tumor cell line were cultured in 12 well flat-bottom microtiter plates in MEM-Dulbecco medium supplemented with 10% fetal calf serum (FCS, Gibco), as recommended by ATCC (American Type Culture Collection). After 24 hours and after 48 hours the cells were treated with indicated concentration of respective TGF-beta 1 specific antisense oligonucleotides for 6 h. For enhancing the cellular uptake additionally Lipofectin^{®} (Invitrogen, USA) with indicated concentrations was added during this 2 x 6 hours. Some experiments were performed without adding Lipofectin^{®}. Between these two periods of 6 h and during the final 3 days cells were treated
with 5 µM of above mentioned TGF-beta 1 specific antisense oligonucleotide in the absence of Lipofectin^{®}. Control cells were cultured in pure medium for the same period. Furthermore reference cells were treated with indicated concentration of Lipofectin^{®}.

Instead of MEM-Dulbecco-medium other culture mediums may be used according to the recommendations of the providers of the cell lines.

Finally cell numbers in all samples were quantified by staining the cells with Trypan blue and counting them in a "Neubauer" hemacytometer.

Alternatively the quantification of living cells was done with the EZ4U-assay according to the manufacturer's protocol, or by electronic cell counting with the Coulter Counter Z2 (Beckmann) according to the manufacturer's instructions.

Additionally, TGF-beta 1 concentrations in supernatants were measured by TGF-beta 1 Enzym-Linked Immunosorbent Assay (TGF-beta 1 ELISA, R&D Systems, USA) according to the manufacturer's instructions. Values from serum-supplemented medium without cells were subtracted to account for background from serum-derived TGF-beta 1.

This proiferation assay was also performed for measuring TGF-beta2 levels. In that case instead of TGF-beta1 specific antisense oligonucleotides, TGF-beta2 specific antisense oligonucleotides were used and instead of measuring TGF-beta1 concentrations, TGF-beta2 concentrations were measured in the supernatants by a TGF-beta2 Enzyme-Linked Immunosorbent Assay (TGF-beta 2 ELISA).

### Example 4

### Proliferation assay without addition of Lipofectin^{®}

About 25.000-200.000 cells per millilitre, dependent on cell diameter and speed of cell proliferation were cultured in 12 well flat-bottom microtiter plates in MEM-Dulbecco medium supplemented with 10% fetal calf serum (FCS, Gibco), as recommended by ATCC (American Type Culture Collection). Additionally, TGF-beta 1 specific antisense oligonucleotides were added during three days, whereas the control cells remained untreated during the three days

After three days cell numbers in all samples were quantified by staining them by trypan blue method and counting them in a "Neubauer" hemacytometer.

Additionally TGF-beta 1 concentrations were measured in the supernatants on day 3 with TGF-beta1 Enzym-Linked Immunosorbent Assay (TGF-beta 1 ELISA, Genzyme, Cambridge, MA, USA) according to the manufacturer instructions. Values from serum-supplemented medium without cells were subtracted to account for background from serum-derived TGF-beta 1.

In other proliferation assays TGF-beta 2 specific antisense oligonucleotides were added. In these experiments the TGF-beta 2 levels were measured in the supernatants on day 3. In experiments enduring 6 days, used for melanoma cell lines, the levels of either TGF-beta1 or TGF-beta2 were measured on day 3 and day 6. TGF-beta1 and TGF-beta2 levels were measured with TGF-beta1 or TGF-beta2 Enzyme-Linked Immunosorbent Assay which were provided by R&D Systems)

In the proliferation assays enduring 6 days the medium was exchanged after the first three days and cells were treated for further three days with the respective antisense oligonucleotides at the indicated concentrations. The cell numbers then were quantified after three and six days. The quantification of the living cells was performed with the EZ4U-assay according to the manufacturer's protocol, or by electronic cell counting with the Coulter Counter Z2 (Beckmann) according to the manufacturere's instructions.

Instead of MEM-Dulbecco medium other culture mediums supplemented with 10% fetal calf serum (FCS, Gibco) were used, as recommended by the providers of the different cell lines.

### Example 5

### Scratch Assay

Tumor cells (about 900,000/well) were seeded in 6-well plates. The next day cells were treated once with the test substance and Lipofectin respectively with Lipofectin for 6h in Optimem (Invitrogen) or left untreated. Then, the confluent monolayer of cells was scratched (start) in a standardized manner with a sterile plastic pipette tip to create a cell-free zone in each well, approximately 1,000µm in width. Afterwards the cells were washed once and incubated at 37°C in standard medium. In vitro migration was documented by photography, and the migration distance was quantified by computer-assisted image analysis using NIH Image 1.6. Each experiment was performed in quadruplicate.

Since migration plays a key role in the formation of metastases this "in vitro" experiment correlates with "in vivo" formation of metastases. Inhibition of "in vitro" migration indicates inhibition of formation of metastases "in vivo".

### Example 6

Spheroid Migration Model:
A spheroid model of migration was established as described elsewhere (Nygaard et al., 1998). Tumor cells were cultured in tissue culture flasks coated with 2% agar. After 3 days multicellular spheroids were transferred into 96-well plates and left untreated, treated with test substance or with recombinant TGF-β2 at 10 ng/ml (R&D Systems). The migration behaviour of the respective cell-line was analyzed by phase contrast microscopy (x10).
Since migration plays a key role in the formation of metastases this "in vitro" experiment correlates with "in vivo" formation of metastases. Inhibition of "in vitro" migration indicates inhibition of formation of metastases "in vivo".

### Example 7

### TGF-β1/β2-ELISA

The amount of TGF-β1 respectively TGF-β2 secreted by tumor cells was determined by ELISA. Briefly, tumor cells (15,000 - 200,000, depending on cell size and cell growth) were seeded into 12-well tissue culture plates and treated with the oligonucleotides under test in the presence of cationic lipid (Lipofectin reagent, Gibco BRL) for 6h in serum-free Optimem medium (Invitrogen) on two consecutive days (final concentrations: AP 11014: 200 nmol/l; Lipofectin: 3 µg/ml) or left untreated. Then, cells were cultured in the presence of 5 µmol/l AP 11014 or left untreated. 72h after the second treatment with Lipofectin and the oligonucleotide under test the cell culture supernatants were collected. The amount of TGF-ß1/ß2 in the supernatants was measured employing a standard TGF-β1-ELISA-Kit respectively TGF-β2 (Quantikine, R&D Systems, USA).

The final concentrations of TGF-beta1 or TGF-beta2 antisense oligonucleotides in this assay were 200nMol/l with Lipofectin concentrations of 3µg/ml, respectively 400 nMol/l with 6µg/ml.

### Example 8 - Colon Cancer

TGF-β1 Suppression:
Analysed with TGF-β1 specific ELISA, as described in Example 7, 200 nM of phosphorothioate from SEQ ID NO 14 in the presence of 3 µg/ml Lipofectin reduced TGF-β1 secretion in colon cancer cell line HCT-116 to about 13,8% compared to untreated control which was set to 100%. In other experiments the proliferation was reduced to about 46 %.

Cell proliferation:
In the proliferation assay according to example 3 SEQ ID NO 14 in the presence of 3 µg/ml Lipofectin reduced proliferation of colon cancer cell line (HCT-116) to about 35% compared to untreated control which was set to 100%. 200 nMol of the SEQ ID NO 14 were used.

### Scratch Assay

In the scratch assays according to Example 5 SEQ ID NO 14 significantly inhibits migration of the colon cancer cell line HCT-116 in a concentration of 200 nM in the presence of Lipofectin 3 µg/ml.

### Example 9 - Hepatocellular Cancer

### TGF-beta 1 Suppression

Analysed with TGF-β1 specific ELISA, as described in Example 7, 400 nM of phosphorothioate from SEQ ID NO 14 in the presence of 6 µg/ml Lipofectin reduced TGF-β1 secretion in hepatocellular cancer cell line Hep G2 to about 75% compared to untreated control which was set to 100%.

Cell proliferation:
In the proliferation assay according to example 3 SEQ ID NO 14 reduced proliferation of the hepatocellular cancer cell line HepG2 to about 39% in a concentration of 400 nM in the presence of 6 µg/ml Lipofectin compared to untreated control which was set to 100%.

### Example 10 - Melanoma

### TGF-beta 1

Analysed with TGF-β1 specific ELISA, as described in Example 7, 400 nM of phosphorothioate from SEQ ID NO 14 in the presence of 1 µg/ml Lipofectin reduced TGF-β1 secretion in melanom cancer cell line MER-116 to about 0% compared to untreated control which was set to 100%.

### TGF-beta 1 Suppression

Analysed with TGF-β1 specific ELISA, as described in Example 7, 10 µM of phosphorothioate from SEQ ID NO 14 reduced TGF-β1 secretion in melanom cancer cell line MES-100a to about 23% compared to untreated control which was set to 100%.

### TGF-beta 2 Suppression

Analysed with TGF-β2 specific ELISA, as described in Example 7, 400 nM of phosphorothioate from SEQ ID NO 30 in the presence of 6 µg/ml Lipofectin reduced TGF-β2 secretion in melanoma cell line RPMI- 7951 to about 14.2 % compared to untreated control which was set to 100%.

### Inhibition of proliferation

In the proliferation assay according to example 3 SEQ ID NO 14 reduced proliferation of melanoma cell line (MER-116) to about 33% in a concentration of 50 nM and to about 23% in a concentration of 200 nM in the presence of 3 µg/ml Lipofectin compared to untreated control which was set to 100%. In other experiments usingA-549 cell line the proliferation was reduced to about 0.4% compared to untreated control, which was set to 100%.

### Inhibition of proliferation

In the proliferation assay according to example 3 SEQ ID NO 30 reduced proliferation of melanoma cell line (RPMI-7951) to about 17.8 % in a concentration of 400 nM in the presence of 6 µg/ml Lipofectin compared to untreated control which was set to 100%.

### Example 11 - NSCLC

### TGF-β1 Suppression

Analysed with TGF-β1 specific ELISA, as described in Example 7, 200 nM of phosphorothioate from SEQ ID NO 14 in the presence of 3 µg/ml Lipofectin reduced TGF-β1 secretion in non small cell lung cancer cell line SW-900 to about 34% and in NCI-H661 to about 38 % compared to untreated control which was set to 100%.

In cancer cell line A-549 TGF under the same conditions the TGF-β1 secretion was reduced to about 0.4%.

Cell proliferation:
In the proliferation assay according to example 3 SEQ ID NO 14 reduced proliferation of NSCLC cell line (SW-900) to about 30% in a concentration of 200 nM in the presence of 3 µg/ml Lipofectin compared to untreated control which was set to 100%. SEQ ID NO 14 was applied in a concentration of 200 nM. In other examples of the proliferation assay according to example 3 SEQ ID NO14 in a concentration of 200 nM reduced proliferation of NSCLC cell line A-549 to about 36% and NCI-H661 to about 44%.

### Scratch Assay

In the scratch assays according to Example 5 SEQ ID NO 14 significantly inhibits migration of the NSCLC cancer cell line SW-900 in a concentration of 200 nM and 400 nM in the presence of Lipofectin 6 µg/ml. The migration after 17 h of the cell treated with both concentrations of SEQ ID NO 14 was about 50 µm, whereas the cell incubated with Lipofectin migrated about 75 µm and the control cells about 80 µm. After 24 h the results were about: control 120µm, Lipofectin treated cells: 115µm and SEQ ID NO 14 treated cell about 60µm. Migration after 48 h was about 250 µm for the control and Lipofectin treated cells and about 150 µm for both concentrations of cells treated with SEQ ID NO 14.

In the scratch assay under the same conditions SEQ ID NO 14 cells incubated with Lipofectin migrated about 75 µm and the control cells about 80 µm. After 24 h the results were about: control 120µm, Lipofectin treated cells: 115µm and SEQ ID NO 14 treated cell about 60µm. Migration after 48 h was about 250 µm for the control and Lipofectin treated cells and about 150 µm for both concentrations of cells treated with SEQ ID NO 14.

### Example 12 - Ovarian cancer

### TGF-beta 1 Suppression

Analysed with TGF-β1 specific ELISA, as described in Example 7, 10 nM of phosphorothioate from SEQ ID NO 14 in the presence of 3 µg/ml Lipofectin reduced TGF-β1 secretion in ovarian cancer Colo 704 to about 54% compared to untreated control which was set to 100%.

### TGF-beta2 Suppression

Analysed with TGF-β2 specific ELISA, as described in Example 7, 200 nM of phosphorothioate from SEQ ID NO 30 in the presence of 3 µg/ml Lipofectin reduced the TGF-β2 secretion in ovarian cancer EFO-21 to about 31 % compared to untreated control which was set to 100%.

### Cell proliferation:

TGF-beta 1: In the proliferation assay according to example 3 SEQ ID NO 14 reduced proliferation of ovarian cancer cell line Colo 704 to about 54% in a concentration of 50 nM in the presence of 3 µg/ml Lipofectin compared to untreated control which was set to 100%.

In another proliferation assay according to example 3 SEQ ID NO 14 reduced proliferation of ovarian cancer cell line Colo 704 to about 40 % in a concentration of 200 nM in the presence of 3 µg/ml Lipofectin compared to untreated control which was set to 100%.

TGF-beta 2: In the proliferation assay according to example 3 SEQ ID NO 30 reduced proliferation of ovarian cancer cell EFO-21 to about 31 % in a concentration of 200 µM and to about 45% in a concentration of 50 nM in the presence of 3 µg/ml Lipofectin compared to untreated control which was set to 100%.

In another proliferation assay according to example 3 SEQ ID NO 30 reduced proliferation of ovarian cancer cell EFO-21 to about 63% in a concentration of 200 nM in the presence of 3 µg/ml Lipofectin compared to untreated control which was set to 100%.

### Example 13 - Pancreas cancer:

### TGF-beta 1 Suppression

Analysed with TGF-β1 specific ELISA, as described in Example 7, 10 µM of phosphorothioate from SEQ ID NO 14 reduced TGF-β1 secretion in pancreatic cancer Hup T3 to about 74% compared to untreated control which was set to 100%.

In another experiment of example 7 analysed with TGF-β1 specific ELISA 200 nM of phosphorothioate from SEQ ID NO 14 in the presence of 3µg/ml Lipofectin reduced TGF-β1 secretion in pancreatic cancer cell line DanG to about 3 % compared to untreated control which was set to 100%.

### TGF-beta 2 Suppression

Analysed with TGF-beta 2 specific ELISA, as described in Example 7, 200 nM of phosphorothioate from SEQ ID NO 30 in the presence of 3 µg/ml Lipofectin reduced TGF-β2 secretion in pancreatic cancer cell line Hup-T3 to about 2% and in PATU-8902 to about 10% compared to untreated control which was set to 100%.

With the TGF-beta2 specific ELISA as described in example 7 200 nM of phosphorothioate from SEQ ID NO 30 in the presence of 3 g/ml Lipofection reduced TGF-β2 secretion in pancreatic cancer cell lines Hup-T4 cells to about 24 %, and in PA-TU-8902 cells to about 6 % compared to untreated control which was set to 100 %.

### Cell Proliferation TGF-beta 2

In the proliferation assay according to example 3 SEQ ID NO 30 reduced proliferation of pancreatic cancer cell line Hup-T3 to about 1% in a concentration of 200 nM in the presence of 3 µg/ml Lipofectin compared to untreated control which was set to 100%, in the cell line PATU-8902 proliferation was reduced to about 10%.

In another proliferation assay according to example 3 SEQ ID NO 30 reduced proliferation of pancreatic cancer cell line Hup-T3 to about 24%, Hup-T4 to about 24 % and PATU-8902 to about 27 % in a concentration of 200 nM in the presence of 3 µg/ml Lipofectin compared to untreated control which was set to 100 %.

### Cell Proliferation TGF-beta 1

In the proliferation assay according to example 4 SEQ ID NO 14 reduced proliferation of pancreatic cancer cell line Hup-T3 to about 13% in a concentration of 10 µM in the presence of 3 µg/ml Lipofectin compared to untreated control which was set to 100%, in the cell line PATU-8902 proliferation was reduced to about 10%.

In another proliferation assay according to example 4 SEQ ID NO 14 reduced proliferation of pancreatic cancer cell line DanG to about 27 % in a concentration of 200 nM in the presence of 3 µg/ml Lipofectin compared to untreated control which was set to 100%.

### Cell Migration

Migration of a pancreatic cell line PATU- 8902 was measured according to the protocol of example 6 treated with SEQ ID NO 30 in a concentration of 5 µMol/l was nearly completely inhibited for about 65 h, compared to the untreated reference whereas the diameter of the sphere of control cells increased about 1000 µm during the same time.

In the same experiment human TGF-beta 2 antibodies were tested which nearly showed no effect, which indicates that the inhibition of migration is highly specific for antisense oligonucleotides and do not only correlate with antagonizing TGF-beta.

### Example 14 - Prostate Cancer

### TGF-beta 1 Suppression

Analysed with TGF-β1 specific ELISA, as described in Example 7, 200 nM of phosphorothioate from SEQ ID NO 14 in the presence of 3 µg/ml Lipofectin reduced TGF-β1 secretion in prostate cancer cell line PC-3 to about 36% and in DU-145 to about 57% compared to untreated control which was set to 100%.

### TGF-beta 2 Suppression

Analysed with TGF-β2 specific ELISA, as described in Example 7, 200 nM of phosphorothioate from SEQ ID NO 14 in the presence of 3 µg/ml Lipofectin reduced TGF-β2 secretion in prostate cancer cell line PC-3 to about 19% and in DU-145 to about 20% compared to untreated control which was set to 100%.

### Cell proliferation TGF-beta1:

In the proliferation assay according to example 3 SEQ ID NO 14 reduced proliferation of prostate carcinoma cell line PC-3 to about 74% in a concentration of 200 nM in the presence of 3 µg/ml Lipofectin compared to untreated control which was set to 100%.

In another experiment according to the example 3 SEQ ID NO 14 reduced proliferation of prostate carcinoma cell line DU-145 to about 81 % in a concentration of 200 nM in the presence of 3 µg/ml Lipofectin compared to untreated control which was set to 100 %.

### Cell proliferation TGF-beta2:

In the proliferation assay according to example 3 SEQ ID NO 30 reduced proliferation of prostate carcinoma cell line PC-3 to about 29 % and of DU-145 to about 34% in a concentration of 200 nM in the presence of 3 µg/ml Lipofectin compared to untreated control which was set to 100%.

### Scratch Assay

In the scratch assays according to Example 5 SEQ ID NO 14 significantly inhibits migration of the prostate cancer cell line PC-3 in a concentration of 400 nM in the presence of Lipofectin 6 µg/ml. The migration after 17 h of the cell treated with SEQ ID NO 14 was about 37 µm, whereas the cell incubated with Lipofectin migrated about 140 µm and the control cells about 165 µm. After 24 h the results were about: control 288 µm, Lipofectin treated cells: 213 µm and SEQ ID NO 14 treated cell about 60 µm. Migration after 48 h was about 366 µm for the control, 328 µm for Lipofectin treated cells and about 150 µm for cells treated with SEQ ID NO 14.

In another experiment the scratch assays according to Example 5 SEQ ID NO 14 significantly inhibits migration of the prostate cancer cell line PC-3 in a concentration of 400 nM in the presence of 6 µg/ml Lipofectin. The migration after 17 h of the cell treated with SEQ ID NO xx was about 118 µm, whereas the cell incubated with Lipofectin migrated about 207 µm and the control cells about 215 µm. After 24 h the results were about: control 288 µm, Lipofectin treated cells: 313 µm and SEQ ID NO 14 treated cell about 166 µm. Migration after 48 h was about 420 µm for the control, 421 µm for Lipofectin treated cells and about 197 µm for cells treated with SEQ ID NO 14.

### Example 15 - Renal Cancer

### TGF-beta 1 Suppression

Analysed with TGF-β1 specific ELISA, as described in Example 7, 200 nM of phosphorothioate from SEQ ID NO 14 in the presence of 3 µg/ml Lipofectin reduced TGF-β1 secretion in renal cancer cell line Caki-1 to about 4% compared to untreated control which was set to 100%.

### Cell proliferation:

In the proliferation assay according to example 3 SEQ ID NO 14 reduced proliferation of the renal carcinoma cell line Caki-1 to about 5% in a concentration of 200 nM in the presence of 3 µg/ml Lipofectin compared to untreated control which was set to 100%.

### Example 16 Antisense complementary to m-RNA of genes TGF-beta 1, TGF-beta 2, TGF-beta 3 IL-10

Antisense complementary to m-RNA of the human transforming growth factor beta 1 (TGF-beta 1):

CTGCAGCCTTGACCTCCCAGGATCAAGTGATCCTCCCACCTTAGCCTCCAGAGTAGCTG
GGACCACAGGTGTACATTTTTTAAAAGTGTTTTGTAGAGATAGGGTCTCACTATGTTACC
CAGGCTGGTCTCAAATGCCTGGATTCAAGTATCCTCCCATCTCTGCCTCCCAAAAGTGC
TAGGATTACAGGCGTGAGCACCCCGCCTGGCCTGAACTACTATCTTTTATTGTCTTCTTC
ACTATCCCCCACTAAAGCAGGTTCCTGGTGGGCAGGAACTCCTCCCTTAACCTCTCTGG
GCTTGTTTCCTCAACCTTTAAAATGGGTGTTATCAGAGTCCCTGCCATCTCAGAGTGTTG
CTATGGTGACTGAATGAGTTCATTAATGTAAGGCACTTCAACAGTGCCCAAGGTGCTCA
ATAAATAGATCTAACTACAGTAGTGTTCCCCACTGGTCCCCTGTGCCTTGATGCCGGGC
AAAGGAATAGTGCAGACAGGCAGGAGGCAGAGAGGGAGAGAGAGGGAGTGGGA
GTGGGGGAACGTCAGGGATGGAGACCCCAGGCAGGCGCCCAATGACACAGAGATCCG
CAGTCCTCTCTCCATCTTTAATGGGGCCCCAGGTGGGCTTGGGGCACGGTGTCCTTAA
ATACAGCCCCCATGGGCAAGGCAGCGGGGGCGGGGCGGGGTGGGGCCGGGCCTGCC
GGGGCGGGGCGGGGCGGGGCGGGACCTCAGCTGCACTTGCAGGAGCGCACGATCAT
GTTGGACAGCTGCTCCACCTTGGGCTTGCGGCCCACGTAGTACACGATGGGCAGCGG

CTCCAGCGCCTGCGGCACGCAGCACGGCGCCGCCGAGGCGCCCGGTTATGCTGGTT
GTACAGGGCCAGGACCTTGCTGTACTGCGTGTCCAGGCTCCAAATGTAGGGGCAGGG
CCCGAGGCAGAAGTTGGCATGGTAGCCCTTGGGCTCGTGGATCCACTTCCAGCCGAG
GTCCTTGCGGAAGTCAATGTACAGCTGCCGCACGCAGCAGTTCTTCTCCGTGGAGCTG
AAGCAATAGTTGGTGTCCAGGGCTCGGCGGTGCCGGAGCTTTGCAGATGCTGGGCC
CTCTCCAGCGGGGTGGCCATGAGAAGCAGGAAAGGCCGGTTCATGCCATGAATGGTG
GCCAGGTCACCTCGGCGGCCGGTAGTGAACCCGTTGATGTCCACTTGCAGTGTGTTAT
CCCTGCTGTCACAGGAGCAGTGGGCGCTAAGGCGAAAGCCCTCAATTTCCCCTCCACG
GCTCAACCACTGCCGCACAACTCCGGTGACATCAAAAGATAACCACTCTGGCGAGTCG
CTGGGTGCCAGCAGCCGGTTGCTGAGGTATCGCCAGGAATTGTTGCTGTATTTCTGGT
ACAGCTCCACGTGCTGCTCCACTTTTAACTTGAGCCTCCTCAGCAGACGCAGCTCTGCC
CGGGAGAGCAACACGGGTTCAGGTACCGCTTCTCGGAGCTCTGATGTGTTGAAGAACA
TATATATGCTGTGTGTACTCTGCTTGAACTTGTCATAGATTTCGTTGTGGGTTTCCACCA
TTAGCACGCGGGTGACCTCCTTGGCGTAGTAGTCGGCCTCAGGCTCGGGCTCCGGTTC
TGCACTCTCCCCGGCCACCCGGTCGCGGGTGCTGTTGTACAGGGCGAGCACGGCCTC
GGGCAGCGGGCCGGGCGGCACCTCCCCCTGGCTCGGGGGGCTGGCGAGCCGCAGCT
TGGACAGGATCTGGCCGCGGATGGCCTCGATGCGCTTCCGCTTCACCAGCTCCATGTC
GATAGTCTTGCAGGTGGATAGTCCCGCGGCCGGCGGGCCAGGCGTCAGCACCAGTAG
CCACAGCAGCGGTAGCAGCAGCGGCAGCAGCCGCAGCCCGGAGGGCGGCATGGGGG
AGGCGGCGCCCCCCGGCACTGCCGAGAGCGCGAACAGGGCTGGTGTGGTGGGGAGG
CCCCGCCCCTGCAGGGGCTGGGGGTCTCCCGGCAAAAGGTAGGAGGGCCTCGAGGG
AAAGCTGAGGCTCCTCAGGGAGAAGGGCGCAGTGGTGGAGGGGAGGCTTGGACCGG
GGGTGTCTCAGTATCCCACGGAAATAACCTAGATGGGCGCGATCTGGTACCAGAAGGT
GGGTGGTCTTGAATAGGGGATCTGTGGCAGGTCGGAGAGAGATCCGTCTCCTGGAGG
AGAAAGGGTCTAGGATGCGCGGGGGCTCAGGAGACAGGCCGGGGATGAAGGCGGCG
TGCAGGGGGTGCGCCCGAGGTCTGGGGAAAAGTCTTTGCGGGAGGCCGGGTCGGCG
ACTCCCGAGGGCTGGTCCGGAATGGGGGCGCCTGAGGGACGCCGTGTAGGGGGCAG
GGAGGGAGCAAGCGTCCCCGGCGGCAAAGGGAGGCGGTCTGGGGTCCCCAAGTCCT
GCCTCCTCGCGGGGCAGCGTCGCGCCAAGAGGTCCCCGCGCCTCCGGCTCCCAGCG
GCAACGGAAAAGTCTCAAAAGTTTTTTCCTCTTCTCCCGACCAGCTCGTCCCTCCTCC
CGCTCCTCCTCCCCCTCCTCCCCGCAGTGGCGGGGGCGGCGGCGGCTCGTCTCAGAC
TCTGGGGCCTCAGGCTGCTCCTCGGCGACTCCTTCCTCCGCTCCGGGCCGAGGCCGG
CCCCGCGGGCGGCTCAGAGCCGGGGGGGTGCCCCGGACGGGGCGTCCCCCCTGC
CCCCGGCCGGGGCCCTCGCTGTCTGGCTGCTCCGCGGAGGGAGGT

Antisense complementary to m-RNA of the human transforming growth factor beta 2 (TGF-beta2):

TTTAAAAAAATTTGCTTCTTGTCTCTCTCACTTACAAAGTAGGTGAAATGTAGAATAAGGC
CTTCAACTTTTTTGTGTCAGATGCCAGTTTAACAAACAGAACACAAACTTCCAAAGTGT
CTGAACTAGTACCGCCTTTTCAAAAATTTTTAACACTGATGAACCAAGGCTCTCTTATGT
TTTCTTGTTACAAGCATCATCGTTGTCGTCGTCATCATCATTATCATCATCATTGTCATTT
TGGTCTTGCCACTTTTCCAAGAATTTTAGCTGCATTTGCAAGACTTTACAATCATATTAGA
AAGCTGTTCAATCTTGGGTGTTTTGCCAATGTAGTAGAGAATGGTTAGAGGTTCTAAATC
TTGGGACACGCAGCAAGGAGAAGCAGATGCTTCTGGATTTATGGTATTATATAAGCTCA
GGACCCTGCTGTGCTGAGTGTCTGAACTCCATAAATACGGGCATGCTCCAGCACAGAA
GTTGGCATTGTACCCTTTGGGTTCGTGTATCCATTTCCACCCTAGATCCCTCTTGAAATC
AATGTAAAGTGGACGTAGGCAGCAATTATCCTGCACATTTCTAAAGCAATAGGCCGCAT
CCAAAGCACGCTTCTTCCGCCGGTTGGTCTGTTGTGACTCAAGTCTGTAGGAGGGCAAT
AACATTAGCAGGAGATGTGGGGTCTTCCCACTGTTTTTTTCCTAGTGGACTTTATAGTT
TTCTGATCACCACTGGTATATGTGGAGGTGCCATCAATACCTGCAAATCTTGCTTCTAGT
TCTTCACTTTTATTTGGGATGATGTAATTATTAGATGGTACAAAAGTGCAGCAGGGACAG
TGTAAGCTTATTTTAAATCCCAGGTTCCTGTCTTTATGGTGAAGCCATTCATGAACAGCA
TCAGTTACATCGAAGGAGAGCCATTCGCCTTCTGCTCTTGTTTTCACAACTTTGCTGTCG
ATGTAGCGCTGGGTTGGAGATGTTAAATCTTTGGACTTGAGAATCTGATATAGCTCAATC
CGTTGTTCAGGCACTCTGGCTTTTGGGTTCTGCAAACGAAAGACTCTGAACTCTGCTTT
CACCAAATTGGAAGCATTCTTCTCCATTGCTGAGACGTCAAATCGAACAATTCTGAAGTA
GGGTCTGTAGAAAGTGGGCGGGATGGCATTTTCGGAGGGGAAGAAGGGCGGCATGTC
TATTTTGTAAACCTCCTTGGCGTAGTACTCTTCGTCGCTCCTCTCGCGCTCGCAGGCGG
CCGCCCTCCGGCTCGCCTTCTCCTGGAGCAAGTCCCTGGTGCTGTTGTAGATGGAAAT
CACCTCCGGGGGGACTTCCTCGGGCTCAGGATAGTCTTCTGGGGGACTGGTGAGCTTC
AGCTTGCTCAGGATCTGCCCGCGGATCGCCTCGATCCTCTTGCGCATGAACTGGTCCA
TATCGAGTGTGCTGCAGGTAGACAGGCTGAGCGCGACCGTGACCAGATGCAGGATCAG
AAAAGCGCTCAGCACACAGTAGTGCATTTTTTAAAAAGTGGAAAAAAAAGTTGTTTTTA
AAAGTCAGAATAAAAAAAAAGAAATCAACAATTCTCAAAGTATAGATCAAGGAGAGTTGT
TTGGTTTTTTGTTGTTGTTGTTTGTTTTTGATGCGAAACTTTTGCAAACAATCTAGTCAAT
GCCCAACAGAAAAACGTATCCTGCTTG

Splice variation of Antisense complementary to m-RNA of the human transforming growth factor beta 2 (TGF-beta2) comprising a further insert compared with the antisense complementary to m-RNA of the human transforming growth factor beta 2 given above. The insert is between the position 812 and 900, starting counting from the top. Oligonucleotides hybridising with parts of this antisense molecule are also within the scope of this invention.

CTGACTGAAAGTTTCTTTTATTAACAGTCTTTTTAGTTTTTGGACCTTACAAGAAGCTTCC
TTAGGCAGCTGATACATAACATGTGTTTTCAGGGTCTTTATCTTAATGCAGACTTTCTCG

GTCATATAATAACTCACTTGGGTTTTCATCCCATTAATATGACCTCTTTTTTTCTGGCTCG
TTTGAGTTCAAGTTCCTTAAGCCATCCATGAGTTTCTGGCAAAGTATTTGGTCTCCACTT
TATACACCTGTTTTATTTTCCAAGGGCAATGAAACGTTCATTATATAGTAACACACAATAA
ATAACTCACTGTTAACTCTAAGAGTAAGAATGGGAAGGGTGCCTATTGCATAGCAATACA
GAAAAATCAAGTGAGGCGCGGGATAGGAACGGTACGTACAGCAACTCCACTTAATGGG
ATTTTCCTGTTTGTTGTTGTTGTTGTTGTCGTTGTTCACATAATTAACACTAATAAATT
CTTCCAGTGTTTATTTTTTTCTTTAAAAAAAATTTGCTTCTTGTCTCTCTCACTTACAAAGT
AGGTGAAATGTAGAATAAGGCCTTCAACTTTTTTTGTGTCAGATGCCAGTTTTAACAAAC
AGAACACAAACTTCCAAAGTGTCTGAACTAGTACCGCCTTTTCAAAAATTTTTTAACACT
GATGAACCAAGGCTCTCTTATGTTTTCTTGTTACAAGCATCATCGTTGTCGTCGTCATCA
TCATTATCATCATCATTGTCATTTTGGTCTTGCCACTTTTCCAAGAATTTTAGCTGCATTT
GCAAGACTTTACAATCATATTAGAAAGCTGTTCAATCTTGGGTGTTTTGCCAATGTAGTA
GAGAATGGTTAGAGGTTCTAAATCTTGGGACACGCAGCAAGGAGAAGCAGATGCTTCT
GGATTTATGGTATTATATAAGCTCAGGACCCTGCTGTGCTGAGTGTCTGAACTCCATAAA
TACGGGCATGCTCCAGCACAGAAGTTGGCATTGTACCCTTTGGGTTCGTGTATCCATTT
CCACCCTAGATCCCTCTTGAAATCAATGTAAAGTGGACGTAGGCAGCAATTATCCTGCA
CATTTCTAAAGCAATAGGCCGCATCCAAAGCACGCTTCTTCCGCCGGTTGGTCTGTTGT
GACTCAAGTCTGTAGGAGGGCAATAACATTAGCAGGAGATGTGGGGTCTTCCCACTGTT
TTTTTTCCTAGTGGACTTTATAGTTTTCTGATCACCACTGGTATATGTGGAGGTGCCATC
AATACCTGCAAATCTTGCTTCTAGTTCTTCACTTTTATTTGGGATGATGTAATTATTAGAT
GGTACAAAAGTGCAGCAGGGACAGTGTAAGCTTATTTTAAATCCCAGGTTCCTGTCTTTA
TGGTGAAGCCATTCATGAACAGCATCAGTTACATCGAAGGAGAGCCATTCGCCTTCTGC
TCTTGTTTTCACAACTTTGCTGTCGATGTAGCGCTGGGTTGGAGATGTTAAATCTTTGGA
CTTGAGAATCTGATATAGCTCAATCCGTTGTTCAGGCACTCTGGCTTTTGGGTTCTGCAA
ACGAAAGACTCTGAACTCTGCTTTCACCAAATTGGAAGCATTCTTCTCCATTGCTGAGAC
GTCAAATCGAACAATTCTGAAGTAGGGTCTGTAGAAAGTGGGCGGATGGCATCAAGG
TACCCACAGAGCACCTGGGACTGTCTGGAGCACAAGCTGCCCACTGAGCCAGAGGGT
GTTGTAACAACTGGGCAGACAGTTTCGGAGGGGAAGAAGGGCGGCATGTCTATTTTGT
AAACCTCCTTGGCGTAGTACTCTTCGTCGCTCCTCTCGCGCTCGCAGGCGGCCGCCCT
CCGGCTCGCCTTCTCCTGGAGCAAGTCCCTGGTGCTGTTGTAGATGGAAATCACCTCC
GGGGGGACTTCCTCGGGCTCAGGATAGTCTTCTGGGGGACTGGTGAGCTTCAGCTTGC
TCAGGATCTGCCCGCGGATCGCCTCGATCCTCTTGCGCATGAACTGGTCCATATCGAG
TGTGCTGCAGGTAGACAGGCTGAGCGCGACCGTGACCAGATGCAGGATCAGAAAAGC
GCTCAGCACACAGTAGTGCATTTTTTAAAAAAGTGGAAAAAAAAGTTGTTTTTAAAAGTC
AGAATAAAAAAAAGAAATCAACAATTCTCAAAGTATAGATCAAGGAGAGTTGTTTGGTTT
TTTGTTGTTGTTGTTTTTGATGCGAAACTTTTGCAAACAATCTAGTCAATGCCCAACAGAA
AAACGTATCCTGCTTGAATTCCTTTAAAAAGAAAAGGCCAGTAGTTCCAAAAGTGGAAAT

ATTAATACGGGACGGGCAGAGGGAACCCTGACTTTGGCGAGTAAGAAGAAAAAAATTCT
TGGAGCAATGAGATGGGGAAAAAAAGAGACGAGTGGCTATTAAGTAGAAATAAATTTAA
GAGGAGGAAGTGGAGTTCAGTGTGTCAGTCTCGGGTGCGGAGTGGCGGATCTGAACT
CGGCTCCTCCGGCCAAAAGGGAAGAGATGAAAAGGTCCCGGGGCTGGCAGCTGGCGA
ACTGACGGGAGGGGC

Antisense complementary to m-RNA of the human transforming growth factor beta 3 (TGF-beta 3)

CAGGATGCCCCAAAAATATTTATTTATACAAAGATTTTGAGAGTAATATTCATACTTGTCT
TTATACCTCAGTCTATGCGTCTGGGGCCAAGTCACTGTGTGGCACATGTCGAGCTTCCC
CGAATGCCTCACATGTTGTCGCACCTGCTTCCAGGAACACCAAATGAACACAGGGTCTT
GGAGGGGAAGTGGGGGAAGAACCCATAATGCCCCAACCCTGCATGGAACCACAATCCA
GAAATGTGCATCCTGACCTGGAAGGCGTCTAACCAAGTGTCCAAGGGGAAATATGATC
GAGGGAGAGGTGAGAGGAGGGACCCAGAGGCAGACAGGAGAGGGTTGATTTCCACCC
TTTCTTCTGCGTTCAGCATATCCAAAAGGCCCAATACAGTTGATGGGCCAGGAACTGCA
TGACCTGGATTTTCTCCCTGTAGTGACCCACGATGTTAATTGATGTAGAGGACAGTTTG
CAAAAGTAATAGATTTGCCCTTAATCCCAGACAGTATGAGATACAATTCTGGGACTTTGT
CTTCGTAACCTGTCTTTAAAAAAAAAAAAAAATGCTTGCCTTGTATAACATAATCCAGATT
CCCTAGAGCAGATGTGGTACAGCAATGAGCAAATCCAACCTCAGATCTGAAGTGTCTTC
CAGTCTGGCCCTGACCCAGCCATTCTCTGCCCTTCCTTCTCCCTTTAGGGTAGCCCAAA
TCCCATTGCCACACAACATCTCAACTTACCATCCCTTTCCTCTATCCCCATCCCCTCTGT
CTGCGTCACAGAAAGTCTGTGTGTTCTGAAGAGTTCAGCCTTCCTCTAACCAAACCCAC
ACTTTCTTTACCACCGTGATTCTCAGAGCCAGCAAGAAAGAAATGTTCCAAAAGGAAAC
CTCCATCTCAGCCATTTGCCCGGAGCCGAAGGTTGTGGGCTCCAGGCCTCTCAGTGAG
GTTTGTTGCTTGTGTGTTTCCCGAGGAGCGGGCAGTCAGGCAGTGGTGGTTCTCTCTC
CCCTCTCTCTGTCGCACGTGGGGTCTCAGCTACATTTACAAGACTTCACCACCATGTTG
GAGAGCTGCTCCACTTTGGGGGTCCTCCCAACATAGTACAGGATGGTCAGGGGCTCCA
GGTCCTGGGGCACGCAGCAAGGCGAGGCAGATGCTTCAGGGTTCAGAGTGTTGTACA
GTCCCAGCACCGTGCTGTGGGTTGTGTCTGCACTGCGGAGGTATGGGCAAGGGCCTG
AGCAGAAGTTGGCATAGTAGCCCTTAGGTTCATGGACCCACTTCCAGCCCAGATCCTGT
CGGAAGTCAATGTAGAGGGGGCGCACACAGCAGTTCTCCTCCAAGTTGCGGAAGCAGT
AATTGGTGTCCAAAGCCCGCTTCTTCCTCTGACCCCCCTGGCCCGGGTTGTCGAGCCG
GTGTGGGGGAATCATCATGAGGATTAGATGAGGGTTGTGGTGATCCTTCTGCTTCTTGA
GGCGCCCCAGATCTCCACGGCCATGGTCATCCTCATTGTCCACGCCTTTGAATTTGATT
TCCATCACCTCGTGAATGTTTTCCAGGATATCTCCATTGGGCTGAAAGGTGTGACATGG
ACAGTGAATGCTGATTTCTAGACCTAAGTTGGACTCTCTTCTCAACAGCCACTCACGCA

CAGTGTCAGTGACATCAAAGGACAGCCACTCGGCAGTGCCCCGTGTGGGCAGATTCTT
GCCACCGATATAGCGCTGTTTGGCAATGTGCTCATCTGGCCGAAGGATCTGGAAGAGC
TCGATCCTCTGCTCATTCCGCTTAGAGCTGGGGTTGGGCACCCGCAAGACCCGGAATT
CTGCTCGGAATAGGTTGGTTCTATTTTTCTCCACTGAGGACACATTGAAGCGGAAAACC
TTGGAGGTAATTCCTTTAGGGCAGACAGCCAGTTCGTTGTGCTCCGCCAGCCCCTGGA
TCATGTCGAATTTATGGATTTCTTTGGCATAGTATTCCGACTCGGTGTTTTCCTGGGTGC
AGCCTTCCTCCCTCTCCCCATGCATCTCCTCCAGCAGCTCCCGGGTGCTGTTGTAAAGG
GCCAGGACCTGATAGGGGACGTGGGTCATCACCGTTGGCTCAGGGGGGCTGGTGAGC
CTGAGCTTGCTCAAGATCTGTCCCCTAATGGCTTCCACCCTCTTCTTCTTGATGTGGCC
GAAGTCCAAGGTGGTGCAAGTGGACAGAGAGAGGCTGACCGTGGCAAAGTTCAGCAG
GGCCAGGACCACCAGAGCCCTTTGCAAGTGCATCTTCATGTGTGAGCTGGGAAGAGAG
GCCAGGGGGACGGCAAGGCCTGGAGAGGAAGAGACCCCAGCAGACGTGCAGAAGGA
GGGAGGAAAACCAGGCGGCCTCCCCAGATCCCAAAGACTGAGGCTTGGCAAGAAGGT
GCATGAACTCACTGCACTGCGAGAGCTTCAGGACTTCCAGGAAGCGCTGGCAACCCTG
AGGACGAAGAAGCGGACTGTGTGCCTTGTAGCGCTGGGATTCTTGTCCATGTGTCTAAA
CAGGTTTTGCTGG

Antisense of m-RNA of human Interleukin 10

TCACCCTATGGAAACAGCTTAAAAACAGGTGAAAATAATAAATATTGAAAAAATTATAAT
ATTGGGCTTCTTTCTAAATCGTTCACAGAGAAGCTCAGTAAATAAATAGAAATGGGGGTT
GAGGTATCAGAGGTAATAAATATTCTATAAGAGAGGTACAATAAGGTTTCTCAAGGGGC
TGGGTCAGCTATCCCAGAGCCCCAGATCCGATTTTGGAGACCTCTAATTTATGTCCTAG
AGTCTATAGAGTCGCCACCCTGATGTCTCAGTTTCGTATCTTCATTGTCATGTAGGCTTC
TATGTAGTTGATGAAGATGTCAAACTCACTCATGGCTTTGTAGATGCCTTTCTCTTGGAG
CTTATTAAAGGCATTCTTCACCTGCTCCACGGCCTTGCTCTTGTTTTCACAGGGAAGAAA
TCGATGACAGCGCCGTAGCCTCAGCCTGAGGGTCTTCAGGTTCTCCCCCAGGGAGTTC
ACATGCGCCTTGATGTCTGGGTCTTGGTTCTCAGCTTGGGGCATCACCTCCTCCAGGTA
AAACTGGATCATCTCAGACAAGGCTTGGCAACCCAGGTAACCCTTAAAGTCCTCCAGCA
AGGACTCCTTTAACAACAAGTTGTCCAGCTGATCCTTCATTTGAAAGAAAGTCTTCACTC
TGCTGAAGGCATCTCGGAGATCTCGAAGCATGTTAGGCAGGTTGCCTGGGAAGTGGGT
GCAGCTGTTCTCAGACTGGGTGCCCTGGCCTGGGCTGGCCCTCACCCCAGTCAGGAG
GACCAGGCAACAGAGCAGTGCTGAGCTGTGCATGCCTTCTTTTGCAAGTCTGTCTTGTG
GTTTGGTTTTGCAAGAGCAACCCCCTGATGTGTAGACCTTCACCTCTCTGTCCCCCTTTT
ATATTGTAAGCTCAGGGAGGCCTCTTCATTCATTAAAAAGCCACAATCAAGGTTTCCCG
GCACAGGATTTTTTCTGCTTAGAGCTCCTCCTTCTCTAACCTCTCTAATAAACTTAGTTTT
CAATTTTTGCATCGTAAGCAAAAATGATTGGTTGAACATGAACTTCTGCATTACAGCTATT
TTTAGGATGGGCTACCTCTCTTAGAATAATTTTTTAGCTTCTCAATTAAAAAAAGTTGATT

TCCTGGGGAGAACAGCTGTTCTGTCCGCAGAGGCCCTCAGCTGTGGGTTCTCATTCGC
GTGTTCCTAGGTCACAGTGACGTGGACAAATTGCCCATTCCAGAATACAATGGGATTGA
GAAATAATTGG

### Example 17 -Synthesis of oligonucleotides

On method for synthesizing oligondesoxy-nucleotides is performed by stepwise 5 addition of protected nucleosides using phosphite triester chemistry. The first nucleotide is introduced as 5'-dimethoxytrityl-deoxyadenosine(N<4>benzoyl)-N N'diisopropyl-2-cyanoethyl phosphoramidite (01 1 M); C is introduced by a 5'dimethoxytrityl-deoxycytidine (N<4>benzoyl)-N, N'-diisopropyl-2-cyanoethyl phosphoramidite; G is introduced as 5'-dimethoxytrityl-deoxyguanosine(N<8>isobutyryl)-N, N'diisopropyl-2-cyanoethyl phosphoramidite and the T was introduced as 5'-dimethoxytrityl-deoxythymidine-N, N'-di-isopropyl-2-cyanoethyl phosphoramidite. The nucleosides were preferably applied in 0.1 M concentration dissolved in acetonitril.

Synthesis is performed on controlled pore glass particles of about 150 Fm diameter (pore diameter about 500 Â) to which the most 3 nucleoside is covalently attached via a long chain alkylamin linker (average loading about 30 Fmol/g solid support).

The solid support is loaded into a cylindrical synthesis column capped on both ends with filters which permit adequate flow of reagents but hold back the solid synthesis support. Reagents are delivered and withdrawn from the synthesis column using positive pressure of inert gas The nucleotides were added to the growing oligonucleotide chain in 3' -> 5' direction. Each nucleotide was coupled using one round of the following synthesis cycle.

Cleaving 5'DMT (dimethoxytrityl) protecting group of the previous nucleotide with 3-chloroacetic acid in dichloromethane followed by washing the column with anhydrous acetonitrile. Then simultaneously one of the bases in form of their protected derivative depending on the sequence is added plus tetrazole in acetonitrile. After reaction the reaction mixture has been withdrawn and the phosphite is oxidized with a mixture of sulfur (S8) in carbon disulfid/pyridine/triethylamine. After the oxidation reaction the mixture is withdrawn and the column was washed with acetonitrile. The unreacted 5'-hydroxyl groups are capped with simultaneous addition of 1-methyltmidazole and acetic anhydryide/lutidine/tetrahydrofuran. Thereafter the synthesis column is washed with acetonitrile and the next cycle was started.

The work up procedure and purification of the synthesis products occurs as follows:
After the addition of the last nucleotide the deoxynucleotides were cleaved from the solid support by incubation in ammonia solution. Exoxyclic base protecting groups are removed by further incubation in ammonia. Then the ammonia is evaporated under vacuum. Full-length synthesis products still bearing the 5'DMT protecting group are separated from shorter failure contaminants using reverse phase high performance liquid chromatography on silica C18 stationary phase. Eluents from the product peak are collected dried under vacuum and the 5'-DMT protecting group cleaved by incubation in acetic acid which is evaporated thereafter under vacuum. The synthesis products are solubilized in the deionized water and extracted three times with diethylether. Then the products are dried in vacuo. Another HPLC-AX chromatography is performed and the eluents from the product peak are dialysed against excess of Tris-buffer and then dialysed against deionized water. The final products are lyophilized and stored dry.

### Example 18

Cell mediated cytotoxicity assays were performed according to protocol of Example 2 with phosphorthioated antisense oligonucleotides of TGF beta 2: SEQ ID NO 30 respectively TGF-beta 1: SEQ ID NO 14.

TGF-beta 2 - pancreatic cancer cell line PA-TU-8902 Surprisingly cell mediated cytotoxicity inhibited by high levels of TGF-beta 2, was nearly completely restored in the pancreatic cancer cell line PA-TU-8902 by 200 nM of SEQ ID NO 30 in the presence of 3µg/ml Lipofectin. The test was consolidated by different ratios of peripheral blood mononuclear cells (PBMCs) to tumor cells (10:1, 5:1, 2,5:1). The respective restoration of the cell mediated cytotoxicity was 80%, 88% and 100%. Results were taken from triplicates. Comparable results were found in non-small cell lung carcinoma cell line K562, colon cancer cell line HCT-116.

### TGF-beta 2 - pancreatic cancer cell line PA-TU-8902

Surprisingly, cell mediated cytotoxicity on Hup-T3 target cells of PBMC cultured in the cell culture supernatants of PA-TU-8902 cells which were treated with 400 nM of SEQ ID NO 30 in the presence of 6 µg/ml Lipofectin, was enhanced by about 140-400 % compared to untreated control at the effector:target cell ratios (20:1, 10:1, 5:1, 2,5:1, 1,25:1). Results were taken from quadruplicates.

### TGF-beta 1 - colon cancer cell line K562

Cell mediated cytotoxicity inhibited by high levels of TGF-beta 1, was completely restored in the colon cancer cell line K562 by 400 nM of SEQ ID NO 30 in the presence of 6 jug/ml Lipofectin-The test was consolidated by different ratios of peripheral blood mononuclear cells (PBMCs) to tumor cells (20:1, 10:1, 5:1, 2,5:1, 1,25:1). The respective restoration of the cell mediated cytotoxicity at all of these ratios was 100%. Results were taken from triplicates.

### TGF-beta 1 - colon cancer cell line HCT-116

Surprisingly, cell mediated cytotoxicity on K562 target cells of PBMC cultured in the cell culture supernatants of HCT-116 cells which were treated with 400 nM of SEQ ID NO 30 in the presence of 6 µg/ml Lipofectin, was enhanced by about 170-285% compared to untreated control at the indicated effector:target cell ratios (20:1, 10:1, 5:1, 2.5:1, 1.25:1). Results were taken from quadruplicates.

TGF-beta 1: non-small cell lung (NSCLC) carcinoma cell line HCl-H661 Cell mediated cytotoxicity inhibited by high levels of TGF-beta 1, was also was completely restored in the non-small cell lung carcinoma cell line HCl-H661 by 200 nM of SEQ ID NO 30 in the presence of 3 µL/g/ml Lipofectin. The test was consolidated by different ratios of peripheral blood mononuclear cells (PBMCs) to tumor cells (20:1, 10:1, 5:1,2,5:1). The respective restoration of the cell mediated cytotoxicity in all of these ratios was 100%. Results were taken from triplicates.

### TGF-beta 1: non-small cell lung (NSCLC) carcinoma cell line A-549

Surprisingly, cell mediated cytotoxicity on Hup-T3 target cells of PBMC cultured in the cell culture supernatants of PA-TU-8902 cells which were treated with 200 nM of SEQ ID NO 30 in the presence of 3 µg/ml Lipofectin, was enhanced by about 250-415% at the indicated effector:target cell ratios (10:1, 5:1,2.5:1,1.25:1,0.6251). Results were taken from quadruplicates.

### TGF-beta 1: prostate cancer cell line PC-3

Surprisingly, cell mediated cytotoxicity on K562 target cells of PBMC cultured in the cell culture supernatants of PC-3 cells which were treated with 400 nM of SEQ ID NO 30 in the presence of 6 µg/ml Lipofectin, was enhanced by about 130-270% at the indicated effector:target cell ratios (20:1,10:1,5:1,2.5:1,1.25:1). Results were taken from quadruplicates.

### Example 19

TGF-beta1 antisense oligonucleotides:

ctgcagccttgacctccc, aggatcaagtgatcctcc, caccttagcctccagagt, agctgggaccacaggtgt, acatttttaaaagtgtt,
ttgtagagatagggtctc, actatgttacccaggctg, gtctcaaatgcctggatt, caagtatcctcccatctc, tgcctcccaaaagtgcta,
ggattacaggcgtgagca, ccccgcctggcctgaact, actatcttttattgtctt, cttcactatcccccacta, aagcaggttcctggtggg,
caggaactcctcccttaa, cctctctgggcttgtttc, ctcaacctttaaaatggg, tgttatcagagtccctgc, catctcagagtgttgcta,
tggtgactgaatgagttc, attaatgtaaggcacttc, aacagtgcccaaggtgct, caataaatagatctaact, acagtagtgttccccact,
ggtcccctgtgccttgat, gccgggcaaaggaatagt, gcagacaggcaggaggag, gcagagagggagagagag, ggagtgggagtgggggaa,
cgtcagggatggagaccc, caggcaggcgcccaatga, cacagagatccgcagtcc, tctctccatctttaatgg, ggccccaggtgggcttgg,
ggcacggtgtccttaaat, acagcccccatgggcaag, gcagcgggggcggggcgg, ggtggggccgggcctgcc, ggggcggggcggggcggg,
gcgggacctcagctgcac, ttgcaggagcgcacgatc, atgttggacagctgctcc, accttgggcttgcggccc, acgtagtacacgatgggc,
agcggctccagcgcctgc, ggcacgcagcacggcgcc, gccgaggcgcccgggtta, tgctggttgtacagggcc, aggaccttgctgtactgc,
gtgtccaggctccaaatg, taggggcagggcccgagg, cagaagttggcatggtag, cccttgggctcgtggatc, cacttccagccgaggtcc,
ttgcggaagtcaatgtac, agctgccgcacgcagcag, ttcttctccgtggagctg, aagcaatagttggtgtcc, agggctcggcggtgccgg,
gagctttgcagatgctgg, gccctctccagcggggtg, gccatgagaagcaggaaa, ggccggttcatgccatga, atggtggccaggtcacct,
cggcggccggtagtgaac, ccgttgatgtccacttgc, agtgtgttatccctgctg, tcacaggagcagtgggcg, ctaaggcgaaagccctca,
atttcccctccacggctc, aaccactgccgcacaact, ccggtgacatcaaaagat, aaccactctggcgagtcg, ctgggtgccagcagccgg,
ttgctgaggtatcgccag, gaattgttgctgtatttc, tggtacagctccacgtgc, tgctccacttttaacttg, agcctcctcagcagacgc,
agctctgcccgggagagc, aacacgggttcaggtacc, gcttctcggagctctgat, gtgttgaagaacatatat, atgctgtgtgtactctgc,
ttgaacttgtcatagatt, tcgttgtgggtttccacc, attagcacgcgggtgacc, tccttggcgtagtagtcg, gcctcaggctcgggctcc,
ggttctgcactctccccg, gccacccggtcgcgggtg, ctgttgtacagggcgagc, acggcctcgggcagcggg, ccgggcggcacctccccc,
tggctcgggggctggcg, agccgcagcttggacagg, atctggccgcggatggcc, tcgatgcgcttccgcttc, accagctccatgtcgata,
gtcttgcaggtggatagt, cccgcggccggcggcca, ggcgtcagcaccagtagc, cacagcagcggtagcagc, agcggcagcagccgcagc,
ccggagggcggcatgggg, gaggcggcgcccccggc, actgccgagagcgcgaac, agggctggtgtggtgggg, aggccccgcccctgcagg,
ggctgggggtctcccggc, aaaaggtaggagggcctc, gagggaaagctgaggctc, ctcagggagaagggcgca, gtggtggagggggaggctt,
ggaccgggggtgtctcag, tatcccacggaaataacc, tagatgggcgcgatctgg, taccagaaggtgggtggt, cttgaataggggatctgt,
ggcaggtcggagagagat, ccgtctcctggaggagaa, agggtctaggatgcgcgg, gggctcaggagacaggcc, ggggatgaaggcggcgtg,
caggggggtgcgcccgagg, tctggggaaaagtctttg, cgggaggccgggtcggcg, actcccgaggctggtcc, ggaatgggggcgcctgag,
ggacgccgtgtagggggc, agggagggagcaagcgtc, cccggcggcaaagggagg, cggtctggggtccccaag, tcctgcctcctcgcgggg,
cagcgtcgcgccaagagg, tccccgcgcctccggctc, ccagcggcaacggaaaag, tctcaaaagtttttttcc, tcttctcccgaccagctc,
gtccctcctcccgctcct, cctccccctcctccccgc, agtggcgggggcggcggc, ggctcgtctcagactctg, gggcctcaggctgctcct,
cggcgactccttcctccg, ctccgggccgaggccggc, cccgcgggcggctcagag, ccgggggggggtgccccgg, acggggcgtccccccctgc,
ccccggccggggccctcg, ctgtctggctgctccgcg, tgcagccttgacctccca, ggatcaagtgatcctccc, accttagcctccagagta,
gctgggaccacaggtgta, catttttaaaagtgttt, tgtagagatagggtctca, ctatgttacccaggctgg, tctcaaatgcctggattc,
aagtatcctcccatctct, gcctcccaaaagtgctag, gattacaggcgtgagcac, cccgcctggcctgaacta, ctatcttttattgtcttc,
ttcactatcccccactaa, agcaggttcctggtgggc, aggaactcctcccttaac, ctctctgggcttgtttcc, tcaacctttaaaatgggt,
gttatcagagtccctgcc, atctcagagtgttgctat, ggtgactgaatgagttca, ttaatgtaaggcacttca, acagtgcccaaggtgctc,
aataaatagatctaacta, cagtagtgttccccactg, gtcccctgtgccttgatg, ccgggcaaaggaatagtg, cagacaggcaggaggagg,
cagagagggagagagagg, gagtgggagtgggggaac, gtcagggatggagacccc, aggcaggcgcccaatgac, acagagatccgcagtcct,
ctctccatctttaatggg, gccccaggtgggcttggg, gcacggtgtccttaaata, cagcccccatgggcaagg, cagcgggggcggggcggg,
gtggggccgggcctgccg, gggcggggcggggcgggg, cgggacctcagctgcact, tgcaggagcgcacgatca, tgttggacagctgctcca,
ccttgggcttgcggccca, cgtagtacacgatgggca, gcggctccagcgcctgcg, gcacgcagcacggcgccg, ccgaggcgcccgggttat,
gctggttgtacagggcca, ggaccttgctgtactgcg, tgtccaggctccaaatgt, aggggcagggcccgaggc, agaagttggcatggtagc,
ccttgggctcgtggatcc, acttccagccgaggtcct, tgcggaagtcaatgtaca, gctgccgcacgcagcagt, tcttctccgtggagctga,
agcaatagttggtgtcca, gggctcggcggtgccggg, agctttgcagatgctggg, ccctctccagcggggtgg, ccatgagaagcaggaaag,
gccggttcatgccatgaa, tggtggccaggtcacctc, ggcggccggtagtgaacc, cgttgatgtccacttgca, gtgtgttatccctgctgt,
cacaggagcagtgggcgc, taaggcgaaagccctcaa, tttcccctccacggctca, accactgccgcacaactc, cggtgacatcaaaagata,
accactctggcgagtcgc, tgggtgccagcagccggt, tgctgaggtatcgccagg, aattgttgctgtatttct, ggtacagctccacgtgct,
gctccacttttaacttga, gcctcctcagcagacgca, gctctgcccgggagagca, acacgggttcaggtaccg, cttctcggagctctgatg,
tgttgaagaacatatata, tgctgtgtgtactctgct, tgaacttgtcatagattt, cgttgtgggtttccacca, ttagcacgcgggtgacct,
ccttggcgtagtagtcgg, cctcaggctcgggctccg, gttctgcactctccccgg, ccacccggtcgcgggtgc, tgttgtacagggcgagca,
cggcctcgggcagcgggc, cgggcggcacctccccct, ggctcgggggctggcga, gccgcagcttggacagga, tctggccgcggatggcct,
cgatgcgcttccgcttca, ccagctccatgtcgatag, tcttgcaggtggatagtc, ccgcggccggcggccag, gcgtcagcaccagtagcc,
acagcagcggtagcagca, gcggcagcagccgcagcc, cggagggcggcatgggg, aggcggcgcccccggca, ctgccgagagcgcgaaca,

gggctggtgtggtgggga, ggccccgcccctgcaggg, gctggggtctcccggca, aaaggtaggagggcctcg, agggaaagctgaggctcc,
tcagggagaagggcgcag, tggtggagggggaggcttg, gaccgggggtgtctcagt, atcccacggaaataacct, agatgggcgcgatctggt,
accagaaggtgggtggtc, ttgaataggggatctgtg, gcaggtcggagagagatc, cgtctcctggaggagaaa, gggtctaggatgcgcggg,
ggctcaggagacaggccg, gggatgaaggcggcgtgc, aggggtgcgcccgaggt, ctggggaaaagtctttgc, gggaggccgggtcggcga,
ctcccgagggctggtccg, gaatgggggcgcctgagg, gacgccgtgtagggggca, gggagggagcaagcgtcc, ccggcggcaaagggaggc,
ggtctggggtccccaagt, cctgcctcctcgcggggc, agcgtcgcgccaagaggt, ccccgcgcctccggctcc, cagcggcaacggaaaagt,
ctcaaaagtttttttcct, cttctcccgaccagctcg, tccctcctcccgctcctc, ctccccctcctccccgca, gtggcgggggcggcggcg,
gctcgtctcagactctgg, ggcctcaggctgctcctc, ggcgactccttcctccgc, tccgggccgaggccggcc, ccgcgggcggctcagagc,
cgggggggggtgccccgga, cggggcgtccccctgcc, cccggccggggccctcgc, tgtctggctgctccgcgg, gcagccttgacctcccag,
gatcaagtgatcctccca, ccttagcctccagagtag, ctgggaccacaggtgtac, atttttaaaagtgtttt, gtagagatagggtctcac,
tatgttacccaggctggt, ctcaaatgcctggattca, agtatcctcccatctctg, cctcccaaaagtgctagg, attacaggcgtgagcacc,
ccgcctggcctgaactac, tatcttttattgtcttct, tcactatcccccactaaa, gcaggttcctggtgggca, ggaactcctcccttaacc,
tctctgggcttgtttcct, caacctttaaaatgggtg, ttatcagagtccctgcca, tctcagagtgttgctatg, gtgactgaatgagttcat,
taatgtaaggcacttcaa, cagtgcccaaggtgctca, ataaatagatctaactac, agtagtgttccccactgg, tcccctgtgccttgatgc,
cgggcaaaggaatagtgc, agacaggcaggaggaggc, agagagggagagagaggg, agtgggagtgggggaacg, tcagggatggagacccca,
ggcaggcgcccaatgaca, cagagatccgcagtcctc, tctccatctttaatgggg, ccccaggtgggcttgggg, cacggtgtccttaaatac,
agcccccatgggcaaggc, agcgggggcggggcgggg, tggggccgggcctgccgg, ggcggggcggggcggggc, gggacctcagctgcactt,
gcaggagcgcacgatcat, gttggacagctgctccac, cttgggcttgcggcccac, gtagtacacgatgggcag, cggctccagcgcctgcgg,
cacgcagcacggcgccgc, cgaggcgcccgggttatg, ctggttgtacagggccag, gaccttgctgtactgcgt, gtccaggctccaaatgta,
ggggcagggcccgaggca, gaagttggcatggtagcc, cttgggctcgtggatcca, cttccagccgaggtcctt, gcggaagtcaatgtacag,
ctgccgcacgcagcagtt, cttctccgtggagctgaa, gcaatagttggtgtccag, ggctcggcggtgccggga, gctttgcagatgctgggc,
cctctccagcggggtggc, catgagaagcaggaaagg, ccggttcatgccatgaat, ggtggccaggtcacctcg, gcggccggtagtgaaccc,
gttgatgtccacttgcag, tgtgttatccctgctgtc, acaggagcagtgggcgct, aaggcgaaagccctcaat, ttcccctccacggctcaa,
ccactgccgcacaactcc, ggtgacatcaaaagataa, ccactctggcgagtcgct, gggtgccagcagccggtt, gctgaggtatcgccagga,
attgttgctgtatttctg, gtacagctccacgtgctg, ctccacttttaacttgag, cctcctcagcagacgcag, ctctgcccgggagagcaa,
cacgggttcaggtaccgc, ttctcggagctctgatgt, gttgaagaacatatatat, gctgtgtgtactctgctt, gaacttgtcatagatttc,
gttgtgggtttccaccat, tagcacgcgggtgacctc, cttggcgtagtagtcggc, ctcaggctcgggctccgg, ttctgcactctcccggc,
cacccggtcgcgggtgct, gttgtacagggcgagcac, ggcctcgggcagcgggcc, gggcggcacctcccctg, gctcgggggctggcgag,
ccgcagcttggacaggat, ctggccgcggatggcctc, gatgcgcttccgcttcac, cagctccatgtcgatagt, cttgcaggtggatagtcc,
cgcggccggcgggccagg, cgtcagcaccagtagcca, cagcagcggtagcagcag, cggcagcagccgcagccc, ggagggcggcatggggga,
ggcggcgcccccggcac, tgccgagagcgcgaacag, ggctggtgtggtggggag, gccccgcccctgcagggg, ctggggtctcccggcaa,
aaggtaggagggcctcga, gggaaagctgaggctcct, cagggagaagggcgcagt, ggtggagggggaggcttgg, accgggggtgtctcagta,
tcccacggaaataaccta, gatgggcgcgatctggta, ccagaaggtgggtggtct, tgaataggggatctgtgg, caggtcggagagagatcc,
gtctcctggaggagaaag, ggtctaggatgcgcgggg, gctcaggagacaggccgg, ggatgaaggcggcgtgca, ggggtgcgcccgaggtc,
tggggaaaagtctttgcg, ggaggccgggtcggcgac, tcccgagggctggtccgg, aatgggggcgcctgaggg, acgccgtgtagggggcag,
ggagggagcaagcgtccc, cggcggcaaagggaggcg, gtctggggtccccaagtc, ctgcctcctcgcggggca, gcgtcgcgccaagaggtc,
cccgcgcctccggctccc, agcggcaacggaaaagtc, tcaaaagtttttttcctc, ttctcccgaccagctcgt, ccctcctcccgctcctcc,
tccccctcctccccgcag, tggcgggggcggcggcgg, ctcgtctcagactctggg, gcctcaggctgctcctcg, gcgactccttcctccgct,
ccgggccgaggccggccc, cgcgggcggctcagagcc, gggggggggtgccccggac, ggggcgtccccctgccc, ccggccggggccctcgct,
gtctggctgctccgcgga, cagccttgacctcccagg, atcaagtgatcctcccac, cttagcctccagagtagc, tgggaccacaggtgtaca,
tttttaaaagtgttttg, tagagatagggtctcact, atgttacccaggctggtc, tcaaatgcctggattcaa, gtatcctcccatctctgc,
ctcccaaaagtgctagga, ttacaggcgtgagcaccc, cgcctggcctgaactact, atcttttattgtcttctt, cactatcccccactaaag,
caggttcctggtgggcag, gaactcctcccttaacct, ctctgggcttgtttcctc, aacctttaaaatgggtgt, tatcagagtccctgccat,
ctcagagtgttgctatgg, tgactgaatgagttcatt, aatgtaaggcacttcaac, agtgcccaaggtgctcaa, taaatagatctaactaca,
gtagtgttccccactggt, cccctgtgccttgatgcc, gggcaaaggaatagtgca, gacaggcaggaggaggca, gagagggagagagaggga,
gtgggagtgggggaacgt, cagggatggagaccccag, gcaggcgcccaatgacac, agagatccgcagtcctct, ctccatctttaatggggc,
cccaggtgggcttggggc, acggtgtccttaaataca, gcccccatgggcaaggca, gcgggggcggggcggggt, ggggccgggcctgccggg,
gcggggcggggcggggcg, ggacctcagctgcacttg, caggagcgcacgatcatg, ttggacagctgctccacc, ttgggcttgcggcccacg,
tagtacacgatgggcagc, ggctccagcgcctgcggc, acgcagcacggcgccgcc, gaggcgcccgggttatgc, tggttgtacagggccagg,
accttgctgtactgcgtg, tccaggctccaaatgtag, gggcagggcccgaggcag, aagttggcatggtagccc, ttgggctcgtggatccac,
ttccagccgaggtccttg, cggaagtcaatgtacagc, tgccgcacgcagcagttc, ttctccgtggagctgaag, caatagttggtgtccagg,
gctcggcggtgccgggag, ctttgcagatgctgggcc, ctctccagcggggtggcc, atgagaagcaggaaaggc, cggttcatgccatgaatg,
gtggccaggtcacctcgg, cggccggtagtgaacccg, ttgatgtccacttgcagt, gtgttatccctgctgtca, caggagcagtgggcgcta,
aggcgaaagccctcaatt, tcccctccacggctcaac, cactgccgcacaactccg, gtgacatcaaaagataac, cactctggcgagtcgctg,
ggtgccagcagccggttg, ctgaggtatcgccaggaa, ttgttgctgtatttctgg, tacagctccacgtgctgc, tccacttttaacttgagc,
ctcctcagcagacgcagc, tctgcccgggagagcaac, acgggttcaggtaccgct, tctcggagctctgatgtg, ttgaagaacatatatatg,
ctgtgtgtactctgcttg, aacttgtcatagatttcg, ttgtgggtttccaccatt, agcacgcgggtgacctcc, ttggcgtagtagtcggcc,
tcaggctcgggctccggt, tctgcactctcccggcc, acccggtcgcgggtgctg, ttgtacagggcgagcacg, gcctcgggcagcgggccg,
ggcggcacctcccctgg, ctcgggggctggcgagc, cgcagcttggacaggatc, tggccgcggatggcctcg, atgcgcttccgcttcacc,
agctccatgtcgatagtc, ttgcaggtggatagtccc, gcggccggcgggccaggc, gtcagcaccagtagccac, agcagcggtagcagcagc,
ggcagcagccgcagcccg, gagggcggcatgggggag, gcggcgcccccggcact, gccgagagcgcgaacagg, gctggtgtggtggggagg,
ccccgcccctgcaggggc, tggggtctcccggcaaa, aggtaggagggcctcgag, ggaaagctgaggctcctc, agggagaagggcgcagtg,
gtggagggggaggcttgga, ccgggggtgtctcagtat, cccacggaaataacctag, atgggcgcgatctggtac, cagaaggtgggtggtctt,
gaataggggatctgtggc, aggtcggagagagatccg, tctcctggaggagaaagg, gtctaggatgcgcggggg, ctcaggagacaggccggg,
gatgaaggcggcgtgcag, ggggtgcgcccgaggtct, ggggaaaagtctttgcgg, gaggccgggtcggcgact, cccgagggctggtccgga,
atgggggcgcctgaggga, cgccgtgtagggggcagg, gagggagcaagcgtcccc, ggcggcaaagggaggcgg, tctggggtccccaagtcc,
tgcctcctcgcggggcag, cgtcgcgccaagaggtcc, ccgcgcctccggctccca, gcggcaacggaaaagtct, caaaagtttttttcctct,
tctcccgaccagctcgtc, cctcctcccgctcctcct, cccccctcctccccgcagt, ggcgggggcggcggcggc, tcgtctcagactctgggg,

cctcaggctgctcctcgg, cgactccttcctccgctc, cgggccgaggccggcccc, gcgggcggctcagagccg, ggggggtgccccggacg,
gggcgtccccccctgcccc, cggccggggccctcgctg, tctggctgctccgcggag, agccttgacctcccagga, tcaagtgatcctcccacc,
ttagcctccagagtagct, gggaccacaggtgtacat, tttttaaaagtgttttgt, agagatagggtctcacta, tgttacccaggctggtct,
caaatgcctggattcaag, tatcctcccatctctgcc, tcccaaaagtgctaggat, tacaggcgtgagcacccc, gcctggcctgaactacta,
tcttttattgtcttcttc, actatcccccactaaagc, aggttcctggtgggcagg, aactcctcccttaacctc, tctgggcttgtttcctca,
acctttaaaatgggtgtt, atcagagtccctgccatc, tcagagtgttgctatggt, gactgaatgagttcatta, atgtaaggcacttcaaca,
gtgcccaaggtgctcaat, aaatagatctaactacag, tagtgttccccactggtc, ccctgtgccttgatgccg, ggcaaaggaatagtgcag,
acaggcaggaggaggcag, agagggagagagagggag, tgggagtgggggaacgtc, agggatggagaccccagg, caggcgcccaatgacaca
gagatccgcagtcctctc, tccatctttaatggggcc, ccaggtgggcttggggca, cggtgtccttaaatacag, cccccatgggcaaggcag,
cgggggcggggcggggtg, gggccgggcctgccgggg, cggggcggggcggggcgg, gacctcagctgcacttgc, aggagcgcacgatcatgt,
tggacagctgctccacct, tgggcttgcggcccacgt, agtacacgatgggcagcg, gctccagcgcctgcggca, cgcagcacggcgccgccg,
aggcgcccgggttatgct, ggttgtacagggccagga, ccttgctgtactgcgtgt, ccaggctccaaatgtagg, ggcagggcccgaggcaga,
agttggcatggtagccct, tgggctcgtggatccact, tccagccgaggtccttgc, ggaagtcaatgtacagct, gccgcacgcagcagttct,
tctccgtggagctgaagc, aatagttggtgtccaggg, ctcggcggtgccgggagc, tttgcagatgctgggccc, tctccagcggggtggcca,
tgagaagcaggaaaggcc, ggttcatgccatgaatgg, tggccaggtcacctcggc, ggccggtagtgaacccgt, tgatgtccacttgcagtg,
tgttatccctgctgtcac, aggagcagtgggcgctaa, ggcgaaagccctcaattt, cccctccacggctcaacc, actgccgcacaactccgg,
tgacatcaaaagataacc, actctggcgagtcgctgg, gtgccagcagccggttgc, tgaggtatcgccaggaat, tgttgctgtatttctggt,
acagctccacgtgctgct, ccactttaacttgagcc, tcctcagcagacgcagct, ctgcccgggagagcaaca, cgggttcaggtaccgctt,
ctcggagctctgatgtgt, tgaagaacatatatatgc, tgtgtgtactctgcttga, acttgtcatagatttcgt, tgtgggtttccaccatta,
gcacgcgggtgacctcct, tggcgtagtagtcggcct, caggctcgggctccggtt, ctgcactctccccggcca, cccggtcgcgggtgctgt,
tgtacagggcgagcacgg, cctcgggcagcgggccgg, gcggcacctcccccctggc, tcggggggctggcgagcc, gcagcttggacaggatct,
ggccgcggatggcctcga, tgcgcttccgcttcacca, gctccatgtcgatagtct, tgcaggtggatagtcccg, cggccggcgggccaggcg,
tcagcaccagtagccaca, gcagcggtagcagcagcg, gcagcagccgcagcccgg, agggcggcatgggggagg, cggcgcccccccggcactg,
ccgagagcgcgaacaggg, ctggtgtggtggggaggc, cccgcccctgcaggggct, ggggggtctcccggcaaaa, ggtaggagggcctcgagg,
gaaagctgaggctcctca, gggagaagggcgcagtgg, tggaggggaggcttggac, cgggggtgtctcagtatc, ccacggaaataacctaga,
tgggcgcgatctggtacc, agaaggtgggtggtcttg, aataggggatctgtggca, ggtcggagagagatccgt, ctcctggaggagaaaggg,
tctaggatgcgcgggggc, tcaggagacaggccgggg, atgaaggcggcgtgcagg, gggtgcgcccgaggtctg, gggaaaagtctttgcggg,
aggccgggtcggcgactc, ccgagggctggtccggaa, tgggggcgcctgagggac, gccgtgtagggggcaggg, agggagcaagcgtccccg,
gcggcaaagggaggcggt, ctggggtccccaagtcct, gcctcctcgcggggcagc, gtcgcgccaagaggtccc, cgcgcctccggctcccag,
cggcaacggaaaagtctc, aaaagtttttttcctctt, ctcccgaccagctcgtcc, ctcctcccgctcctcctc, cccctcctccccgcagtg,
gcgggggcggcggcggct, cgtctcagactctggggc, ctcaggctgctcctcggc, gactccttcctccgctcc, gggccgaggccggccccg,
cgggcggctcagagccgg, ggggggtgccccggacgg, ggcgtccccccctgcccc, ggccggggccctcgctgt, ctggctgctccgcggagg,
gccttgacctcccaggat, caagtgatcctcccacct, tagcctccagagtagctg, ggaccacaggtgtacatt, ttttaaaagtgttttgta,
gagatagggtctcactat, gttacccaggctggtctc, aaatgcctggattcaagt, atcctcccatctctgcct, cccaaaagtgctaggatt,
acaggcgtgagcaccccg, cctggcctgaactactat, cttttattgtcttcttca, ctatcccccactaaagca, ggttcctggtgggcagga,
actcctcccttaacctct, ctgggcttgtttcctcaa, cctttaaaatgggtgtta, tcagagtccctgccatct, cagagtgttgctatggtg,
actgaatgagttcattaa, tgtaaggcacttcaacag, tgcccaaggtgctcaata, aatagatctaactacagt, agtgttccccactggtcc,
cctgtgccttgatgccgg, gcaaaggaatagtgcaga, caggcaggaggaggcaga, gagggagagagagggagt, gggagtgggggaacgtca,
gggatggagaccccaggc, aggcgcccaatgacacag, agatccgcagtcctctct, ccatctttaatggggccc, caggtgggcttggggcac,
ggtgtccttaaatacagc, ccccatgggcaaggcagc, gggggcggggcggggtgg, ggccgggcctgccggggc, ggggcggggcggggcggg,
acctcagctgcacttgca, ggagcgcacgatcatgtt, ggacagctgctccacctt, gggcttgcggcccacgta, gtacacgatgggcagcgg,
ctccagcgcctgcggcac, gcagcacggcgccgccga, ggcgcccgggttatgctg, gttgtacagggccaggac, cttgctgtactgcgtgtc,
caggctccaaatgtaggg, gcagggcccgaggcagaa, gttggcatggtagccctt, gggctcgtggatccactt, ccagccgaggtccttgcg,
gaagtcaatgtacagctg, ccgcacgcagcagttctt, ctccgtggagctgaagca, atagttggtgtccagggc, tcggcggtgccgggagct,
ttgcagatgctgggccct, ctccagcggggtggccat, gagaagcaggaaaggccg, gttcatgccatgaatggt, ggccaggtcacctcggcg,
gccggtagtgaacccgtt, gatgtccacttgcagtgt, gttatccctgctgtcaca, ggagcagtgggcgctaag, gcgaaagccctcaatttc,
ccctccacggctcaacca, ctgccgcacaactccggt, gacatcaaaagataacca, ctctggcgagtcgctggg, tgccagcagccggttgct,
gaggtatcgccaggaatt, gttgctgtatttctggta, cagctccacgtgctgctc, cactttaacttgagcct, cctcagcagacgcagctc,
tgcccgggagagcaacac, gggttcaggtaccgcttc, tcggagctctgatgtgtt, gaagaacatatatatgct, gtgtgtactctgcttgaa,
cttgtcatagatttcgtt, gtgggtttccaccattag, cacgcgggtgacctcctt, ggcgtagtagtcggcctc, aggctcgggctccggttc,
tgcactctccccggccac, ccggtcgcgggtgctgtt, gtacagggcgagcacggc, ctcgggcagcgggccggg, cggcacctcccccctggct,
cggggggctggcgagccg, cagcttggacaggatctg, gccgcggatggcctcgat, gcgcttccgcttcaccag, ctccatgtcgatagtctt,
gcaggtggatagtcccgc, ggccggcgggccaggcgt, cagcaccagtagccacag, cagcggtagcagcagcgg, cagcagccgcagcccgga,
gggcggcatgggggaggc, ggcgcccccccggcactgc, cgagagcgcgaacagggc, tggtgtggtggggaggcc, ccgcccctgcaggggctg,
ggggtctcccggcaaaag, gtaggagggcctcgaggg, aaagctgaggctcctcag, ggagaagggcgcagtggt, ggaggggaggcttggacc,
gggggtgtctcagtatcc, cacggaaataacctagat, gggcgcgatctggtacca, gaaggtgggtggtcttga, ataggggatctgtggcag,
gtcggagagagatccgtc, tcctggaggagaaagggt, ctaggatgcgcgggggct, caggagacaggccgggga, tgaaggcggcgtgcaggg,
ggtgcgcccgaggtctgg, ggaaaagtctttgcggga, ggccgggtcggcgactcc, cgagggctggtccggaat, gggggcgcctgagggacg,
ccgtgtagggggcaggga, gggagcaagcgtccccgg, cggcaaagggaggcggtc, tggggtccccaagtcctg, cctcctcgcggggcagcg,
tcgcgccaagaggtcccc, gcgcctccggctcccagc, ggcaacggaaaagtctca, aaagtttttttcctcttc, tcccgaccagctcgtccc,
tcctcccgctcctcctcc, ccctcctccccgcagtgg, cgggggcggcggcggctc, gtctcagactctggggcc, tcaggctgctcctcggcg,
actccttcctccgctccg, ggccgaggccggccccgc, gggcggctcagagccggg, gggggtgccccggacggg, gcgtccccccctgcccccg,
gccggggccctcgctgtc, tggctgctccgcggaggg, ccttgacctcccaggatc, aagtgatcctcccacctt, agcctccagagtagctgg,
gaccacaggtgtacattt, tttaaaagtgttttgtag, agatagggtctcactatg, ttacccaggctggtctca, aatgcctggattcaagta,
tcctcccatctctgcctc, ccaaaagtgctaggatta, caggcgtgagcaccccgc, ctggcctgaactactatc, ttttattgtcttcttcac,
tatcccccactaaagcag, gttcctggtgggcaggaa, ctcctcccttaacctctc, tgggcttgtttcctcaac, ctttaaaatgggtgttat,
cagagtccctgccatctc, agagtgttgctatggtga, ctgaatgagttcattaat, gtaaggcacttcaacagt, gcccaaggtgctcaataa,
atagatctaactacagta, gtgttccccactggtccc, ctgtgccttgatgccggg, caaaggaatagtgcagac, aggcaggaggaggcagag,

agggagagagagggagtg, ggagtgggggaacgtcag, ggatggagaccccaggca, ggcgcccaatgacacaga, gatccgcagtcctctctc,
catctttaatgggcccc, aggtgggcttggggcacg, gtgtccttaaatacagcc, cccatgggcaaggcagcg, ggggcggggcggggtggg,
gccgggcctgccggggcg, gggcggggcggggcggga, cctcagctgcacttgcag, gagcgcacgatcatgttg, gacagctgctccaccttg,
ggcttgcggcccacgtag, tacacgatgggcagcggc, tccagcgcctgcggcacg, cagcacggcgccgccgag, gcgcccgggttatgctgg,
ttgtacagggccaggacc, ttgctgtactgcgtgtcc, aggctccaaatgtagggg, cagggcccgaggcagaag, ttggcatggtagcccttg,
ggctcgtggatccacttc, cagccgaggtccttgcgg, aagtcaatgtacagctgc, cgcacgcagcagttcttc, tccgtggagctgaagcaa,
tagttggtgtccagggct, cggcggtgccgggagctt, tgcagatgctgggccctc, tccagcggggtggccatg, agaagcaggaaaggccgg,
ttcatgccatgaatggtg, gccaggtcacctcggcgg, ccggtagtgaacccgttg, atgtccacttgcagtgtg, ttatccctgctgtcacag,
gagcagtgggcgctaagg, cgaaagccctcaatttcc, cctccacggctcaaccac, tgccgcacaactccggtg, acatcaaaagataaccac,
tctggcgagtcgctgggt, gccagcagccggttgctg, aggtatcgccaggaattg, ttgctgtatttctggtac, agctccacgtgctgctcc,
acttttaacttgagcctc, ctcagcagacgcagctct, gcccgggagagcaacacg, ggttcaggtaccgcttct, cggagctctgatgtgttg,
aagaacatatatatgctg, tgtgtactctgcttgaac, ttgtcatagatttcgttg, tgggtttccaccattagc, acgcgggtgacctccttg,
gcgtagtagtcggcctca, ggctcgggctccggttct, gcactctccccggccacc, cggtcgcgggtgctgttg, tacagggcgagcacggcc,
tcgggcagcgggccgggc, ggcacctccccctggctc, ggggggctggcgagccgc, agcttggacaggatctgg, ccgcggatggcctcgatg,
cgcttccgcttcaccagc, tccatgtcgatagtcttg, caggtggatagtcccgcg, gccggcgggccaggcgtc, agcaccagtagccacagc,
agcggtagcagcagcggc, agcagccgcagcccggag, ggcggcatgggggaggcg, gcgcccccggcactgcc, gagagcgcgaacagggct,
ggtgtggtggggaggccc, cgcccctgcaggggctgg, gggtctcccggcaaaagg, taggagggcctcgaggga, aagctgaggctcctcagg,
gagaagggcgcagtggtg, gaggggaggcttggaccg, ggggtgtctcagtatccc, acggaaataacctagatg, ggcgcgatctggtaccag,
aaggtgggtggtcttgaa, taggggatctgtggcagg, tcggagagagatccgtct, cctggaggagaaagggtc, taggatgcgcgggggctc,
aggagacaggccggggat, gaaggcggcgtgcagggg, gtgcgcccgaggtctggg, gaaaagtctttgcgggag, gccgggtcggcgactccc,
gagggctggtccggaatg, ggggcgcctgagggacgc, cgtgtagggggcagggag, ggagcaagcgtccccggc, ggcaaagggaggcggtct,
ggggtccccaagtcctgc, ctcctcgcggggcagcgt, cgcgccaagaggtccccg, cgcctccggctcccagcg, gcaacggaaaagtctcaa,
aagtttttttcctcttct, cccgaccagctcgtccct, cctcccgctcctcctccc, cctcctccccgcagtggc, gggggcggcggcggctcg,
tctcagactctggggcct, caggctgctcctcggcga, ctccttcctccgctccgg, gccgaggccggccccgcg, ggcggctcagagccgggg,
ggggtgccccggacgggg, cgtccccccctgcccccgg, ccggggccctcgctgtct, ggctgctccgcggaggga, cttgacctcccaggatca,
agtgatcctcccacctta, gcctccagagtagctggg, accacaggtgtacatttt, ttaaaagtgttttgtaga, gatagggtctcactatgt,
tacccaggctggtctcaa, atgcctggattcaagtat, cctcccatctctgcctcc, caaaagtgctaggattac, aggcgtgagcaccccgcc,
tggcctgaactactatct, tttattgtcttcttcact, atcccccactaaagcagg, ttcctggtgggcaggaac, tcctcccttaacctctct,
gggcttgtttcctcaacc, tttaaaatgggtgttatc, agagtccctgccatctca, gagtgttgctatggtgac, tgaatgagttcattaatg,
taaggcacttcaacagtg, cccaaggtgctcaataaa, tagatctaactacagtag, tgttccccactggtcccc, tgtgccttgatgccgggc,
aaaggaatagtgcagaca, ggcaggaggaggcagaga, gggagagagagggagtgg, gagtgggggaacgtcagg, gatggagaccccaggcag,
gcgcccaatgacacagag, atccgcagtcctctctcc, atctttaatgggcccca, ggtgggcttggggcacgg, tgtccttaaatacagccc,
ccatgggcaaggcagcgg, gggcggggcggggtgggg, ccgggcctgccggggcgg, ggcggggcggggcgggac, ctcagctgcacttgcagg,
agcgcacgatcatgttgg, acagctgctccaccttgg, gcttgcggcccacgtagt, acacgatgggcagcggct, ccagcgcctgcggcacgc,
agcacggcgccgccgagg, cgcccgggttatgctggt, tgtacagggccaggacct, tgctgtactgcgtgtcca, ggctccaaatgtaggggc,
agggcccgaggcagaagt, tggcatggtagcccttgg, gctcgtggatccacttcc, agccgaggtccttgcgga, agtcaatgtacagctgcc,
gcacgcagcagttcttct, ccgtggagctgaagcaat, agttggtgtccagggctc, ggcggtgccgggagcttt, gcagatgctgggccctct,
ccagcggggtggccatga, gaagcaggaaaggccggt, tcatgccatgaatggtgg, ccaggtcacctcggcggc, cggtagtgaacccgttga,
tgtccacttgcagtgtgt, tatccctgctgtcacagg, agcagtgggcgctaaggc, gaaagccctcaatttccc, ctccacggctcaaccact,
gccgcacaactccggtga, catcaaaagataaccact, ctggcgagtcgctgggtg, ccagcagccggttgctga, ggtatcgccaggaattgt,
tgctgtatttctggtaca, gctccacgtgctgctcca, cttttaacttgagcctcc, tcagcagacgcagctctg, cccgggagagcaacacgg,
gttcaggtaccgcttctc, ggagctctgatgtgttga, agaacatatatatgctgt, gtgtactctgcttgaact, tgtcatagatttcgttgt,
gggtttccaccattagca, cgcgggtgacctccttgg, cgtagtagtcggcctcag, gctcgggctccggttctg, cactctccccggccaccc,
ggtcgcgggtgctgttgt, acagggcgagcacggcct, cgggcagcgggccgggcg, gcacctccccctggctcg, gggggctggcgagccgca,
gcttggacaggatctggc, cgcggatggcctcgatgc, gcttccgcttcaccagct, ccatgtcgatagtcttgc, aggtggatagtcccgcgg,
ccggcgggccaggcgtca, gcaccagtagccacagca, gcggtagcagcagcggca, gcagccgcagcccggagg, gcggcatgggggaggcgg,
cgcccccggcactgccg, agagcgcgaacagggctg, gtgtggtggggaggcccc, gcccctgcaggggctggg, ggtctcccggcaaaaggt,
aggagggcctcgagggaa, agctgaggctcctcaggg, agaagggcgcagtggtgg, aggggaggcttggaccgg, gggtgtctcagtatccca,
cggaaataacctagatgg, gcgcgatctggtaccaga, aggtgggtggtcttgaat, aggggatctgtggcaggt, cggagagagatccgtctc,
ctggaggagaaagggtct, aggatgcgcgggggctca, ggagacaggccggggatg, aaggcggcgtgcaggggg, tgcgcccgaggtctgggg,
aaaagtctttgcgggagg, ccgggtcggcgactcccg, agggctggtccggaatgg, gggcgcctgagggacgcc, gtgtagggggcagggagg,
gagcaagcgtccccggcg, gcaaagggaggcggtctg, gggtccccaagtcctgcc, tcctcgcggggcagcgtc, gcgccaagaggtccccgc,
gcctccggctcccagcgg, caacggaaaagtctcaaa, agtttttttcctcttctc, ccgaccagctcgtccctc, ctcccgctcctcctcccc,
ctcctccccgcagtggcg, ggggcggcggcggctcgt, ctcagactctggggcctc, aggctgctcctcggcgac, tccttcctccgctccggg,
ccgaggccggccccgcgg, gcggctcagagccggggg, gggtgccccggacggggc, gtccccccctgcccccggc, cggggccctcgctgtctg,
gctgctccgcggagggag, ttgacctcccaggatcaa, gtgatcctcccaccttag, cctccagagtagctggga, ccacaggtgtacattttt,
taaaagtgttttgtagag, atagggtctcactatgtt, acccaggctggtctcaaa, tgcctggattcaagtatc, ctcccatctctgcctccc,
aaaagtgctaggattaca, ggcgtgagcaccccgcct, ggcctgaactactatctt, ttattgtcttcttcacta, tcccccactaaagcaggt,
tcctggtgggcaggaact, cctcccttaacctctctg, ggcttgtttcctcaacct, ttaaaatgggtgttatca, gagtccctgccatctcag,
agtgttgctatggtgact, gaatgagttcattaatgt, aaggcacttcaacagtgc, ccaaggtgctcaataaat, agatctaactacagtagt,
gttccccactggtcccct, gtgccttgatgccgggca, aaggaatagtgcagacag, gcaggaggaggcagagag, ggagagagagggagtggg,
agtgggggaacgtcaggg, atggagaccccaggcagg, cgcccaatgacacagaga, tccgcagtcctctctcca, tctttaatgggccccag,
gtgggcttggggcacggt, gtccttaaatacagcccc, catgggcaaggcagcggg, ggcggggcggggtggggc, cgggcctgccggggcggg,
gcggggcggggcgggacc, tcagctgcacttgcagga, gcgcacgatcatgttgga, cagctgctccaccttggg, cttgcggcccacgtagta,
cacgatgggcagcggctc, cagcgcctgcggcacgca, gcacggcgccgccgaggc, gcccgggttatgctggtt, gtacagggccaggacctt,
gctgtactgcgtgtccag, gctccaaatgtaggggca, gggcccgaggcagaagtt, ggcatggtagcccttggg, ctcgtggatccacttcca,
gccgaggtccttgcggaa, gtcaatgtacagctgccg, cacgcagcagttcttctc, cgtggagctgaagcaata, gttggtgtccagggctcg,
gcggtgccgggagctttg, cagatgctgggccctctc, cagcggggtggccatgag, aagcaggaaaggccggtt, catgccatgaatggtggc,

caggtcacctcggcggcc, ggtagtgaacccgttgat, gtccacttgcagtgtgtt, atccctgctgtcacagga, gcagtgggcgctaaggcg,
aaagccctcaatttcccc, tccacggctcaaccactg, ccgcacaactccggtgac, atcaaaagataaccactc, tggcgagtcgctgggtgc,
cagcagccggttgctgag, gtatcgccaggaattgtt, gctgtatttctggtacag, ctccacgtgctgctccac, ttttaacttgagcctcct,
cagcagacgcagctctgc, ccgggagagcaacacggg, ttcaggtaccgcttctcg, gagctctgatgtgttgaa, gaacatatatatgctgtg,
tgtactctgcttgaactt, gtcatagatttcgttgtg, ggtttccaccattagcac, gcgggtgacctccttggc, gtagtagtcggcctcagg,
ctcgggctccggttctgc, actctccccggccacccg, gtcgcgggtgctgttgta, cagggcgagcacggcctc, gggcagcgggccgggcgg,
cacctccccctggctcgg, ggggctggcgagccgcag, cttggacaggatctggcc, gcggatggcctcgatgcg, cttccgcttcaccagctc,
catgtcgatagtcttgca, ggtggatagtcccgcggc, cggcgggccaggcgtcag, caccagtagccacagcag, cggtagcagcagcggcag,
cagccgcagcccggaggg, cggcatgggggaggcggc, gccccccggcactgccga, gagcgcgaacagggctgg, tgtggtggggaggccccg,
cccctgcaggggctgggg, gtctcccggcaaaaggta, ggagggcctcgagggaaa, gctgaggctcctcaggga, gaagggcgcagtggtgga,
ggggaggcttggaccggg, ggtgtctcagtatcccac, ggaaataacctagatggg, cgcgatctggtaccagaa, ggtgggtggtcttgaata,
ggggatctgtggcaggtc, ggagagagatccgtctcc, tggaggagaaagggtcta, ggatgcgcgggggctcag, gagacaggccggggatga,
aggcggcgtgcaggggggt, gcgcccgaggtctgggga, aaagtctttgcgggaggc, cgggtcggcgactcccga, gggctggtccggaatggg,
ggcgcctgagggacgccg, tgtagggggcagggaggg, agcaagcgtccccggcgg, caaagggaggcggtctgg, ggtccccaagtcctgcct,
cctcgcggggcagcgtcg, cgccaagaggtccccgcg, cctccggctcccagcggc, aacggaaaagtctcaaaa, gtttttttcctcttctcc,
cgaccagctcgtccctcc, tcccgctcctcctccccc, tcctccccgcagtggcgg, gggcggcggcggctcgtc, tcagactctggggcctca,
ggctgctcctcggcgact, ccttcctccgctccgggc, cgaggccggccccgcggg, cggctcagagccgggggg, ggtgccccggacggggcg,
tccccccctgcccccggcc, ggggccctcgctgtctgg, ctgctccgcggagggagg, tgacctcccaggatcaag, tgatcctcccaccttagc,
ctccagagtagctgggac, cacaggtgtacattttttt, aaaagtgttttgtagaga, tagggtctcactatgtta, cccaggctggtctcaaat,
gcctggattcaagtatcc, tcccatctctgcctccca, aaagtgctaggattacag, gcgtgagcaccccgcctg, gcctgaactactatcttt,
tattgtcttcttcactat, cccccactaaagcaggtt, cctggtgggcaggaactc, ctcccttaacctctctgg, gcttgtttcctcaacctt,
taaaatgggtgttatcag, agtccctgccatctcaga, gtgttgctatggtgactg, aatgagttcattaatgta, aggcacttcaacagtgcc,
caaggtgctcaataaata, gatctaactacagtagtg, ttccccactggtcccctg, tgccttgatgccgggcaa, aggaatagtgcagacagg,
caggaggaggcagagagg, gagagagagggagtggga, gtgggggaacgtcaggga, tggagaccccaggcaggc, gcccaatgacacagagat,
ccgcagtcctctctccat, ctttaatggggccccagg, tgggcttggggcacggtg, tccttaaatacagcccccc, atgggcaaggcagcgggg,
gcggggcggggtggggcc, gggcctgccggggcgggg, cggggcggggcggacct, cagctgcacttgcaggag, cgcacgatcatgttggac,
agctgctccaccttgggc, ttgcggcccacgtagtac, acgatgggcagcggctcc, agcgcctgcggcacgcag, cacggcgccgccgaggcg,
cccgggttatgctggttg, tacagggccaggaccttg, ctgtactgcgtgtccagg, ctccaaatgtaggggcag, ggcccgaggcagaagttg,
gcatggtagcccttgggc, tcgtggatccacttccag, ccgaggtccttgcggaag, tcaatgtacagctgccgc, acgcagcagttcttctcc,
gtggagctgaagcaatag, ttggtgtccagggctcgg, cggtgccgggagctttgc, agatgctgggccctctcc, agcggggtggccatgaga,
agcaggaaaggccggttc, atgccatgaatggtggcc, aggtcacctcggcggccg, gtagtgaacccgttgatg, tccacttgcagtgtgtta,
tccctgctgtcacaggag, cagtgggcgctaaggcga, aagccctcaatttcccct, ccacggctcaaccactgc, cgcacaactccggtgaca,
tcaaaagataaccactct, ggcgagtcgctgggtgcc, agcagccggttgctgagg, tatcgccaggaattgttg, ctgtatttctggtacagc,
tccacgtgctgctccact, tttaacttgagcctcctc, agcagacgcagctctgcc, cgggagagcaacacgggt, tcaggtaccgcttctcgg,
agctctgatgtgttgaag, aacatatatatgctgtgt, gtactctgcttgaacttg, tcatagatttcgttgtgg, gtttccaccattagcacg,
cgggtgacctccttggcg, tagtagtcggcctcaggc, tcgggctccggttctgca, ctctccccggccacccgg, tcgcgggtgctgttgtac,
agggcgagcacggcctcg, ggcagcgggccgggcggc, acctccccctggctcggg, gggctggcgagccgcagc, ttggacaggatctggccg,
cggatggcctcgatgcgc, ttccgcttcaccagctcc, atgtcgatagtcttgcag, gtggatagtcccgcggcc, ggcgggccaggcgtcagc,
accagtagccacagcagc, ggtagcagcagcggcagc, agccgcagcccggagggc, ggcatgggggaggcggcg, ccccccggcactgccgag,
agcgcgaacagggctggt, gtggtggggaggccccgc, ccctgcaggggctggggg, tctcccggcaaaaggtag, gagggcctcgagggaaag,
ctgaggctcctcagggag, aagggcgcagtggtggag, gggaggcttggaccgggg, gtgtctcagtatcccacg, gaaataacctagatgggc,
gcgatctggtaccagaag, gtgggtggtcttgaatag, gggatctgtggcaggtcg, gagagagatccgtctcct, ggaggagaaagggtctag,
gatgcgcgggggctcagg, agacaggccggggatgaa, ggcggcgtgcaggggggtg, cgcccgaggtctggggaa, aagtctttgcgggaggcc,
gggtcggcgactcccgag, ggctggtccggaatgggg, gcgcctgagggacgccgt, gtagggggcagggaggga, gcaagcgtccccggcggc,
aaagggaggcggtctggg, gtccccaagtcctgcctc, ctcgcggggcagcgtcgc, gccaagaggtccccgcgc, ctccggctcccagcggca,
acggaaaagtctcaaaag, tttttttcctcttctccc, gaccagctcgtccctcct, cccgctcctcctccccct, cctccccgcagtggcggg,
ggcggcggcggctcgtct, cagactctggggcctcag, gctgctcctcggcgactc, cttcctccgctccgggcc, gaggccggccccgcgggc,
ggctcagagccgggggggg, gtgccccggacggggcgt, cccccctgcccccggccg, gggccctcgctgtctggc, tgctccgcggagggaggt,
gacctcccaggatcaagt, gatcctcccaccttagcc, tccagagtagctgggacc, acaggtgtacattttttta, aaagtgttttgtagagat,
agggtctcactatgttac, ccaggctggtctcaaatg, cctggattcaagtatcct, cccatctctgcctcccaa, aagtgctaggattacagg,
cgtgagcaccccgcctgg, cctgaactactatctttt, attgtcttcttcactatc, ccccactaaagcaggttc, ctggtgggcaggaactcc,
tcccttaacctctctggg, cttgtttcctcaaccttt, aaaatgggtgttatcaga, gtccctgccatctcagag, tgttgctatggtgactga,
atgagttcattaatgtaa, ggcacttcaacagtgccc, aaggtgctcaataaatag, atctaactacagtagtgt, tccccactggtcccctgt,
gccttgatgccgggcaaa, ggaatagtgcagacaggc, aggaggaggcagagaggg, agagagagggagtgggag, tgggggaacgtcagggat,
ggagaccccaggcaggcg, cccaatgacacagagatc, cgcagtcctctctccatc, tttaatggggccccaggt, gggcttggggcacggtgt,
ccttaaatacagcccccca, tgggcaaggcagcggggg, cggggcggggtggggccg, ggcctgccggggcggggc, ggggcggggcggacctc,
agctgcacttgcaggagc, gcacgatcatgttggaca, gctgctccaccttgggct, tgcggcccacgtagtaca, cgatgggcagcggctcca,
gcgcctgcggcacgcagc, acggcgccgccgaggcgc, ccgggttatgctggttgt, acagggccaggaccttgc, tgtactgcgtgtccaggc,
tccaaatgtaggggcagg, gcccgaggcagaagttgg, catggtagcccttgggct, cgtggatccacttccagc, cgaggtccttgcggaagt,
caatgtacagctgccgca, cgcagcagttcttctccg, tggagctgaagcaatagt, tggtgtccagggctcggc, ggtgccgggagctttgca,
gatgctgggccctctcca, gcggggtggccatgagaa, gcaggaaaggccggttca, tgccatgaatggtggcca, ggtcacctcggcggccgg,
tagtgaacccgttgatgt, ccacttgcagtgtgttat, ccctgctgtcacaggagc, agtgggcgctaaggcgaa, agccctcaatttcccctc,
cacggctcaaccactgcc, gcacaactccggtgacat, caaaagataaccactctg, gcgagtcgctgggtgcca, gcagccggttgctgaggt,
atcgccaggaattgttgc, tgtatttctggtacagct, ccacgtgctgctccactt, ttaacttgagcctcctca, gcagacgcagctctgccc,
gggagagcaacacgggtt, caggtaccgcttctcgga, gctctgatgtgttgaaga, acatatatatgctgtgtg, tactctgcttgaacttgt,
catagatttcgttgtggg, tttccaccattagcacgc, gggtgacctccttggcgt, agtagtcggcctcaggct, cgggctccggttctgcac,
tctccccggccacccggt, cgcgggtgctgttgtaca, gggcgagcacggcctcgg, gcagcgggccgggcggca, cctccccctggctcgggg,
ggctggcgagccgcagct, tggacaggatctggccgc, ggatggcctcgatgcgct, tccgcttcaccagctcca, tgtcgatagtcttgcagg,

tggatagtcccgcggccg, gcgggccaggcgtcagca, ccagtagccacagcagcg, gtagcagcagcggcagca, gccgcagcccggagggcg,
gcatgggggaggcggcgc, cccccggcactgccgaga, gcgcgaacagggctggtg, tggtggggaggccccgcc, cctgcaggggctggggt,
ctcccggcaaaaggtagg, agggcctcgagggaaagc, tgaggctcctcagggaga, agggcgcagtggtggagg, ggaggcttggaccggggg,
tgtctcagtatcccacgg, aaataacctagatgggcg, cgatctggtaccagaagg, tgggtggtcttgaatagg, ggatctgtggcaggtcgg,
agagagatccgtctcctg, gaggagaaagggtctagg, atgcgcgggggctcagga, gacaggccggggatgaag, gcggcgtgcaggggggtgc,
gcccgaggtctggggaaa, agtctttgcgggaggccg, ggtcggcgactcccgagg, gctggtccggaatggggg, cgcctgagggacgccgtg,
taggggggcagggagggag, caagcgtccccggcggca, aagggaggcggtctgggg, tccccaagtcctgcctcc, tcgcggggcagcgtcgcg,
ccaagaggtccccgcgcc, tccggctcccagcggcaa, cggaaaagtctcaaaagt, tttttcctcttctcccg, accagctcgtccctcctc,
ccgctcctcctccccctc, ctccccgcagtggcgggg, gcggcggcggctcgtctc, agactctggggcctcagg, ctgctcctcggcgactcc,
ttcctccgctccgggccg, aggccggccccgcgggcg, gctcagagccgggggggg, tgccccggacggggcgtc, ccccctgcccccggccgg,
ggccctcgctgtctggct, gctccgcggagggaggt, , acctcccaggatcaagtg, atcctcccaccttagcct, ccagagtagctgggacca,
caggtgtacatttttttaa, aagtgttttgtagagata, gggtctcactatgttacc, caggctggtctcaaatgc, ctggattcaagtatcctc,
ccatctctgcctcccaaa, agtgctaggattacaggc, gtgagcaccccgcctggc, ctgaactactatcttttta, ttgtcttcttcactatcc,
cccactaaagcaggttcc, tggtgggcaggaactcct, cccttaacctctctgggc, ttgtttcctcaacctttta, aaatgggtgttatcagag,
tccctgccatctcagagt, gttgctatggtgactgaa, tgagttcattaatgtaag, gcacttcaacagtgccca, aggtgctcaataaataga,
tctaactacagtagtgtt, ccccactggtcccctgtg, ccttgatgccgggcaaag, gaatagtgcagacaggca, ggaggaggcagagaggga,
gagagagggagtgggagt, gggggaacgtcagggatg, gagaccccaggcaggcgc, ccaatgacacagagatcc, gcagtcctctctccatct,
ttaatggggccccaggtg, ggcttggggcacggtgtc, cttaaatacagcccccat, gggcaaggcagcgggggc, ggggcggggtgggccgg,
gcctgccggggcggggcg, gggcggggcgggacctca, gctgcacttgcaggagcg, cacgatcatgttggacag, ctgctccaccttgggctt,
gcggcccacgtagtacac, gatgggcagcggctccag, cgcctgcggcacgcagca, cggcgccgccgaggcgcc, cgggttatgctggttgta,
cagggccaggaccttgct, gtactgcgtgtccaggct, ccaaatgtaggggcaggg, cccgaggcagaagttggc, atggtagcccttgggctc,
gtggatccacttccagcc, gaggtccttgcggaagtc, aatgtacagctgccgcac, gcagcagttcttctccgt, ggagctgaagcaatagtt,
ggtgtccagggctcggcg, gtgccgggagctttgcag, atgctgggccctctccag, cggggtggccatgagaag, caggaaaggccggttcat,
gccatgaatggtggccag, gtcacctcggcggccggt, agtgaacccgttgatgtc, cacttgcagtgtgttatc, cctgctgtcacaggagca,
gtgggcgctaaggcgaaa, gccctcaatttcccctcc, acggctcaaccactgccg, cacaactccggtgacatc, aaaagataaccactctgg,
cgagtcgctgggtgccag, cagccggttgctgaggta, tcgccaggaattgttgct, gtatttctggtacagctc, cacgtgctgctccacttt,
taacttgagcctcctcag, cagacgcagctctgcccg, ggagagcaacacgggttc, aggtaccgcttctcggag, ctctgatgtgttgaagaa,
catatatatgctgtgtgt, actctgcttgaacttgtc, atagatttcgttgtgggt, ttccaccattagcacgcg, ggtgacctccttggcgta,
gtagtcggcctcaggctc, gggctccggttctgcact, ctccccggccacccggtc, gcgggtgctgttgtacag, ggcgagcacggcctcggg,
cagcgggccgggcggcac, ctccccctggctcggggg, gctggcgagccgcagctt, ggacaggatctggccgcg, gatggcctcgatgcgctt,
ccgcttcaccagctccat, gtcgatagtcttgcaggt, ggatagtcccgcggccgg, cgggccaggcgtcagcac, cagtagccacagcagcgg,
tagcagcagcggcagcag, ccgcagcccggagggcgg, catgggggaggcggcgcc, ccccggcactgccgagag, cgcgaacagggctggtgt,
ggtggggaggccccgccc, ctgcaggggctgggggtc, tcccggcaaaaggtagga, gggcctcgagggaaagct, gaggctcctcagggagaa,
gggcgcagtggtggaggg, gaggcttggaccgggggt, gtctcagtatcccacgga, aataacctagatgggcgc, gatctggtaccagaaggt,
gggtggtcttgaataggg, gatctgtggcaggtcgga, gagagatccgtctcctgg, aggagaaagggtctagga, tgcgcgggggctcaggag,
acaggccggggatgaagg, cggcgtgcaggggggtgcg, cccgaggtctggggaaaa, gtctttgcgggaggccgg, gtcggcgactcccgaggg,
ctggtccggaatgggggc, gcctgagggacgccgtgt, agggggcagggagggagc, aagcgtccccggcggcaa, agggaggcggtctggggt,
ccccaagtcctgcctcct, cgcggggcagcgtcgcgc, caagaggtccccgcgcct, ccggctcccagcggcaac, ggaaaagtctcaaaagtt,
tttttcctcttctcccga, ccagctcgtccctcctcc, cgctcctcctccccctcc, tccccgcagtggcggggg, cggcggcggctcgtctca,
gactctggggcctcaggc, tgctcctcggcgactcct, tcctccgctccgggccga, ggccggccccgcgggcgg, ctcagagccgggggggggt,
gccccggacggggcgtcc, cccctgcccccggccggg, gccctcgctgtctggctg, cctcccaggatcaagtga, tcctcccaccttagcctc,
cagagtagctgggaccac, aggtgtacatttttttaaa, agtgttttgtagagatag, ggtctcactatgttaccc, aggctggtctcaaatgcc,
tggattcaagtatcctcc, catctctgcctcccaaaa, gtgctaggattacaggcg, tgagcaccccgcctggcc, tgaactactatcttttat,
tgtcttcttcactatccc, ccactaaagcaggttcct, ggtgggcaggaactcctc, ccttaacctctctgggct, tgtttcctcaacctttaa,
aatgggtgttatcagagt, ccctgccatctcagagtg, ttgctatggtgactgaat, gagttcattaatgtaagg, cacttcaacagtgcccaa,
ggtgctcaataaatagat, ctaactacagtagtgttc, cccactggtcccctgtgc, cttgatgccgggcaaagg, aatagtgcagacaggcag,
gaggaggcagagagggag, agagagggagtgggagtg, ggggaacgtcagggatgg, agaccccaggcaggcgcc, caatgacacagagatccg,
cagtcctctctccatctt, taatggggccccaggtgg, gcttggggcacggtgtcc, ttaaatacagcccccatg, ggcaaggcagcgggggcg,
gggcggggtgggccggg, cctgccggggcggggcgg, ggcggggcgggacctcag, ctgcacttgcaggagcgc, acgatcatgttggacagc,
tgctccaccttgggcttg, cggcccacgtagtacacg, atgggcagcggctccagc, gcctgcggcacgcagcac, ggcgccgccgaggcgccc,
gggttatgctggttgtac, agggccaggaccttgctg, tactgcgtgtccaggctc, caaatgtaggggcagggc, ccgaggcagaagttggca,
tggtagcccttgggctcg, tggatccacttccagccg, aggtccttgcggaagtca, atgtacagctgccgcacg, cagcagttcttctccgtg,
gagctgaagcaatagttg, gtgtccagggctcggcgg, tgccgggagctttgcaga, tgctgggccctctccagc, ggggtggccatgagaagc,
aggaaaggccggttcatg, ccatgaatggtggccagg, tcacctcggcggccggta, gtgaacccgttgatgtcc, acttgcagtgtgttatcc,
ctgctgtcacaggagcag, tgggcgctaaggcgaaag, ccctcaatttcccctcca, cggctcaaccactgccgc, acaactccggtgacatca,
aaagataaccactctggc, gagtcgctgggtgccagc, agccggttgctgaggtat, cgccaggaattgttgctg, tatttctggtacagctcc,
acgtgctgctccactttt, aacttgagcctcctcagc, agacgcagctctgcccgg, gagagcaacacgggttca, ggtaccgcttctcggagc,
tctgatgtgttgaagaac, atatatatgctgtgtgta, ctctgcttgaacttgtca, tagatttcgttgtgggtt, tccaccattagcacgcgg,
gtgacctccttggcgtag, tagtcggcctcaggctcg, ggctccggttctgcactc, tccccggccacccggtcg, cgggtgctgttgtacagg,
gcgagcacggcctcgggc, agcgggccgggcggcacc, tccccctggctcgggggg, ctggcgagccgcagcttg, gacaggatctggccgcgg,
atggcctcgatgcgcttc, cgcttcaccagctccatg, tcgatagtcttgcaggtg, gatagtcccgcggccggc, gggccaggcgtcagcacc,
agtagccacagcagcggt, agcagcagcggcagcagc, cgcagcccggagggcggc, atgggggaggcggcgccc, cccggcactgccgagagc,
gcgaacagggctggtgtg, gtggggaggccccgcccc, tgcaggggctgggggtct, cccggcaaaaggtaggag, ggcctcgagggaaagctg,
aggctcctcagggagaag, ggcgcagtggtggagggg, aggcttggaccgggggtg, tctcagtatcccacggaa, ataacctagatgggcgcg,
atctggtaccagaaggtg, ggtggtcttgaatagggg, atctgtggcaggtcggag, agagatccgtctcctgga, ggagaaagggtctaggat,
gcgcgggggctcaggaga, caggccggggatgaaggc, ggcgtgcaggggggtgcgc, ccgaggtctggggaaaag, tctttgcgggaggccggg,
tcggcgactcccgagggc, tggtccggaatgggggcg, cctgagggacgccgtgta, gggggcagggagggagca, agcgtccccggcggcaaa,
gggaggcggtctggggtc, cccaagtcctgcctcctc, gcggggcagcgtcgcgcc, aagaggtccccgcgcctc, cggctcccagcggcaacg,

gaaaagtctcaaaagttt, ttttcctcttctcccgac, cagctcgtccctcctccc, gctcctcctccccctcct, ccccgcagtggcgggggc,
ggcggcggctcgtctcag, actctggggcctcaggct, gctcctcggcgactcctt, cctccgctccgggccgag, gccggccccgcgggcggc,
tcagagccgggggggtg, ccccggacggggcgtccc, ccctgcccccggccgggg, ccctcgctgtctggctgc, ctcccaggatcaagtgat,
cctcccaccttagcctcc, agagtagctgggaccaca, ggtgtacatttttaaaa, gtgttttgtagagatagg, gtctcactatgttaccca,
ggctggtctcaaatgcct, ggattcaagtatcctccc, atctctgcctcccaaaag, tgctaggattacaggcgt, gagcaccccgcctggcct,
gaactactatcttttatt, gtcttcttcactatcccc, cactaaagcaggttcctg, gtgggcaggaactcctcc, cttaacctctctgggctt,
gtttcctcaacctttaaa, atgggtgttatcagagtc, cctgccatctcagagtgt, tgctatggtgactgaatg, agttcattaatgtaaggc,
acttcaacagtgcccaag, gtgctcaataaatagatc, taactacagtagtgttcc, ccactggtcccctgtgcc, ttgatgccgggcaaagga,
atagtgcagacaggcagg, aggaggcagagagggaga, gagagggagtgggagtgg, gggaacgtcagggatgga, gaccccaggcaggcgccc,
aatgacacagagatccgc, agtcctctctccatcttt, aatggggccccaggtggg, cttggggcacggtgtcct, taaatacagcccccatgg,
gcaaggcagcgggggcgg, ggcggggtggggccgggc, ctgccggggcggggcggg, gcggggcgggacctcagc, tgcacttgcaggagcgca,
cgatcatgttggacagct, gctccaccttgggcttgc, ggcccacgtagtacacga, tgggcagcggctccagcg, cctgcggcacgcagcacg,
gcgccgccgaggcgcccg, ggttatgctggttgtaca, gggccaggaccttgctgt, actgcgtgtccaggctcc, aaatgtaggggcagggcc,
cgaggcagaagttggcat, ggtagcccttgggctcgt, ggatccacttccagccga, ggtccttgcggaagtcaa, tgtacagctgccgcacgc,
agcagttcttctccgtgg, agctgaagcaatagttgg, tgtccagggctcggcggt, gccgggagctttgcagat, gctgggccctctccagcg,
gggtggccatgagaagca, ggaaaggccggttcatgc, catgaatggtggccaggt, cacctcggcggccggtag, tgaacccgttgatgtcca,
cttgcagtgtgttatccc, tgctgtcacaggagcagt, gggcgctaaggcgaaagc, cctcaatttcccctccac, ggctcaaccactgccgca,
caactccggtgacatcaa, aagataaccactctggcg, agtcgctgggtgccagca, gccggttgctgaggtatc, gccaggaattgttgctgt,
atttctggtacagctcca, cgtgctgctccactttta, acttgagcctcctcagca, gacgcagctctgcccggg, agagcaacacgggttcag,
gtaccgcttctcggagct, ctgatgtgttgaagaaca, tatatatgctgtgtgtac, tctgcttgaacttgtcat, agatttcgttgtgggttt,
ccaccattagcacgcggg, tgacctccttggcgtagt, agtcggcctcaggctcgg, gctccggttctgcactct, ccccggccacccggtcgc,
gggtgctgttgtacaggg, cgagcacggcctcgggca, gcgggccgggcggcacct, ccccctggctcgggggc, tggcgagccgcagcttgg,
acaggatctggccgcgga, tggcctcgatgcgcttcc, gcttcaccagctccatgt, cgatagtcttgcaggtgg, atagtcccgcggccggcg,
ggccaggcgtcagcacca, gtagccacagcagcggta, gcagcagcggcagcagcc, gcagcccggagggcggca, tgggggaggcggcgcccc,
ccggcactgccgagagcg, cgaacagggctggtgtgg, tggggaggccccgcccct, gcaggggctgggggtctc, ccggcaaaaggtaggagg,
gcctcgagggaaagctga, ggctcctcagggagaagg, gcgcagtggtggaggga, ggcttggaccgggggtgt, ctcagtatcccacggaaa,
taacctagatgggcgcga, tctggtaccagaaggtgg, gtggtcttgaatagggga, tctgtggcaggtcggaga, gagatccgtctcctggag,
gagaaagggtctaggatg, cgcgggggctcaggagac, aggccggggatgaaggcg, gcgtgcaggggggtgcgcc, cgaggtctggggaaaagt,
ctttgcgggaggccgggt, cggcgactcccgagggct, ggtccggaatgggggcgc, ctgagggacgccgtgtag, ggggcagggagggagcaa,
gcgtccccggcggcaaag, ggaggcggtctggggtcc, ccaagtcctgcctcctcg, cggggcagcgtcgcgcca, agaggtccccgcgcctcc,
ggctcccagcggcaacgg, aaaagtctcaaaagtttt, tttcctcttctcccgacc, agctcgtccctcctcccg, ctcctcctccccctcctc,
cccgcagtggcgggggcg, gcggcggctcgtctcaga, ctctggggcctcaggctg, ctcctcggcgactccttc, ctccgctccgggccgagg,
ccggccccgcgggcggct, cagagccgggggggtgc, cccggacggggcgtcccc, cctgcccccggccggggc, cctcgctgtctggctgct,
tcccaggatcaagtgatc, ctcccaccttagcctcca, gagtagctgggaccacag, gtgtacatttttaaaag, tgttttgtagagataggg,
tctcactatgttacccag, gctggtctcaaatgcctg, gattcaagtatcctccca, tctctgcctcccaaaagt, gctaggattacaggcgtg,
agcaccccgcctggcctg, aactactatcttttattg, tcttcttcactatccccc, actaaagcaggttcctgg, tgggcaggaactcctccc,
ttaacctctctgggcttg, tttcctcaacctttaaaa, tgggtgttatcagagtcc, ctgccatctcagagtgtt, gctatggtgactgaatga,
gttcattaatgtaaggca, cttcaacagtgcccaagg, tgctcaataaatagatct, aactacagtagtgttccc, cactggtcccctgtgcct,
tgatgccgggcaaaggaa, tagtgcagacaggcagga, ggaggcagagagggagag, agagggagtgggagtggg, ggaacgtcagggatggag,
accccaggcaggcgccca, atgacacagagatccgca, gtcctctctccatcttta, atggggccccaggtgggc, ttggggcacggtgtcctt,
aaatacagcccccatggg, caaggcagcgggggcggg, gcggggtggggccgggcc, tgccggggcggggcgggg, cggggcgggacctcagct,
gcacttgcaggagcgcac, gatcatgttggacagctg, ctccaccttgggcttgcg, gcccacgtagtacacgat, gggcagcggctccagcgc,
ctgcggcacgcagcacgg, cgccgccgaggcgcccgg, gttatgctggttgtacag, ggccaggaccttgctgta, ctgcgtgtccaggctcca,
aatgtaggggcagggccc, gaggcagaagttggcatg, gtagcccttgggctcgtg, gatccacttccagccgag, gtccttgcggaagtcaat,
gtacagctgccgcacgca, gcagttcttctccgtgga, gctgaagcaatagttggt, gtccagggctcggcggtg, ccgggagctttgcagatg,
ctgggccctctccagcgg, ggtggccatgagaagcag, gaaaggccggttcatgcc, atgaatggtggccaggtc, acctcggcggccggtagt,
gaacccgttgatgtccac, ttgcagtgtgttatccct, gctgtcacaggagcagtg, ggcgctaaggcgaaagcc, ctcaatttcccctccacg,
gctcaaccactgccgcac, aactccggtgacatcaaa, agataaccactctggcga, gtcgctgggtgccagcag, ccggttgctgaggtatcg,
ccaggaattgttgctgta, tttctggtacagctccac, gtgctgctccacttttaa, cttgagcctcctcagcag, acgcagctctgcccggga,
gagcaacacgggttcagg, taccgcttctcggagctc, tgatgtgttgaagaacat, atatatgctgtgtgtact, ctgcttgaacttgtcata,
gatttcgttgtgggtttc, caccattagcacgcgggt, gacctccttggcgtagta, gtcggcctcaggctcggg, ctccggttctgcactctc,
cccggccacccggtcgcg, ggtgctgttgtacagggc, gagcacggcctcgggcag, cgggccgggcggcacctc, cccctggctcgggggct,
ggcgagccgcagcttgga, caggatctggccgcggat, ggcctcgatgcgcttccg, cttcaccagctccatgtc, gatagtcttgcaggtgga,
tagtcccgcggccggcgg, gccaggcgtcagcaccag, tagccacagcagcggtag, cagcagcggcagcagccg, cagcccggagggcggcat,
gggggaggcggcgccccc, cggcactgccgagagcgc, gaacagggctggtgtggt, ggggaggccccgcccctg, caggggctgggggtctcc,
cggcaaaaggtaggaggg, cctcgagggaaagctgag, gctcctcagggagaaggg, cgcagtggtggaggggag, gcttggaccgggggtgtc,
tcagtatcccacggaaat, aacctagatgggcgcgat, ctggtaccagaaggtggg, tggtcttgaataggggat, ctgtggcaggtcggagag,
agatccgtctcctggagg, agaaagggtctaggatgc, gcgggggctcaggagaca, ggccggggatgaaggcgg, cgtgcaggggggtgcgccc,
gaggtctggggaaaagtc, tttgcgggaggccgggtc, ggcgactcccgagggctg, gtccggaatgggggcgcc, tgagggacgccgtgtagg,
gggcagggagggagcaag, cgtccccggcggcaaagg, gaggcggtctggggtccc, caagtcctgcctcctcgc, ggggcagcgtcgcgccaa,
gaggtccccgcgcctccg, gctcccagcggcaacgga, aaagtctcaaaagttttt, ttcctcttctcccgacca, gctcgtccctcctcccgc,
tcctcctccccctcctcc, ccgcagtggcgggggcgg, cggcggctcgtctcagac, tctggggcctcaggctgc, tcctcggcgactccttcc,
tccgctccgggccgaggc, cggccccgcgggcggctc, agagccgggggggtgcc, ccggacggggcgtccccc, ctgcccccggccggggcc,
ctcgctgtctggctgctc, cccaggatcaagtgatcc, tcccaccttagcctccag, agtagctgggaccacagg, tgtacatttttaaaagt,
gttttgtagagatagggt, ctcactatgttacccagg, ctggtctcaaatgcctgg, attcaagtatcctcccat, ctctgcctcccaaaagtg,
ctaggattacaggcgtga, gcaccccgcctggcctga, actactatcttttattgt, cttcttcactatccccca, ctaaagcaggttcctggt,
gggcaggaactcctccct, taacctctctgggcttgt, ttcctcaacctttaaaat, gggtgttatcagagtccc, tgccatctcagagtgttg,
ctatggtgactgaatgag, ttcattaatgtaaggcac, ttcaacagtgcccaaggt, gctcaataaatagatcta, actacagtagtgttcccc,

actggtcccctgtgccttt, gatgccgggcaaaggaat, agtgcagacaggcaggag, gaggcagagagggagaga, gagggagtgggagtgggg,
gaacgtcagggatggaga, ccccaggcaggcgcccaa, tgacacagagatccgcag, tcctctctccatctttaa, tggggccccaggtgggct,
tggggcacggtgtcctta, aatacagcccccatgggc, aaggcagcgggggcgggg, cggggtggggccgggcct, gccggggcggggcggggc,
ggggcgggacctcagctg, cacttgcaggagcgcacg, atcatgttggacagctgc, tccaccttgggcttgcgg, cccacgtagtacacgatg,
ggcagcggctccagcgcc, tgcggcacgcagcacggc, gccgccgaggcgcccggg, ttatgctggttgtacagg, gccaggaccttgctgtac,
tgcgtgtccaggctccaa, atgtaggggcagggcccg, aggcagaagttggcatgg, tagcccttgggctcgtgg, atccacttccagccgagg,
tccttgcggaagtcaatg, tacagctgccgcacgcag, cagttcttctccgtggag, ctgaagcaatagttggtg, tccagggctcggcggtgc,
cgggagctttgcagatgc, tgggccctctccagcggg, gtggccatgagaagcagg, aaaggccggttcatgcca, tgaatggtggccaggtca,
cctcggcggccggtagtg, aacccgttgatgtccact, tgcagtgtgttatccctg, ctgtcacaggagcagtgg, gcgctaaggcgaaagccc,
tcaatttcccctccacgg, ctcaaccactgccgcaca, actccggtgacatcaaaa, gataaccactctggcgag, tcgctgggtgccagcagc,
cggttgctgaggtatcgc, caggaattgttgctgtat, ttctggtacagctccacg, tgctgctccacttttaac, ttgagcctcctcagcaga,
cgcagctctgcccgggag, agcaacacgggttcaggt, accgcttctcggagctct, gatgtgttgaagaacata, tatatgctgtgtgtactc,
tgcttgaacttgtcatag, atttcgttgtgggtttcc, accattagcacgcgggtg, acctccttggcgtagtag, tcggcctcaggctcgggc,
tccggttctgcactctcc, ccggccacccggtcgcgg, gtgctgttgtacagggcg, agcacggcctcgggcagc, gggccgggcggcacctcc,
ccctggctcgggggctg, gcgagccgcagcttggac, aggatctggccgcggatg, gcctcgatgcgcttccgc, ttcaccagctccatgtcg,
atagtcttgcaggtggat, agtcccgcggccggcggg, ccaggcgtcagcaccagt, agccacagcagcggtagc, agcagcggcagcagccgc,
agcccggagggcggcatg, ggggaggcggcgcccccc, ggcactgccgagagcgcg, aacagggctggtgtggtg, gggaggccccgcccctgc,
aggggctgggggtctccc, ggcaaaaggtaggagggc, ctcgagggaaagctgagg, ctcctcagggagaagggc, gcagtggtggaggggagg,
cttggaccgggggtgtct, cagtatcccacggaaata, acctagatgggcgcgatc, tggtaccagaaggtgggt, ggtcttgaataggggatc,
tgtggcaggtcggagaga, gatccgtctcctggagga, gaaagggtctaggatgcg, cgggggctcaggagacag, gccggggatgaaggcggc,
gtgcagggggtgcgcccg, aggtctggggaaaagtct, ttgcgggaggccgggtcg, gcgactcccgagggctgg, tccggaatgggggcgcct,
gagggacgccgtgtaggg, ggcagggagggagcaagc, gtccccggcggcaaaggg, aggcggtctggggtcccc, aagtcctgcctcctcgcg,
gggcagcgtcgcgccaag, aggtccccgcgcctccgg, ctcccagcggcaacggaa, aagtctcaaaagtttttt, tcctcttctcccgaccag,
ctcgtccctcctcccgct, cctcctccccctcctccc, cgcagtggcgggggcggc, ggcggctcgtctcagact, ctggggcctcaggctgct,
cctcggcgactccttcct, ccgctccgggccgaggcc, ggccccgcgggcggctca, gagccgggggggggtgccc, cggacggggcgtcccccc,
tgcccccggccggggccc, tcgctgtctggctgctcc, ccaggatcaagtgatcct, cccaccttagcctccaga, gtagctgggaccacaggt,
gtacatttttttaaaagtg, ttttgtagagatagggtc, tcactatgttacccaggc, tggtctcaaatgcctgga, ttcaagtatcctcccatc,
tctgcctcccaaaagtgc, taggattacaggcgtgag, caccccgcctggcctgaa, ctactatcttttattgtc, ttcttcactatcccccac,
taaagcaggttcctggtg, ggcaggaactcctccctt, aacctctctgggcttgtt, tcctcaacctttaaaatg, ggtgttatcagagtccct,
gccatctcagagtgttgc, tatggtgactgaatgagt, tcattaatgtaaggcact, tcaacagtgcccaaggtg, ctcaataaatagatctaa,
ctacagtagtgttcccca, ctggtcccctgtgccttg, atgccgggcaaaggaata, gtgcagacaggcaggagg, aggcagagagggagagag,
agggagtgggagtggggg, aacgtcagggatggagac, cccaggcaggcgcccaat, gacacagagatccgcagt, cctctctccatctttaat,
ggggccccaggtgggctt, ggggcacggtgtccttaa, atacagcccccatgggca, aggcagcgggggcggggc, ggggtggggccgggcctg,
ccggggcggggcggggcg, gggcgggacctcagctgc, acttgcaggagcgcacga, tcatgttggacagctgct, ccaccttgggcttgcggc,
ccacgtagtacacgatgg, gcagcggctccagcgcct, gcggcacgcagcacggcg, ccgccgaggcgcccgggt, tatgctggttgtacaggg,
ccaggaccttgctgtact, gcgtgtccaggctccaaa, tgtaggggcagggcccga, ggcagaagttggcatggt, agcccttgggctcgtgga,
tccacttccagccgaggt, ccttgcggaagtcaatgt, acagctgccgcacgcagc, agttcttctccgtggagc, tgaagcaatagttggtgt,
ccagggctcggcggtgcc, gggagctttgcagatgct, gggccctctccagcgggg, tggccatgagaagcagga, aaggccggttcatgccat,
gaatggtggccaggtcac, ctcggcggccggtagtga, acccgttgatgtccactt, gcagtgtgttatccctgc, tgtcacaggagcagtggg,
cgctaaggcgaaagccct, caatttcccctccacggc, tcaaccactgccgcacaa, ctccggtgacatcaaaag, ataaccactctggcgagt,
cgctgggtgccagcagcc, ggttgctgaggtatcgcc, aggaattgttgctgtatt, tctggtacagctccacgt, gctgctccacttttaact,
tgagcctcctcagcagac, gcagctctgcccgggaga, gcaacacgggttcaggta, ccgcttctcggagctctg, atgtgttgaagaacatat,
atatgctgtgtgtactct, gcttgaacttgtcataga, tttcgttgtgggtttcca, ccattagcacgcgggtga, cctccttggcgtagtagt,
cggcctcaggctcgggct, ccggttctgcactctccc, cggccacccggtcgcggg, tgctgttgtacagggcga, gcacggcctcgggcagcg,
ggccgggcggcacctccc, cctggctcgggggctgg, cgagccgcagcttggaca, ggatctggccgcggatgg, cctcgatgcgcttccgct,
tcaccagctccatgtcga, tagtcttgcaggtggata, gtcccgcggccggcgggc, caggcgtcagcaccagta, gccacagcagcggtagca,
gcagcggcagcagccgca, gcccggagggcggcatgg, gggaggcggcgcccccccg, gcactgccgagagcgcga, acagggctggtgtggtgg,
ggaggccccgcccctgca, ggggctgggggtctcccg, gcaaaaggtaggagggcc, tcgagggaaagctgaggc, tcctcagggagaagggcg,
cagtggtggaggggaggc, ttggaccgggggtgtctc, agtatcccacggaaataa, cctagatgggcgcgatct, ggtaccagaaggtgggtg,
gtcttgaataggggatct, gtggcaggtcggagagag, atccgtctcctggaggag, aaagggtctaggatgcgc, gggggctcaggagacagg,
ccggggatgaaggcggcg, tgcagggggtgcgcccga, ggtctggggaaaagtctt, tgcgggaggccgggtcgg, cgactcccgagggctggt,
ccggaatgggggcgcctg, agggacgccgtgtagggg, gcagggagggagcaagcg, tccccggcggcaaaggga, ggcggtctggggtcccca,
agtcctgcctcctcgcgg, ggcagcgtcgcgccaaga, ggtccccgcgcctccggc, tcccagcggcaacggaaa, agtctcaaaagttttttt,
cctcttctcccgaccagc, tcgtccctcctcccgctc, ctcctccccctcctcccc, gcagtggcgggggcggcg, gcggctcgtctcagactc,
tggggcctcaggctgctc, ctcggcgactccttcctc, cgctccgggccgaggccg, gccccgcgggcggctcag, agccgggggggggtgcccc,
ggacggggcgtcccccct, gcccccggccggggccct, cgctgtctggctgctccg,

### Example 20

TGF-beta2 antisense oligonucleotides: 
tttaaaaaaatttgcttc, ttgtctctctcacttaca, aagtaggtgaaatgtaga, ataaggccttcaactttt, tttgtgtcagatgccagt, tttaacaaacagaacaca,
aacttccaaagtgtctga, actagtaccgccttttca, aaaatttttaacactga, tgaaccaaggctctctta, tgtttcttgttacaagc, atcatcgttgtcgtcgtc,
atcatcattatcatcatc, attgtcattttggtcttg, ccacttttccaagaattt, tagctgcatttgcaagac, tttacaatcatattagaa, agctgttcaatcttgggt,
gttttgccaatgtagtag, agaatggttagaggttct, aaatcttgggacacgcag, caaggagaagcagatgct, tctggatttatggtatta, tataagctcaggaccctg,
ctgtgctgagtgtctgaa, ctccataaatacgggcat, gctccagcacagaagttg, gcattgtaccctttgggt, tcgtgtatccatttccac, cctagatccctcttgaaa,
tcaatgtaaagtggacgt, aggcagcaattatcctgc, acatttctaaagcaatag, gccgcatccaaagcacgc, ttcttccgccggttggtc, tgttgtgactcaagtctg,
taggagggcaataacatt, agcaggagatgtggggtc, ttcccactgttttttttc, ctagtggactttatagtt, ttctgatcaccactggta, tatgtggaggtgccatca,
atacctgcaaatcttgct, tctagttcttcactttta, tttgggatgatgtaatta, ttagatggtacaaaagtg, cagcagggacagtgtaag, cttatttaaatcccagg,
ttcctgtctttatggtga, agccattcatgaacagca, tcagttacatcgaaggag, agccattcgccttctgct, cttgttttcacaactttg, ctgtcgatgtagcgctgg,
gttggagatgttaaatct, ttggacttgagaatctga, tatagctcaatccgttgt, tcaggcactctggctttt, gggttctgcaaacgaaag, actctgaactctgctttc,
accaaattggaagcattc, ttctccattgctgagacg, tcaaatcgaacaattctg, aagtagggtctgtagaaa, gtgggcgggatggcattt, tcggaggggaagaagggc,
ggcatgtctatttgtaa, acctccttggcgtagtac, tcttcgtcgctcctctcg, cgctcgcaggcggccgcc, ctccggctcgccttctcc, tggagcaagtccctggtg,
ctgttgtagatggaaatc, acctccgggggggacttcc, tcggctcaggatagtct, tctgggggactggtgagc, ttcagcttgctcaggatc, tgcccgcggatcgcctcg,
atcctcttgcgcatgaac, tggtccatatcgagtgtg, ctgcaggtagacaggctg, agcgcgaccgtgaccaga, tgcaggatcagaaaagcg, ctcagcacacagtagtgc,
atttttaaaaaagtgga, aaaaaaagttgtttttaa, aagtcagaataaaaaaaa, agaaatcaacaattctca, aagtatagatcaaggaga, gttgtttggtttttttgtt,
gttgttgtttgtttttga, tgcgaaactttgcaaac, aatctagtcaatgcccaa, cagaaaaacgtatcctgc, ttaaaaaaatttgcttct, tgtctctctcacttacaa,
agtaggtgaaatgtagaa, taaggccttcaactttt, ttgtgtcagatgccagtt, ttaacaaacagaacacaa, acttccaaagtgtctgaa, ctagtaccgccttttcaa,
aaatttttaacactgat, gaaccaaggctctcttat, gtttcttgttacaagca, tcatcgttgtcgtcgtca, tcatcattatcatcatca, ttgtcattttggtcttgc,
cacttttccaagaatttt, agctgcatttgcaagact, ttacaatcatattagaaa, gctgttcaatcttgggtg, ttttgccaatgtagtaga, gaatggttagaggttcta,
aatcttgggacacgcagc, aaggagaagcagatgctt, ctggatttatggtattat, ataagctcaggaccctgc, tgtgctgagtgtctgaac, tccataaatacgggcatg,
ctccagcacagaagttgg, cattgtaccctttgggtt, cgtgtatccatttccacc, ctagatccctcttgaaat, caatgtaaagtggacgta, ggcagcaattatcctgca,
catttctaaagcaatagg, ccgcatccaaagcacgct, tcttccgccggttggtct, gttgtgactcaagtctgt, aggagggcaataacatta, gcaggagatgtggggtct,
tcccactgttttttttcc, tagtggactttatagttt, tctgatcaccactggtat, atgtggaggtgccatcaa, tacctgcaaatcttgctt, ctagttcttcacttttat,
ttgggatgatgtaattat, tagatggtacaaaagtgc, agcagggacagtgtaagc, ttatttaaatcccaggt, tcctgtctttatggtgaa, gccattcatgaacagcat,
cagttacatcgaaggaga, gccattcgccttctgctc, ttgttttcacaactttgc, tgtcgatgtagcgctggg, ttggagatgttaaatctt, tggacttgagaatctgat,
atagctcaatccgttgtt, caggcactctggcttttg, ggttctgcaaacgaaaga, ctctgaactctgctttca, ccaaattggaagcattct, tctccattgctgagacgt,
caaatcgaacaattctga, agtagggtctgtagaaag, tgggcgggatggcatttt, cggaggggaagaagggcg, gcatgtctatttgtaaa, cctccttggcgtagtact,
cttcgtcgctcctctcgc, gctcgcaggcggccgccc, tccggctcgccttctcct, ggagcaagtccctggtgc, tgttgtagatggaaatca, cctccgggggggacttcct,
cggctcaggatagtctt, ctgggggactggtgagct, tcagcttgctcaggatct, gcccgcggatcgcctcga, tcctcttgcgcatgaact, ggtccatatcgagtgtgc,
tgcaggtagacaggctga, gcgcgaccgtgaccagat, gcaggatcagaaaagcgc, tcagcacacagtagtgca, tttttaaaaaagtggaa, aaaaaagttgtttttaaa,
agtcagaataaaaaaaaa, gaaatcaacaattctcaa, agtatagatcaaggagag, ttgtttggtttttttgttg, ttgttgtttgtttttgat, gcgaaactttgcaaaca,
atctagtcaatgcccaac, agaaaaacgtatcctgct, taaaaaaatttgcttctt, gtctctctcacttacaaa, gtaggtgaaatgtagaat, aaggccttcaactttttt,
tgtgtcagatgccagttt, taacaaacagaacacaaa, cttccaaagtgtctgaac, tagtaccgccttttcaaa, aatttttaacactgatg, aaccaaggctctcttatg,
tttcttgttacaagcat, catcgttgtcgtcgtcat, catcattatcatcatcat, tgtcattttggtcttgcc, acttttccaagaatttta, gctgcatttgcaagactt,
tacaatcatattagaaag, ctgttcaatcttgggtgt, tttgccaatgtagtagag, aatggttagaggttctaa, atcttgggacacgcagca, aggagaagcagatgcttc,
tggatttatggtattata, taagctcaggaccctgct, gtgctgagtgtctgaact, ccataaatacgggcatgc, tccagcacagaagttggc, attgtaccctttgggttc,
gtgtatccatttccaccc, tagatccctcttgaaatc, aatgtaaagtggacgtag, gcagcaattatcctgcac, atttctaaagcaataggc, cgcatccaaagcacgctt,
cttccgccggttggtctg, ttgtgactcaagtctgta, ggagggcaataacattag, caggagatgtggggtctt, cccactgttttttttcct, agtggactttatagtttt,
ctgatcaccactggtata, tgtggaggtgccatcaat, acctgcaaatcttgcttc, tagttcttcacttttatt, tgggatgatgtaattatt, agatggtacaaaagtgca,
gcagggacagtgtaagct, tatttaaatcccaggtt, cctgtctttatggtgaag, ccattcatgaacagcatc, agttacatcgaaggagag, ccattcgccttctgctct,
tgttttcacaactttgct, gtcgatgtagcgctgggt, tggagatgttaaatcttt, ggacttgagaatctgata, tagctcaatccgttgttc, aggcactctggcttttgg,
gttctgcaaacgaaagac, tctgaactctgctttcac, caaattggaagcattctt, ctccattgctgagacgtc, aaatcgaacaattctgaa, gtagggtctgtagaaagt,
gggcgggatggcattttc, ggaggggaagaagggcgg, catgtctatttgtaaac, ctccttggcgtagtactc, ttcgtcgctcctctcgcg, ctcgcaggcggccgccct,
ccggctcgccttctcctg, gagcaagtccctggtgct, gttgtagatggaaatcac, ctccgggggggacttcctc, ggctcaggatagtcttc, tgggggactggtgagctt,
cagcttgctcaggatctg, cccgcggatcgcctcgat, cctcttgcgcatgaactg, gtccatatcgagtgtgct, gcaggtagacaggctgag, cgcgaccgtgaccagatg,
caggatcagaaaagcgct, cagcacacagtagtgcat, ttttaaaaaagtggaaa, aaaaagttgtttttaaaa, gtcagaataaaaaaaaag, aaatcaacaattctcaaa,
gtatagatcaaggagagt, tgtttggtttttttgttgt, tgttgtttgtttttgatg, cgaaactttgcaaacaa, tctagtcaatgcccaaca, gaaaaacgtatcctgctt,
aaaaaaatttgcttcttg, tctctctcacttacaaag, taggtgaaatgtagaata, aggccttcaacttttttt, gtgtcagatgccagtttt, aacaaacagaacacaaac,
ttccaaagtgtctgaact, agtaccgccttttcaaaa, atttttaacactgatga, accaaggctctcttatgt, ttcttgttacaagcatc, atcgttgtcgtcgtcatc,
atcattatcatcatcatt, gtcattttggtcttgcca, cttttccaagaattttag, ctgcatttgcaagacttt, acaatcatattagaaagc, tgttcaatcttgggtgtt,
ttgccaatgtagtagaga, atggttagaggttctaaa, tcttgggacacgcagcaa, ggagaagcagatgcttct, ggatttatggtattatat, aagctcaggaccctgctg,
tgctgagtgtctgaactc, cataaatacgggcatgct, ccagcacagaagttggca, ttgtaccctttgggttcg, tgtatccatttccaccct, agatccctcttgaaatca,
atgtaaagtggacgtagg, cagcaattatcctgcaca, tttctaaagcaataggcc, gcatccaaagcacgcttc, ttccgccggttggtctgt, tgtgactcaagtctgtag,
gagggcaataacattagc, aggagatgtggggtcttc, ccactgttttttttccta, gtggactttatagttttc, tgatcaccactggtatat, gtggaggtgccatcaata,
cctgcaaatcttgcttct, agttcttcacttttattt, gggatgatgtaattatta, gatggtacaaaagtgcag, cagggacagtgtaagctt, atttaaatcccaggttc,
ctgtctttatggtgaagc, cattcatgaacagcatca, gttacatcgaaggagagc, cattcgccttctgctctt, gttttcacaactttgctg, tcgatgtagcgctgggtt,
ggagatgttaaatctttg, gacttgagaatctgatat, agctcaatccgttgttca, ggcactctggcttttggg, ttctgcaaacgaaagact, ctgaactctgctttcacc,
aaattggaagcattcttc, tccattgctgagacgtca, aatcgaacaattctgaag, tagggtctgtagaaagtg, ggcgggatggcattttcg, gaggggaagaagggcggc,
atgtctatttgtaaacc, tccttggcgtagtactct, tcgtcgctcctctcgcgc, tcgcaggcggccgccctc, cggctcgccttctcctgg, agcaagtccctggtgctg,
ttgtagatggaaatcacc, tccgggggggacttcctcg, ggctcaggatagtcttct, gggggactggtgagcttc, agcttgctcaggatctgc, ccgcggatcgcctcgatc,
ctcttgcgcatgaactgg, tccatatcgagtgtgctg, caggtagacaggctgagc, gcgaccgtgaccagatgc, aggatcagaaaagcgctc, agcacacagtagtgcatt,
tttaaaaaagtggaaaa, aaaagttgtttttaaaag, tcagaataaaaaaaaaga, aatcaacaattctcaaag, tatagatcaaggagagtt, gtttggtttttttgttgtt,
gttgtttgtttttgatgc, gaaactttgcaaacaat, ctagtcaatgcccaacag, aaaaacgtatcctgcttg, aaaaaatttgcttcttgt, ctctctcacttacaaagt,
aggtgaaatgtagaataa, ggccttcaactttttttg, tgtcagatgccagtttta, acaaacagaacacaaact, tccaaagtgtctgaacta, gtaccgccttttcaaaaa,
tttttaacactgatgaa, ccaaggctctcttatgtt, ttcttgttacaagcatca, tcgttgtcgtcgtcatca, tcattatcatcatcattg, tcattttggtcttgccac,
ttttccaagaattttagc, tgcatttgcaagacttta, caatcatattagaaagct, gttcaatcttgggtgttt, tgccaatgtagtagagaa, tggttagaggttctaaat,
cttgggacacgcagcaag, gagaagcagatgcttctg, gatttatggtattatata, agctcaggaccctgctgt, gctgagtgtctgaactcc, ataaatacgggcatgctc,
cagcacagaagttggcat, tgtaccctttgggttcgt, gtatccatttccacccta, gatccctcttgaaatcaa, tgtaaagtggacgtaggc, agcaattatcctgcacat,
ttctaaagcaataggccg, catccaaagcacgcttct, tccgccggttggtctgtt, gtgactcaagtctgtagg, agggcaataacattagca, ggagatgtggggtcttcc,
 
cactgttttttttcctag, tggacttatagttttct, gatcaccactggtatatg, tggaggtgccatcaatac, ctgcaaatcttgcttcta, gttcttcacttttatttg,
ggatgatgtaattattag, atggtacaaaagtgcagc, agggacagtgtaagctta, ttttaaatcccaggttcc, tgtctttatggtgaagcc, attcatgaacagcatcag,
ttacatcgaaggagagcc, attcgccttctgctcttg, ttttcacaactttgctgt, cgatgtagcgctgggttg, gagatgttaaatctttgg, acttgagaatctgatata,
gctcaatccgttgttcag, gcactctggcttttgggt, tctgcaaacgaaagactc, tgaactctgctttcacca, aattggaagcattcttct, ccattgctgagacgtcaa,
atcgaacaattctgaagt, agggtctgtagaaagtgg, gcgggatggcatttcgg, aggggaagaagggcggca, tgtctatttgtaaacct, ccttggcgtagtactctt,
cgtcgctcctctcgcgct, cgcaggcggccgccctcc, ggctcgccttctcctgga, gcaagtccctggtgctgt, tgtagatggaaatcacct, ccggggggacttcctcgg,
gctcaggatagtcttctg, ggggactggtgagcttca, gcttgctcaggatctgcc, cgcggatcgcctcgatcc, tcttgcgcatgaactggt, ccatatcgagtgtgctgc,
aggtagacaggctgagcg, cgaccgtgaccagatgca, ggatcagaaaagcgctca, gcacacagtagtgcattt, ttaaaaaagtggaaaaa, aaagttgtttttaaaagt,
cagaataaaaaaaaagaa, atcaacaattctcaaagt, atagatcaaggagagttg, tttggtttttgttgttg, ttgtttgttttgatgcg, aaacttttgcaaacaatc,
tagtcaatgcccaacaga, aaaaatttgcttcttgtc, tctctcacttacaaagta, ggtgaaatgtagaataag, gccttcaactttttttgt, gtcagatgccagttttaa,
caaacagaacacaaactt, ccaaagtgtctgaactag, taccgccttttcaaaaat, ttttaacactgatgaac, caaggctctcttatgttt, cttgttacaagcatcat,
cgttgtcgtcgtcatcat, cattatcatcatcattgt, catttttggtcttgccact, tttccaagaattttagct, gcatttgcaagactttac, aatcatattagaaagctg,
ttcaatcttgggtgtttt, gccaatgtagtagagaat, ggttagaggttctaaatc, ttgggacacgcagcaagg, agaagcagatgcttctgg, atttatggtattatataa,
gctcaggaccctgctgtg, ctgagtgtctgaactcca, taaatacgggcatgctcc, agcacagaagttggcatt, gtaccctttgggttcgtg, tatccatttccaccctag,
atccctcttgaaatcaat, gtaaagtggacgtaggca, gcaattatcctgcacatt, tctaaagcaataggccgc, atccaaagcacgcttctt, ccgccggttggtctgttg,
tgactcaagtctgtagga, gggcaataacattagcag, gagatgtggggtcttccc, actgtttttttcctagt, ggacttatagttttctg, atcaccactggtatatgt,
ggaggtgccatcaatacc, tgcaaatcttgcttctag, ttcttcacttttatttgg, gatgatgtaattattaga, tggtacaaaagtgcagca, gggacagtgtaagcttat,
tttaaatcccaggttcct, gtctttatggtgaagcca, ttcatgaacagcatcagt, tacatcgaaggagagcca, ttcgccttctgctcttgt, tttcacaactttgctgtc,
gatgtagcgctgggttgg, agatgttaaatctttgga, cttgagaatctgatatag, ctcaatccgttgttcagg, cactctggcttttgggtt, ctgcaaacgaaagactct,
gaactctgctttcaccaa, attggaagcattcttctc, cattgctgagacgtcaaa, tcgaacaattctgaagta, gggtctgtagaaagtggg, cgggatggcatttcgga,
ggggaagaagggcggcat, gtctatttgtaaacctc, cttggcgtagtactcttc, gtcgctcctctcgcgctc, gcaggcggccgccctccg, gctcgccttctcctggag,
caagtccctggtgctgtt, gtagatggaaatcacctc, cggggggacttcctcggg, ctcaggatagtcttctgg, gggactggtgagcttcag, cttgctcaggatctgccc,
gcggatcgcctcgatcct, cttgcgcatgaactggtc, catatcgagtgtgctgca, ggtagacaggctgagcgc, gaccgtgaccagatgcag, gatcagaaaagcgctcag,
cacacagtagtgcatttt, ttaaaaaagtggaaaaaa, aagttgtttttaaaagtc, agaataaaaaaaaagaaa, tcaacaattctcaaagta, tagatcaaggagagttgt,
ttggtttttgttgttgt, tgtttgttttgatgcga, aacttttgcaaacaatct, agtcaatgcccaacagaa, aaaatttgcttcttgtct, ctctcacttacaaagtag,
gtgaaatgtagaataagg, ccttcaactttttttgtg, tcagatgccagttttaac, aaacagaacacaaacttc, caaagtgtctgaactagt, accgccttttcaaaaatt,
tttaacactgatgaacc, aaggctctcttatgtttc, ttgttacaagcatcatc, gttgtcgtcgtcatcatc, attatcatcatcattgtc, atttttggtcttgccactt,
ttccaagaattttagctg, catttgcaagactttaca, atcatattagaaagctgt, tcaatcttgggtgttttg, ccaatgtagtagagaatg, gttagaggttctaaatct,
tgggacacgcagcaagga, gaagcagatgcttctgga, tttatggtattatataag, ctcaggaccctgctgtgc, tgagtgtctgaactccat, aaatacgggcatgctcca,
gcacagaagttggcattg, taccctttgggttcgtgt, atccatttccaccctaga, tccctcttgaaatcaatg, taaagtggacgtaggcag, caattatcctgcacattt,
ctaaagcaataggccgca, tccaaagcacgcttcttc, cgccggttggtctgttgt, gactcaagtctgtaggag, ggcaataacattagcagg, agatgtggggtcttccca,
ctgtttttttcctagtg, gacttatagttttctga, tcaccactggtatatgtg, gaggtgccatcaatacct, gcaaatcttgcttctagt, tcttcacttttatttggg,
atgatgtaattattagat, ggtacaaaagtgcagcag, ggacagtgtaagcttatt, ttaaatcccaggttcctg, tctttatggtgaagccat, tcatgaacagcatcagtt,
acatcgaaggagagccat, tcgccttctgctcttgtt, ttcacaactttgctgtcg, atgtagcgctgggttgga, gatgttaaatctttggac, ttgagaatctgatatagc,
tcaatccgttgttcaggc, actctggcttttgggttc, tgcaaacgaaagactctg, aactctgctttcaccaaa, ttggaagcattcttctcc, attgctgagacgtcaaat,
cgaacaattctgaagtag, ggtctgtagaaagtgggc, gggatggcatttcggag, gggaagaagggcggcatg, tctatttgtaaacctcc, ttggcgtagtactcttcg,
tcgctcctctcgcgctcg, caggcggccgccctccgg, ctcgccttctcctggagc, aagtccctggtgctgttg, tagatggaaatcacctcc, ggggggacttcctcgggc,
tcaggatagtcttctggg, ggactggtgagcttcagc, ttgctcaggatctgcccg, cggatcgcctcgatcctc, ttgcgcatgaactggtcc, atatcgagtgtgctgcag,
gtagacaggctgagcgcg, accgtgaccagatgcagg, atcagaaaagcgctcagc, acacagtagtgcattttt, taaaaaagtggaaaaaaa, agttgtttttaaaagtca,
gaataaaaaaaaagaaat, caacaattctcaaagtat, agatcaaggagagttgtt, tggtttttgttgttgttg, gtttgttttgatgcgaa, acttttgcaaacaatcta,
gtcaatgcccaacagaaa, aaatttgcttcttgtctc, tctcacttacaaagtagg, tgaaatgtagaataaggc, cttcaactttttttgtgt, cagatgccagttttaaca,
aacagaacacaaacttcc, aaagtgtctgaactagta, ccgccttttcaaaaattt, ttaacactgatgaacca, aggctctcttatgtttc, ttgttacaagcatcatcg,
ttgtcgtcgtcatcatca, ttatcatcatcattgtca, tttttggtcttgccactt, tccaagaattttagctgc, atttgcaagactttacaa, tcatattagaaagctgtt,
caatcttgggtgttttgc, caatgtagtagagaatgg, ttagaggttctaaatctt, gggacacgcagcaaggag, aagcagatgcttctggat, ttatggtattatataagc,
tcaggaccctgctgtgct, gagtgtctgaactccata, aatacgggcatgctccag, cacagaagttggcattgt, accctttgggttcgtgta, tccatttccaccctagat,
ccctcttgaaatcaatgt, aaagtggacgtaggcagc, aattatcctgcacatttc, taaagcaataggccgcat, ccaaagcacgcttcttcc, gccggttggtctgttgtg,
actcaagtctgtaggagg, gcaataacattagcagga, gatgtggggtcttcccac, tgtttttttcctagtgg, acttatagttttctgat, caccactggtatatgtgg,
aggtgccatcaatacctg, caaatcttgcttctagtt, cttcacttttatttggga, tgatgtaattattagatg, gtacaaaagtgcagcagg, gacagtgtaagcttattt,
taaatcccaggttcctgt, ctttatggtgaagccatt, catgaacagcatcagtta, catcgaaggagagccatt, cgccttctgctcttgttt, tcacaactttgctgtcga,
tgtagcgctgggttggag, atgttaaatctttggact, tgagaatctgatatagct, caatccgttgttcaggca, ctctggcttttgggttct, gcaaacgaaagactctga,
actctgctttcaccaaat, tggaagcattcttctcca, ttgctgagacgtcaaatc, gaacaattctgaagtagg, gtctgtagaaagtgggcg, ggatggcatttcggagg,
ggaagaagggcggcatgt, ctatttgtaaacctcct, tggcgtagtactcttcgt, cgctcctctcgcgctcgc, aggcggccgccctccggc, tcgccttctcctggagca,
agtccctggtgctgttgt, agatggaaatcacctccg, gggggacttcctcgggct, caggatagtcttctgggg, gactggtgagcttcagct, tgctcaggatctgcccgc,
ggatcgcctcgatcctct, tgcgcatgaactggtcca, tatcgagtgtgctgcagg, tagacaggctgagcgcga, ccgtgaccagatgcagga, tcagaaaagcgctcagca,
cacagtagtgcattttt, aaaaaagtggaaaaaaa, gttgtttttaaaagtcag, aataaaaaaaaagaaatc, aacaattctcaaagtata, gatcaaggagagttgttt,
ggtttttgttgttgttg, tttgttttgatgcgaaa, cttttgcaaacaatctag, tcaatgcccaacagaaaa, aatttgcttcttgtctct, ctcacttacaaagtaggt,
gaaatgtagaataaggcc, ttcaactttttttgtgtc, agatgccagttttaacaa, acagaacacaaacttcca, aagtgtctgaactagtac, cgccttttcaaaaatttt,
taacactgatgaaccaa, ggctctcttatgtttct, tgttacaagcatcatcgt, tgtcgtcgtcatcatcat, tatcatcatcattgtcat, ttggtcttgccactttt,
ccaagaattttagctgca, tttgcaagactttacaat, catattagaaagctgttc, aatcttgggtgttttgcc, aatgtagtagagaatggt, tagaggttctaaatcttg,
ggacacgcagcaaggaga, agcagatgcttctggatt, tatggtattatataagct, caggaccctgctgtgctg, agtgtctgaactccataa, atacgggcatgctccagc,
acagaagttggcattgta, ccctttgggttcgtgtat, ccatttccaccctagatc, cctcttgaaatcaatgta, aagtggacgtaggcagca, attatcctgcacatttct,
aaagcaataggccgcatc, caaagcacgcttcttccg, ccggttggtctgttgtga, ctcaagtctgtaggaggg, caataacattagcaggag, atgtggggtcttcccact,
gtttttttcctagtgga, cttatagttttctgatc, accactggtatatgtgga, ggtgccatcaatacctgc, aaatcttgcttctagttc, ttcacttttatttgggat,
gatgtaattattagatgg, tacaaaagtgcagcaggg, acagtgtaagcttatttt, aaatcccaggttcctgtc, tttatggtgaagccattc, atgaacagcatcagttac,
atcgaaggagagccattc, gccttctgctcttgtttt, cacaactttgctgtcgat, gtagcgctgggttggaga, tgttaaatctttggactt, gagaatctgatatagctc,
aatccgttgttcaggcac, tctggcttttgggttctg, caaacgaaagactctgaa, ctctgctttcaccaaatt, ggaagcattcttctccat, tgctgagacgtcaaatcg,
aacaattctgaagtaggg, tctgtagaaagtgggcgg, gatggcatttcggaggg, gaagaagggcggcatgtc, tatttgtaaacctcctt, ggcgtagtactcttcgtc,
gctcctctcgcgctcgca, ggcggccgccctccggct, cgccttctcctggagcaa, gtccctggtgctgttgta, gatggaaatcacctccgg, ggggacttcctcgggctc,
aggatagtcttctggggg, actggtgagcttcagctt, gctcaggatctgcccgcg, gatcgcctcgatcctctt, gcgcatgaactggtccat, atcgagtgtgctgcaggt,
agacaggctgagcgcgac, cgtgaccagatgcaggat, cagaaaagcgctcagcac, acagtagtgcattttta, aaaaagtggaaaaaaag, ttgtttttaaaagtcaga,
ataaaaaaaaagaaatca, acaattctcaaagtatag, atcaaggagagttgtttg, gtttttgttgttgttgt, ttgttttgatgcgaaac, ttttgcaaacaatctagt,
caatgcccaacagaaaaa, atttgcttcttgtctctc, tcacttacaaagtaggtg, aaatgtagaataaggcct, tcaactttttttgtgtca, gatgccagttttaacaaa,
cagaacacaaacttccaa, agtgtctgaactagtacc, gccttttcaaaaattttt, aacactgatgaaccaag, gctctcttatgtttctt, gttacaagcatcatcgtt,
gtcgtcgtcatcatcatt, atcatcatcattgtcatt, ttggtcttgccactttc, caagaattttagctgcat, ttgcaagactttacaatc, atattagaaagctgttca,
atcttgggtgttttgcca, atgtagtagagaatggtt, agaggttctaaatcttgg, gacacgcagcaaggagaa, gcagatgcttctggattt, atggtattatataagctc,
aggaccctgctgtgctga, gtgtctgaactccataaa, tacgggcatgctccagca, cagaagttggcattgtac, cctttgggttcgtgtatc, catttccaccctagatcc,
ctcttgaaatcaatgtaa, agtggacgtaggcagcaa, ttatcctgcacatttcta, aagcaataggccgcatcc, aaagcacgcttcttccgc, cggttggtctgttgtgac,
 
tcaagtctgtaggagggc, aataacattagcaggaga, tgtggggtcttcccactg, tttttttcctagtggac, tttatagtttctgatca, ccactggtatatgtggag,
gtgccatcaatacctgca, aatcttgcttctagttct, tcacttttatttgggatg, atgtaattattagatggt, acaaaagtgcagcaggga, cagtgtaagcttatttta,
aatcccaggttcctgtct, ttatggtgaagccattca, tgaacagcatcagttaca, tcgaaggagagccattcg, ccttctgctcttgtttc, acaactttgctgtcgatg,
tagcgctgggttggagat, gttaaatctttggacttg, agaatctgatatagctca, atccgttgttcaggcact, ctggcttttgggttctgc, aaacgaaagactctgaac,
tctgctttcaccaaattg, gaagcattcttctccatt, gctgagacgtcaaatcga, acaattctgaagtagggt, ctgtagaaagtgggcggg, atggcattttcggagggg,
aagaagggcggcatgtct, attttgtaaacctccttg, gcgtagtactcttcgtcg, ctcctctcgcgctcgcag, gcggccgccctccggctc, gccttctcctggagcaag,
tccctggtgctgttgtag, atggaaatcacctccggg, gggacttcctcgggctca, ggatagtcttctgggga, ctggtgagcttcagcttg, ctcaggatctgcccgcgg,
atcgcctcgatcctcttg, cgcatgaactggtccata, tcgagtgtgctgcaggta, gacaggctgagcgcgacc, gtgaccagatgcaggatc, agaaaagcgctcagcaca,
cagtagtgcatttttaa, aaaagtggaaaaaaaagt, tgtttttaaaagtcagaa, taaaaaaaaagaaatcaa, caattctcaaagtataga, tcaaggagagttgtttgg,
tttttgttgttgttgtt, tgtttttgatgcgaaact, tttgcaaacaatctagtc, aatgcccaacagaaaaac, tttgcttcttgtctctct, cacttacaaagtaggtga,
aatgtagaataaggcctt, caactttttttgtgtcag, atgccagttttaacaaac, agaacacaaacttccaaa, gtgtctgaactagtaccg, cctttcaaaaattttt,
aacactgatgaaccaagg, ctctcttatgttttcttg, ttacaagcatcatcgttg, tcgtcgtcatcatcatta, tcatcatcattgtcattt, tggtcttgccactttcc,
aagaatttagctgcatt, tgcaagactttacaatca, tattagaaagctgttcaa, tcttgggtgttttgccaa, tgtagtagagaatggtta, gaggttctaaatcttggg,
acacgcagcaaggagaag, cagatgcttctggattta, tggtattatataagctca, ggaccctgctgtgctgag, tgtctgaactccataaat, acgggcatgctccagcac,
agaagttggcattgtacc, ctttgggttcgtgtatcc, atttccaccctagatccc, tcttgaaatcaatgtaaa, gtggacgtaggcagcaat, tatcctgcacatttctaa,
agcaataggccgcatcca, aagcacgcttcttccgcc, ggttggtctgttgtgact, caagtctgtaggagggca, ataacattagcaggagat, gtggggtcttcccactgt,
ttttttcctagtggact, ttatagtttctgatcac, cactggtatatgtggagg, tgccatcaatacctgcaa, atcttgcttctagttctt, cacttttatttgggatga,
tgtaattattagatggta, caaaagtgcagcagggac, agtgtaagcttattttaa, atcccaggttcctgtctt, tatggtgaagccattcat, gaacagcatcagttacat,
cgaaggagagccattcgc, cttctgctcttgttttca, caactttgctgtcgatgt, agcgctgggttggagatg, ttaaatctttggacttga, gaatctgatatagctcaa,
tccgttgttcaggcactc, tggcttttgggttctgca, aacgaaagactctgaact, ctgctttcaccaaattgg, aagcattcttctccattg, ctgagacgtcaaatcgaa,
caattctgaagtagggtc, tgtagaaagtgggcggga, tggcattttcggagggga, agaagggcggcatgtcta, ttttgtaaacctccttgg, cgtagtactcttcgtcgc,
tcctctcgcgctcgcagg, cggccgccctccggctcg, ccttctcctggagcaagt, ccctggtgctgttgtaga, tggaaatcacctccgggg, ggacttcctcgggctcag,
gatagtcttctgggggac, tggtgagcttcagcttgc, tcaggatctgcccgcgga, tcgcctcgatcctcttgc, gcatgaactggtccatat, cgagtgtgctgcaggtag,
acaggctgagcgcgaccg, tgaccagatgcaggatca, gaaaagcgctcagcacac, agtagtgcatttttaaa, aaagtggaaaaaaaagtt, gtttttaaaagtcagaat,
aaaaaaaaagaaatcaac, aattctcaaagtatagat, caaggagagttgtttggt, ttttgttgttgttgttt, gtttttgatgcgaaactt, ttgcaaacaatctagtca,
atgcccaacagaaaaacg, ttgcttcttgtctctctc, acttacaaagtaggtgaa, atgtagaataaggccttc, aactttttttgtgtcaga, tgccagttttaacaaaca,
gaacacaaacttccaaag, tgtctgaactagtaccgc, ctttcaaaaattttta, acactgatgaaccaaggc, tctcttatgttttcttgt, tacaagcatcatcgttgt,
cgtcgtcatcatcattat, catcatcattgtcatttt, ggtcttgccactttcca, agaatttagctgcattt, gcaagactttacaatcat, attagaaagctgttcaat,
cttgggtgttttgccaat, gtagtagagaatggttag, aggttctaaatcttggga, cacgcagcaaggagaagc, agatgcttctggatttat, ggtattatataagctcag,
gaccctgctgtgctgagt, gtctgaactccataaata, cgggcatgctccagcaca, gaagttggcattgtaccc, tttgggttcgtgtatcca, tttccaccctagatccct,
cttgaaatcaatgtaaag, tggacgtaggcagcaatt, atcctgcacatttctaaa, gcaataggccgcatccaa, agcacgcttcttccgccg, gttggtctgttgtgactc,
aagtctgtaggagggcaa, taacattagcaggagatg, tggggtcttcccactgtt, tttttcctagtggactt, tatagtttctgatcacc, actggtatatgtggaggt,
gccatcaatacctgcaaa, tcttgcttctagttcttc, acttttatttgggatgat, gtaattattagatggtac, aaaagtgcagcagggaca, gtgtaagcttattttaaa,
tcccaggttcctgtcttt, atggtgaagccattcatg, aacagcatcagttacatc, gaaggagagccattcgcc, ttctgctcttgttttcac, aactttgctgtcgatgta,
gcgctgggttggagatgt, taaatctttggacttgag, aatctgatatagctcaat, ccgttgttcaggcactct, ggcttttgggttctgcaa, acgaaagactctgaactc,
tgctttcaccaaattgga, agcattcttctccattgc, tgagacgtcaaatcgaac, aattctgaagtagggtct, gtagaaagtgggcgggat, ggcattttcggaggggaa,
gaagggcggcatgtctat, tttgtaaacctccttggc, gtagtactcttcgtcgct, cctctcgcgctcgcaggc, ggccgccctccggctcgc, cttctcctggagcaagtc,
cctggtgctgttgtagat, ggaaatcacctccggggg, gacttcctcgggctcagg, atagtcttctgggggact, ggtgagcttcagcttgct, caggatctgcccgcggat,
cgcctcgatcctcttgcg, catgaactggtccatatc, gagtgtgctgcaggtaga, caggctgagcgcgaccgt, gaccagatgcaggatcag, aaaagcgctcagcacaca,
gtagtgcatttttaaaa, aagtggaaaaaaaagttg, tttttaaaagtcagaata, aaaaaaaagaaatcaaca, attctcaaagtatagatc, aaggagagttgtttggtt,
tttgttgttgttgtttg, tttttgatgcgaaacttt, tgcaaacaatctagtcaa, tgcccaacagaaaaacgt, tgcttcttgtctctctca, cttacaaagtaggtgaaa,
tgtagaataaggccttca, actttttttgtgtcagat, gccagttttaacaaacag, aacacaaacttccaaagt, gtctgaactagtaccgcc, tttcaaaaatttttaa,
cactgatgaaccaaggct, ctcttatgttttcttgtt, acaagcatcatcgttgtc, gtcgtcatcatcattatc, atcatcattgtcattttg, gtcttgccactttccaa,
gaatttagctgcatttg, caagactttacaatcata, ttagaaagctgttcaatc, ttgggtgttttgccaatg, tagtagagaatggttaga, ggttctaaatcttgggac,
acgcagcaaggagaagca, gatgcttctggatttatg, gtattatataagctcagg, accctgctgtgctgagtg, tctgaactccataaatac, gggcatgctccagcacag,
aagttggcattgtaccct, ttgggttcgtgtatccat, ttccaccctagatccctc, ttgaaatcaatgtaaagt, ggacgtaggcagcaatta, tcctgcacatttctaaag,
caataggccgcatccaaa, gcacgcttcttccgccgg, ttggtctgttgtgactca, agtctgtaggagggcaat, aacattagcaggagatgt, ggggtcttcccactgttt,
ttttcctagtggacttt, atagtttctgatcacca, ctggtatatgtggaggtg, ccatcaatacctgcaaat, cttgcttctagttcttca, cttttatttgggatgatg,
taattattagatggtaca, aaagtgcagcagggacag, tgtaagcttattttaaat, cccaggttcctgtcttta, tggtgaagccattcatga, acagcatcagttacatcg,
aaggagagccattcgcct, tctgctcttgttttcaca, actttgctgtcgatgtag, cgctgggttggagatgtt, aaatctttggacttgaga, atctgatatagctcaatc,
cgttgttcaggcactctg, gcttttgggttctgcaaa, cgaaagactctgaactct, gctttcaccaaattggaa, gcattcttctccattgct, gagacgtcaaatcgaaca,
attctgaagtagggtctg, tagaaagtgggcgggatg, gcattttcggaggggaag, aagggcggcatgtctatt, ttgtaaacctccttggcg, tagtactcttcgtcgctc,
ctctcgcgctcgcaggcg, gccgccctccggctcgcc, ttctcctggagcaagtcc, ctggtgctgttgtagatg, gaaatcacctccgggggg, acttcctcgggctcagga,
tagtcttctgggggactg, gtgagcttcagcttgctc, aggatctgcccgcggatc, gcctcgatcctcttgcgc, atgaactggtccatatcg, agtgtgctgcaggtagac,
aggctgagcgcgaccgtg, accagatgcaggatcaga, aaagcgctcagcacacag, tagtgcatttttaaaaa, agtggaaaaaaaagttgt, ttttaaaagtcagaataa,
aaaaaaagaaatcaacaa, ttctcaaagtatagatca, aggagagttgtttggttt, ttgttgttgttgtttgt, ttttgatgcgaaactttt, gcaaacaatctagtcaat,
gcccaacagaaaaacgta, gcttcttgtctctctcac, ttacaaagtaggtgaaat, gtagaataaggccttcaa, ctttttttgtgtcagatg, ccagttttaacaaacaga,
acacaaacttccaaagtg, tctgaactagtaccgcct, ttcaaaaatttttaac, actgatgaaccaaggctc, tcttatgttttcttgtta, caagcatcatcgttgtcg,
tcgtcatcatcattatca, tcatcattgtcattttgg, tcttgccactttccaag, aatttagctgcatttgc, aagactttacaatcatat, tagaaagctgttcaatct,
tgggtgttttgccaatgt, agtagagaatggttagag, gttctaaatcttgggaca, cgcagcaaggagaagcag, atgcttctggatttatgg, tattatataagctcagga,
ccctgctgtgctgagtgt, ctgaactccataaatacg, ggcatgctccagcacaga, agttggcattgtaccctt, tgggttcgtgtatccatt, tccaccctagatccctct,
tgaaatcaatgtaaagtg, gacgtaggcagcaattat, cctgcacatttctaaagc, aataggccgcatccaaag, cacgcttcttccgccggt, tggtctgttgtgactcaa,
gtctgtaggagggcaata, acattagcaggagatgtg, gggtcttcccactgtttt, tttcctagtggactta, tagtttctgatcaccac, tggtatatgtggaggtgc,
catcaatacctgcaaatc, ttgcttctagttcttcac, ttttatttgggatgatgt, aattattagatggtacaa, aagtgcagcagggacagt, gtaagcttattttaaatc,
ccaggttcctgtctttat, ggtgaagccattcatgaa, cagcatcagttacatcga, aggagagccattcgcctt, ctgctcttgttttcacaa, ctttgctgtcgatgtagc,
gctgggttggagatgtta, aatctttggacttgagaa, tctgatatagctcaatcc, gttgttcaggcactctgg, cttttgggttctgcaaac, gaaagactctgaactctg,
ctttcaccaaattggaag, cattcttctccattgctg, agacgtcaaatcgaacaa, ttctgaagtagggtctgt, agaaagtgggcgggatgg, cattttcggaggggaaga,
agggcggcatgtctattt, tgtaaacctccttggcgt, agtactcttcgtcgctcc, tctcgcgctcgcaggcgg, ccgccctccggctcgcct, tctcctggagcaagtccc,
tggtgctgttgtagatgg, aaatcacctccgggggga, cttcctcgggctcaggat, agtcttctgggggactgg, tgagcttcagcttgctca, ggatctgcccgcggatcg,
cctcgatcctcttgcgca, tgaactggtccatatcga, gtgtgctgcaggtagaca, ggctgagcgcgaccgtga, ccagatgcaggatcagaa, aagcgctcagcacacagt,
agtgcatttttaaaaaa, gtggaaaaaaaagttgtt, tttaaaagtcagaataaa, aaaaaagaaatcaacaat, tctcaaagtatagatcaa, ggagagttgtttggtttt,
tgttgttgttgtttgtt, tttgatgcgaaacttttg, caaacaatctagtcaatg, cccaacagaaaaacgtat, cttcttgtctctctcact, tacaaagtaggtgaaatg,
tagaataaggccttcaac, tttttttgtgtcagatgc, cagttttaacaaacagaa, cacaaacttccaaagtgt, ctgaactagtaccgcctt, ttcaaaaatttttaaca,
ctgatgaaccaaggctct, cttatgttttcttgttac, aagcatcatcgttgtcgt, cgtcatcatcattatcat, catcattgtcattttggt, cttgccactttccaaga,
atttagctgcatttgca, agactttacaatcatatt, agaaagctgttcaatctt, gggtgttttgccaatgta, gtagagaatggttagagg, ttctaaatcttgggacac,
gcagcaaggagaagcaga, tgcttctggatttatggt, attatataagctcaggac, cctgctgtgctgagtgtc, tgaactccataaatacgg, gcatgctccagcacagaa,
gttggcattgtacccttt, gggttcgtgtatccattt, ccaccctagatccctctt, gaaatcaatgtaaagtgg, acgtaggcagcaattatc, ctgcacatttctaaagca,
 
ataggccgcatccaaagc, acgcttcttccgccggtt, ggtctgttgtgactcaag, tctgtaggagggcaataa, cattagcaggagatgtgg, ggtcttcccactgttttt,
tttcctagtggactttat, agtttctgatcaccact, ggtatatgtggaggtgcc, atcaatacctgcaaatct, tgcttctagttcttcact, tttatttgggatgatgta,
attattagatggtacaaa, agtgcagcagggacagtg, taagcttattttaaatcc, caggttcctgtctttatg, gtgaagccattcatgaac, agcatcagttacatcgaa,
ggagagccattcgccttc, tgctcttgttttcacaac, tttgctgtcgatgtagcg, ctgggttggagatgttaa, atctttggacttgagaat, ctgatatagctcaatccg,
ttgttcaggcactctggc, ttttgggttctgcaaacg, aaagactctgaactctgc, tttcaccaaattggaagc, attcttctccattgctga, gacgtcaaatcgaacaat,
tctgaagtagggtctgta, gaaagtgggcgggatggc, attttcggaggggaagaa, gggcggcatgtctatttt, gtaaacctccttggcgta, gtactcttcgtcgctcct,
ctcgcgctcgcaggcggc, cgccctccggctcgcctt, ctcctggagcaagtccct, ggtgctgttgtagatgga, aatcacctccgggggac, ttcctcgggctcaggata,
gtcttctggggggactggt, gagcttcagcttgctcag, gatctgcccgcggatcgc, ctcgatcctcttgcgcat, gaactggtccatatcgag, tgtgctgcaggtagacag,
gctgagcgcgaccgtgac, cagatgcaggatcagaaa, agcgctcagcacacagta, gtgcatttttaaaaaag, tggaaaaaaaagttgttt, ttaaaagtcagaataaaa,
aaaaagaaatcaacaatt, ctcaaagtatagatcaag, gagagttgtttggttttt, tgttgttgttgtttgttt, ttgatgcgaaacttttgc, aaacaatctagtcaatgc,
ccaacagaaaaacgtatc, ttcttgtctctctcactt, acaaagtaggtgaaatgt, agaataaggccttcaact, ttttttgtgtcagatgcc, agttttaacaaacagaac,
acaaacttccaaagtgtc, tgaactagtaccgccttt, tcaaaaatttttaacac, tgatgaaccaaggctctc, ttatgtttcttgttaca, agcatcatcgttgtcgtc,
gtcatcatcattatcatc, atcattgtcattttggtc, ttgccacttttccaagaa, ttttagctgcatttgcaa, gactttacaatcatatta, gaaagctgttcaatcttg,
ggtgttttgccaatgtag, tagagaatggttagaggt, tctaaatcttgggacacg, cagcaaggagaagcagat, gcttctggatttatggta, ttatataagctcaggacc,
ctgctgtgctgagtgtct, gaactccataaatacggg, catgctccagcacagaag, ttggcattgtaccctttg, ggttcgtgtatccatttc, caccctagatccctcttg,
aaatcaatgtaaagtgga, cgtaggcagcaattatcc, tgcacatttctaaagcaa, taggccgcatccaaagca, cgcttcttccgccggttg, gtctgttgtgactcaagt,
ctgtaggagggcaataac, attagcaggagatgtggg, gtcttcccactgtttttt, ttcctagtggactttata, gtttctgatcaccactg, gtatatgtggaggtgcca,
tcaatacctgcaaatctt, gcttctagttcttcactt, ttatttgggatgatgtaa, ttattagatggtacaaaa, gtgcagcagggacagtgt, aagcttattttaaatccc,
aggttcctgtctttatgg, tgaagccattcatgaaca, gcatcagttacatcgaag, gagagccattcgccttct, gctcttgttttcacaact, ttgctgtcgatgtagcgc,
tgggttggagatgttaaa, tctttggacttgagaatc, tgatatagctcaatccgt, tgttcaggcactctggct, tttgggttctgcaaacga, aagactctgaactctgct,
ttcaccaaattggaagca, ttcttctccattgctgag, acgtcaaatcgaacaatt, ctgaagtagggtctgtag, aaagtgggcgggatggca, ttttcggaggggaagaag,
ggcggcatgtctattttg, taaacctccttggcgtag, tactcttcgtcgctcctc, tcgcgctcgcaggcggcc, gccctccggctcgccttc, tcctggagcaagtccctg,
gtgctgttgtagatggaa, atcacctccgggggact, tcctcgggctcaggatag, tcttctggggggactggtg, agcttcagcttgctcagg, atctgcccgcggatcgcc,
tcgatcctcttgcgcatg, aactggtccatatcgagt, gtgctgcaggtagacagg, ctgagcgcgaccgtgacc, agatgcaggatcagaaaa, gcgctcagcacacagtag,
tgcatttttaaaaaagt, ggaaaaaaaagttgtttt, taaaagtcagaataaaaa, aaaagaaatcaacaattc, tcaaagtatagatcaagg, agagttgtttggtttttt,
gttgttgttgtttgtttt, tgatgcgaaacttttgca, aacaatctagtcaatgcc, caacagaaaaacgtatcc, tcttgtctctctcactta, caaagtaggtgaaatgta,
gaataaggccttcaactt, tttttgtgtcagatgcca, gttttaacaaacagaaca, caaacttccaaagtgtct, gaactagtaccgcctttt, caaaaatttttaacact,
gatgaaccaaggctctct, tatgtttcttgttacaa, gcatcatcgttgtcgtcg, tcatcatcattatcatca, tcattgtcattttggtct, tgccacttttccaagaat,
tttagctgcatttgcaag, actttacaatcatattag, aaagctgttcaatcttgg, gtgttttgccaatgtagt, agagaatggttagaggtt, ctaaatcttgggacacgc,
agcaaggagaagcagatg, cttctggatttatggtat, tatataagctcaggaccc, tgctgtgctgagtgtctg, aactccataaatacgggc, atgctccagcacagaagt,
tggcattgtaccctttgg, gttcgtgtatccatttcc, accctagatccctcttga, aatcaatgtaaagtggac, gtaggcagcaattatcct, gcacatttctaaagcaat,
aggccgcatccaaagcac, gcttcttccgccggttgg, tctgttgtgactcaagtc, tgtaggagggcaataaca, ttagcaggagatgtgggg, tcttcccactgttttttt,
tcctagtggactttatag, tttctgatcaccactgg, tatatgtggaggtgccat, caatacctgcaaatcttg, cttctagttcttcacttt, tatttgggatgatgtaat,
tattagatggtacaaaag, tgcagcagggacagtgta, agcttattttaaatccca, ggttcctgtctttatggt, gaagccattcatgaacag, catcagttacatcgaagg,
agagccattcgccttctg, ctcttgttttcacaactt, tgctgtcgatgtagcgct, gggttggagatgttaaat, ctttggacttgagaatct, gatatagctcaatccgtt,
gttcaggcactctggctt, ttgggttctgcaaacgaa, agactctgaactctgctt, tcaccaaattggaagcat, tcttctccattgctgaga, cgtcaaatcgaacaattc,
tgaagtagggtctgtaga, aagtgggcgggatggcat, tttcggaggggaagaagg, gcggcatgtctattttgt, aaacctccttggcgtagt, actcttcgtcgctcctct,
cgcgctcgcaggcggccg, ccctccggctcgccttct, cctggagcaagtccctgg, tgctgttgtagatggaaa, tcacctccgggggactt, cctcgggctcaggatagt,
cttctggggggactggtga, gcttcagcttgctcagga, tctgcccgcggatcgcct, cgatcctcttgcgcatga, actggtccatatcgagtg, tgctgcaggtagacaggc,
tgagcgcgaccgtgacca, gatgcaggatcagaaaag, cgctcagcacacagtagt, gcatttttaaaaaagtg, gaaaaaaaagttgttttt, aaaagtcagaataaaaaa,
aaagaaatcaacaattct, caaagtatagatcaagga, gagttgtttggttttttg, ttgttgttgtttgttttt, gatgcgaaacttttgcaa, acaatctagtcaatgccc,
aacagaaaaacgtatcct

Splice - non coding region:
agcacacagtagtgcatt, ttttaaaaaagtggaaaa, aaaagttgtttttaaaag, tcagaataaaaaaagaa, atcaacaattctcaaagt, atagatcaaggagagttg,
tttggtttttgttgttg, ttgtttttgatgcgaaac, ttttgcaaacaatctagt, caatgcccaacagaaaaa, cgtatcctgcttgaattc, ctttaaaaagaaaaggcc,
agtagttccaaaagtgga, aatattaatacgggacgg, gcagagggaaccctgact, ttggcgagtaagaagaaa, aaaattcttggagcaatg, agatggggaaaaaaagag,
acgagtggctattaagta, gaaataaatttaagagga, ggaagtggagttcagtgt, gtcagtctcgggtgcgga, gtggcggatctgaactcg, gctcctccggccaaaagg,
gaagagatgaaaaggtcc, cggggctggcagctggcg, gcacacagtagtgcattt, tttaaaaaagtggaaaaa, aaagttgtttttaaaagt, cagaataaaaaaagaaa,
tcaacaattctcaaagta, tagatcaaggagagttgt, ttggtttttgttgttgt, tgtttttgatgcgaaact, tttgcaaacaatctagtc, aatgcccaacagaaaaac,
gtatcctgcttgaattcc, tttaaaaagaaaaggcca, gtagttccaaaagtggaa, atattaatacgggacggg, cagagggaaccctgactt, tggcgagtaagaagaaaa,
aaattcttggagcaatga, gatggggaaaaaaagaga, cgagtggctattaagtag, aaataaatttaagaggag, gaagtggagttcagtgtg, tcagtctcgggtgcggag,
tggcggatctgaactcgg, ctcctccggccaaaaggg, aagagatgaaaaggtccc, ggggctggcagctggcga, cacacagtagtgcatttt, ttaaaaaagtggaaaaaa,
aagttgtttttaaaagtc, agaataaaaaaagaaat, caacaattctcaaagtat, agatcaaggagagttgtt, tggtttttgttgttgtt, gtttttgatgcgaaactt,
ttgcaaacaatctagtca, atgcccaacagaaaaacg, tatcctgcttgaattcct, ttaaaaagaaaaggccag, tagttccaaaagtggaaa, tattaatacgggacgggc,
agagggaaccctgacttt, ggcgagtaagaagaaaaa, aattcttggagcaatgag, atggggaaaaaaagagac, gagtggctattaagtaga, aataaatttaagaggagg,
aagtggagttcagtgtgt, cagtctcgggtgcggagt, ggcggatctgaactcggc, tcctccggccaaaaggga, agagatgaaaaggtcccg, gggctggcagctggcgaa,
acacagtagtgcattttt, taaaaaagtggaaaaaaa, agttgtttttaaaagtca, gaataaaaaaagaaatc, aacaattctcaaagtata, gatcaaggagagttgttt,
ggtttttgttgttgttg, tttttgatgcgaaacttt, tgcaaacaatctagtcaa, tgcccaacagaaaaacgt, atcctgcttgaattcctt, taaaaagaaaaggccagt,
agttccaaaagtggaaat, attaatacgggacgggca, gagggaaccctgactttg, gcgagtaagaagaaaaaa, attcttggagcaatgaga, tggggaaaaaaagagacg,
agtggctattaagtagaa, ataaatttaagaggagga, agtggagttcagtgtgtc, agtctcgggtgcggagtg, gcggatctgaactcggct, cctccggccaaaagggaa,
gagatgaaaaggtcccgg, ggctggcagctggcgaac, cacagtagtgcattttt, aaaaaagtggaaaaaaaa, gttgtttttaaaagtcag, aataaaaaaagaaatca,
acaattctcaaagtatag, atcaaggagagttgtttg, gtttttgttgttgttgt, ttttgatgcgaaactttt, gcaaacaatctagtcaat, gcccaacagaaaaacgta,
tcctgcttgaattccttt, aaaaagaaaaggccagta, gttccaaaagtggaaata, ttaatacgggacgggcag, agggaaccctgactttgg, cgagtaagaagaaaaaaa,
ttcttggagcaatgagat, ggggaaaaaaagagacga, gtggctattaagtagaaa, taaatttaagaggaggaa, gtggagttcagtgtgtca, gtctcgggtgcggagtgg,
cggatctgaactcggctc, ctccggccaaaagggaag, agatgaaaaggtcccggg, gctggcagctggcgaact, acagtagtgcattttta, aaaaagtggaaaaaaaag,
ttgtttttaaaagtcaga, ataaaaaaagaaatcaa, caattctcaaagtataga, tcaaggagagttgtttgg, tttttgttgttgttgtt, ttgatgcgaaacttttg,
caaacaatctagtcaatg, cccaacagaaaaacgtat, cctgcttgaattccttta, aaaagaaaaggccagtag, ttccaaaagtggaaatat, taatacgggacgggcaga,
gggaaccctgactttggc, gagtaagaagaaaaaaat, tcttggagcaatgagatg, gggaaaaaaagagacgag, tggctattaagtagaaat, aaatttaagaggaggaag,
tggagttcagtgtgtcag, tctcgggtgcggagtggc, ggatctgaactcggctcc, tccggccaaaagggaaga, gatgaaaaggtcccgggg, ctggcagctggcgaactg,
cagtagtgcatttttaa, aaaagtggaaaaaaaagt, tgtttttaaaagtcagaa, taaaaaaagaaatcaac, aattctcaaagtatagat, caaggagagttgtttggt,
ttttgttgttgttgttt, ttgatgcgaaacttttgc, aaacaatctagtcaatgc, ccaacagaaaaacgtatc, ctgcttgaattcctttaa, aaagaaaaggccagtagt,
tccaaaagtggaaatatt, aatacgggacgggcagag, ggaaccctgactttggcg, agtaagaagaaaaaaatt, cttggagcaatgagatgg, ggaaaaaaagagacgagt,
ggctattaagtagaaata, aatttaagaggaggaagt, ggagttcagtgtgtcagt, ctcgggtgcggagtggcg, gatctgaactcggctcct, ccggccaaaagggaagag,
atgaaaaggtcccggggc, tggcagctggcgaactga, agtagtgcatttttaaa, aaagtggaaaaaaaagtt, gtttttaaaagtcagaat, aaaaaaagaaatcaaca,
attctcaaagtatagatc, aaggagagttgtttggtt, tttgttgttgttgtttt, tgatgcgaaacttttgca, aacaatctagtcaatgcc, caacagaaaaacgtatcc,
tgcttgaattcctttaaa, aagaaaaggccagtagtt, ccaaaagtggaaatatta, atacgggacgggcagagg, gaaccctgactttggcga, gtaagaagaaaaaaattc,
 
ttggagcaatgagatggg, gaaaaaagagacgagtg, gctattaagtagaaataa, atttaagaggaggaagtg, gagttcagtgtgtcagtc, tcgggtgcggagtggcgg,
atctgaactcggctcctc, cggccaaaagggaagaga, tgaaaaggtcccggggct, ggcagctggcgaactgac, gtagtgcatttttaaaaa, aagtggaaaaaaaagttg,
tttttaaaagtcagaata, aaaaaaagaaatcaacaa, ttctcaaagtatagatca, aggagagttgtttggttt, tttgttgttgttgttttt, gatgcgaaactttgcaa,
acaatctagtcaatgccc, aacagaaaaacgtatcct, gcttgaattcctttaaaa, agaaaaggccagtagttc, caaaagtggaaatattaa, tacgggacgggcagaggg,
aaccctgactttggcgag, taagaagaaaaaaattct, tggagcaatgagatgggg, aaaaaaagagacgagtgg, ctattaagtagaaataaa, tttaagaggaggaagtgg,
agttcagtgtgtcagtct, cgggtgcggagtggcgga, tctgaactcggctcctcc, ggccaaaagggaagagat, gaaaaggtcccggggctg, gcagctggcgaactgacg,
tagtgcatttttaaaaaa, agtggaaaaaaaagttgt, ttttaaaagtcagaataa, aaaaaagaaatcaacaat, tctcaaagtatagatcaa, ggagagttgtttggtttt,
ttgttgttgttgtttttg, atgcgaaactttgcaaa, caatctagtcaatgccca, acagaaaaacgtatcctg, cttgaattcctttaaaaa, gaaaaggccagtagttcc,
aaaagtggaaatattaat, acgggacgggcagaggga, accctgactttggcgagt, aagaagaaaaaaattctt, ggagcaatgagatgggga, aaaaaagagacgagtggc,
tattaagtagaaataaat, ttaagaggaggaagtgga, gttcagtgtgtcagtctc, gggtgcggagtggcggat, ctgaactcggctcctccg, gccaaaagggaagagatg,
aaaaggtcccggggctgg, cagctggcgaactgacgg, agtgcatttttaaaaaaa, gtggaaaaaaaagttgtt, tttaaaagtcagaataaa, aaaaagaaatcaacaatt,
ctcaaagtatagatcaag, gagagttgtttggttttt, tgttgttgttgtttttga, tgcgaaactttgcaaac, aatctagtcaatgcccaa, cagaaaaacgtatcctgc,
ttgaattcctttaaaaag, aaaaggccagtagttcca, aaagtggaaatattaata, cgggacgggcagagggaa, ccctgactttggcgagta, agaagaaaaaaattcttg,
gagcaatgagatggggaa, aaaaagagacgagtggct, attaagtagaaataaatt, taagaggaggaagtggag, ttcagtgtgtcagtctcg, ggtgcggagtggcggatc,
tgaactcggctcctccgg, ccaaaagggaagagatga, aaaggtcccggggctggc, agctggcgaactgacggg, gtgcatttttaaaaaaag, tggaaaaaaaagttgttt,
ttaaaagtcagaataaaa, aaaagaaatcaacaattc, tcaaagtatagatcaagg, agagttgtttggtttttt, gttgttgttgtttttgat, gcgaaactttgcaaaca,
atctagtcaatgcccaac, agaaaaacgtatcctgct, tgaattcctttaaaaaga, aaaggccagtagttccaa, aagtggaaatattaatac, gggacgggcagagggaac,
cctgactttggcgagtaa, gaagaaaaaaattcttgg, agcaatgagatggggaaa, aaaagagacgagtggcta, ttaagtagaaataaattt, aagaggaggaagtggagt,
tcagtgtgtcagtctcgg, gtgcggagtggcggatct, gaactcggctcctccggc, caaaagggaagagatgaa, aaggtcccggggctggca, gctggcgaactgacggga,
tgcatttttaaaaaaagt, ggaaaaaaaagttgtttt, taaaagtcagaataaaaa, aaagaaatcaacaattct, caaagtatagatcaagga, gagttgtttggttttttg,
ttgttgttgtttttgatg, cgaaactttgcaaacaa, tctagtcaatgcccaaca, gaaaaacgtatcctgctt, gaattcctttaaaaagaa, aaggccagtagttccaaa,
agtggaaatattaatacg, ggacgggcagagggaacc, ctgactttggcgagtaag, aagaaaaaaattcttgga, gcaatgagatggggaaaa, aaagagacgagtggctat,
taagtagaaataaattta, agaggaggaagtggagtt, cagtgtgtcagtctcggg, tgcggagtggcggatctg, aactcggctcctccggcc, aaaagggaagagatgaaa,
aggtcccggggctggcag, ctggcgaactgacgggag, gcatttttaaaaaaagtg, gaaaaaaaagttgttttt, aaaagtcagaataaaaaa, aagaaatcaacaattctc,
aaagtatagatcaaggag, agttgtttggttttttgt, tgttgttgtttttgatgc, gaaactttgcaaacaat, ctagtcaatgcccaacag, aaaaacgtatcctgcttg,
aattcctttaaaaagaaa, aggccagtagttccaaaa, gtggaaatattaatacgg, gacgggcagagggaaccc, tgactttggcgagtaaga, agaaaaaaattcttggag,
caatgagatggggaaaaa, aagagacgagtggctatt, aagtagaaataaatttaa, gaggaggaagtggagttc, agtgtgtcagtctcgggt, gcggagtggcggatctga,
actcggctcctccggcca, aaagggaagagatgaaaa, ggtcccggggctggcagc, tggcgaactgacgggagg, catttttaaaaaaagtgg, aaaaaaagttgttttta,
aaagtcagaataaaaaaa, agaaatcaacaattctca, aagtatagatcaaggaga, gttgtttggttttttgtt, gttgttgtttttgatgcg, aaactttgcaaacaatc,
tagtcaatgcccaacaga, aaaacgtatcctgcttga, attcctttaaaaagaaaa, ggccagtagttccaaaag, tggaaatattaatacggg, acgggcagagggaaccct,
gactttggcgagtaagaa, gaaaaaaattcttggagc, aatgagatggggaaaaaa, agagacgagtggctatta, agtagaaataaatttaag, aggaggaagtggagttca,
gtgtgtcagtctcgggtg, cggagtggcggatctgaa, ctcggctcctccggccaa, aagggaagagatgaaaag, gtcccggggctggcagct, ggcgaactgacgggaggg,
atttttaaaaaaagtgga, aaaaaaagttgttttaa, aagtcagaataaaaaaaa, gaaatcaacaattctcaa, agtatagatcaaggagag, ttgtttggttttttgttg,
ttgttgtttttgatgcga, aactttgcaaacaatct, agtcaatgcccaacagaa, aaacgtatcctgcttgaa, ttcctttaaaaagaaaag, gccagtagttccaaaagt,
ggaaatattaatacggga, cgggcagagggaaccctg, actttggcgagtaagaag, aaaaaaattcttggagca, atgagatggggaaaaaaa, gagacgagtggctattaa,
gtagaaataaatttaaga, ggaggaagtggagttcag, tgtgtcagtctcgggtgc, ggagtggcggatctgaac, tcggctcctccggccaaa, agggaagagatgaaaagg,
tcccggggctggcagctg, gcgaactgacgggagggg, tttttaaaaaaagtggaa, aaaaaagttgttttaaa, agtcagaataaaaaaaag, aaatcaacaattctcaaa,
gtatagatcaaggagagt, tgtttggttttttgttgt, tgttgtttttgatgcgaa, actttgcaaacaatcta, gtcaatgcccaacagaaa, aacgtatcctgcttgaat,
tcctttaaaaagaaaagg, ccagtagttccaaaagtg, gaaatattaatacgggac, gggcagagggaaccctga, ctttggcgagtaagaaga, aaaaaattcttggagcaa,
tgagatggggaaaaaaag, agacgagtggctattaag, tagaaataaatttaagag, gaggaagtggagttcagt, gtgtcagtctcgggtgcg, gagtggcggatctgaact,
cggctcctccggccaaaa, gggaagagatgaaaaggt, cccggggctggcagctgg, cgaactgacgggaggggc
 Splice - Insert: 
agtgggcgggatggcatc, aaggtacccacagagcac, ctgggactgtctggagca, caagctgcccactgagcc, agagggtgttgtaacaac, tgggcagacagtttcgga,
gtgggcgggatggcatca, aggtacccacagagcacc, tgggactgtctggagcac, aagctgcccactgagcca, gagggtgttgtaacaact, gggcagacagtttcggag,
tgggcgggatggcatcaa, ggtacccacagagcacct, gggactgtctggagcaca, agctgcccactgagccag, agggtgttgtaacaactg, ggcagacagtttcggagg,
gggcgggatggcatcaag, gtacccacagagcacctg, ggactgtctggagcacaa, gctgcccactgagccaga, gggtgttgtaacaactgg, cagacagtttcggaggg,
ggcgggatggcatcaagg, tacccacagagcacctgg, gactgtctggagcacaag, ctgcccactgagccagag, ggtgttgtaacaactggg, cagacagtttcggagggg,
gcgggatggcatcaaggt, acccacagagcacctggg, actgtctggagcacaagc, tgcccactgagccagagg, gtgttgtaacaactgggc, agacagtttcggagggga,
cgggatggcatcaaggta, cccacagagcacctggga, ctgtctggagcacaagct, gcccactgagccagaggg, tgttgtaacaactgggca, gacagtttcggaggggaa,
gggatggcatcaaggtac, ccacagagcacctgggac, tgtctggagcacaagctg, cccactgagccagagggt, gttgtaacaactgggcag, acagtttcggaggggaag,
ggatggcatcaaggtacc, cacagagcacctgggact, gtctggagcacaagctgc, ccactgagccagagggtg, ttgtaacaactgggcaga, cagtttcggaggggaaga,
gatggcatcaaggtaccc, acagagcacctgggactg, tctggagcacaagctgcc, cactgagccagagggtgt, tgtaacaactgggcagac, agtttcggaggggaagaa,
atggcatcaaggtaccca, cagagcacctgggactgt, ctggagcacaagctgccc, actgagccagagggtgtt, gtaacaactgggcagaca, gtttcggaggggaagaag,
tggcatcaaggtacccac, agagcacctgggactgtc, tggagcacaagctgccca, ctgagccagagggtgttg, taacaactgggcagacag, tttcggaggggaagaagg,
ggcatcaaggtacccaca, gagcacctgggactgtct, ggagcacaagctgcccac, tgagccagagggtgttgt, aacaactgggcagacagt, gcatcaaggtacccacag,
agcacctgggactgtctg, gagcacaagctgcccact, gagccagagggtgttgta, acaactgggcagacagtt, catcaaggtacccacaga, gcacctgggactgtctgg,
agcacaagctgcccactg, agccagagggtgttgtaa, caactgggcagacagttt, atcaaggtacccacagag, cacctgggactgtctgga, gcacaagctgcccactga,
gccagagggtgttgtaac, aactgggcagacagtttc, tcaaggtacccacagagc, acctgggactgtctggag, cacaagctgcccactgag, ccagagggtgttgtaaca,
actgggcagacagtttcg
 Splice - end

aagaaaaaaataaacact, ggaagaatttattagtgt, taattatgtgaacaacga, caacaacaacaacaacaa, caaacaggaaaatcccat, taagtggagttgctgtac,
gtaccgttcctatcccgc, gcctcacttgatttttct, gtattgctatgcaatagg, caccccttcccattcttac, tcttagagttaacagtga, gttatttattgtgtgtta,
ctatataatgaacgtttc, attgcccttggaaaataa, aacaggtgtataaagtgg, agaccaaatactttgcca, gaaactcatggatggctt, aaggaacttgaactcaaa,
cgagccagaaaaaaagag, gtcatattaatgggatga, aaacccaagtgagttatt, atatgaccgagaaagtct, gcattaagataaagaccc, tgaaaacacatgttatgt,
atcagctgcctaaggaag, cttcttgtaaggtccaaa, aactaaaaagactgttaa, taaaagaaactttcagtc, agaaaaaaataaacactg, gaagaatttattagtgtt,
aattatgtgaacaacgac, aacaacaacaacaacaac, aaacaggaaaatcccatt, aagtggagttgctgtacg, taccgttcctatcccgcg, cctcacttgatttttctg,
tattgctatgcaataggc, accccttcccattcttact, cttagagttaacagtgag, ttatttattgtgtgttac, tatataatgaacgtttca, ttgcccttggaaaataaa,
acaggtgtataaagtgga, gaccaaatactttgccag, aaactcatggatggctta, aggaacttgaactcaaac, gagccagaaaaaaagagg, tcatattaatgggatgaa,
aacccaagtgagttatta, tatgaccgagaaagtctg, cattaagataaagaccct, gaaaacacatgttatgta, tcagctgcctaaggaagc, ttcttgtaaggtccaaaa,
actaaaaagactgttaat, aaaagaaactttcagtca, gaaaaaaataaacactgg, aagaatttattagtgtta, attatgtgaacaacgaca, acaacaacaacaacaaca,
aacaggaaaatcccatta, agtggagttgctgtacgt, accgttcctatcccgcgc, ctcacttgatttttctgt, attgctatgcaataggca, ccccttcccattcttactc,
ttagagttaacagtgagt, tatttattgtgtgttact, atataatgaacgtttcat, tgcccttggaaaataaaa, caggtgtataaagtggag, accaaatactttgccaga,
aactcatggatggcttaa, ggaacttgaactcaaacg, agccagaaaaaaagaggt, catattaatgggatgaaa, acccaagtgagttattat, atgaccgagaaagtctgc,
attaagataaagaccctg, aaaacacatgttatgtat, cagctgcctaaggaagct, tcttgtaaggtccaaaaa, ctaaaaagactgttaata, aaagaaactttcagtcag,
aaaaaaataaacactgga, agaatttattagtgttaa, ttatgtgaacaacgacaa, caacaacaacaacaacaa, acaggaaaatcccattaa, gtggagttgctgtacgta,

ccgttcctatcccgcgcc, tcacttgattttctgta, ttgctatgcaataggcac, ccttcccattcttactct, tagagttaacagtgagtt, atttattgtgtgttacta, tataatgaacgtttcatt,
gcccttggaaaataaaac, aggtgtataaagtggaga, ccaaatactttgccagaa, actcatggatggcttaag, gaacttgaactcaaacga, gccagaaaaaaagaggtc,
atattaatgggatgaaaa, cccaagtgagttattata, tgaccgagaaagtctgca, ttaagataaagaccctga, aaacacatgttatgtatc, agctgcctaaggaagctt,
cttgtaaggtccaaaaac, taaaaagactgttaataa, aaaaaataaacactggaa, gaatttattagtgttaat, tatgtgaacaacgacaac, aacaacaacaacaacaaa,
caggaaaatcccattaag, tggagttgctgtacgtac, cgttcctatcccgcgcct, cacttgattttctgtat, tgctatgcaataggcacc, cttcccattcttactctt,
agagttaacagtgagtta, tttattgtgtgttactat, ataatgaacgtttcattg, cccttggaaaataaaaca, ggtgtataaagtggagac, caaatactttgccagaaa,
ctcatggatggcttaagg, aacttgaactcaaacgag, ccagaaaaaaagaggtca, tattaatgggatgaaaac, ccaagtgagttattatat, gaccgagaaagtctgcat,
taagataaagaccctgaa, aacacatgttatgtatca, gctgcctaaggaagcttc, ttgtaaggtccaaaaact, aaaaagactgttaataaa, aaaaataaacactggaag,
aatttattagtgttaatt, atgtgaacaacgacaaca, acaacaacaacaacaaac, aggaaaatcccattaagt, ggagttgctgtacgtacc, gttcctatcccgcgcctc,
acttgattttctgtatt, gctatgcaataggcaccc, ttcccattcttactctta, gagttaacagtgagttat, ttattgtgtgttactata, taatgaacgtttcattgc,
ccttggaaaataaaacag, gtgtataaagtggagacc, aaatactttgccagaaac, tcatggatggcttaagga, acttgaactcaaacgagc, cagaaaaaaagaggtcat,
attaatgggatgaaaacc, caagtgagttattatatg, accgagaaagtctgcatt, aagataaagaccctgaaa, acacatgttatgtatcag, ctgcctaaggaagcttct,
tgtaaggtccaaaaacta, aaaagactgttaataaaa, aaaataaacactggaaga, atttattagtgttaatta, tgtgaacaacgacaacaa, caacaacaacaacaaaca,
ggaaaatcccattaagtg, gagttgctgtacgtaccg, ttcctatcccgcgcctca, cttgattttctgtattg, ctatgcaataggcaccct, tcccattcttactcttag,
agttaacagtgagttatt, tattgtgtgttactatat, aatgaacgtttcattgcc, cttggaaaataaaacagg, tgtataaagtggagacca, aatactttgccagaaact,
catggatggcttaaggaa, cttgaactcaaacgagcc, agaaaaaaagaggtcata, ttaatgggatgaaaaccc, aagtgagttattatatga, ccgagaaagtctgcatta,
agataaagaccctgaaaa, cacatgttatgtatcagc, tgcctaaggaagcttctt, gtaaggtccaaaaactaa, aaagactgttaataaaag, aaataaacactggaagaa,
tttattagtgttaattat, gtgaacaacgacaacaac, aacaacaacaacaaacag, gaaaatcccattaagtgg, agttgctgtacgtaccgt, tcctatcccgcgcctcac,
ttgattttctgtattgc, tatgcaataggcaccctt, cccattcttactcttaga, gttaacagtgagttattt, attgtgtgttactatata, atgaacgtttcattgccc,
ttggaaaataaaacaggt, gtataaagtggagaccaa, atactttgccagaaactc, atggatggcttaaggaac, ttgaactcaaacgagcca, gaaaaaaagaggtcatat,
taatgggatgaaaaccca, agtgagttattatatgac, cgagaaagtctgcattaa, gataaagaccctgaaaac, acatgttatgtatcagct, gcctaaggaagcttcttg,
taaggtccaaaaactaaa, aagactgttaataaaaga, aataaacactggaagaat, ttattagtgttaattatg, tgaacaacgacaacaaca, acaacaacaacaaacagg,
aaaatcccattaagtgga, gttgctgtacgtaccgtt, cctatcccgcgcctcact, tgattttctgtattgct, atgcaataggcacccttc, ccattcttactcttagag,
ttaacagtgagttattta, ttgtgtgttactatataa, tgaacgtttcattgccct, tggaaaataaaacaggtg, tataaagtggagaccaaa, tactttgccagaaactca,
tggatggcttaaggaact, tgaactcaaacgagccag, aaaaaaagaggtcatatt, aatgggatgaaaacccaa, gtgagttattatatgacc, gagaaagtctgcattaag,
ataaagaccctgaaaaca, catgttatgtatcagctg, cctaaggaagcttcttgt, aaggtccaaaaactaaaa, agactgttaataaaagaa, ataaacactggaagaatt,
tattagtgttaattatgt, gaacaacgacaacaacaa, caacaacaacaaacagga, aaatcccattaagtggag, ttgctgtacgtaccgttc, ctatcccgcgcctcactt,
gattttctgtattgcta, tgcaataggcacccttcc, cattcttactcttagagt, taacagtgagttatttat, tgtgtgttactatataat, gaacgtttcattgccctt,
ggaaaataaaacaggtgt, ataaagtggagaccaaat, actttgccagaaactcat, ggatggcttaaggaactt, gaactcaaacgagccaga, aaaaaagaggtcatatta,
atgggatgaaaacccaag, tgagttattatatgaccg, agaaagtctgcattaaga, taaagaccctgaaaacac, atgttatgtatcagctgc, ctaaggaagcttcttgta,
aggtccaaaaactaaaaa, gactgttaataaaagaaa, taaacactggaagaattt, attagtgttaattatgtg, aacaacgacaacaacaac, aacaacaacaaacaggaa,
aatcccattaagtggagt, tgctgtacgtaccgttcc, tatcccgcgcctcacttg, attttctgtattgctat, gcaataggcacccttccc, attcttactcttagagtt,
aacagtgagttatttatt, gtgtgttactatataatg, aacgtttcattgcccttg, gaaaataaaacaggtgta, taaagtggagaccaaata, ctttgccagaaactcatg,
gatggcttaaggaacttg, aactcaaacgagccagaa, aaaaagaggtcatattaa, tgggatgaaaacccaagt, gagttattatatgaccga, gaaagtctgcattaagat,
aaagaccctgaaaacaca, tgttatgtatcagctgcc, taaggaagcttcttgtaa, ggtccaaaaactaaaaag, actgttaataaaagaaac, aaacactggaagaattta,
ttagtgttaattatgtga, acaacgacaacaacaaca, acaacaacaaacaggaaa, atcccattaagtggagtt, gctgtacgtaccgttcct, atcccgcgcctcacttga,
ttttctgtattgctatg, caataggcacccttccca, ttcttactcttagagtta, acagtgagttatttattg, tgtgttactatataatga, acgtttcattgcccttgg,
aaaataaaacaggtgtat, aaagtggagaccaaatac, tttgccagaaactcatgg, atggcttaaggaacttga, actcaaacgagccagaaa, aaaagaggtcatattaat,
gggatgaaaacccaagtg, agttattatatgaccgag, aaagtctgcattaagata, aagaccctgaaaacacat, gttatgtatcagctgcct, aaggaagcttcttgtaag,
gtccaaaaactaaaaaga, ctgttaataaaagaaact, aacactggaagaatttat, tagtgttaattatgtgaa, caacgacaacaacaacaa, caacaacaaacaggaaaa,
tcccattaagtggagttg, ctgtacgtaccgttccta, tcccgcgcctcacttgat, tttctgtattgctatgc, aataggcacccttcccat, tcttactcttagagttaa,
cagtgagttatttattgt, gtgttactatataatgaa, cgtttcattgcccttgga, aaataaaacaggtgtata, aagtggagaccaaatact, ttgccagaaactcatgga,
tggcttaaggaacttgaa, ctcaaacgagccagaaaa, aaagaggtcatattaatg, ggatgaaaacccaagtga, gttattatatgaccgaga, aagtctgcattaagataa,
agaccctgaaaacacatg, ttatgtatcagctgccta, aggaagcttcttgtaagg, tccaaaaactaaaaagac, tgttaataaaagaaactt, acactggaagaatttatt,
agtgttaattatgtgaac, aacgacaacaacaacaac, aacaacaaacaggaaaat, cccattaagtggagttgc, tgtacgtaccgttcctat, cccgcgcctcacttgatt,
ttctgtattgctatgca, ataggcacccttcccatt, cttactcttagagttaac, agtgagttatttattgtg, tgttactatataatgaac, gtttcattgcccttggaa,
aataaaacaggtgtataa, agtggagaccaaatactt, tgccagaaactcatggat, ggcttaaggaacttgaac, tcaaacgagccagaaaaa, aagaggtcatattaatgg,
gatgaaaacccaagtgag, ttattatatgaccgagaa, agtctgcattaagataaa, gaccctgaaaacacatgt, tatgtatcagctgcctaa, ggaagcttcttgtaaggt,
ccaaaaactaaaaagact, gttaataaaagaaacttt, cactggaagaatttatta, gtgttaattatgtgaaca, acgacaacaacaacaaca, acaacaaacaggaaaatc,
ccattaagtggagttgct, gtacgtaccgttcctatc, ccgcgcctcacttgattt, tctgtattgctatgcaa, taggcacccttcccattc, ttactcttagagttaaca,
gtgagttatttattgtgt, gttactatataatgaacg, tttcattgcccttggaaa, ataaaacaggtgtataaa, gtggagaccaaatacttt, gccagaaactcatggatg,
gcttaaggaacttgaact, caaacgagccagaaaaaa, agaggtcatattaatggg, atgaaaacccaagtgagt, tattatatgaccgagaaa, gtctgcattaagataaag,
accctgaaaacacatgtt, atgtatcagctgcctaag, gaagcttcttgtaaggtc, caaaaactaaaaagactg, ttaataaaagaaactttc, actggaagaatttattag,
tgttaattatgtgaacaa, cgacaacaacaacaacaa, caacaaacaggaaaatcc, cattaagtggagttgctg, tacgtaccgttcctatcc, cgcgcctcacttgatttt,
tctgtattgctatgcaat, aggcacccttcccattct, tactcttagagttaacag, tgagttatttattgtgtg, ttactatataatgaacgt, ttcattgcccttggaaaa,
taaaacaggtgtataaag, tggagaccaaatactttg, ccagaaactcatggatgg, cttaaggaacttgaactc, aaacgagccagaaaaaaa, gaggtcatattaatggga,
tgaaaacccaagtgagtt, attatatgaccgagaaag, tctgcattaagataaaga, ccctgaaaacacatgtta, tgtatcagctgcctaagg, aagcttcttgtaaggtcc,
aaaaactaaaaagactgt, taataaaagaaactttca, ctggaagaatttattagt, gttaattatgtgaacaac, gacaacaacaacaacaac, aacaaacaggaaaatccc,
attaagtggagttgctgt, acgtaccgttcctatccc, gcgcctcacttgattttt, ctgtattgctatgcaata, ggcacccttcccattctt, actcttagagttaacagt,
gagttatttattgtgtgt, tactatataatgaacgtt, tcattgcccttggaaaat, aaaacaggtgtataaagt, ggagaccaaatactttgc, cagaaactcatggatggc,
ttaaggaacttgaactca, aacgagccagaaaaaaag, aggtcatattaatgggat, gaaaacccaagtgagtta, ttatatgaccgagaaagt, ctgcattaagataaagac,
cctgaaaacacatgttat, gtatcagctgcctaagga, agcttcttgtaaggtcca, aaaactaaaaagactgtt, aataaaagaaactttcag

### Example 21

TGF-beta3 antisense oligonucleotides:

caggatgccccaaaaata, tttatttatacaaagatt, ttgagagtaatattcata, cttgtctttatacctcag, tctatgcgtctggggcca, agtcactgtgtggcacat,
gtcgagcttccccgaatg, cctcacatgttgtcgcac, ctgcttccaggaacacca, aatgaacacagggtcttg, gaggggaagtgggggaag, aacccataatgccccaac,
cctgcatggaaccacaat, ccagaaatgtgcatcctg, acctggaaggcgtctaac, caagtgtccaaggggaaa, tatgatcgagggagaggt,
gagaggagggacccagag, gcagacaggagagggttg, atttccaccctttcttct, gcgttcagcatatccaaa, aggcccaatacagttgat, gggccaggaactgcatga,
cctggatttctccctgt, agtgacccacgatgttaa, ttgatgtagaggacagtt, tgcaaaagtaatagattt, gcccttaatcccagacag, tatgagatacaattctgg,
gactttgtcttcgtaacc, tgtctttaaaaaaaaaaa, aaaatgcttgccttgtat, aacataatccagattccc, tagagcagatgtggtaca, gcaatgagcaaatccaac,
ctcagatctgaagtgtct, tccagtctggccctgacc, cagccattctctgccctt, ccttctccctttagggta, gcccaaatcccattgcca, cacaacatctcaacttac,
catccctttcctctatcc, ccatcccctctgtctgcg, tcacagaaagtctgtgtg, ttctgaagagttcagcct, tcctctaaccaaacccac, actttctttaccaccgtg,
attctcagagccagcaag, aaagaaatgttccaaaag, gaaacctccatctcagcc, atttgcccggagccgaag, gttgtgggctccaggcct, ctcagtgaggtttgttgc,
ttgtgtgtttcccgagga, gcgggcagtcaggcagtg, gtggttctctctcccctc, tctctgtcgcacgtgggg, tctcagctacatttacaa, gacttcaccaccatgttg,
gagagctgctccactttg, ggggtcctcccaacatag, tacaggatggtcaggggc, tccaggtcctggggcacg, cagcaaggcgaggcagat, gcttcagggttcagagtg,
ttgtacagtcccagcacc, gtgctgtgggttgtgtct, gcactgcggaggtatggg, caagggcctgagcagaag, ttggcatagtagccctta, ggttcatggacccacttc,
cagcccagatcctgtcgg, aagtcaatgtagaggggg, cgcacacagcagttctcc, tccaagttgcggaagcag, taattggtgtccaaagcc, cgcttcttcctctgaccc,
ccctggcccgggttgtcg, agccggtgtgggggaatc, atcatgaggattagatga, gggttgtggtgatccttc, tgcttcttgaggcgcccc, agatctccacggccatgg,
tcatcctcattgtccacg, cctttgaatttgatttcc, atcacctcgtgaatgttt, tccaggatatctccattg, ggctgaaaggtgtgacat, ggacagtgaatgctgatt,
tctagacctaagttggac, tctcttctcaacagccac, tcacgcacagtgtcagtg, acatcaaaggacagccac, tcggcagtgccccgtgtg, ggcagattcttgccaccg,
atatagcgctgtttggca, atgtgctcatctggccga, aggatctggaagagctcg, atcctctgctcattccgc, ttagagctggggttgggc, acccgcaagacccggaat,
tctgctcggaataggttg, gttctattttctccact, gaggacacattgaagcgg, aaaaccttggaggtaatt, cctttagggcagacagcc, agttcgttgtgctccgcc,
agcccctggatcatgtcg, aatttatggatttctttg, gcatagtattccgactcg, gtgttttcctgggtgcag, ccttcctccctctcccca, tgcatctcctccagcagc,
tcccgggtgctgttgtaa, agggccaggacctgatag, gggacgtgggtcatcacc, gttggctcaggggggctg, gtgagcctgagcttgctc, aagatctgtcccctaatg,
gcttccaccctcttcttc, ttgatgtggccgaagtcc, aaggtggtgcaagtggac, agagagaggctgaccgtg, gcaaagttcagcagggcc, aggaccaccagagccctt,
tgcaagtgcatcttcatg, tgtgagctgggaagagag, gccaggggacggcaagg, cctggagaggaagagacc, ccagcagacgtgcagaag,
gagggaggaaaaccaggc, ggcctccccagatcccaa, agactgaggcttggcaag, aaggtgcatgaactcact, gcactgcgagagcttcag,
gacttccaggaagcgctg, gcaaccctgaggacgaag, aagcggactgtgtgcctt, gtagcgctgggattcttg, tccatgtgtctaaacagg, aggatgccccaaaaatat,
ttatttatacaaagattt, tgagagtaatattcatac, ttgtctttatacctcagt, ctatgcgtctggggccaa, gtcactgtgtggcacatg, tcgagcttccccgaatgc,
ctcacatgttgtcgcacc, tgcttccaggaacaccaa, atgaacacagggtcttgg, aggggaagtgggggaaga, acccataatgccccaacc, ctgcatggaaccacaatc,
cagaaatgtgcatcctga, cctggaaggcgtctaacc, aagtgtccaaggggaaat, atgatcgagggagaggtg, agaggagggacccagagg,
cagacaggagagggttga, tttccaccctttcttctg, cgttcagcatatccaaaa, ggcccaatacagttgatg, ggccaggaactgcatgac, ctggatttctccctgta,
gtgacccacgatgttaat, tgatgtagaggacagttt, gcaaaagtaatagatttg, cccttaatcccagacagt, atgagatacaattctggg, actttgtcttcgtaacct,
gtctttaaaaaaaaaaaa, aaatgcttgccttgtata, acataatccagattccct, agagcagatgtggtacag, caatgagcaaatccaacc, tcagatctgaagtgtctt,
ccagtctggccctgaccc, agccattctctgcccttc, cttctccctttagggtag, cccaaatcccattgccac, acaacatctcaacttacc, atccctttcctctatccc,
catcccctctgtctgcgt, cacagaaagtctgtgtgt, tctgaagagttcagcctt, cctctaaccaaacccaca, ctttctttaccaccgtga, ttctcagagccagcaaga,
aagaaatgttccaaaagg, aaacctccatctcagcca, tttgcccggagccgaagg, ttgtgggctccaggcctc, tcagtgaggtttgttgct, tgtgtgtttcccgaggag,
cgggcagtcaggcagtgg, tggttctctctcccctct, ctctgtcgcacgtggggt, ctcagctacatttacaag, acttcaccaccatgttgg, agagctgctccactttgg,
gggtcctcccaacatagt, acaggatggtcaggggct, ccaggtcctggggcacgc, agcaaggcgaggcagatg, cttcagggttcagagtgt, tgtacagtcccagcaccg,
tgctgtgggttgtgtctg, cactgcggaggtatgggc, aagggcctgagcagaagt, tggcatagtagcccttag, gttcatggacccacttcc, agcccagatcctgtcgga,
agtcaatgtagagggggc, gcacacagcagttctcct, ccaagttgcggaagcagt, aattggtgtccaaagccc, gcttcttcctctgacccc, cctggcccgggttgtcga,
gccggtgtgggggaatca, tcatgaggattagatgag, ggttgtggtgatccttct, gcttcttgaggcgcccca, gatctccacggccatggt, catcctcattgtccacgc,
ctttgaatttgatttcca, tcacctcgtgaatgtttt, ccaggatatctccattgg, gctgaaaggtgtgacatg, gacagtgaatgctgattt, ctagacctaagttggact,
ctcttctcaacagccact, cacgcacagtgtcagtga, catcaaaggacagccact, cggcagtgccccgtgtgg, gcagattcttgccaccga, tatagcgctgtttggcaa,
tgtgctcatctggccgaa, ggatctggaagagctcga, tcctctgctcattccgct, tagagctggggttgggca, cccgcaagacccggaatt, ctgctcggaataggttgg,
ttctattttctccactg, aggacacattgaagcgga, aaaccttggaggtaattc, ctttagggcagacagcca, gttcgttgtgctccgcca, gcccctggatcatgtcga,
atttatggatttctttgg, catagtattccgactcgg, tgttttcctgggtgcagc, cttcctccctctccccat, gcatctcctccagcagct, cccgggtgctgttgtaaa,
gggccaggacctgatagg, ggacgtgggtcatcaccg, ttggctcaggggggctgg, tgagcctgagcttgctca, agatctgtcccctaatgg, cttccaccctcttcttct,
tgatgtggccgaagtcca, aggtggtgcaagtggaca, gagagaggctgaccgtgg, caaagttcagcagggcca, ggaccaccagagcccttt, gcaagtgcatcttcatgt,
gtgagctgggaagagagg, ccaggggacggcaaggc, ctggagaggaagagaccc, cagcagacgtgcagaagg, agggaggaaaaccaggcg,
gcctccccagatcccaaa, gactgaggcttggcaaga, aggtgcatgaactcactg, cactgcgagagcttcagg, acttccaggaagcgctgg,
caaccctgaggacgaaga, agcggactgtgtgccttg, tagcgctgggattcttgt, ccatgtgtctaaacaggt, ggatgccccaaaaatatt, tatttatacaaagatttt,
gagagtaatattcatact, tgtctttatacctcagtc, tatgcgtctggggccaag, tcactgtgtggcacatgt, cgagcttccccgaatgcc, tcacatgttgtcgcacct,
gcttccaggaacaccaaa, tgaacacagggtcttgga, ggggaagtgggggaagaa, cccataatgccccaaccc, tgcatggaaccacaatcc,
agaaatgtgcatcctgac, ctggaaggcgtctaacca, agtgtccaaggggaaata, tgatcgagggagaggtga, gaggagggacccagaggc,
agacaggagagggttgat, ttccaccctttcttctgc, gttcagcatatccaaaag, gcccaatacagttgatgg, gccaggaactgcatgacc, tggatttctccctgtag,
tgacccacgatgttaatt, gatgtagaggacagtttg, caaaagtaatagatttgc, ccttaatcccagacagta, tgagatacaattctggga, ctttgtcttcgtaacctg,
tctttaaaaaaaaaaaaa, aatgcttgccttgtataa, cataatccagattcccta, gagcagatgtggtacagc, aatgagcaaatccaacct, cagatctgaagtgtcttc,
cagtctggccctgaccca, gccattctctgcccttcc, ttctccctttagggtagc, ccaaatcccattgccaca, caacatctcaacttacca, tccctttcctctatcccc,
atcccctctgtctgcgtc, acagaaagtctgtgtgtt, ctgaagagttcagccttc, ctctaaccaaacccacac, tttctttaccaccgtgat, tctcagagccagcaagaa,
agaaatgttccaaaagga, aacctccatctcagccat, ttgcccggagccgaaggt, tgtgggctccaggcctct, cagtgaggtttgttgctt, gtgtgtttcccgaggagc,
gggcagtcaggcagtggt, ggttctctctcccctctc, tctgtcgcacgtggggtc, tcagctacatttacaaga, cttcaccaccatgttgga, gagctgctccactttggg,
ggtcctcccaacatagta, caggatggtcaggggctc, caggtcctggggcacgca, gcaaggcgaggcagatgc, ttcagggttcagagtgtt, gtacagtcccagcaccgt,
gctgtgggttgtgtctgc, actgcggaggtatgggca, agggcctgagcagaagtt, ggcatagtagcccttagg, ttcatggacccacttcca, gcccagatcctgtcggaa,
gtcaatgtagagggggcg, cacacagcagttctcctc, caagttgcggaagcagta, attggtgtccaaagcccg, cttcttcctctgaccccc, ctggcccgggttgtcgag,
ccggtgtgggggaatcat, catgaggattagatgagg, gttgtggtgatccttctg, cttcttgaggcgccccag, atctccacggccatggtc, atcctcattgtccacgcc,
tttgaatttgatttccat, cacctcgtgaatgttttc, caggatatctccattggg, ctgaaaggtgtgacatgg, acagtgaatgctgatttc, tagacctaagttggactc,
tcttctcaacagccactc, acgcacagtgtcagtgac, atcaaaggacagccactc, ggcagtgccccgtgtggg, cagattcttgccaccgat, atagcgctgtttggcaat,
gtgctcatctggccgaag, gatctggaagagctcgat, cctctgctcattccgctt, agagctggggttgggcac, ccgcaagacccggaattc, tgctcggaataggttggt,
tctattttctccactga, ggacacattgaagcggaa, aaccttggaggtaattcc, tttagggcagacagccag, ttcgttgtgctccgccag, cccctggatcatgtcgaa,
tttatggatttctttggc, atagtattccgactcggt, gttttcctgggtgcagcc, ttcctccctctccccatg, catctcctccagcagctc, ccgggtgctgttgtaaag,
ggccaggacctgataggg, gacgtgggtcatcaccgt, tggctcaggggggctggt, gagcctgagcttgctcaa, gatctgtcccctaatggc, ttccaccctcttcttctt,
gatgtggccgaagtccaa, ggtggtgcaagtggacag, agagaggctgaccgtggc, aaagttcagcagggccag, gaccaccagagccctttg, caagtgcatcttcatgtg,
tgagctgggaagagaggc, caggggacggcaaggcc, tggagaggaagagacccc, agcagacgtgcagaagga, gggaggaaaaccaggcgg,
cctccccagatcccaaag, actgaggcttggcaagaa, ggtgcatgaactcactgc, actgcgagagcttcagga, cttccaggaagcgctggc,
aaccctgaggacgaagaa, gcggactgtgtgccttgt, agcgctgggattcttgtc, catgtgtctaaacaggtt, gatgccccaaaaatattt, atttatacaaagatttg,
agagtaatattcatactt, gtctttatacctcagtct, atgcgtctggggccaagt, cactgtgtggcacatgtc, gagcttccccgaatgcct, cacatgttgtcgcacctg,

cttccaggaacaccaaat, gaacacagggtcttggag, gggaagtggggaagaac, ccataatgccccaaccct, gcatggaaccacaatcca,
gaaatgtgcatcctgacc, tggaaggcgtctaaccaa, gtgtccaaggggaaatat, gatcgagggagaggtgag, aggagggacccagaggca,
gacaggagagggttgatt, tccaccctttcttctgcg, ttcagcatatccaaaagg, cccaatacagttgatggg, ccaggaactgcatgacct, ggattttctccctgtagt,
gacccacgatgttaattg, atgtagaggacagtttgc, aaaagtaatagatttgcc, cttaatcccagacagtat, gagatacaattctgggac, tttgtcttcgtaacctgt,
ctttaaaaaaaaaaaaaa, atgcttgccttgtataac, ataatccagattccctag, agcagatgtggtacagca, atgagcaaatccaacctc, agatctgaagtgtcttcc,
agtctggccctgacccag, ccattctctgcccttcct, tctccctttagggtagcc, caaatcccattgccacac, aacatctcaacttaccat, cccttcctctatcccca,
tcccctctgtctgcgtca, cagaaagtctgtgtgttc, tgaagagttcagccttcc, tctaaccaaacccacact, ttctttaccaccgtgatt, ctcagagccagcaagaaa,
gaaatgttccaaaaggaa, acctccatctcagccatt, tgcccggagccgaaggtt, gtgggctccaggcctctc, agtgaggtttgttgcttg, tgtgtttcccgaggagcg,
ggcagtcaggcagtggtg, gttctctctcccctctct, ctgtcgcacgtggggtct, cagctacatttacaagac, ttcaccaccatgttggag, agctgctccactttgggg,
gtcctcccaacatagtac, aggatggtcaggggctcc, aggtcctggggcacgcag, caaggcgaggcagatgct, tcagggttcagagtgttg, tacagtcccagcaccgtg,
ctgtgggttgtgtctgca, ctgcggaggtatgggcaa, gggcctgagcagaagttg, gcatagtagcccttaggt, tcatggacccacttccag, cccagatcctgtcggaag,
tcaatgtagagggggcgc, acacagcagttctcctcc, aagttgcggaagcagtaa, ttggtgtccaaagcccgc, ttcttcctctgacccccc, tggcccgggttgtcgagc,
cggtgtgggggaatcatc, atgaggattagatgaggg, ttgtggtgatccttctgc, ttcttgaggcgccccaga, tctccacggccatggtca, tcctcattgtccacgcct,
ttgaatttgatttccatc, acctcgtgaatgttttcc, aggatatctccattgggc, tgaaaggtgtgacatgga, cagtgaatgctgatttct, agacctaagttggactct,
cttctcaacagccactca, cgcacagtgtcagtgaca, tcaaaggacagccactcg, gcagtgccccgtgtgggc, agattcttgccaccgata, tagcgctgtttggcaatg,
tgctcatctggccgaagg, atctggaagagctcgatc, ctctgctcattccgctta, gagctggggttgggcacc, cgcaagacccggaattct, gctcggaataggttggtt,
ctatttttctccactgag, gacacattgaagcggaaa, accttggaggtaattcct, ttagggcagacagccagt, tcgttgtgctccgccagc, ccctggatcatgtcgaat,
ttatggatttcttggca, tagtattccgactcggtg, ttttcctgggtgcagcct, tcctccctctccccatgc, atctcctccagcagctcc, cgggtgctgttgtaaagg,
gccaggacctgatagggg, acgtgggtcatcaccgtt, ggctcaggggggctggtg, agcctgagcttgctcaag, atctgtcccctaatggct, tccaccctcttcttcttg,
atgtggccgaagtccaag, gtggtgcaagtggacaga, gagaggctgaccgtggca, aagttcagcagggccagg, accaccagagccctttgc, aagtgcatcttcatgtgt,
gagctgggaagagaggcc, agggggacggcaaggcct, ggagaggaagagacccca, gcagacgtgcagaaggag, ggaggaaaaccaggcggc,
ctccccagatcccaaaga, ctgaggcttggcaagaag, gtgcatgaactcactgca, ctgcgagagcttcaggac, ttccaggaagcgctggca,
accctgaggacgaagaag, cggactgtgtgccttgta, gcgctgggattcttgtcc, atgtgtctaaacaggttt, atgccccaaaaatattta, tttatacaaagatttga,
gagtaatattcatacttg, tctttatacctcagtcta, tgcgtctggggccaagtc, actgtgtggcacatgtcg, agcttccccgaatgcctc, acatgttgtcgcacctgc,
ttccaggaacaccaaatg, aacacagggtcttggagg, ggaagtggggaagaacc, cataatgccccaaccctg, catggaaccacaatccag,
aaatgtgcatcctgacct, ggaaggcgtctaaccaag, tgtccaaggggaaatatg, atcgagggagaggtgaga, ggagggacccagaggcag,
acaggagagggttgattt, ccaccctttcttctgcgt, tcagcatatccaaaaggc, ccaatacagttgatgggc, caggaactgcatgacctg, gattttctccctgtagtg,
acccacgatgttaattga, tgtagaggacagtttgca, aaagtaatagatttgccc, ttaatcccagacagtatg, agatacaattctgggact, ttgtcttcgtaacctgtc,
tttaaaaaaaaaaaaaaa, tgcttgccttgtataaca, taatccagattccctaga, gcagatgtggtacagcaa, tgagcaaatccaacctca, gatctgaagtgtcttcca,
gtctggccctgacccagc, cattctctgcccttcctt, ctccctttagggtagccc, aaatcccattgccacaca, acatctcaacttaccatc, cctttcctctatccccat,
cccctctgtctgcgtcac, agaaagtctgtgtgttct, gaagagttcagccttcct, ctaaccaaacccacactt, tctttaccaccgtgattc, tcagagccagcaagaaag,
aaatgttccaaaaggaaa, cctccatctcagccattt, gcccggagccgaaggttg, tgggctccaggcctctca, gtgaggtttgttgcttgt, gtgtttcccgaggagcgg,
gcagtcaggcagtggtgg, ttctctctcccctctctc, tgtcgcacgtggggtctc, agctacatttacaagact, tcaccaccatgttggaga, gctgctccactttggggg,
tcctcccaacatagtaca, ggatggtcaggggctcca, ggtcctggggcacgcagc, aaggcgaggcagatgctt, cagggttcagagtgttgt, acagtcccagcaccgtgc,
tgtgggttgtgtctgcac, tgcggaggtatgggcaag, ggcctgagcagaagttgg, catagtagcccttaggtt, catggacccacttccagc, ccagatcctgtcggaagt,
caatgtagagggggcgca, cacagcagttctcctcca, agttgcggaagcagtaat, tggtgtccaaagcccgct, tcttcctctgacccccct, ggcccgggttgtcgagcc,
ggtgtgggggaatcatca, tgaggattagatgagggt, tgtggtgatccttctgct, tcttgaggcgccccagat, ctccacggccatggtcat, cctcattgtccacgcctt,
tgaatttgatttccatca, cctcgtgaatgttttcca, ggatatctccattgggct, gaaaggtgtgacatggac, agtgaatgctgatttcta, gacctaagttggactctc,
ttctcaacagccactcac, gcacagtgtcagtgacat, caaaggacagccactcgg, cagtgccccgtgtgggca, gattcttgccaccgatat, agcgctgtttggcaatgt,
gctcatctggccgaagga, tctggaagagctcgatcc, tctgctcattccgcttag, agctggggttgggcaccc, gcaagacccggaattctg, ctcggaataggttggttc,
tatttttctccactgagg, acacattgaagcggaaaa, ccttggaggtaattcctt, tagggcagacagccagtt, cgttgtgctccgccagcc, cctggatcatgtcgaatt,
tatggatttcttggcat, agtattccgactcggtgt, tttcctgggtgcagcctt, cctccctctccccatgca, tctcctccagcagctccc, gggtgctgttgtaaaggg,
ccaggacctgatagggga, cgtgggtcatcaccgttg, gctcaggggggctggtga, gcctgagcttgctcaaga, tctgtcccctaatggctt, ccaccctcttcttcttga,
tgtggccgaagtccaagg, tggtgcaagtggacagag, agaggctgaccgtggcaa, agttcagcagggccagga, ccaccagagccctttgca, agtgcatcttcatgtgtg,
agctgggaagagaggcca, gggggacggcaaggcctg, gagaggaagagaccccag, cagacgtgcagaaggagg, gaggaaaaccaggcggcc,
tccccagatcccaaagac, tgaggcttggcaagaagg, tgcatgaactcactgcac, tgcgagagcttcaggact, tccaggaagcgctggcaa,
ccctgaggacgaagaagc, ggactgtgtgccttgtag, cgctgggattcttgtcca, tgtgtctaaacaggtttt, tgccccaaaaatatttat, ttatacaaagattttgag,
agtaatattcatacttgt, ctttatacctcagtctat, gcgtctggggccaagtca, ctgtgtggcacatgtcga, gcttccccgaatgcctca, catgttgtcgcacctgct,
tccaggaacaccaaatga, acacagggtcttggaggg, gaagtggggaagaaccc, ataatgccccaaccctgc, atggaaccacaatccaga,
aatgtgcatcctgacctg, gaaggcgtctaaccaagt, gtccaaggggaaatatga, tcgagggagaggtgagag, gagggacccagaggcaga,
caggagagggttgatttc, caccctttcttctgcgtt, cagcatatccaaaaggcc, caatacagttgatgggcc, aggaactgcatgacctgg, attttctccctgtagtga,
cccacgatgttaattgat, gtagaggacagtttgcaa, aagtaatagatttgccct, taatcccagacagtatga, gatacaattctgggactt, tgtcttcgtaacctgtct,
ttaaaaaaaaaaaaaaat, gcttgccttgtataacat, aatccagattccctagag, cagatgtggtacagcaat, gagcaaatccaacctcag, atctgaagtgtcttccag,
tctggccctgacccagcc, attctctgcccttccttc, tccctttagggtagccca, aatcccattgccacacaa, catctcaacttaccatcc, ctttcctctatccccatc,
ccctctgtctgcgtcaca, gaaagtctgtgtgttctg, aagagttcagccttcctc, taaccaaacccacacttt, ctttaccaccgtgattct, cagagccagcaagaaaga,
aatgttccaaaaggaaac, ctccatctcagccatttg, cccggagccgaaggttgt, gggctccaggcctctcag, tgaggtttgttgcttgtg, tgtttcccgaggagcggg,
cagtcaggcagtggtggt, tctctctcccctctctct, gtcgcacgtggggtctca, gctacatttacaagactt, caccaccatgttggagag, ctgctccactttgggggt,
cctcccaacatagtacag, gatggtcaggggctccag, gtcctggggcacgcagca, aggcgaggcagatgcttc, agggttcagagtgttgta, cagtcccagcaccgtgct,
gtgggttgtgtctgcact, gcggaggtatgggcaagg, gcctgagcagaagttggc, atagtagcccttaggttc, atggacccacttccagcc, cagatcctgtcggaagtc,
aatgtagagggggcgcac, acagcagttctcctccaa, gttgcggaagcagtaatt, ggtgtccaaagcccgctt, cttcctctgacccccctg, gcccgggttgtcgagccg,
gtgtgggggaatcatcat, gaggattagatgagggtt, gtggtgatccttctgctt, cttgaggcgccccagatc, tccacggccatggtcatc, ctcattgtccacgccttt,
gaatttgatttccatcac, ctcgtgaatgttttccag, gatatctccattgggctg, aaaggtgtgacatggaca, gtgaatgctgatttctag, acctaagttggactctct,
tctcaacagccactcacg, cacagtgtcagtgacatc, aaaggacagccactcggc, agtgccccgtgtgggcag, attcttgccaccgatata, gcgctgtttggcaatgtg,
ctcatctggccgaaggat, ctggaagagctcgatcct, ctgctcattccgcttaga, gctggggttgggcacccg, caagacccggaattctgc, tcggaataggttggttct,
atttttctccactgagga, cacattgaagcggaaaac, cttggaggtaattccttt, agggcagacagccagttc, gttgtgctccgccagccc, ctggatcatgtcgaattt,
atggatttcttggcata, gtattccgactcggtgtt, ttcctgggtgcagccttc, ctccctctccccatgcat, ctcctccagcagctcccg, ggtgctgttgtaaagggc,
caggacctgataggggac, gtgggtcatcaccgttgg, ctcaggggggctggtgag, cctgagcttgctcaagat, ctgtcccctaatggcttc, caccctcttcttcttgat,
gtggccgaagtccaaggt, ggtgcaagtggacagaga, gaggctgaccgtggcaaa, gttcagcagggccaggac, caccagagccctttgcaa, gtgcatcttcatgtgtga,
gctgggaagagaggccag, ggggacggcaaggcctgg, agaggaagagaccccagc, agacgtgcagaaggaggg, aggaaaaccaggcggcct,
ccccagatcccaaagact, gaggcttggcaagaaggt, gcatgaactcactgcact, gcgagagcttcaggactt, ccaggaagcgctggcaac,
cctgaggacgaagaagcg, gactgtgtgccttgtagc, gctgggattcttgtccat, gtgtctaaacaggttttg, gccccaaaaatatttatt, tatacaaagattttgaga,
gtaatattcatacttgtc, tttatacctcagtctatg, cgtctggggccaagtcac, tgtgtggcacatgtcgag, cttccccgaatgcctcac, atgttgtcgcacctgctt,
ccaggaacaccaaatgaa, cacagggtcttggagggg, aagtggggaagaaccca, taatgccccaaccctgca, tggaaccacaatccagaa,
atgtgcatcctgacctgg, aaggcgtctaaccaagtg, tccaaggggaaatatgat, cgagggagaggtgagagg, agggacccagaggcagac,
aggagagggttgatttcc, accctttcttctgcgttc, agcatatccaaaaggccc, aatacagttgatgggcca, ggaactgcatgacctgga, tttctccctgtagtgac,
ccacgatgttaattgatg, tagaggacagtttgcaaa, agtaatagatttgccctt, aatcccagacagtatgag, atacaattctgggacttt, gtcttcgtaacctgtctt,
taaaaaaaaaaaaaaatg, cttgccttgtataacata, atccagattccctagagc, agatgtggtacagcaatg, agcaaatccaacctcaga, tctgaagtgtcttccagt,

ctggccctgacccagcca, ttctctgcccttccttct, cccttagggtagcccaa, atcccattgccacacaac, atctcaacttaccatccc, tttcctctatccccatcc,
cctctgtctgcgtcacag, aaagtctgtgtgttctga, agagttcagccttcctct, aaccaaacccacactttc, tttaccaccgtgattctc, agagccagcaagaaagaa,
atgttccaaaaggaaacc, tccatctcagccatttgc, ccggagccgaaggttgtg, ggctccaggcctctcagt, gaggtttgttgcttgtgt, gtttcccgaggagcgggc,
agtcaggcagtggtggtt, ctctctcccctctctctg, tcgcacgtggggtctcag, ctacatttacaagacttc, accaccatgttggagagc, tgctccactttggggtc,
ctcccaacatagtacagg, atggtcaggggctccagg, tcctggggcacgcagcaa, ggcgaggcagatgcttca, gggttcagagtgttgtac, agtcccagcaccgtgctg,
tgggttgtgtctgcactg, cggaggtatgggcaaggg, cctgagcagaagttggca, tagtagcccttaggttca, tggacccacttccagccc, agatcctgtcggaagtca,
atgtagaggggcgcaca, cagcagttctcctccaag, ttgcggaagcagtaattg, gtgtccaaagcccgcttc, ttcctctgaccccctgg, cccgggttgtcgagccgg,
tgtggggaatcatcatg, aggattagatgagggttg, tggtgatccttctgcttc, ttgaggcgccccagatct, ccacggccatggtcatcc, tcattgtccacgcctttg,
aatttgatttccatcacc, tcgtgaatgttttccagg, atatctccattgggctga, aaggtgtgacatggacag, tgaatgctgatttctaga, cctaagttggactctctt,
ctcaacagccactcacgc, acagtgtcagtgacatca, aaggacagccactcggca, gtgccccgtgtgggcaga, ttcttgccaccgatatag, cgctgtttggcaatgtgc,
tcatctggccgaaggatc, tggaagagctcgatcctc, tgctcattccgcttagag, ctggggttgggcacccgc, aagacccggaattctgct, cggaataggttggttcta,
tttttctccactgaggac, acattgaagcggaaaacc, ttggaggtaattccttta, gggcagacagccagttcg, ttgtgctccgccagcccc, tggatcatgtcgaattta,
tggatttcttggcatag, tattccgactcggtgttt, tcctgggtgcagccttcc, tccctctccccatgcatc, tcctccagcagctcccgg, gtgctgttgtaaagggcc,
aggacctgataggggacg, tgggtcatcaccgttggc, tcagggggggctggtgagc, ctgagcttgctcaagatc, tgtcccctaatggcttcc, accctcttcttcttgatg,
tggccgaagtccaaggtg, gtgcaagtggacagagag, aggctgaccgtggcaaag, ttcagcagggccaggacc, accagagccctttgcaag, tgcatcttcatgtgtgag,
ctgggaagagaggccagg, gggacggcaaggcctgga, gaggaagagaccccagca, gacgtgcagaaggaggga, ggaaaaccaggcggcctc,
cccagatcccaaagactg, aggcttggcaagaaggtg, catgaactcactgcactg, cgagagcttcaggacttc, caggaagcgctggcaacc,
ctgaggacgaagaagcgg, actgtgtgccttgtagcg, ctgggattcttgtccatg, tgtctaaacaggttttgc, ccccaaaaatatttattt, atacaaagatttgagag,
taatattcatacttgtct, ttatacctcagtctatgc, gtctggggccaagtcact, gtgtggcacatgtcgagc, ttccccgaatgcctcaca, tgttgtcgcacctgcttc,
caggaacaccaaatgaac, acagggtcttggaggggga, agtgggggaagaacccat, aatgccccaaccctgcat, ggaaccacaatccagaaa,
tgtgcatcctgacctgga, aggcgtctaaccaagtgt, ccaaggggaaatatgatc, gagggagaggtgagagga, gggacccagaggcagaca,
ggagagggttgatttcca, ccctttcttctgcgttca, gcatatccaaaaggccca, atacagttgatgggccag, gaactgcatgacctggat, tttctccctgtagtgacc,
cacgatgttaattgatgt, agaggacagtttgcaaaa, gtaatagattgcccttta, atcccagacagtatgaga, tacaattctgggactttg, tcttcgtaacctgtcttt,
aaaaaaaaaaaaaaatgc, ttgccttgtataacataa, tccagattccctagagca, gatgtggtacagcaatga, gcaaatccaacctcagat, ctgaagtgtcttccagtc,
tggccctgacccagccat, tctctgcccttccttctc, cctttagggtagcccaaa, tcccattgccacacaaca, tctcaacttaccatccct, ttcctctatccccatccc,
ctctgtctgcgtcacaga, aagtctgtgtgttctgaa, gagttcagccttcctcta, accaaacccacactttct, ttaccaccgtgattctca, gagccagcaagaaagaaa,
tgttccaaaaggaaacct, ccatctcagccatttgcc, cggagccgaaggttgtgg, gctccaggcctctcagtg, aggtttgttgcttgtgtg, tttcccgaggagcgggca,
gtcaggcagtggtggttc, tctctcccctctctctgt, cgcacgtggggtctcagc, tacatttacaagacttca, ccaccatgttggagagct, gctccactttggggtcc,
tcccaacatagtacagga, tggtcaggggctccaggt, cctggggcacgcagcaag, gcgaggcagatgcttcag, ggttcagagtgttgtaca, gtcccagcaccgtgctgt,
gggttgtgtctgcactgc, ggaggtatgggcaagggc, ctgagcagaagttggcat, agtagcccttaggttcat, ggacccacttccagccca, gatcctgtcggaagtcaa,
tgtagaggggcgcacac, agcagttctcctccaagt, tgcggaagcagtaattgg, tgtccaaagcccgcttct, tcctctgaccccctggc, ccgggttgtcgagccggt,
gtggggaatcatcatga, ggattagatgagggttgt, ggtgatccttctgcttct, tgaggcgccccagatctc, cacggccatggtcatcct, cattgtccacgcctttga,
atttgatttccatcacct, cgtgaatgttttccagga, tatctccattgggctgaa, aggtgtgacatggacagt, gaatgctgatttctagac, ctaagttggactctcttc,
tcaacagccactcacgca, cagtgtcagtgacatcaa, aggacagccactcggcag, tgccccgtgtgggcagat, tcttgccaccgatatagc, gctgtttggcaatgtgct,
catctggccgaaggatct, ggaagagctcgatcctct, gctcattccgcttagagc, tggggttgggcacccgca, agacccggaattctgctc, ggaataggttggttctat,
ttttctccactgaggaca, cattgaagcggaaaacct, tggaggtaattcctttag, ggcagacagccagttcgt, tgtgctccgccagcccct, ggatcatgtcgaatttat,
ggatttcttggcatagt, attccgactcggtgtttt, cctgggtgcagccttcct, ccctctccccatgcatct, cctccagcagctcccggg, tgctgttgtaaagggcca,
ggacctgataggggacgt, gggtcatcaccgttggct, caggggggctggtgagcc, tgagcttgctcaagatct, gtcccctaatggcttcca, ccctcttcttcttgatgt,
ggccgaagtccaaggtgg, tgcaagtggacagagaga, ggctgaccgtggcaaagt, tcagcagggccaggacca, ccagagccctttgcaagt, gcatcttcatgtgtgagc,
tgggaagagaggccaggg, ggacggcaaggcctggag, aggaagagaccccagcag, acgtgcagaaggagggag, gaaaaccaggcggcctcc,
ccagatcccaaagactga, ggcttggcaagaaggtgc, atgaactcactgcactgc, gagagcttcaggacttcc, aggaagcgctggcaaccc,
tgaggacgaagaagcgga, ctgtgtgccttgtagcgc, tgggattcttgtccatgt, gtctaaacaggttttgct, cccaaaaatatttattta, tacaaagatttgagagt,
aatattcatacttgtctt, tatacctcagtctatgcg, tctggggccaagtcactg, tgtggcacatgtcgagct, tccccgaatgcctcacat, gttgtcgcacctgcttcc,
aggaacaccaaatgaaca, cagggtcttggaggggaa, gtgggggaagaacccata, atgccccaaccctgcatg, gaaccacaatccagaaat,
gtgcatcctgacctggaa, ggcgtctaaccaagtgtc, caaggggaaatatgatcg, agggagaggtgagaggag, ggacccagaggcagacag,
gagagggttgatttccac, cctttcttctgcgttcag, catatccaaaaggcccaa, tacagttgatgggccagg, aactgcatgacctggatt, ttctccctgtagtgaccc,
acgatgttaattgatgta, gaggacagtttgcaaaag, taatagattgcccttaa, tcccagacagtatgagat, acaattctgggactttgt, cttcgtaacctgtcttta,
aaaaaaaaaaaaaatgct, tgccttgtataacataat, ccagattccctagagcag, atgtggtacagcaatgag, caaatccaacctcagatc, tgaagtgtcttccagtct,
ggccctgacccagccatt, ctctgcccttccttctcc, ctttagggtagcccaaat, cccattgccacacaacat, ctcaacttaccatccctt, tcctctatccccatcccc,
tctgtctgcgtcacagaa, agtctgtgtgttctgaag, agttcagccttcctctaa, ccaaacccacactttctt, taccaccgtgattctcag, agccagcaagaaagaaat,
gttccaaaaggaaacctc, catctcagccatttgccc, ggagccgaaggttgtggg, ctccaggcctctcagtga, ggtttgttgcttgtgtgt, ttcccgaggagcgggcag,
tcaggcagtggtggttct, ctctcccctctctctgtc, gcacgtggggtctcagct, acatttacaagacttcac, caccatgttggagagctg, ctccactttggggtcct,
cccaacatagtacaggat, ggtcaggggctccaggtc, ctggggcacgcagcaagg, cgaggcagatgcttcagg, gttcagagtgttgtacag, tcccagcaccgtgctgtg,
ggttgtgtctgcactgcg, gaggtatgggcaagggcc, tgagcagaagttggcata, gtagcccttaggttcatg, gacccacttccagcccag, atcctgtcggaagtcaat,
gtagaggggcgcacaca, gcagttctcctccaagtt, gcggaagcagtaattggt, gtccaaagcccgcttctt, cctctgaccccctggcc, cgggttgtcgagccggtg,
tggggaatcatcatgag, gattagatgagggttgtg, gtgatccttctgcttctt, gaggcgccccagatctcc, acggccatggtcatcctc, attgtccacgcctttgaa,
tttgatttccatcacctc, gtgaatgttttccaggat, atctccattgggctgaaa, ggtgtgacatggacagtg, aatgctgatttctagacc, taagttggactctcttct,
caacagccactcacgcac, agtgtcagtgacatcaaa, ggacagccactcggcagt, gccccgtgtgggcagatt, cttgccaccgatatagcg, ctgtttggcaatgtgctc,
atctggccgaaggatctg, gaagagctcgatcctctg, ctcattccgcttagagct, ggggttgggcacccgcaa, gacccggaattctgctcg, gaataggttggttctatt,
tttctccactgaggacac, attgaagcggaaaacctt, ggaggtaattcctttagg, gcagacagccagttcgtt, gtgctccgccagcccctg, gatcatgtcgaatttatg,
gatttcttggcatagta, ttccgactcggtgttttc, ctgggtgcagccttcctc, cctctccccatgcatctc, ctccagcagctcccgggt, gctgttgtaaagggccag,
gacctgataggggacgtg, ggtcatcaccgttggctc, aggggggctggtgagcct, gagcttgctcaagatctg, tcccctaatggcttccac, cctcttcttcttgatgtg,
gccgaagtccaaggtggt, gcaagtggacagagagag, gctgaccgtggcaaagtt, cagcagggccaggaccac, cagagccctttgcaagtg, catcttcatgtgtgagct,
gggaagagaggccagggg, gacggcaaggcctggaga, ggaagagaccccagcaga, cgtgcagaaggagggagg, aaaaccaggcggcctccc,
cagatcccaaagactgag, gcttggcaagaaggtgca, tgaactcactgcactgcg, agagcttcaggacttcca, ggaagcgctggcaaccct,
gaggacgaagaagcggac, tgtgtgccttgtagcgct, gggattcttgtccatgtg, tctaaacaggttttgctg, ccaaaaatatttatttat, acaaagatttgagagta,
atattcatacttgtcttt, atacctcagtctatgcgt, ctggggccaagtcactgt, gtggcacatgtcgagctt, ccccgaatgcctcacatg, ttgtcgcacctgcttcca,
ggaacaccaaatgaacac, agggtcttggaggggaag, tgggggaagaacccataa, tgccccaaccctgcatgg, aaccacaatccagaaatg,
tgcatcctgacctggaag, gcgtctaaccaagtgtcc, aaggggaaatatgatcga, gggagaggtgagaggagg, gacccagaggcagacagg,
agagggttgatttccacc, ctttcttctgcgttcagc, atatccaaaaggcccaat, acagttgatgggccagga, actgcatgacctggattt, tctccctgtagtgaccca,
cgatgttaattgatgtag, aggacagtttgcaaaagt, aatagattgcccttaat, cccagacagtatgagata, caattctgggactttgtc, ttcgtaacctgtctttaa,
aaaaaaaaaaaaatgctt, gccttgtataacataatc, cagattccctagagcaga, tgtggtacagcaatgagc, aaatccaacctcagatct, gaagtgtcttccagtctg,
gccctgacccagccattc, tctgcccttccttctccc, tttagggtagcccaaatc, ccattgccacacaacatc, tcaacttaccatcccttt, cctctatccccatcccct,
ctgtctgcgtcacagaaa, gtctgtgtgttctgaaga, gttcagccttcctctaac, caaacccacactttcttt, accaccgtgattctcaga, gccagcaagaaagaaatg,
ttccaaaaggaaacctcc, atctcagccatttgcccg, gagccgaaggttgtgggc, tccaggcctctcagtgag, gtttgttgcttgtgtgtt, tcccgaggagcgggcagt,
caggcagtggtggttctc, tctcccctctctctgtcg, cacgtggggtctcagcta, catttacaagacttcacc, accatgttggagagctgc, tccactttggggtcctc,
ccaacatagtacaggatg, gtcaggggctccaggtcc, tggggcacgcagcaaggc, gaggcagatgcttcaggg, ttcagagtgttgtacagt, cccagcaccgtgctgtgg,

gttgtgtctgcactgcgg, aggtatgggcaagggcct, gagcagaagttggcatag, tagcccttaggttcatgg, acccacttccagcccaga, tcctgtcggaagtcaatg,
tagaggggcgcacacag, cagttctcctccaagttg, cggaagcagtaattggtg, tccaaagcccgcttcttc, ctctgaccccctggccc, gggttgtcgagccggtgt,
ggggaatcatcatgagg, attagatgagggttgtgg, tgatccttctgcttcttg, aggcgccccagatctcca, cggccatggtcatcctca, ttgtccacgcctttgaat,
ttgatttccatcacctcg, tgaatgttttccaggata, tctccattgggctgaaag, gtgtgacatggacagtga, atgctgatttctagacct, aagttggactctcttctc,
aacagccactcacgcaca, gtgtcagtgacatcaaag, gacagccactcggcagtg, ccccgtgtgggcagattc, ttgccaccgatatagcgc, tgtttggcaatgtgctca,
tctggccgaaggatctgg, aagagctcgatcctctgc, tcattccgcttagagctg, gggttgggcacccgcaag, acccggaattctgctcgg, aataggttggttctattt,
ttctccactgaggacaca, ttgaagcggaaaaccttg, gaggtaattcctttaggg, cagacagccagttcgttg, tgctccgccagcccctgg, atcatgtcgaatttatgg,
atttctttggcatagtat, tccgactcggtgttttcc, tgggtgcagccttcctcc, ctctccccatgcatctcc, tccagcagctcccgggtg, ctgttgtaaagggccagg,
acctgataggggacgtgg, gtcatcaccgttggctca, ggggggctggtgagcctg, agcttgctcaagatctgt, cccctaatggcttccacc, ctcttcttcttgatgtgg,
ccgaagtccaaggtggtg, caagtggacagagagagg, ctgaccgtggcaaagttc, agcagggccaggaccacc, agagccctttgcaagtgc, atcttcatgtgtgagctg,
ggaagagaggccaggggg, acggcaaggcctggagag, gaagagaccccagcagac, gtgcagaaggagggagga, aaaccaggcggcctcccc,
agatcccaaagactgagg, cttggcaagaaggtgcat, gaactcactgcactgcga, gagcttcaggacttccag, gaagcgctggcaaccctg,
aggacgaagaagcggact, gtgtgccttgtagcgctg, ggattcttgtccatgtgt, ctaaacaggttttgctgg, , caaaaatatttatttata, caaagattttgagagtaa,
tattcatacttgtcttta, tacctcagtctatgcgtc, tggggccaagtcactgtg, tggcacatgtcgagcttc, cccgaatgcctcacatgt, tgtcgcacctgcttccag,
gaacaccaaatgaacaca, gggtcttggaggggaagt, gggggaagaacccataat, gccccaaccctgcatgga, accacaatccagaaatgt,
gcatcctgacctggaagg, cgtctaaccaagtgtcca, aggggaaatatgatcgag, ggagaggtgagaggaggg, acccagaggcagacagga,
gagggttgatttccaccc, ttcttctgcgttcagca, tatccaaaaggcccaata, cagttgatgggccaggaa, ctgcatgacctggatttt, ctccctgtagtgacccac,
gatgttaattgatgtaga, ggacagtttgcaaaagta, atagatttgcccttaatc, ccagacagtatgagatac, aattctgggactttgtct, tcgtaacctgtctttaaa,
aaaaaaaaaaaatgcttg, ccttgtataacataatcc, agattccctagagcagat, gtggtacagcaatgagca, aatccaacctcagatctg, aagtgtcttccagtctgg,
ccctgacccagccattct, ctgcccttccttctccct, ttagggtagcccaaatcc, cattgccacacaacatct, caacttaccatccctttc, ctctatccccatcccctc,
tgtctgcgtcacagaaag, tctgtgtgttctgaagag, ttcagccttcctctaacc, aaacccacactttcttta, ccaccgtgattctcagag, ccagcaagaaagaaatgt,
tccaaaaggaaacctcca, tctcagccatttgcccgg, agccgaaggttgtgggct, ccaggcctctcagtgagg, tttgttgcttgtgtgttt, cccgaggagcgggcagtc,
aggcagtggtggttctct, ctcccctctctctgtcgc, acgtggggtctcagctac, atttacaagacttcacca, ccatgttggagagctgct, ccactttggggggtcctcc,
caacatagtacaggatgg, tcaggggctccaggtcct, ggggcacgcagcaaggcg, aggcagatgcttcagggt, tcagagtgttgtacagtc, ccagcaccgtgctgtggg,
ttgtgtctgcactgcgga, ggtatgggcaagggcctg, agcagaagttggcatagt, agcccttaggttcatgga, cccacttccagcccagat, cctgtcggaagtcaatgt,
agaggggcgcacacagc, agttctcctccaagttgc, ggaagcagtaattggtgt, ccaaagcccgcttcttcc, tctgaccccctggcccg, ggttgtcgagccggtgtg,
gggaatcatcatgagga, ttagatgagggttgtggt, gatccttctgcttcttga, ggcgccccagatctccac, ggccatggtcatcctcat, tgtccacgcctttgaatt,
tgatttccatcacctcgt, gaatgttttccaggatat, ctccattgggctgaaagg, tgtgacatggacagtgaa, tgctgatttctagaccta, agttggactctcttctca,
acagccactcacgcacag, tgtcagtgacatcaaagg, acagccactcggcagtgc, cccgtgtgggcagattct, tgccaccgatatagcgct, gtttggcaatgtgctcat,
ctggccgaaggatctgga, agagctcgatcctctgct, cattccgcttagagctgg, ggttgggcacccgcaaga, cccggaattctgctcgga, ataggttggttctatttt,
tctccactgaggacacat, tgaagcggaaaaccttgg, aggtaattcctttagggc, agacagccagttcgttgt, gctccgccagcccctgga, tcatgtcgaatttatgga,
tttctttggcatagtatt, ccgactcggtgttttcct, gggtgcagccttcctccc, tctccccatgcatctcct, ccagcagctcccgggtgc, tgttgtaaagggccagga,
cctgataggggacgtggg, tcatcaccgttggctcag, gggggctggtgagcctga, gcttgctcaagatctgtc, ccctaatggcttccaccc, tcttcttcttgatgtggc,
cgaagtccaaggtggtgc, aagtggacagagagaggc, tgaccgtggcaaagttca, gcagggccaggaccacca, gagccctttgcaagtgca, tcttcatgtgtgagctgg,
gaagagaggccaggggga, cggcaaggcctggagagg, aagagaccccagcagacg, tgcagaaggagggaggaa, aaccaggcggcctcccca,
gatcccaaagactgaggc, ttggcaagaaggtgcatg, aactcactgcactgcgag, agcttcaggacttccagg, aagcgctggcaaccctga,
ggacgaagaagcggactg, tgtgccttgtagcgctgg, gattcttgtccatgtgtc, aaaaatatttatttatac, aaagattttgagagtaat, attcatacttgtctttat,
acctcagtctatgcgtct, ggggccaagtcactgtgt, ggcacatgtcgagcttcc, ccgaatgcctcacatgtt, gtcgcacctgcttccagg, aacaccaaatgaacacag,
ggtcttggaggggaagtg, ggggaagaacccataatg, ccccaaccctgcatggaa, ccacaatccagaaatgtg, catcctgacctggaaggc, gtctaaccaagtgtccaa,
ggggaaatatgatcgagg, gagaggtgagaggaggga, cccagaggcagacaggag, agggttgatttccaccct, ttcttctgcgttcagcat, atccaaaaggcccaatac,
agttgatgggccaggaac, tgcatgacctggattttc, tccctgtagtgacccacg, atgttaattgatgtagag, gacagtttgcaaaagtaa, tagatttgcccttaatcc,
cagacagtatgagataca, attctgggactttgtctt, cgtaacctgtctttaaaa, aaaaaaaaaaatgcttgc, cttgtataacataatcca, gattccctagagcagatg,
tggtacagcaatgagcaa, atccaacctcagatctga, agtgtcttccagtctggc, cctgacccagccattctc, tgcccttccttctccctt, tagggtagcccaaatccc,
attgccacacaacatctc, aacttaccatccctttcc, tctatccccatcccctct, gtctgcgtcacagaaagt, ctgtgtgttctgaagagt, tcagccttcctctaacca,
aacccacactttctttac, caccgtgattctcagagc, cagcaagaaagaaatgtt, ccaaaaggaaacctccat, ctcagccatttgcccgga, gccgaaggttgtgggctc,
caggcctctcagtgaggt, ttgttgcttgtgtgtttc, ccgaggagcgggcagtca, ggcagtggtggttctctc, tcccctctctctgtcgca, cgtggggtctcagctaca,
tttacaagacttcaccac, catgttggagagctgctc, cactttggggggtcctccc, aacatagtacaggatggt, caggggctccaggtcctg, gggcacgcagcaaggcga,
ggcagatgcttcagggtt, cagagtgttgtacagtcc, cagcaccgtgctgtgggt, tgtgtctgcactgcggag, gtatgggcaagggcctga, gcagaagttggcatagta,
gcccttaggttcatggac, ccacttccagcccagatc, ctgtcggaagtcaatgta, gaggggcgcacacagca, gttctcctccaagttgcg, gaagcagtaattggtgtc,
caaagcccgcttcttcct, ctgaccccctggcccgg, gttgtcgagccggtgtgg, gggaatcatcatgaggat, tagatgagggttgtggtg, atccttctgcttcttgag,
gcgccccagatctccacg, gccatggtcatcctcatt, gtccacgcctttgaattt, gatttccatcacctcgtg, aatgttttccaggatatc, tccattgggctgaaaggt,
gtgacatggacagtgaat, gctgatttctagacctaa, gttggactctcttctcaa, cagccactcacgcacagt, gtcagtgacatcaaagga, cagccactcggcagtgcc,
ccgtgtgggcagattctt, gccaccgatatagcgctg, tttggcaatgtgctcatc, tggccgaaggatctggaa, gagctcgatcctctgctc, attccgcttagagctggg,
gttgggcacccgcaagac, ccggaattctgctcggaa, taggttggttctattttt, ctccactgaggacacatt, gaagcggaaaaccttgga, ggtaattcctttagggca,
gacagccagttcgttgtg, ctccgccagcccctggat, catgtcgaatttatggat, ttctttggcatagtattc, cgactcggtgttttcctg, ggtgcagccttcctccct,
ctccccatgcatctcctc, cagcagctcccgggtgct, gttgtaaagggccaggac, ctgataggggacgtgggt, catcaccgttggctcagg, ggggctggtgagcctgag,
cttgctcaagatctgtcc, cctaatggcttccaccct, cttcttcttgatgtggcc, gaagtccaaggtggtgca, agtggacagagagaggct, gaccgtggcaaagttcag,
cagggccaggaccaccag, agccctttgcaagtgcat, cttcatgtgtgagctggg, aagagaggccagggggac, ggcaaggcctggagagga,
agagaccccagcagacgt, gcagaaggagggaggaaa, accaggcggcctccccag, atcccaaagactgaggct, tggcaagaaggtgcatga,
actcactgcactgcgaga, gcttcaggacttccagga, agcgctggcaaccctgag, gacgaagaagcggactgt, gtgccttgtagcgctggg, attcttgtccatgtgtct,
aaaatatttatttataca, aagattttgagagtaata, ttcatacttgtctttata, cctcagtctatgcgtctg, gggccaagtcactgtgtg, gcacatgtcgagcttccc,
cgaatgcctcacatgttg, tcgcacctgcttccagga, acaccaaatgaacacagg, gtcttggaggggaagtgg, gggaagaacccataatgc, cccaaccctgcatggaac,
cacaatccagaaatgtgc, atcctgacctggaaggcg, tctaaccaagtgtccaag, gggaaatatgatcgaggg, agaggtgagaggagggac,
ccagaggcagacaggaga, gggttgatttccaccctt, tcttctgcgttcagcata, tccaaaaggcccaataca, gttgatgggccaggaact, gcatgacctggattttct,
ccctgtagtgacccacga, tgttaattgatgtagagg, acagtttgcaaaagtaat, agatttgcccttaatccc, agacagtatgagatacaa, ttctgggactttgtcttc,
gtaacctgtctttaaaaa, aaaaaaaaaatgcttgcc, ttgtataacataatccag, attccctagagcagatgt, ggtacagcaatgagcaaa, tccaacctcagatctgaa,
gtgtcttccagtctggcc, ctgacccagccattctct, gcccttccttctcccttt, agggtagcccaaatccca, ttgccacacaacatctca, acttaccatccctttcct,
ctatccccatcccctctg, tctgcgtcacagaaagtc, tgtgtgttctgaagagtt, cagccttcctctaaccaa, acccacactttctttacc, accgtgattctcagagcc,
agcaagaaagaaatgttc, caaaaggaaacctccatc, tcagccatttgcccggag, ccgaaggttgtgggctcc, aggcctctcagtgaggtt, tgttgcttgtgtgtttcc,
cgaggagcgggcagtcag, gcagtggtggttctctct, cccctctctctgtcgcac, gtggggtctcagctacat, ttacaagacttcaccacc, atgttggagagctgctcc,
actttggggggtcctccca, acatagtacaggatggtc, aggggctccaggtcctgg, ggcacgcagcaaggcgag, gcagatgcttcagggttc, agagtgttgtacagtccc,
agcaccgtgctgtgggtt, gtgtctgcactgcggagg, tatgggcaagggcctgag, cagaagttggcatagtag, cccttaggttcatggacc, cacttccagcccagatcc,
tgtcggaagtcaatgtag, aggggcgcacacagcag, ttctcctccaagttgcgg, aagcagtaattggtgtcc, aaagcccgcttcttcctc, tgaccccctggcccggg,
ttgtcgagccggtgtggg, ggaatcatcatgaggatt, agatgagggttgtggtga, tccttctgcttcttgagg, cgccccagatctccacgg, ccatggtcatcctcattg,
tccacgcctttgaatttg, atttccatcacctcgtga, atgttttccaggatatct, ccattgggctgaaaggtg, tgacatggacagtgaatg, ctgatttctagacctaag,
ttggactctcttctcaac, agccactcacgcacagtg, tcagtgacatcaaaggac, agccactcggcagtgccc, cgtgtgggcagattcttg, ccaccgatatagcgctgt,
ttggcaatgtgctcatct, ggccgaaggatctggaag, agctcgatcctctgctca, ttccgcttagagctgggg, ttgggcacccgcaagacc, cggaattctgctcggaat,

aggttggttctatttttc, tccactgaggacacattg, aagcggaaaaccttggag, gtaattcctttagggcag, acagccagttcgttgtgc, tccgccagcccctggatc,
atgtcgaatttatggatt, tctttggcatagtattcc, gactcggtgttttcctgg, gtgcagccttcctccctc, tccccatgcatctcctcc, agcagctcccgggtgctg,
ttgtaaagggccaggacc, tgataggggacgtgggtc, atcaccgttggctcaggg, gggctggtgagcctgagc, ttgctcaagatctgtccc, ctaatggcttccaccctc,
ttcttcttgatgtggccg, aagtccaaggtggtgcaa, gtggacagagagaggctg, accgtggcaaagttcagc, agggccaggaccaccaga, gccctttgcaagtgcatc,
ttcatgtgtgagctggga, agagaggccaggggacg, gcaaggcctggagaggaa, gagaccccagcagacgtg, cagaaggagggaggaaaa,
ccaggcggcctccccaga, tcccaaagactgaggctt, ggcaagaaggtgcatgaa, ctcactgcactgcgagag, cttcaggacttccaggaa,
gcgctggcaaccctgagg, acgaagaagcggactgtg, tgccttgtagcgctggga, ttcttgtccatgtgtcta, aaatatttatttatacaa, agattttgagagtaatat,
tcatacttgtctttatac, ctcagtctatgcgtctgg, ggccaagtcactgtgtgg, cacatgtcgagcttcccc, gaatgcctcacatgttgt, cgcacctgcttccaggaa,
caccaaatgaacacaggg, tcttggaggggaagtggg, ggaagaacccataatgcc, ccaaccctgcatggaacc, acaatccagaaatgtgca,
tcctgacctggaaggcgt, ctaaccaagtgtccaagg, ggaaatatgatcgaggga, gaggtgagaggagggacc, cagaggcagacaggagag, ggttgatttccacccttt,
cttctgcgttcagcatat, ccaaaaggcccaatacag, ttgatgggccaggaactg, catgacctggattttctc, cctgtagtgacccacgat, gttaattgatgtagagga,
cagtttgcaaaagtaata, gatttgcccttaatccca, gacagtatgagatacaat, tctgggactttgtcttcg, taacctgtctttaaaaaa, aaaaaaaaatgcttgcct,
tgtataacataatccaga, ttccctagagcagatgtg, gtacagcaatgagcaaat, ccaacctcagatctgaag, tgtcttccagtctggccc, tgacccagccattctctg,
cccttccttctcccttta, gggtagcccaaatcccat, tgccacacaacatctcaa, cttaccatccctttcctc, tatccccatcccctctgt, ctgcgtcacagaaagtct,
gtgtgttctgaagagttc, agccttcctctaaccaaa, cccacactttctttacca, ccgtgattctcagagcca, gcaagaaagaaatgttcc, aaaaggaaacctccatct,
cagccatttgcccggagc, cgaaggttgtgggctcca, ggcctctcagtgaggttt, gttgcttgtgtgtttccc, gaggagcgggcagtcagg, cagtggtggttctctctc,
ccctctctctgtcgcacg, tggggtctcagctacatt, tacaagacttcaccacca, tgttggagagctgctcca, ctttgggggtcctcccaa, catagtacaggatggtca,
ggggctccaggtcctggg, gcacgcagcaaggcgagg, cagatgcttcagggttca, gagtgttgtacagtccca, gcaccgtgctgtgggttg, tgtctgcactgcggaggt,
atgggcaagggcctgagc, agaagttggcatagtagc, ccttaggttcatggaccc, acttccagcccagatcct, gtcggaagtcaatgtaga, gggggcgcacacagcagt,
tctcctccaagttgcgga, agcagtaattggtgtcca, aagcccgcttcttcctct, gacccccctggcccgggt, tgtcgagccggtgtgggg, gaatcatcatgaggatta,
gatgagggttgtggtgat, ccttctgcttcttgaggc, gccccagatctccacggc, catggtcatcctcattgt, ccacgcctttgaatttga, tttccatcacctcgtgaa,
tgttttccaggatatctc, cattgggctgaaaggtgt, gacatggacagtgaatgc, tgatttctagacctaagt, tggactctcttctcaaca, gccactcacgcacagtgt,
cagtgacatcaaaggaca, gccactcggcagtgcccc, gtgtgggcagattcttgc, caccgatatagcgctgtt, tggcaatgtgctcatctg, gccgaaggatctggaaga,
gctcgatcctctgctcat, tccgcttagagctggggt, tgggcacccgcaagaccc, ggaattctgctcggaata, ggttggttctatttttct, ccactgaggacacattga,
agcggaaaaccttggagg, taattcctttagggcaga, cagccagttcgttgtgct, ccgccagcccctggatca, tgtcgaatttatggattt, ctttggcatagtattccg,
actcggtgttttcctggg, tgcagccttcctccctct, ccccatgcatctcctcca, gcagctcccgggtgctgt, tgtaaagggccaggacct, gataggggacgtgggtca,
tcaccgttggctcagggg, ggctggtgagcctgagct, tgctcaagatctgtcccc, taatggcttccaccctct, tcttcttgatgtggccga, agtccaaggtggtgcaag,
tggacagagagaggctga, ccgtggcaaagttcagca, gggccaggaccaccagag, ccctttgcaagtgcatct, tcatgtgtgagctgggaa,
gagaggccaggggacgg, caaggcctggagaggaag, agaccccagcagacgtgc, agaaggagggaggaaaac, caggcggcctccccagat,
cccaaagactgaggcttg, gcaagaaggtgcatgaac, tcactgcactgcgagagc, ttcaggacttccaggaag, cgctggcaaccctgagga,
cgaagaagcggactgtgt, gccttgtagcgctgggat, tcttgtccatgtgtctaa, aatatttatttatacaaa, gattttgagagtaatatt, catacttgtctttatacc,
tcagtctatgcgtctggg, gccaagtcactgtgtggc, acatgtcgagcttccccg, aatgcctcacatgttgtc, gcacctgcttccaggaac, accaaatgaacacagggt,
cttggaggggaagtgggg, gaagaacccataatgccc, caaccctgcatggaacca, caatccagaaatgtgcat, cctgacctggaaggcgtc,
taaccaagtgtccaaggg, gaaatatgatcgagggag, aggtgagaggagggaccc, agaggcagacaggagagg, gttgatttccaccctttc, ttctgcgttcagcatatc,
caaaaggcccaatacagt, tgatgggccaggaactgc, atgacctggattttctcc, ctgtagtgacccacgatg, ttaattgatgtagaggac, agtttgcaaaagtaatag,
atttgcccttaatcccag, acagtatgagatacaatt, ctgggactttgtcttcgt, aacctgtctttaaaaaaa, aaaaaaaatgcttgcctt, gtataacataatccagat,
tccctagagcagatgtgg, tacagcaatgagcaaatc, caacctcagatctgaagt, gtcttccagtctggccct, gacccagccattctctgc, ccttccttctccctttag,
ggtagcccaaatcccatt, gccacacaacatctcaac, ttaccatccctttcctct, atccccatcccctctgtc, tgcgtcacagaaagtctg, tgtgttctgaagagttca,
gccttcctctaaccaaac, ccacactttctttaccac, cgtgattctcagagccag, caagaaagaaatgttcca, aaaggaaacctccatctc, agccatttgcccggagcc,
gaaggttgtgggctccag, gcctctcagtgaggtttg, ttgcttgtgtgtttcccg, aggagcgggcagtcaggc, agtggtggttctctctcc, cctctctctgtcgcacgt,
ggggtctcagctacattt, acaagacttcaccaccat, gttggagagctgctccac, tttgggggtcctcccaac, atagtacaggatggtcag, gggctccaggtcctgggg,
cacgcagcaaggcgaggc, agatgcttcagggttcag, agtgttgtacagtcccag, caccgtgctgtgggttgt, gtctgcactgcggaggta, tgggcaagggcctgagca,
gaagttggcatagtagcc, cttaggttcatggaccca, cttccagcccagatcctg, tcggaagtcaatgtagag, ggggcgcacacagcagtt, ctcctccaagttgcggaa,
gcagtaattggtgtccaa, agcccgcttcttcctctg, acccccctggcccgggtt, gtcgagccggtgtggggg, aatcatcatgaggattag, atgagggttgtggtgatc,
cttctgcttcttgaggcg, ccccagatctccacggcc, atggtcatcctcattgtc, cacgcctttgaatttgat, ttccatcacctcgtgaat, gttttccaggatatctcc,
attgggctgaaaggtgtg, acatggacagtgaatgct, gatttctagacctaagtt, ggactctcttctcaacag, ccactcacgcacagtgtc, agtgacatcaaaggacag,
ccactcggcagtgccccg, tgtgggcagattcttgcc, accgatatagcgctgttt, ggcaatgtgctcatctgg, ccgaaggatctggaagag, ctcgatcctctgctcatt,
ccgcttagagctggggtt, gggcacccgcaagacccg, gaattctgctcggaatag, gttggttctatttttctc, cactgaggacacattgaa, gcggaaaaccttggaggt,
aattcctttagggcagac, agccagttcgttgtgctc, cgccagcccctggatcat, gtcgaatttatggatttc, tttggcatagtattccga, ctcggtgttttcctgggt,
gcagccttcctccctctc, cccatgcatctcctccag, cagctcccgggtgctgtt, gtaaagggccaggacctg, ataggggacgtgggtcat, caccgttggctcaggggg,
gctggtgagcctgagctt, gctcaagatctgtcccct, aatggcttccaccctctt, cttcttgatgtggccgaa, gtccaaggtggtgcaagt, ggacagagagaggctgac,
cgtggcaaagttcagcag, ggccaggaccaccagagc, cctttgcaagtgcatctt, catgtgtgagctgggaag, agaggccaggggacggc,
aaggcctggagaggaaga, gaccccagcagacgtgca, gaaggagggaggaaaacc, aggcggcctccccagatc, ccaaagactgaggcttgg,
caagaaggtgcatgaact, cactgcactgcgagagct, tcaggacttccaggaagc, gctggcaaccctgaggac, gaagaagcggactgtgtg, ccttgtagcgctgggatt,
cttgtccatgtgtctaaa, atatttatttatacaaag, attttgagagtaatattc, atacttgtctttatacct, cagtctatgcgtctgggg, ccaagtcactgtgtggca,
catgtcgagcttccccga, atgcctcacatgttgtcg, cacctgcttccaggaaca, ccaaatgaacacagggtc, ttggaggggaagtggggg, aagaacccataatgcccc,
aaccctgcatggaaccac, aatccagaaatgtgcatc, ctgacctggaaggcgtct, aaccaagtgtccaagggg, aaatatgatcgagggaga,
ggtgagaggagggaccca, gaggcagacaggagaggg, ttgatttccaccctttct, tctgcgttcagcatatcc, aaaaggcccaatacagtt, gatgggccaggaactgca,
tgacctggattttctccc, tgtagtgacccacgatgt, taattgatgtagaggaca, gtttgcaaaagtaataga, tttgcccttaatcccaga, cagtatgagatacaattc,
tgggactttgtcttcgta, acctgtctttaaaaaaaa, aaaaaaatgcttgccttg, tataacataatccagatt, ccctagagcagatgtggt, acagcaatgagcaaatcc,
aacctcagatctgaagtg, tcttccagtctggccctg, acccagccattctctgcc, cttccttctccctttagg, gtagcccaaatcccattg, ccacacaacatctcaact,
taccatccctttcctcta, tccccatcccctctgtct, gcgtcacagaaagtctgt, gtgttctgaagagttcag, ccttcctctaaccaaacc, cacactttctttaccacc,
gtgattctcagagccagc, aagaaagaaatgttccaa, aaggaaacctccatctca, gccatttgcccggagccg, aaggttgtgggctccagg, cctctcagtgaggtttgt,
tgcttgtgtgtttcccga, ggagcgggcagtcaggca, gtggtggttctctctccc, ctctctctgtcgcacgtg, gggtctcagctacattta, caagacttcaccaccatg,
ttggagagctgctccact, ttgggggtcctcccaaca, tagtacaggatggtcagg, ggctccaggtcctggggc, acgcagcaaggcgaggca, gatgcttcagggttcaga,
gtgttgtacagtcccagc, accgtgctgtgggttgtg, tctgcactgcggaggtat, gggcaagggcctgagcag, aagttggcatagtagccc, ttaggttcatggacccac,
ttccagcccagatcctgt, cggaagtcaatgtagagg, gggcgcacacagcagttc, tcctccaagttgcggaag, cagtaattggtgtccaaa, gcccgcttcttcctctga,
cccccctggcccgggttg, tcgagccggtgtggggga, atcatcatgaggattaga, tgagggttgtggtgatcc, ttctgcttcttgaggcgc, cccagatctccacggcca,
tggtcatcctcattgtcc, acgcctttgaatttgatt, tccatcacctcgtgaatg, ttttccaggatatctcca, ttgggctgaaaggtgtga, catggacagtgaatgctg,
atttctagacctaagttg, gactctcttctcaacagc, cactcacgcacagtgtca, gtgacatcaaaggacagc, cactcggcagtgccccgt, gtgggcagattcttgcca,
ccgatatagcgctgtttg, gcaatgtgctcatctggc, cgaaggatctggaagagc, tcgatcctctgctcattc, cgcttagagctggggttg, ggcacccgcaagacccgg,
aattctgctcggaatagg, ttggttctatttttctcc, actgaggacacattgaag, cggaaaaccttggaggta, attcctttagggcagaca, gccagttcgttgtgctcc,
gccagcccctggatcatg, tcgaatttatggatttct, ttggcatagtattccgac, tcggtgttttcctgggtg, cagccttcctccctctcc, ccatgcatctcctccagc,
agctcccgggtgctgttg, taaagggccaggacctga, taggggacgtgggtcatc, accgttggctcagggggg, ctggtgagcctgagcttg, ctcaagatctgtccccta,
atggcttccaccctcttc, ttcttgatgtggccgaag, tccaaggtggtgcaagtg, gacagagagaggctgacc, gtggcaaagttcagcagg, gccaggaccaccagagcc,
ctttgcaagtgcatcttc, atgtgtgagctgggaaga, gaggccaggggacggca, aggcctggagaggaagag, accccagcagacgtgcag,
aaggagggaggaaaacca, ggcggcctccccagatcc, caaagactgaggcttggc, aagaaggtgcatgaactc, actgcactgcgagagctt,

caggacttccaggaagcg, ctggcaaccctgaggacg, aagaagcggactgtgtgc, cttgtagcgctgggattc, ttgtccatgtgtctaaac, tatttatttatacaaaga,
ttttgagagtaatattca, tacttgtcttatacctc, agtctatgcgtctggggc, caagtcactgtgtggcac, atgtcgagcttccccgaa, tgcctcacatgttgtcgc,
acctgcttccaggaacac, caaatgaacacagggtct, tggaggggaagtggggga, agaacccataatgcccca, accctgcatggaaccaca,
atccagaaatgtgcatcc, tgacctggaaggcgtcta, accaagtgtccaaggga, aatatgatcgagggagag, gtgagaggagggacccag,
aggcagacaggagagggt, tgatttccacccttcttt, ctgcgttcagcatatcca, aaaggcccaatacagttg, atgggccaggaactgcat, gacctggattttctccct,
gtagtgacccacgatgtt, aattgatgtagaggacag, tttgcaaaagtaatagat, ttgcccttaatcccagac, agtatgagatacaattct, gggactttgtcttcgtaa,
cctgtctttaaaaaaaaa, aaaaaatgcttgccttgt, ataacataatccagattc, cctagagcagatgtggta, cagcaatgagcaaatcca, acctcagatctgaagtgt,
cttccagtctggccctga, cccagccattctctgccc, ttccttctccctttaggg, tagcccaaatcccattgc, cacacaacatctcaactt, accatccctttcctctat,
ccccatcccctctgtctg, cgtcacagaaagtctgtg, tgttctgaagagttcagc, cttcctctaaccaaaccc, acactttctttaccaccg, tgattctcagagccagca,
agaaagaaatgttccaaa, aggaaacctccatctcag, ccatttgcccggagccga, aggttgtgggctccaggc, ctctcagtgaggtttgtt, gcttgtgtgtttcccgag,
gagcgggcagtcaggcag, tggtggttctctctcccc, tctctctgtcgcacgtgg, ggtctcagctacatttac, aagacttcaccaccatgt, tggagagctgctccactt,
tggggggtcctcccaacat, agtacaggatggtcaggg, gctccaggtcctggggca, cgcagcaaggcgaggcag, atgcttcagggttcagag, tgttgtacagtcccagca,
ccgtgctgtgggttgtgt, ctgcactgcggaggtatg, ggcaagggcctgagcaga, agttggcatagtagccct, taggttcatggacccact, tccagcccagatcctgtc,
ggaagtcaatgtagaggg, ggcgcacacagcagttct, cctccaagttgcggaagc, agtaattggtgtccaaag, cccgcttcttcctctgac, ccccctggcccgggttgt,
cgagccggtgtgggggaa, tcatcatgaggattagat, gagggttgtggtgatcct, tctgcttcttgaggcgcc, ccagatctccacggccat, ggtcatcctcattgtcca,
cgcctttgaatttgattt, ccatcacctcgtgaatgt, tttccaggatatctccat, tgggctgaaaggtgtgac, atggacagtgaatgctga, tttctagacctaagttgg,
actctcttctcaacagcc, actcacgcacagtgtcag, tgacatcaaaggacagcc, actcggcagtgccccgtg, tgggcagattcttgccac, cgatatagcgctgtttgg,
caatgtgctcatctggcc, gaaggatctggaagagct, cgatcctctgctcattcc, gcttagagctggggttgg, gcacccgcaagacccgga, attctgctcggaataggt,
tggttctatttttctcca, ctgaggacacattgaagc, ggaaaaccttggaggtaa, ttcctttagggcagacag, ccagttcgttgtgctccg, ccagcccctggatcatgt,
cgaatttatggatttctt, tggcatagtattccgact, cggtgttttcctgggtgc, agccttcctccctctccc, catgcatctcctccagca, gctcccgggtgctgttgt,
aaagggccaggacctgat, aggggacgtgggtcatca, ccgttggctcaggggggc, tggtgagcctgagcttgc, tcaagatctgtcccctaa, tggcttccaccctcttct,
tcttgatgtggccgaagt, ccaaggtggtgcaagtgg, acagagagaggctgaccg, tggcaaagttcagcaggg, ccaggaccaccagagccc, tttgcaagtgcatcttca,
tgtgtgagctgggaagag, aggccaggggggacggcaa, ggcctggagaggaagaga, ccccagcagacgtgcaga, aggagggaggaaaaccag,
gcggcctccccagatccc, aaagactgaggcttggca, agaaggtgcatgaactca, ctgcactgcgagagcttc, aggacttccaggaagcgc,
tggcaaccctgaggacga, agaagcggactgtgtgcc, ttgtagcgctgggattct, tgtccatgtgtctaaaca,

### Example 22

IL-10 antisense oligonucleotides:

tcaccctatggaaacagc, ttaaaaacaggtgaaaat, aataaatattgaaaaaaa, ttataatattgggcttct, ttctaaatcgttcacaga, gaagctcagtaaataaat,
agaaatggggggttgaggt, atcagaggtaataaatat, tctataagagaggtacaa, taaggtttctcaaggggc, tgggtcagctatcccaga, gccccagatccgattttg,
gagacctctaatttatgt, cctagagtctatagagtc, gccaccctgatgtctcag, tttcgtatcttcattgtc, atgtaggcttctatgtag, ttgatgaagatgtcaaac,
tcactcatggctttgtag, atgcctttctcttggagc, ttattaaaggcattcttc, acctgctccacggccttg, ctcttgttttcacaggga, agaaatcgatgacagcgc,
cgtagcctcagcctgagg, gtcttcaggttctccccc, agggagttcacatgcgcc, ttgatgtctgggtcttgg, ttctcagcttggggcatc, acctcctccaggtaaaac,
tggatcatctcagacaag, gcttggcaacccaggtaa, cccttaaagtcctccagc, aaggactcctttaacaac, aagttgtccagctgatcc, ttcatttgaaagaaagtc,
ttcactctgctgaaggca, tctcggagatctcgaagc, atgttaggcaggttgcct, gggaagtgggtgcagctg, ttctcagactgggtgccc, tggcctgggctggccctc,
accccagtcaggaggacc, aggcaacagagcagtgct, gagctgtgcatgccttct, tttgcaagtctgtcttgt, ggtttggttttgcaagag, caacccccctgatgtgtag,
accttcacctctctgtcc, ccctttatattgtaagc, tcagggaggcctcttcat, tcattaaaaagccacaat, caaggtttcccggcacag, gatttttctgcttagag,
ctcctccttctctaacct, ctctaataaacttagttt, tcaattttgcatcgtaa, gcaaaaatgattggttga, acatgaacttctgcatta, cagctatttttaggatgg,
gctacctctcttagaata, atttttagcttctcaat, taaaaaaagttgatttcc, tggggagaacagctgttc, tgtccgcagaggccctca, gctgtgggttctcattcg,
cgtgttcctaggtcacag, tgacgtggacaaattgcc, cattccagaatacaatgg, caccctatggaaacagct, taaaaacaggtgaaaata, ataaatattgaaaaaaat,
tataatattgggcttctt, tctaaatcgttcacagag, aagctcagtaaataaata, gaaatggggggttgaggta, tcagaggtaataaatatt, ctataagagaggtacaat,
aaggtttctcaaggggct, gggtcagctatcccagag, ccccagatccgattttgg, agacctctaatttatgtc, ctagagtctatagagtcg, ccaccctgatgtctcagt,
ttcgtatcttcattgtca, tgtaggcttctatgtagt, tgatgaagatgtcaaact, cactcatggctttgtaga, tgcctttctcttggagct, tattaaaggcattcttca,
cctgctccacggccttgc, tcttgttttcacagggaa, gaaatcgatgacagcgcc, gtagcctcagcctgaggg, tcttcaggttctccccca, gggagttcacatgcgcct,
tgatgtctgggtcttggt, tctcagcttggggcatca, cctcctccaggtaaaact, ggatcatctcagacaagg, cttggcaacccaggtaac, ccttaaagtcctccagca,
aggactcctttaacaaca, agttgtccagctgatcct, tcatttgaaagaaagtct, tcactctgctgaaggcat, ctcggagatctcgaagca, tgttaggcaggttgcctg,
ggaagtgggtgcagctgt, tctcagactgggtgccct, ggcctgggctggccctca, ccccagtcaggaggacca, ggcaacagagcagtgctg, agctgtgcatgccttctt,
ttgcaagtctgtcttgtg, gtttggttttgcaagagc, aacccccctgatgtgtaga, ccttcacctctctgtccc, cctttatattgtaagct, cagggaggcctcttcatt,
cattaaaaagccacaatc, aaggtttcccggcacagg, atttttctgcttagagc, tcctccttctctaacctc, tctaataaacttagtttt, caattttgcatcgtaag,
caaaaatgattggttgaa, catgaacttctgcattac, agctatttttaggatggg, ctacctctcttagaataa, tttttagcttctcaatt, aaaaaaagttgatttcct,
ggggagaacagctgttct, gtccgcagaggccctcag, ctgtgggttctcattcgc, gtgttcctaggtcacagt, gacgtggacaaattgccc, attccagaatacaatggg,
accctatggaaacagctt, aaaaacaggtgaaaataa, taaatattgaaaaaaatt, ataatattgggcttcttt, ctaaatcgttcacagaga, agctcagtaaataaatag,
aaatggggggttgaggtat, cagaggtaataaatattc, tataagagaggtacaata, aggtttctcaaggggctg, ggtcagctatcccagagc, cccagatccgattttgga,
gacctctaatttatgtcc, tagagtctatagagtcgc, caccctgatgtctcagtt, tcgtatcttcattgtcat, gtaggcttctatgtagtt, gatgaagatgtcaaactc,
actcatggctttgtagat, gcctttctcttggagctt, attaaaggcattcttcac, ctgctccacggccttgct, cttgttttcacagggaag, aaatcgatgacagcgccg,
tagcctcagcctgagggt, cttcaggttctcccccag, ggagttcacatgcgcctt, gatgtctgggtcttggtt, ctcagcttggggcatcac, ctcctccaggtaaaactg,
gatcatctcagacaaggc, ttggcaacccaggtaacc, cttaaagtcctccagcaa, ggactcctttaacaacaa, gttgtccagctgatcctt, catttgaaagaaagtctt,
cactctgctgaaggcatc, tcggagatctcgaagcat, gttaggcaggttgcctgg, gaagtgggtgcagctgtt, ctcagactgggtgccctg, gcctgggctggccctcac,
cccagtcaggaggaccag, gcaacagagcagtgctga, gctgtgcatgccttcttt, tgcaagtctgtcttgtgg, tttggttttgcaagagca, acccccctgatgtgtagac,

cttcacctctctgtcccc, cttttatattgtaagctc, agggaggcctcttcattc, attaaaaagccacaatca, aggtttcccggcacagga, tttttctgcttagagct,
cctccttctctaacctct, ctaataaacttagttttc, aatttttgcatcgtaagc, aaaaatgattggttgaac, atgaacttctgcattaca, gctatttttaggatgggc,
tacctctcttagaataat, tttttagcttctcaatta, aaaaaagttgatttcctg, gggagaacagctgttctg, tccgcagaggccctcagc, tgtgggttctcattcgcg,
tgttcctaggtcacagtg, acgtggacaaattgccca, ttccagaatacaatggga, ccctatggaaacagctta, aaaacaggtgaaaataat, aaatattgaaaaaaatta,
taatattgggcttctttc, taaatcgttcacagagaa, gctcagtaaataaataga, aatgggggttgaggtatc, agaggtaataaatattct, ataagagaggtacaataa,
ggtttctcaaggggctgg, gtcagctatcccagagcc, ccagatccgattttggag, acctctaatttatgtcct, agagtctatagagtcgcc, accctgatgtctcagttt,
cgtatcttcattgtcatg, taggcttctatgtagttg, atgaagatgtcaaactca, ctcatggctttgtagatg, cctttctcttggagctta, ttaaaggcattcttcacc,
tgctccacggccttgctc, ttgttttcacagggaaga, aatcgatgacagcgccgt, agcctcagcctgagggtc, ttcaggttctcccccagg, gagttcacatgcgccttg,
atgtctgggtcttggttc, tcagcttggggcatcacc, tcctccaggtaaaactgg, atcatctcagacaaggct, tggcaacccaggtaaccc, ttaaagtcctccagcaag,
gactcctttaacaacaag, ttgtccagctgatccttc, atttgaaagaaagtcttc, actctgctgaaggcatct, cggagatctcgaagcatg, ttaggcaggttgcctggg,
aagtgggtgcagctgttc, tcagactgggtgccctgg, cctgggctggccctcacc, ccagtcaggaggaccagg, caacagagcagtgctgag, ctgtgcatgccttctttt,
gcaagtctgtcttgtggt, ttggttttgcaagagcaa, ccccctgatgtgtagacc, ttcacctctctgtccccc, ttttatattgtaagctca, gggaggcctcttcattca,
ttaaaaagccacaatcaa, ggtttcccggcacaggat, tttttctgcttagagctc, ctccttctctaacctctc, taataaacttagttttca, atttttgcatcgtaagca,
aaaatgattggttgaaca, tgaacttctgcattacag, ctatttttaggatgggct, acctctcttagaataatt, ttttagcttctcaattaa, aaaaagttgatttcctgg,
ggagaacagctgttctgt, ccgcagaggccctcagct, gtgggttctcattcgcgt, gttcctaggtcacagtga, cgtggacaaattgcccat, tccagaatacaatgggat,
cctatggaaacagcttaa, aaacaggtgaaaataata, aatattgaaaaaaattat, aatattgggcttctttct, aaatcgttcacagagaag, ctcagtaaataaatagaa,
atgggggttgaggtatca, gaggtaataaatattcta, taagagaggtacaataag, gtttctcaaggggctggg, tcagctatcccagagccc, cagatccgattttggaga,
cctctaatttatgtccta, gagtctatagagtcgcca, ccctgatgtctcagtttc, gtatcttcattgtcatgt, aggcttctatgtagttga, tgaagatgtcaaactcac,
tcatggctttgtagatgc, ctttctcttggagcttat, taaaggcattcttcacct, gctccacggccttgctct, tgttttcacagggaagaa, atcgatgacagcgccgta,
gcctcagcctgagggtct, tcaggttctcccccaggg, agttcacatgcgccttga, tgtctgggtcttggttct, cagcttggggcatcacct, cctccaggtaaaactgga,
tcatctcagacaaggctt, ggcaacccaggtaaccct, taaagtcctccagcaagg, actcctttaacaacaagt, tgtccagctgatccttca, tttgaaagaaagtcttca,
ctctgctgaaggcatctc, ggagatctcgaagcatgt, taggcaggttgcctggga, agtgggtgcagctgttct, cagactgggtgccctggc, ctgggctggccctcaccc,
cagtcaggaggaccaggc, aacagagcagtgctgagc, tgtgcatgccttcttttg, caagtctgtcttgtggtt, tggttttgcaagagcaac, cccctgatgtgtagacct,
tcacctctctgtccccct, tttatattgtaagctcag, ggaggcctcttcattcat, taaaaagccacaatcaag, gtttcccggcacaggatt, ttttctgcttagagctcc,
tccttctctaacctctct, aataaacttagttttcaa, tttttgcatcgtaagcaa, aaatgattggttgaacat, gaacttctgcattacagc, tatttttaggatgggcta,
cctctcttagaataattt, tttagcttctcaattaaa, aaaagttgatttcctggg, gagaacagctgttctgtc, cgcagaggccctcagctg, tgggttctcattcgcgtg,
ttcctaggtcacagtgac, gtggacaaattgcccatt, ccagaatacaatgggatt, ctatggaaacagcttaaa, aacaggtgaaaataataa, atattgaaaaaaattata,
atattgggcttctttcta, aatcgttcacagagaagc, tcagtaaataaatagaaa, tgggggttgaggtatcag, aggtaataaatattctat, aagagaggtacaataagg,
tttctcaaggggctgggt, cagctatcccagagcccc, agatccgattttggagac, ctctaatttatgtcctag, agtctatagagtcgccac, cctgatgtctcagtttcg,
tatcttcattgtcatgta, ggcttctatgtagttgat, gaagatgtcaaactcact, catggctttgtagatgcc, tttctcttggagcttatt, aaaggcattcttcacctg,
ctccacggccttgctctt, gttttcacagggaagaaa, tcgatgacagcgccgtag, cctcagcctgagggtctt, caggttctcccccaggga, gttcacatgcgccttgat,
gtctgggtcttggttctc, agcttggggcatcacctc, ctccaggtaaaactggat, catctcagacaaggcttg, gcaacccaggtaaccctt, aaagtcctccagcaagga,
ctcctttaacaacaagtt, gtccagctgatccttcat, ttgaaagaaagtcttcac, tctgctgaaggcatctcg, gagatctcgaagcatgtt, aggcaggttgcctgggaa,
gtgggtgcagctgttctc, agactgggtgccctggcc, tgggctggccctcacccc, agtcaggaggaccaggca, acagagcagtgctgagct, gtgcatgccttcttttgc,
aagtctgtcttgtggttt, ggttttgcaagagcaacc, ccctgatgtgtagacctt, cacctctctgtccccctt, ttatattgtaagctcagg, gaggcctcttcattcatt,
aaaaagccacaatcaagg, tttcccggcacaggattt, tttctgcttagagctcct, ccttctctaacctctcta, ataaacttagttttcaat, ttttgcatcgtaagcaaa,
aatgattggttgaacatg, aacttctgcattacagct, atttttaggatgggctac, ctctcttagaataatttt, ttagcttctcaattaaaa, aaagttgatttcctgggg,
agaacagctgttctgtcc, gcagaggccctcagctgt, gggttctcattcgcgtgt, tcctaggtcacagtgacg, tggacaaattgcccattc, cagaatacaatgggattg,
tatggaaacagcttaaaa, acaggtgaaaataataaa, tattgaaaaaaattataa, tattgggcttctttctaa, atcgttcacagagaagct, cagtaaataaatagaaat,
gggggttgaggtatcaga, ggtaataaatattctata, agagaggtacaataaggt, ttctcaaggggctgggtc, agctatcccagagcccca, gatccgattttggagacc,
tctaatttatgtcctaga, gtctatagagtcgccacc, ctgatgtctcagtttcgt, atcttcattgtcatgtag, gcttctatgtagttgatg, aagatgtcaaactcactc,
atggctttgtagatgcct, ttctcttggagcttatta, aaggcattcttcacctgc, tccacggccttgctcttg, ttttcacagggaagaaat, cgatgacagcgccgtagc,
ctcagcctgagggtcttc, aggttctcccccagggag, ttcacatgcgccttgatg, tctgggtcttggttctca, gcttggggcatcacctcc, tccaggtaaaactggatc,
atctcagacaaggcttgg, caacccaggtaaccctta, aagtcctccagcaaggac, tcctttaacaacaagttg, tccagctgatccttcatt, tgaaagaaagtcttcact,
ctgctgaaggcatctcgg, agatctcgaagcatgtta, ggcaggttgcctgggaag, tgggtgcagctgttctca, gactgggtgccctggcct, gggctggccctcacccca,
gtcaggaggaccaggcaa, cagagcagtgctgagctg, tgcatgccttcttttgca, agtctgtcttgtggtttg, gttttgcaagagcaaccc, cctgatgtgtagaccttc,
acctctctgtcccccttt, tatattgtaagctcaggg, aggcctcttcattcatta, aaaagccacaatcaaggt, ttcccggcacaggatttt, ttctgcttagagctcctc,
cttctctaacctctctaa, taaacttagttttcaatt, tttgcatcgtaagcaaaa, atgattggttgaacatga, acttctgcattacagcta, tttttaggatgggctacc,
tctcttagaataattttt, tagcttctcaattaaaaa, aagttgatttcctgggga, gaacagctgttctgtccg, cagaggccctcagctgtg, ggttctcattcgcgtgtt,
cctaggtcacagtgacgt, ggacaaattgcccattcc, agaatacaatgggattga, atggaaacagcttaaaaa, caggtgaaaataataaat, attgaaaaaaattataat,
attgggcttctttctaaa, tcgttcacagagaagctc, agtaaataaatagaaatg, ggggttgaggtatcagag, gtaataaatattctataa, gagaggtacaataaggtt,
tctcaaggggctgggtca, gctatcccagagccccag, atccgattttggagacct, ctaatttatgtcctagag, tctatagagtcgccaccc, tgatgtctcagtttcgta,
tcttcattgtcatgtagg, cttctatgtagttgatga, agatgtcaaactcactca, tggctttgtagatgcctt, tctcttggagcttattaa, aggcattcttcacctgct,
ccacggccttgctcttgt, tttcacagggaagaaatc, gatgacagcgccgtagcc, tcagcctgagggtcttca, ggttctcccccagggagt, tcacatgcgccttgatgt,
ctgggtcttggttctcag, cttggggcatcacctcct, ccaggtaaaactggatca, tctcagacaaggcttggc, aacccaggtaacccttaa, agtcctccagcaaggact,
cctttaacaacaagttgt, ccagctgatccttcattt, gaaagaaagtcttcactc, tgctgaaggcatctcgga, gatctcgaagcatgttag, gcaggttgcctgggaagt,
gggtgcagctgttctcag, actgggtgccctggcctg, ggctggccctcaccccag, tcaggaggaccaggcaac, agagcagtgctgagctgt, gcatgccttcttttgcaa,
gtctgtcttgtggtttgg, ttttgcaagagcaacccc, ctgatgtgtagaccttca, cctctctgtccccctttt, atattgtaagctcaggga, ggcctcttcattcattaa,
aaagccacaatcaaggtt, tcccggcacaggattttt, tctgcttagagctcctcc, ttctctaacctctctaat, aaacttagttttcaattt, ttgcatcgtaagcaaaaa,
tgattggttgaacatgaa, cttctgcattacagctat, ttttaggatgggctacct, ctcttagaataatttttt, agcttctcaattaaaaaa, agttgatttcctggggag,
aacagctgttctgtccgc, agaggccctcagctgtgg, gttctcattcgcgtgttc, ctaggtcacagtgacgtg, gacaaattgcccattcca, gaatacaatgggattgag,
tggaaacagcttaaaaac, aggtgaaaataataaata, ttgaaaaaaattataata, ttgggcttctttctaaat, cgttcacagagaagctca, gtaaataaatagaaatgg,
gggttgaggtatcagagg, taataaatattctataag, agaggtacaataaggttt, ctcaaggggctgggtcag, ctatcccagagccccaga, tccgattttggagacctc,
taatttatgtcctagagt, ctatagagtcgccaccct, gatgtctcagtttcgtat, cttcattgtcatgtaggc, ttctatgtagttgatgaa, gatgtcaaactcactcat,
ggctttgtagatgccttt, ctcttggagcttattaaa, ggcattcttcacctgctc, cacggccttgctcttgtt, ttcacagggaagaaatcg, atgacagcgccgtagcct,
cagcctgagggtcttcag, gttctcccccagggagtt, cacatgcgccttgatgtc, tgggtcttggttctcagc, ttggggcatcacctcctc, caggtaaaactggatcat,
ctcagacaaggcttggca, acccaggtaacccttaaa, gtcctccagcaaggactc, ctttaacaacaagttgtc, cagctgatccttcatttg, aaagaaagtcttcactct,
gctgaaggcatctcggag, atctcgaagcatgttagg, caggttgcctgggaagtg, ggtgcagctgttctcaga, ctgggtgccctggcctgg, gctggccctcaccccagt,
caggaggaccaggcaaca, gagcagtgctgagctgtg, catgccttcttttgcaag, tctgtcttgtggtttggt, tttgcaagagcaaccccc, tgatgtgtagaccttcac,
ctctctgtccccctttta, tattgtaagctcagggag, gcctcttcattcattaaa, aagccacaatcaaggttt, cccggcacaggattttt, ctgcttagagctcctcct,
tctctaacctctctaata, aacttagttttcaatttt, tgcatcgtaagcaaaaat, gattggttgaacatgaac, ttctgcattacagctatt, tttaggatgggctacctc,
tcttagaataatttttta, gcttctcaattaaaaaaa, gttgatttcctggggaga, acagctgttctgtccgca, gaggccctcagctgtggg, ttctcattcgcgtgttcc,
taggtcacagtgacgtgg, acaaattgcccattccag, aatacaatgggattgaga, ggaaacagcttaaaaaca, ggtgaaaataataaatat, tgaaaaaaattataatat,
tgggcttctttctaaatc, gttcacagagaagctcag, taaataaatagaaatggg, ggttgaggtatcagaggt, aataaatattctataaga, gaggtacaataaggtttc,
tcaaggggctgggtcagc, tatcccagagccccagat, ccgattttggagacctct, aatttatgtcctagagtc, tatagagtcgccaccctg, atgtctcagtttcgtatc,
ttcattgtcatgtaggct, tctatgtagttgatgaag, atgtcaaactcactcatg, gctttgtagatgcctttc, tcttggagcttattaaag, gcattcttcacctgctcc,
acggccttgctcttgttt, tcacagggaagaaatcga, tgacagcgccgtagcctc, agcctgagggtcttcagg, ttctcccccagggagttc, acatgcgccttgatgtct,

gggtcttggttctcagct, tggggcatcacctcctcc, aggtaaaactggatcatc, tcagacaaggcttggcaa, cccaggtaacccttaaag, tcctccagcaaggactcc,
tttaacaacaagttgtcc, agctgatccttcattga, aagaaagtcttcactctg, ctgaaggcatctcggaga, tctcgaagcatgttaggc, aggttgcctgggaagtgg,
gtgcagctgttctcagac, tgggtgccctggcctggg, ctggccctcaccccagtc, aggaggaccaggcaacag, agcagtgctgagctgtgc, atgccttcttttgcaagt,
ctgtcttgtggtttggtt, ttgcaagagcaacccccct, gatgtgtagaccttcacc, tctctgtcccccttttat, attgtaagctcagggagg, cctcttcattcattaaaa,
agccacaatcaaggtttc, ccggcacaggatttttc, tgcttagagctcctcctt, ctctaacctctctaataa, acttagtttcaatttt, gcatcgtaagcaaaaatg,
attggttgaacatgaact, tctgcattacagctattt, ttaggatgggctacctct, cttagaataatttttag, cttctcaattaaaaaaag, ttgatttcctggggagaa,
cagctgttctgtccgcag, aggccctcagctgtgggt, tctcattcgcgtgttcct, aggtcacagtgacgtgga, caaattgcccattccaga, atacaatgggattgagaa,
gaaacagcttaaaaacag, gtgaaaataataaatatt, gaaaaaaattataatatt, gggcttctttctaaatcg, ttcacagagaagctcagt, aaataaatagaaatgggg,
gttgaggtatcagaggta, ataaatattctataagag, aggtacaataaggtttct, caaggggctgggtcagct, atcccagagccccagatc, cgattttggagacctcta,
atttatgtcctagagtct, atagagtcgccaccctga, tgtctcagtttcgtatct, tcattgtcatgtaggctt, ctatgtagttgatgaaga, tgtcaaactcactcatgg,
ctttgtagatgcctttct, cttggagcttattaaagg, cattcttcacctgctcca, cggccttgctcttgtttt, cacagggaagaaatcgat, gacagcgccgtagcctca,
gcctgagggtcttcaggt, tctcccccagggagttca, catgcgccttgatgtctg, ggtcttggttctcagctt, ggggcatcacctcctcca, ggtaaaactggatcatct,
cagacaaggcttggcaac, ccaggtaacccttaaagt, cctccagcaaggactcct, ttaacaacaagttgtcca, gctgatccttcattgaa, agaaagtcttcactctgc,
tgaaggcatctcggagat, ctcgaagcatgttaggca, ggttgcctgggaagtggg, tgcagctgttctcagact, gggtgccctggcctgggc, tggccctcaccccagtca,
ggaggaccaggcaacaga, gcagtgctgagctgtgca, tgccttcttttgcaagtc, tgtcttgtggtttggttt, tgcaagagcaacccccctg, atgtgtagaccttcacct,
ctctgtcccccttttata, ttgtaagctcagggaggc, ctcttcattcattaaaaa, gccacaatcaaggtttcc, cggcacaggatttttct, gcttagagctcctccttc,
tctaacctctctaataaa, cttagtttcaattttg, catcgtaagcaaaaatga, ttggttgaacatgaactt, ctgcattacagctatttt, taggatgggctacctctc,
ttagaataatttttagc, ttctcaattaaaaaaagt, tgatttcctggggagaac, agctgttctgtccgcaga, ggccctcagctgtgggtt, ctcattcgcgtgttccta,
ggtcacagtgacgtggac, aaattgcccattccagaa, tacaatgggattgagaaa, aaacagcttaaaaacagg, tgaaaataataaatattg, aaaaaaattataatattg,
ggcttctttctaaatcgt, tcacagagaagctcagta, aataaatagaaatggggg, ttgaggtatcagaggtaa, taaatattctataagaga, ggtacaataaggtttctc,
aaggggctgggtcagcta, tcccagagccccagatcc, gattttggagacctctaa, tttatgtcctagagtcta, tagagtcgccaccctgat, gtctcagtttcgtatctt,
cattgtcatgtaggcttc, tatgtagttgatgaagat, gtcaaactcactcatggc, tttgtagatgcctttctc, ttggagcttattaaaggc, attcttcacctgctccac,
ggccttgctcttgttttc, acagggaagaaatcgatg, acagcgccgtagcctcag, cctgagggtcttcaggtt, ctcccccagggagttcac, atgcgccttgatgtctgg,
gtcttggttctcagcttg, gggcatcacctcctccag, gtaaaactggatcatctc, agacaaggcttggcaacc, caggtaacccttaaagtc, ctccagcaaggactcctt,
taacaacaagttgtccag, ctgatccttcattgaaa, gaaagtcttcactctgct, gaaggcatctcggagatc, tcgaagcatgttaggcag, gttgcctgggaagtgggt,
gcagctgttctcagactg, ggtgccctggcctgggct, ggccctcaccccagtcag, gaggaccaggcaacagag, cagtgctgagctgtgcat, gccttcttttgcaagtct,
gtcttgtggtttggtttt, gcaagagcaacccccctga, tgtgtagaccttcacctc, tctgtcccccttttatat, tgtaagctcagggaggcc, tcttcattcattaaaaag,
ccacaatcaaggtttccc, ggcacaggatttttctg, cttagagctcctccttct, ctaacctctctaataaac, ttagtttcaattttgc, atcgtaagcaaaaatgat,
tggttgaacatgaacttc, tgcattacagctattttt, aggatgggctacctctct, tagaataatttttagct, tctcaattaaaaaaagtt, gatttcctggggagaaca,
gctgttctgtccgcagag, gccctcagctgtgggttc, tcattcgcgtgttcctag, gtcacagtgacgtggaca, aattgcccattccagaat, acaatgggattgagaaat,
aacagcttaaaaacaggt, gaaaataataaatattga, aaaaaattataatattgg, gcttctttctaaatcgtt, cacagagaagctcagtaa, ataaatagaaatgggggt,
tgaggtatcagaggtaat, aaatattctataagagag, gtacaataaggtttctca, aggggctgggtcagctat, cccagagccccagatccg, attttggagacctctaat,
ttatgtcctagagtctat, agagtcgccaccctgatg, tctcagtttcgtatcttc, attgtcatgtaggcttct, atgtagttgatgaagatg, tcaaactcactcatggct,
ttgtagatgcctttctct, tggagcttattaaaggca, ttcttcacctgctccacg, gccttgctcttgttttca, cagggaagaaatcgatga, cagcgccgtagcctcagc,
ctgagggtcttcaggttc, tcccccagggagttcaca, tgcgccttgatgtctggg, tcttggttctcagcttgg, ggcatcacctcctccagg, taaaactggatcatctca,
gacaaggcttggcaaccc, aggtaacccttaaagtcc, tccagcaaggactccttt, aacaacaagttgtccagc, tgatccttcattgaaag, aaagtcttcactctgctg,
aaggcatctcggagatct, cgaagcatgttaggcagg, ttgcctgggaagtgggtg, cagctgttctcagactgg, gtgccctggcctgggctg, gccctcaccccagtcagg,
aggaccaggcaacagagc, agtgctgagctgtgcatg, ccttcttttgcaagtctg, tcttgtggtttggttttg, caagagcaacccccctgat, gtgtagaccttcacctct,
ctgtcccccttttatatt, gtaagctcagggaggcct, cttcattcattaaaaagc, cacaatcaaggtttcccg, gcacaggatttttctgc, ttagagctcctccttctc,
taacctctctaataaact, tagtttcaattttgca, tcgtaagcaaaaatgatt, ggttgaacatgaacttct, gcattacagctattttta, ggatgggctacctctctt,
agaataatttttagctt, ctcaattaaaaaaagttg, atttcctggggagaacag, ctgttctgtccgcagagg, ccctcagctgtgggttct, cattcgcgtgttcctagg,
tcacagtgacgtggacaa, attgcccattccagaata, caatgggattgagaaata, acagcttaaaaacaggtg, aaaataataaatattgaa, aaaaattataatattggg,
cttctttctaaatcgttc, acagagaagctcagtaaa, taaatagaaatgggggtt, gaggtatcagaggtaata, aatattctataagagagg, tacaataaggtttctcaa,
ggggctgggtcagctatc, ccagagccccagatccga, ttttggagacctctaatt, tatgtcctagagtctata, gagtcgccaccctgatgt, ctcagtttcgtatcttca,
ttgtcatgtaggcttcta, tgtagttgatgaagatgt, caaactcactcatggctt, tgtagatgcctttctctt, ggagcttattaaaggcat, tcttcacctgctccacgg,
ccttgctcttgttttcac, agggaagaaatcgatgac, agcgccgtagcctcagcc, tgagggtcttcaggttct, cccccagggagttcacat, gcgccttgatgtctgggt,
cttggttctcagcttggg, gcatcacctcctccaggt, aaaactggatcatctcag, acaaggcttggcaaccca, ggtaacccttaaagtcct, ccagcaaggactccttta,
acaacaagttgtccagct, gatccttcattgaaaga, aagtcttcactctgctga, aggcatctcggagatctc, gaagcatgttaggcaggt, tgcctgggaagtgggtgc,
agctgttctcagactggg, tgccctggcctgggctgg, ccctcaccccagtcagga, ggaccaggcaacagagca, gtgctgagctgtgcatgc, cttcttttgcaagtctgt,
cttgtggtttggttttgc, aagagcaacccccctgatg, tgtagaccttcacctctc, tgtcccccttttatattg, taagctcagggaggcctc, ttcattcattaaaaagcc,
acaatcaaggtttcccgg, cacaggatttttctgct, tagagctcctccttctct, aacctctctaataaactt, agtttcaattttgcat, cgtaagcaaaaatgattg,
gttgaacatgaacttctg, cattacagctatttttag, gatgggctacctctctta, gaataatttttagcttc, tcaattaaaaaaagttga, tttcctggggagaacagc,
tgttctgtccgcagaggc, cctcagctgtgggttctc, attcgcgtgttcctaggt, cacagtgacgtggacaaa, ttgcccattccagaatac, aatgggattgagaaataa,
cagcttaaaaacaggtga, aaataataaatattgaaa, aaaattataatattgggc, ttctttctaaatcgttca, cagagaagctcagtaaat, aaatagaaatgggggttg,
aggtatcagaggtaataa, atattctataagagaggt, acaataaggtttctcaag, gggctgggtcagctatcc, cagagccccagatccgat, tttggagacctctaattt,
atgtcctagagtctatag, agtcgccaccctgatgtc, tcagtttcgtatcttcat, tgtcatgtaggcttctat, gtagttgatgaagatgtc, aaactcactcatggcttt,
gtagatgcctttctcttg, gagcttattaaaggcatt, cttcacctgctccacggc, cttgctcttgttttcaca, gggaagaaatcgatgaca, gcgccgtagcctcagcct,
gagggtcttcaggttctc, ccccagggagttcacatg, cgccttgatgtctgggtc, ttggttctcagcttgggg, catcacctcctccaggta, aaactggatcatctcaga,
caaggcttggcaacccag, gtaacccttaaagtcctc, cagcaaggactcctttaa, caacaagttgtccagctg, atccttcattgaaagaa, agtcttcactctgctgaa,
ggcatctcggagatctcg, aagcatgttaggcaggtt, gcctgggaagtgggtgca, gctgttctcagactgggt, gccctggcctgggctggc, cctcaccccagtcaggag,
gaccaggcaacagagcag, tgctgagctgtgcatgcc, ttcttttgcaagtctgtc, ttgtggtttggttttgca, agagcaacccccctgatgt, gtagaccttcacctctct,
gtcccccttttatattgt, aagctcagggaggcctct, tcattcattaaaaagcca, caatcaaggtttcccggc, acaggatttttctgctt, agagctcctccttctcta,
acctctctaataaactta, gtttcaattttgcatc, gtaagcaaaaatgattgg, ttgaacatgaacttctgc, attacagctatttttagg, atgggctacctctcttag,
aataatttttagcttct, caattaaaaaaagttgat, ttcctggggagaacagct, gttctgtccgcagaggcc, ctcagctgtgggttctca, ttcgcgtgttcctaggtc,
acagtgacgtggacaaat, tgcccattccagaataca, atgggattgagaaataat, agcttaaaaacaggtgaa, aataataaatattgaaaa, aaattataatattgggct,
tctttctaaatcgttcac, agagaagctcagtaaata, aatagaaatgggggttga, ggtatcagaggtaataaa, tattctataagagaggta, caataaggtttctcaagg,
ggctgggtcagctatccc, agagccccagatccgatt, ttggagacctctaattta, tgtcctagagtctataga, gtcgccaccctgatgtct, cagtttcgtatcttcatt,
gtcatgtaggcttctatg, tagttgatgaagatgtca, aactcactcatggctttg, tagatgcctttctcttgg, agcttattaaaggcattc, ttcacctgctccacggcc,
ttgctcttgttttcacag, ggaagaaatcgatgacag, cgccgtagcctcagcctg, agggtcttcaggttctcc, cccagggagttcacatgc, gccttgatgtctgggtct,
tggttctcagcttggggc, atcacctcctccaggtaa, aactggatcatctcagac, aaggcttggcaacccagg, taacccttaaagtcctcc, agcaaggactcctttaac,
aacaagttgtccagctga, tccttcattgaaagaaa, gtcttcactctgctgaag, gcatctcggagatctcga, agcatgttaggcaggttg, cctgggaagtgggtgcag,
ctgttctcagactgggtg, ccctggcctgggctggcc, ctcaccccagtcaggagg, accaggcaacagagcagt, gctgagctgtgcatgcct, tcttttgcaagtctgtct,
tgtggtttggttttgcaa, gagcaacccccctgatgtg, tagaccttcacctctctg, tcccccttttatattgta, agctcagggaggcctctt, cattcattaaaaagccac,
aatcaaggtttcccggca, caggatttttctgctta, gagctcctccttctctaa, cctctctaataaacttag, tttcaattttgcatcg, taagcaaaaatgattggt,
tgaacatgaacttctgca, ttacagctatttttagga, tgggctacctctcttaga, ataatttttagcttctc, aattaaaaaaagttgatt, tcctggggagaacagctg,
ttctgtccgcagaggccc, tcagctgtgggttctcat, tcgcgtgttcctaggtca, cagtgacgtggacaaatt, gcccattccagaatacaa, tgggattgagaaataatt,
gcttaaaaacaggtgaaa, ataataaatattgaaaaa, aattataatattgggctt, ctttctaaatcgttcaca, gagaagctcagtaaataa, atagaaatgggggttgag,

gtatcagaggtaataaat, attctataagagaggtac, aataaggtttctcaaggg, gctgggtcagctatccca, gagccccagatccgattt, tggagacctctaatttat,
gtcctagagtctatagag, tcgccaccctgatgtctc, agtttcgtatcttcattg, tcatgtaggcttctatgt, agttgatgaagatgtcaa, actcactcatggctttgt,
agatgcctttctcttgga, gcttattaaaggcattct, tcacctgctccacggcct, tgctcttgttttcacagg, gaagaaatcgatgacagc, gccgtagcctcagcctga,
gggtcttcaggttctccc, ccagggagttcacatgcg, ccttgatgtctgggtctt, ggttctcagcttggggca, tcacctcctccaggtaaa, actggatcatctcagaca,
aggcttggcaacccaggt, aacccttaaagtcctcca, gcaaggactcctttaaca, acaagttgtccagctgat, ccttcatttgaaagaaag, tcttcactctgctgaagg,
catctcggagatctcgaa, gcatgttaggcaggttgc, ctgggaagtgggtgcagc, tgttctcagactgggtgc, cctggcctgggctggccc, tcaccccagtcaggagga,
ccaggcaacagagcagtg, ctgagctgtgcatgcctt, cttttgcaagtctgtctt, gtggtttggttttgcaag, agcaacccccctgatgtgt, agaccttcacctctctgt,
cccccttttatattgtaa, gctcagggaggcctcttc, attcattaaaaagccaca, atcaaggtttcccggcac, aggatttttctgcttag, agctcctccttctctaac,
ctctctaataaacttagt, tttcaattttgcatcgt, aagcaaaaatgattggtt, gaacatgaacttctgcat, tacagctattttaggat, gggctacctctcttagaa,
taatttttagcttctca, attaaaaaaagttgattt, cctggggagaacagctgt, tctgtccgcagaggccct, cagctgtgggttctcatt, cgcgtgttcctaggtcac,
agtgacgtggacaaattg, cccattccagaatacaat, gggattgagaaataattg,

### Example 23

TGF-beta1, 2 and 3 antisense oligonucleotides:

gtgccatcaatacctgcaaa, catcagttacatcgaaggag, tcttgggacacgcagcaagg, gaaatcaatgtaaagtggac, catgaactggtccatatcga,
gaggttctaaatcttgggac, gcactctggcttttgggttc, tagctcaatccgttgttcag, ccctagatccctcttgaaat, accaaggctctcttatgttt,
tcgagtgtgctgcaggtaga, tgaacagcatcagttacatc, gctgggttggagatgttaaa, agaggttctaaatcttggga, cgccggttggtctgttgtga,
ctgctttcaccaaattggaa, aagtatagatcaaggagagt, tgctcaggatctgcccgcgg, gtgctgttgtagatggaaat, agggcggcatgtctattttg,
taagcttattttaaatccca, tagctgcatttgcaagactt, tctgttgtgactcaagtctg, aagcaataggccgcatccaa, tcaatgtaaagtggacgtag,
attttagctgcatttgcaag, tgtagatggaaatcacctcc, ttaacactgatgaaccaagg, attgtaccctttgggttcgt, agatccctcttgaaatcaat,
tgtaaagtggacgtaggcag, ccattcgccttctgctcttg, tgttaaatctttggacttga, gaagggcggcatgtctattt, gaccctgctgtgctgagtgt,
gaactagtaccgccttttca, cgatcctcttgcgcatgaac, ccggccaaaagggaagagat, aaagagacgagtggctatta, aagtggaaatattaatacgg,
agatcaaggagagttgtttg, agttgtttttaaaagtcaga, tgtaacaactgggcagacag, ggtgttgtaacaactgggca, tacccacagagcacctggga,
gggatggcatcaaggtaccc, tcgtcatcatcattatcatc, aagggtgcctattgcatagc, ctcactgttaactctaagag, gcaaagtatttggtctccac,
caagttccttaagccatcca, ttatcttaatgcagactttc, cttacaagaagcttccttag, actggtgagcttcagcttgc, acttgagaatctgatatagc,
aggttcctgtctttatggtg, gtgtatccatttccacccta, cagcacagaagttggcattg, gcaaggagaagcagatgctt, agcaaggagaagcagatgct,
ttttccaagaattttagctg, ttcttgttacaagcatcatc, ttaaagaaggagcggttcgg, ctgggctgaaatttatatat, gggcagacagctaggagttt,
gtgtactcaccaaggtaccc, cccagcactttgggaggccg, ggctcacgcctgtaatccca, tgaccgtgaactcactattt, atagtggtgatggctataca,
ttttggttacctgcaaatct, gaacactcaccctgctgtgc, gaatggctctttaaacccta, gaagaaatggagttcagtgt, tttctcctggaagggagagg,
aaatgcaacgcgttcccaac, aatacgaaactttgcaaag, actagtaattctcagagcgg, aagaaactagtaattctcag, agtgcatgtttttaaaagga,
cagtagtgcatgtttttaaa, ctcagcacacagtagtgcat, agatgcaggagcaaaaaggt, caggtagacagactgagcgc, gcctcgatcctcttgcgcat,
gcggatggcctcgatcctct, ctcaggatctgcccgcggat, gctccggatagtcttccggg, agatggaaatcacctccggg, gttgtagatggaaatcacct,
ctggtactgttgtagatgga, aggcggctgccctccggctt, aacctccttggcgtagtact, attttataaacctccttggc, cggcatgtcgattttataaa,
cgggatggcatttcggagg, gtagggtctgtagaaagtgg, tgaagtagggtctgtagaaa, attctgaagtagggtctgta, aagcggacgattctgaagta,
cccaggttcctgtctttgtg, ggcagtgtaaacttatttta, ccatcaatacctgcaaatct, aggtgccatcaatacctgca, agttttctgatcaccactgg,
ttatagttttctgatcacca, cctagtggactttatagttt, acattagcaggagatgtggg, agggcaacaacattagcagg, actccagtctgtaggagggc,
tcctgcacatttctaaagca, cagcaattatcctgcacatt, atgtaaagagggcgaaggca, ctcttaaaatcaatgtaaag, ccaagatccctcttaaaatc,
cctttgggttcatggatcca, gcattgtaccctttgggttc, gcacagaagttagcattgta, ctgaggactttggtgtgttg, tcctgggacacacagcaagg,
tttagctgcatttacaagac, caaggactttagctgcattt, gtcattgtcaccgtgatttt, ccagttttaacaaacagaac, agatgccagttttaacaaac,
gttcattatatagtaacaca, atgaaaggttcattatatag, ttccaagggtaatgaaaggt, cttaagccatccatgagttt, cctggcttatttgagttcaa,
ttagtcctataacaactcac, gcaaagaaccatttacaatt, cttgcttaaactggcaaaga, acatgtaaagtagttactgt, acacattacatgtaaagtag,
taagatctacacattacatg, attcaaaggtactggccagc, tttgtagtgcaagtcaaaat, catgtcattaaatggacaat, cctacatttgtgcgaacttc,
ttcccccttttgaaaaactca, tttttaatcagcctgcaaag, actgggcagacagtttcgga, taacaactgggcagacagtt, tgttgtaacaactgggcaga,
cacagagcacctgggactgt, gtacccacagagcacctggg, tcaaggtacccacagagcac, tggcatcaaggtacccacag,

ggcgggatggcatcaaggta, tttgcaggtattgatggcac, ctccttcgatgtaactgatg, ccttgctgcgtgtcccaaga, tcgatatggaccagttcatg,
gtcccaagatttagaacctc, gaacccaaaagccagagtgc, atttcaagagggatctaggg, aaacataagagagccttggt, tctacctgcagcacactcga,
tcacaacagaccaaccggcg, ttccaatttggtgaaagcag, actctccttgatctatactt, ccgcgggcagatcctgagca, caaaatagacatgccgccct,
tgggatttaaaataagctta, cagacttgagtcacaacaga, ttggatgcggcctattgctt, ctacgtccactttacattga, ggaggtgatttccatctaca,
ccttggttcatcagtgttaa, acgaacccaaagggtacaat, attgatttcaagagggatct, ctgcctacgtccactttaca, caagagcagaaggcgaatgg,
tcaagtccaaagatttaaca, aaatagacatgccgcccttc, acactcagcacagcagggtc, tgaaaaggcggtactagttc, gttcatgcgcaagaggatcg,
atctcttccctttggccgg, taatagccactcgtctcttt, ccgtattaatatttccactt, caaacaactctccttgatct, ctgtctgcccagttgttaca,
tgcccagttgttacaacacc, tcccaggtgctctgtgggta, gatgataatgatgatgacga, gctatgcaataggcaccctt, ctcttagagttaacagtgag,
gtggagaccaaatactttgc, gaaagtctgcattaagataa, ctaaggaagcttcttgtaag, gcaagctgaagctcaccagt, tagggtggaaatggatacac,
caatgccaacttctgtgctg, aagcatctgcttctccttgc, cagctaaaattcttggaaaa, gatgatgcttgtaacaagaa, aaactcctagctgtctgccc,
gggtaccttggtgagtacac, cggcctcccaaagtgctggg, tgggattacaggcgtgagcc, tgtatagccatcaccactat, acactgaactccatttcttc,
cctctcccttccaggagaaa, gttgggaacgcgttgcattt, ccgctctgagaattactagt, ctgagaattactagtttctt, tttaaaaacatgcactactg,
atgcactactgtgtgctgag, gcgctcagtctgtctacctg, atgcgcaagaggatcgaggc, agaggatcgaggccatccgc, atccgcgggcagatcctgag,
cccggaagactatccggagc, cccggaggtgatttccatct, aggtgatttccatctacaac, tccatctacaacagtaccag, aagccggagggcagccgcct,
agtactacgccaaggaggtt, gccaaggaggtttataaaat, tttataaaatcgacatgccg, cctccgaaaatgccatcccg, ccactttctacagaccctac,
tttctacagaccctacttca, tacagaccctacttcagaat, tacttcagaatcgtccgctt, cacaaagacaggaacctggg, taaaataagtttacactgcc,
tgcaggtattgatggcacct, ccagtggtgatcagaaaact, tggtgatcagaaaactataa, cctgctaatgttgttgccct, gccctcctacagactggagt,
tgctttagaaatgtgcagga, tgccttcgccctctttacat, gattttaagagggatcttgg, tggatccatgaacccaaagg, gaacccaaagggtacaatgc,
tacaatgctaacttctgtgc, ccttgctgtgtgtcccagga, gtcttgtaaatgcagctaaa, aaatgcagctaaagtccttg, aaaatcacggtgacaatgac,
gttctgtttgttaaaactgg, gtttgttaaaactggcatct, tgtgttactatataatgaac, acctttcattacccttggaa, aaactcatggatggcttaag,
aattgtaaatggttctttgc, tctttgccagtttaagcaag, acagtaactactttacatgt, ctactttacatgtaatgtgt, catgtaatgtgtagatctta,
gctggccagtacctttgaat, ctttgcaggctgattaaaaa, aactgtctgcccagttgtta, tctgcccagttgttacaaca, acagtcccaggtgctctgtg,
cccaggtgctctgtgggtac, gtgctctgtgggtaccttga, ctgtgggtaccttgatgcca, taccttgatgccatcccgcc, ttccaccattagcacgcggg,
ccgtgaccagatgcaggatc,

### Example 24

Antisense oligonucleotides complementary to m-RNA of TGF-beta1:

cgatagtcttgcag, gtcgatagtcttgc, cttggacaggatct, ccaggaattgttgc, cctcaatttcccct, gatgtccacttgca, ctccaaatgtaggg, accttgctgtactg,
gtagtacacgatgg, cacgtagtacacga, catgttggacagct, gcacgatcatgttg, tgtactctgcttgaac, ctgatgtgttgaagaaca, ctctgatgtgttgaag,
ggaagtcaatgtacag, catgtcgatagtcttgca, agctgaagcaatagttgg, gtcatagatttcgttgtg, ctccacttttaacttgag, tgctgtatttctggtaca,
tgcaggtggatagt, ccatgtcgatagtc, ctccatgtcgatag, tgctgttgtacagg, gtgctgttgtacag, ttggcgtagtagtc, tccaccattagcac, gatttcgttgtggg,
gtgtactctgcttg, tgctgtgtgtactc, gctctgatgtgttg, gagctctgatgtgt, cacttttaacttgagcct, ttgctgaggtatcg, gataaccactctgg,
caaaagataaccactctg, cggtgacatcaaaag, gttatccctgctgt, gcagtgtgttatcc, tagtgaacccgttg, tgccatgaatggtg, gttcatgccatgaatg,
catgagaagcagga, gctttgcagatgct, gagctttgcagatg, tagttggtgtccag, ctgaagcaatagttgg, ggagctgaagcaat, caatgtacagctgc,
cggaagtcaatgtac, gcggaagtcaatgt, agttggcatggtag, gcagaagttggcat, tgctggttgtacag, ggttatgctggttg, cgtagtacacgatg,
acctccttggcgtagta, cgggggcggggcgggg, cggggcggggcggggcg, cggcgccgccgaggcgcccg, ccgaggtccttgcgg, cggcggtgccggga,
ctcggcggccggtag, cgctaaggcg, ccgcacaactccgg, gcgagtcgctgg, cggttgctgaggtatcg, ccgggagagcaacacgg, cgcttctcg,
ccattagcacgcggg, cgggctccg, ccggccacccggtcgcgg, cgagcacggcctcg, cgggcagcgggccgggcg, cgcggatggcctcg, cgatgcgcttccg,
cccgcggccggcggg, cgcagcccggagggcg, cggcgccccccg, cggcactgccgagagcgcg, cggggatgaaggcggcg, cgggtcggcgactcccg,
cgcctgagggacgccg, aagcgtccccggcg, cgcggggcagcgtcgcg, ccccgcgcctccgg, cggcggcggctcg, cgctccgggccgaggccg,
cggccccgcgggcg, cggacggggcgtcc, cggccggggccctcg, gggaaagctgaggc, tcgagggaaagctga, cctcgagggaaagc, gggctggtgtggtg,
gaacagggctggtgtg, gaacagggctggtg, agagcgcgaacagg, gagagcgcgaacagg, cgagagcgcgaacag, cccctggctcggggg, ccctggctcgggg,
cccctggctcgggg, tccccctggctcgg, ctccccctggctcg, tgcgcttccgcttcac, cctcgatgcgcttc, gatggcctcgatgc, ggatggcctcgatgc,
atggcctcgatgcgctt, cccggagggcggcatgggga, cctcagggagaagggcgc, gtaggagggcctcgaggg, ctgcaggggctgggggtc,
agggctggtgtggtgggg, ggcatggggaggcggcg, ccggagggcggcatgggg, gggggctggcgagccgc, ggacaggatctggccgcggatgg,
ccccctggctcgggggc, gggccgggcggcacctcc, gggcagcgggccgggcgg, acggcctcgggcagcggg, gggtgctgttgtacaggg,
gggtttccaccattagcacgcggg, tcatagatttcgtt, ttgtcatagattt, aagaacatatatatg, aagaacatatatat, ttgaagaacatatata,

cgatagtcttgcag, gtcgatagtcttgc, cttggacaggatct, ccaggaattgttgc, cctcaatttcccct, gatgtccacttgca, ctccaaatgtaggg, accttgctgtactg,
gtagtacacgatgg, cacgtagtacacga, catgttggacagct, gcacgatcatgttg, tgtactctgcttgaac, ctgatgtgttgaagaaca, ctctgatgtgttgaag,
ggaagtcaatgtacag, catgtcgatagtcttgca, agctgaagcaatagttgg, gtcatagatttcgttgtg, ctccacttttaacttgag, tgctgtatttctggtaca,
tgcaggtggatagt, ccatgtcgatagtc, ctccatgtcgatag, tgctgttgtacagg, gtgctgttgtacag, ttggcgtagtagtc, tccaccattagcac, gatttcgttgtggg,
gtgtactctgcttg, tgctgtgtgtactc, gctctgatgtgttg, gagctctgatgtgt, cacttttaacttgagcct, ttgctgaggtatcg, gataaccactctgg,
caaaagataaccactctg, cggtgacatcaaaag, gttatccctgctgt, gcagtgtgttatcc, tagtgaacccgttg, tgccatgaatggtg, gttcatgccatgaatg,
catgagaagcagga, gctttgcagatgct, gagctttgcagatg, tagttggtgtccag, ctgaagcaatagttgg, ggagctgaagcaat, caatgtacagctgc,
cggaagtcaatgtac, gcggaagtcaatgt, agttggcatggtag, gcagaagttggcat, tgctggttgtacag, ggttatgctggttg, cgtagtacacgatg,
acctccttggcgtagta, cgggggcggggcgggg, cggggcggggcggggcg, cggcgccgccgaggcgcccg, ccgaggtccttgcgg, cggcggtgccggga,
ctcggcggccggtag, cgctaaggcg, ccgcacaactccgg, gcgagtcgctgg, cggttgctgaggtatcg, ccgggagagcaacacgg, cgcttctcg,
ccattagcacgcggg, cgggctccg, ccggccacccggtcgcgg, cgagcacggcctcg, cgggcagcgggccgggcg, cgcggatggcctcg, cgatgcgcttccg,
cccgcggccggcggg, cgcagcccggagggcg, cggcgcccccg, cggcactgccgagagcgcg, cggggatgaaggcggcg, cgggtcggcgactcccg,
cgcctgagggacgccg, aagcgtccccggcg, cgcgggggcagcgtcgcg, ccccgcgcctccgg, cggcggcggctcg, cgctccgggccgaggccg,
cggccccgcgggcg, cggacggggcgtcc, cggccggggccctcg, gggaaagctgaggc, tcgagggaaagctga, cctcgagggaaagc, gggctggtgtggtg,
gaacagggctggtgtg, gaacagggctggtg, agagcgcgaacagg, gagagcgcgaacagg, cgagagcgcgaacag, cccctggctcggggg, ccctggctcgggg,
cccctggctcgggg, tccccctggctcgg, ctccccctggctcg, tgcgcttccgcttcac, cctcgatgcgcttc, gatggcctcgatgc, ggatggcctcgatgc,
atggcctcgatgcgctt, cccggagggcggcatgggga, cctcagggagaagggcgc, gtaggagggcctcgaggg, ctgcaggggctgggggtc,
agggctggtgtggtgggg, ggcatgggggaggcggcg, ccggagggcggcatgggg, ggggggctggcgagccgc, ggacaggatctggccgcggatgg,
ccccctggctcggggggc, gggccgggcggcacctcc, gggcagcgggccgggcgg, acggcctcgggcagcggg, gggtgctgttgtacaggg,
gggtttccaccattagcacgcggg, tcatagatttcgtt, ttgtcatagattt, aagaacatatatatg, aagaacatatatat, ttgaagaacatatata,

Antisense oligonucleotides complementary to m-RNA of TGF-beta2:

cacacagtagtgca, gcacacagtagtgc, gcttgctcaggatctgc, tactcttcgtcgct, cttggcgtagtact, gtaaacctccttgg, gtctattttgtaaacctcc,
gcatgtctattttgtaaacc, cggcatgtctattttgta, ctgtagaaagtggg, acaattctgaagtagggt, tcaccaaattggaagcat, gctttcaccaaattggaagc,
ctggcttttgggtt, tctgatatagctcaatcc, tcctagtggactttatag, tttttcctagtggact, caattatcctgcacatttc, gcaattatcctgcaca, gcagcaattatcctgc,
tggcattgtaccct, tgtgctgagtgtct, cctgctgtgctgagtg, cttgggtgttttgc, tttagctgcatttgcaag, gccacttttccaag, gatcagaaaagcgc,
accgtgaccagatg, gtagacaggctgag, tatcgagtgtgctg, ttgcgcatgaactg, ttgctcaggatctg, actggtgagcttca, atagtcttctgggg, gctcaggatagtct,
tgtagatggaaatcacct, tggtgctgttgtag, ttctcctggagcaa, ttttcggaggggaa, cgggatggcatttt, attgctgagacgtcaaat, tctccattgctgag,
ctctgaactctgct, aacgaaagactctgaact, tgggttctgcaaac, gttgttcaggcact, tctttggacttgagaatc, tgggttggagatgt, tgctgtcgatgtag,
acaactttgctgtcga, attcgccttctgct, gaaggagagccatt, tcagttacatcgaagg, tgaagccattcatgaaca, tcctgtctttatggtg, aaatcccaggttcc,
ggacagtgtaagcttatt, gtacaaaagtgcagca, tagatggtacaaaagtgc, cacttttatttgggatgatg, gcaaatcttgcttctagt, gtgccatcaatacc,
ggtatatgtggagg, tctgatcaccactg, agatgtggggtctt, caataacattagcagg, aagtctgtaggagg, tctgttgtgactcaag, gttggtctgttgtg,
caaagcacgcttct, tttctaaagcaataggcc, acgtaggcagcaat, atcaatgtaaagtggacg, ctagatccctcttg, ccatttccacccta, tgggttcgtgtatc,
tccagcacagaagt, ataaatacgggcatgc, agtgtctgaactcc, ataagctcaggacc, aggagaagcagatg, agcaaggagaagca, aatcttgggacacg,
tagagaatggttagaggt, gttttgccaatgtagtag, gcaagactttacaatc, gcatttgcaagactttac, ttggtcttgccact, cagcacacagtagt,
ctttcaccaaattggaag, caccaaattggaagc, tcaccaaattggaagc, ctctggcttttggg, cggcatgtctattttg, catcgttgtcgtcg, cgcttcttccgccg,
cgaaggagagccattcg, cgatgtagcg, cgtcaaatcg, cgtagtactcttcgtcg, cgcgctcgcaggcg, cggccgccctccggctcg, cgcggatcgcctcg,
gagcgcgaccgtgac, acctccttggcgtagta, agggcggcatgtctattttg, cagaagttggcattgtac, agggcggcatgtctattttgta, tgggacacgcagcaagg,
gcaggatcagaaaagc, gcaggtagacaggc, gcaagtccctggtgc, cctggagcaagtcc, cgtagtactcttcg, gagaatctgatatagctc,
ggagatgttaaatctttgg, gctgtcgatgtagc, ccaggttcctgtctttatgg, cagcagggacagtg, cttgcttctagttcttcac, gccatcaatacctgc,
ggtgccatcaatacc, ccactggtatatgtgg, ggactttatagttttctg, ctcaagtctgtaggag, ggtctgttgtgactc, ggcagcaattatcc, ggttcgtgtatccatttcc,
gcacagaagttggc, ccagcacagaagttgg, gtgctgagtgtctg, gctcaggaccctgc, gcagcaaggagaagc, ccaatgtagtagagaatgg, gctgcatttgcaag,
aaaaaagaaatcaa, aaaaaaagaaatcaa, aaaaaaaagaaatcaa, cagaataaaaaaa, tcagaataaaaaaa, ttgtttttaaaagt, agttgtttttaaaa,
aagttgtttttaaaa, aaagttgtttttaaaa, aaaagttgtttttaaaa, aaaaagttgtttttaaaa, aaaaaagttgtttttaaaa, aaaaaaagttgtttttaaaa,
aaaaaaaagttgtttttaaa, ttttaaaaaagtg, tttttaaaaaagtg, atttttaaaaaagtg, catttttaaaaaagt, gcatttttaaaaaa, tgcatttttaaaaaa,
agcttattttaaat, aagcttattttaaat, taagcttattttaaat, tgtaattattagat, atgtaattattagat, tgatgtaattatta, atgatgtaattatta, atggtattatataa,
tatggtattatataa, ttatggtattatataa, tttatggtattatataa, atttatggtattatataa, aatcatattagaaa, ttacaatcatatta, tttacaatcatatta

### Sequences, Sequence-Listing

| | No. | Sequences | Length | No. int. |
|---|---|---|---|---|
| TGF-beta 1 | 1 | CGATAGTCTTGCAG | 14 | 1 |
| | 2 | GTCGATAGTCTTGC | 14 | 2 |
| | 3 | CTTGGACAGGATCT | 14 | 3 |
| | 4 | CCAGGAATTGTTGC | 14 | 4 |
| | 5 | CCTCAATTTCCCCT | 14 | 5 |
| | 6 | GATGTCCACTTGCA | 14 | 6 |
| | 7 | CTCCAAATGTAGGG | 14 | 7 |
| | 8 | ACCTTGCTGTACTG | 14 | 8 |
| | 9 | GTAGTACACGATGG | 14 | 9 |
| | 10 | CACGTAGTACACGA | 14 | 10 |
| | 11 | CATGTTGGACAGCT | 14 | 11 |
| | 12 | GCACGATCATGTTG | 14 | 12 |
| | 13 | TGTACTCTGCTTGAAC | 16 | 13 |
| | 14 | CTGATGTGTTGAAGAACA | 18 | 14 |
| | 15 | CTCTGATGTGTTGAAG | 16 | 15 |
| | 16 | GGAAGTCAATGTACAG | 16 | 16 |
| | 17 | CATGTCGATAGTCTTGCA | 18 | 17 |
| | 18 | AGCTGAAGCAATAGTTGG | 18 | 18 |
| | 19 | GTCATAGATTTCGTTGTG | 18 | 19 |
| | 20 | CTCCACTTTTAACTTGAG | 18 | 20 |
| | 21 | TGCTGTATTTCTGGTACA | 18 | 21 |
| TGF-beta 2 | 22 | CACACAGTAGTGCA | 14 | 1 |
| | 23 | GCACACAGTAGTGC | 14 | 2 |
| | 24 | GCTTGCTCAGGATCTGC | 17 | 3 |
| | 25 | TACTCTTCGTCGCT | 14 | 4 |
| | 26 | CTTGGCGTAGTACT | 14 | 5 |
| | 27 | GTAAACCTCCTTGG | 14 | 6 |
| | 28 | GTCTATTTTGTAAACCTCC | 19 | 7 |
| | 29 | GCATGTCTATTTTGTAAACC | 20 | 8 |
| | 30 | CGGCATGTCTATTTTGTA | 18 | 9 |
| | 31 | GGCATCAAGGTACC | 14 | 10 |
| | 32 | CTGTAGAAAGTGGG | 14 | 11 |
| | 33 | ACAATTCTGAAGTAGGGT | 18 | 12 |
| | 34 | TCACCAAATTGGAAGCAT | 18 | 13 |
| | 35 | GCTTTCACCAAATTGGAAGC | 20 | 14 |
| | 36 | CTGGCTTTTGGGTT | 14 | 15 |
| | 37 | TCTGATATAGCTCAATCC | 18 | 16 |
| | 38 | TCCTAGTGGACTTTATAG | 18 | 17 |
| | 39 | TTTTTCCTAGTGGACT | 16 | 18 |
| | 40 | CAATTATCCTGCACATTTC | 19 | 19 |
| | 41 | GCAATTATCCTGCACA | 16 | 20 |
| | 42 | GCAGCAATTATCCTGC | 16 | 21 |
| | 43 | TGGCATTGTACCCT | 14 | 22 |
| | 44 | TGTGCTGAGTGTCT | 14 | 23 |
| | 45 | CCTGCTGTGCTGAGTG | 16 | 24 |
| | 46 | CTTGGGTGTTTTGC | 14 | 25 |
| | 47 | TTTAGCTGCATTTGCAAG | 18 | 26 |
| | 48 | GCCACTTTTCCAAG | 14 | 27 |
| IL-10 | 49 | CTTCTTTTGCAAGTCTGT | 18 | |
| | 50 | TGAGCTGTGCATGCCTTC | 18 | |
| | 51 | AGTCAGGAGGACCAG | 15 | |
| | 52 | TGGGTGCCCTGGCCT | 15 | |
| | 53 | CATGTTAGGCAGGTT | 15 | |
| | 54 | AGGCATCTCGGAGATCT | 17 | |
| | 55 | AAAGTCTTCACTCTGC | 16 | |
| | 56 | AACAAGTTGTCCAGCTG | 17 | |
| | 57 | GTAAAACTGGATCATCTC | 18 | |
| | 58 | CATCACCTCCTCCAG | 15 | |
| | 59 | GGGTCTTCAGGTTCTCCC | 18 | |
| | 60 | CACGGCCTTGCTCTTGTT | 18 | |
| | 61 | TTATTAAAGGCATTCTTC | 18 | |
| | 62 | AAGATGTCAAACTCACTC | 18 | |
| | 63 | GTAGTTGATGAAGATGTC | 18 | |
| | 64 | GATTTTGGAGACCTCT | 16 | |
| | 65 | TCAGCTATCCCAGAGC | 16 | |
| | 66 | GGCTGGGTCAGCTAT | 15 | |
| | 67 | AAATCGTTCACAGAGAAG | 18 | |
| | 68 | TCTTTCTAAATCGTTCAC | 18 | |
| TGF-beta3 | 69 | TCGAGCTTCCCCGA | | Immun 107 |
| | 70 | CCCGGAGCCGAAGG | | Immun 108 |
| | 71 | CCCGAGGAGCGGG | | Immun 109 |
| | 72 | ACGCAGCAAGGCGA | | Immun 110 |
| | 73 | CGGGTTGTCGAGCCG | | Immun 111 |
| | 74 | CGGCAGTGCCCCG | | Immun 112 |
| | 75 | CGGAATTCTGCTCG | | Immun 113 |
| | 76 | TTCGTTGTGCTCCG | | Immun 114 |
| | 77 | ATTCCGACTCGGTG | | Immun 115 |
| | 78 | ACGTGGGTCATCACCGT | | Immun116 |
| | 79 | CGAAGAAGCG | | Immun 117 |
| | 80 | CCTAATGGCTTCCA | | Immun 118 |
| | 81 | TCAGCAGGGCCAGG | | Immun 187 |
| | 82 | GCAAAGTTCAGCAGGGC | | Immun 188 |
| | 83 | GGCAAAGTTCAGCAGG | | Immun 189 |
| | 84 | GTGGCAAAGTTCAGCAGG | | Immun 190 |
| | 85 | GTGGCAAAGTTCAG | | Immun 191 |
| | 86 | GACCGTGGCAAAGTTCAG | | Immun 192 |
| | 87 | AGAGAGGCTGACCGT | | Immun 193 |
| | 88 | GACAGAGAGAGGCTGAC | | Immun 194 |
| | 89 | ACAGAGAGAGGCTGA | | Immun 195 |
| | 90 | GTGGACAGAGAGAGG | | Immun 196 |
| | 91 | CAAGTGGACAGAGAGAGG | | Immun 197 |
| | 92 | TCTTCTTGATGTGGCC | | Immun 198 |
| | 93 | CCCTCTTCTTCTTGATG | | Immun 199 |
| | 94 | CACCCTCTTCTTCT | | Immun 200 |
| | 95 | ATG GATTTCTTTGGCAT | | Immun 201 |
| | 96 | GGATTTCTTTGGC | | Immun 202 |
| | 97 | AAGTTGGACTCTCTTCTC | | Immun 203 |
| | 98 | TAAGTTG GACTCTCTTCT | | Immun 204 |
| | 99 | GACCTAAGTTGGACTC | | Immun 205 |
| | 100 | TTTCTAGACCTAAGTTGG | | Immun 206 |
| | 101 | CTGATTTCTAGACCTAAG | | Immun 207 |
| | 102 | GAAGCAGTAATTGGTGT | | Immun 208 |
| | 103 | GGAATCATCATGAGG | | Immun 209 |
| | 104 | GGGAATCATCATGAG | | Immun 210 |
| | 105 | GGTTGTCGAGCCGGT | | Immun 211 |
| | 106 | GTCCTCCCAACATAGTA | | Immun 212 |
| | 107 | GGGTCCTCCCAACA | | Immun 213 |

## Claims

1. At least one TGF-beta2 antisense oligonucleotide of SEQ ID NO: 30 or its active derivative which is a morpholino oligonucleotide, or comprises a locked nucleic acid, or comprises 1 to 20 additional nucleotides at the 3'- and/or 5'-end, for use in inhibiting the formation of metastasis in pancreatic cancer.

2. Antisense oligonucleotide or its active derivative for use according to claim 1, wherein the TGF-beta2 antisense oligonucleotide is inhibiting the synthesis of proteins involved in the formation of metastasis in pancreatic cancer.

3. Antisense oligonucleotide or its active derivative for use according to claim 1 or 2, wherein the cancer is pancreatic cancer.

4. Pharmaceutical composition comprising the TGF-beta2 antisense oligonucleotide or its active derivative according to claim 1 for use in inhibiting the formation of metastasis in pancreatic cancer.

## Patentansprüche

1. Mindestens ein TGF-beta2 Antisense-Oligonukleotid von SEQ ID NO: 30 oder sein aktives Derivat, welches ein Morpholino-Oligonukleotid ist, oder eine verschlossene Nukleinsäure (locked nucleic acid, LNA) umfasst, oder 1 bis 20 zusätzliche Nukleotide an dem 3'- und/ oder 5'-Ende umfasst, zur Verwendung bei der Hemmung der Metastasenbildung bei Bauchspeicheldrüsenkrebs.

2. Antisense-Oligonukleotid oder sein aktives Derivat zur Verwendung nach Anspruch 1, wobei
das TGF-beta2 Antisense-Oligonukleotid die Synthese von Proteinen hemmt, die an der Metastasenbildung bei Bauchspeicheldrüsenkrebs beteiligt sind.

3. Antisense-Oligonukleotid oder sein aktives Derivat zur Verwendung nach Anspruch 1 oder 2,
wobei der Krebs Bauchspeicheldrüsenkrebs ist.

4. Pharmazeutische Zusammensetzung, umfassend das TGF-beta2 Antisense-Oligonukleotid oder sein aktives Derivat nach Anspruch 1, zur Verwendung bei der Hemmung der Metastasenbildung bei Bauchspeicheldrüsenkrebs.

## Revendications

1. Au moins un oligonucléotide antisens TGF-beta2 de SEQ ID NO: 30 ou son dérivé actif qui est un oligonucléotide morpholino, ou qui comprend un acide nucléique verrouillé, ou qui comprend 1 à 20 nucléotides supplémentaires à l'extrémité 3' et/ou 5', à utiliser afin d'inhiber la formation de métastase dans le cancer du pancréas.

2. Oligonucléotide antisens ou son dérivé actif à utiliser selon la revendication 1, dans lequel l'oligonucléotide antisens TGF-beta2 inhibe la synthèse de protéines impliquées dans la formation de métastase dans le cancer du pancréas.

3. Oligonucléotide antisens ou son dérivé actif à utiliser selon la revendication 1 ou 2, dans lequel le cancer est le cancer du pancréas.

4. Composition pharmaceutique comprenant l'oligonucléotide antisens TGF-beta2 ou son dérivé actif selon la revendication 1 à utiliser afin d'inhiber la formation de métastase dans le cancer du pancréas.


### SEQUENCE LISTING

<110> Antisense Pharma GmbH
<120> Pharmaceutical composition
<130> A30067
<150> EP 05 715 605.1
   <151> 2005-02-28
<160> 107
<170> PatentIn version 3.3
<210> 1
   <211> 14
   <212> DNA
   <213> Artificial
<220>
   <223> Antisense oligonucleotide
<400> 1
   cgatagtctt gcag 14
<210> 2
   <211> 14
   <212> DNA
   <213> Artificial
<220>
   <223> Antisense oligonucleotide
<400> 2
   gtcgatagtc ttgc 14
<210> 3
   <211> 14
   <212> DNA
   <213> Artificial
<220>
   <223> Antisense oligonucleotide
<400> 3
   cttggacagg atct 14
<210> 4
   <211> 14
   <212> DNA
   <213> Artificial
<220>
   <223> Antisense oligonucleotide
<400> 4
   ccaggaattg ttgc 14
<210> 5
   <211> 14
   <212> DNA
   <213> Artificial
<220>
   <223> Antisense oligonucleotide
<400> 5
   cctcaatttc ccct 14
<210> 6
   <211> 14
   <212> DNA
   <213> Artificial
<220>
   <223> Antisense oligonucleotide
<400> 6
   gatgtccact tgca 14
<210> 7
   <211> 14
   <212> DNA
   <213> Artificial
<220>
   <223> Antisense oligonucleotide
<400> 7
   ctccaaatgt aggg 14
<210> 8
   <211> 14
   <212> DNA
   <213> Artificial
<220>
   <223> Antisense oligonucleotide
<400> 8
   accttgctgt actg 14
<210> 9
   <211> 14
   <212> DNA
   <213> Artificial
<220>
   <223> Antisense oligonucleotide
<400> 9
   gtagtacacg atgg 14
<210> 10
   <211> 14
   <212> DNA
   <213> Artificial
<220>
   <223> Antisense oligonucleotide
<400> 10
   cacgtagtac acga 14
<210> 11
   <211> 14
   <212> DNA
   <213> Artificial
<220>
   <223> Antisense oligonucleotide
<400> 11
   catgttggac agct 14
<210> 12
   <211> 14
   <212> DNA
   <213> Artificial
<220>
   <223> Antisense oligonucleotide
<400> 12
   gcacgatcat gttg 14
<210> 13
   <211> 16
   <212> DNA
   <213> Artificial
<220>
   <223> Antisense oligonucleotide
<400> 13
   tgtactctgc ttgaac 16
<210> 14
   <211> 18
   <212> DNA
   <213> Artificial
<220>
   <223> Antisense oligonucleotide
<400> 14
   ctgatgtgtt gaagaaca 18
<210> 15
   <211> 16
   <212> DNA
   <213> Artificial
<220>
   <223> Antisense oligonucleotide
<400> 15
   ctctgatgtg ttgaag 16
<210> 16
   <211> 16
   <212> DNA
   <213> Artificial
<220>
   <223> Antisense oligonucleotide
<400> 16
   ggaagtcaat gtacag 16
<210> 17
   <211> 18
   <212> DNA
   <213> Artificial
<220>
   <223> Antisense oligonucleotide
<400> 17
   catgtcgata gtcttgca 18
<210> 18
   <211> 18
   <212> DNA
   <213> Artificial
<220>
   <223> Antisense oligonucleotide
<400> 18
   agctgaagca atagttgg 18
<210> 19
   <211> 18
   <212> DNA
   <213> Artificial
<220>
   <223> Antisense oligonucleotide
<400> 19
   gtcatagatt tcgttgtg 18
<210> 20
   <211> 18
   <212> DNA
   <213> Artificial
<220>
   <223> Antisense oligonucleotide
<400> 20
   ctccactttt aacttgag 18
<210> 21
   <211> 18
   <212> DNA
   <213> Artificial
<220>
   <223> Antisense oligonucleotide
<400> 21
   tgctgtattt ctggtaca 18
<210> 22
   <211> 14
   <212> DNA
   <213> Artificial
<220>
   <223> Antisense oligonucleotide
<400> 22
   cacacagtag tgca 14
<210> 23
   <211> 14
   <212> DNA
   <213> Artificial
<220>
   <223> Antisense oligonucleotide
<400> 23
   gcacacagta gtgc 14
<210> 24
   <211> 17
   <212> DNA
   <213> Artificial
<220>
   <223> Antisense oligonucleotide
<400> 24
   gcttgctcag gatctgc 17
<210> 25
   <211> 14
   <212> DNA
   <213> Artificial
<220>
   <223> Antisense oligonucleotide
<400> 25
   tactcttcgt cgct 14
<210> 26
   <211> 14
   <212> DNA
   <213> Artificial
<220>
   <223> Antisense oligonucleotide
<400> 26
   cttggcgtag tact 14
<210> 27
   <211> 14
   <212> DNA
   <213> Artificial
<220>
   <223> Antisense oligonucleotide
<400> 27
   gtaaacctcc ttgg 14
<210> 28
   <211> 19
   <212> DNA
   <213> Artificial
<220>
   <223> Antisense oligonucleotide
<400> 28
   gtctattttg taaacctcc 19
<210> 29
   <211> 20
   <212> DNA
   <213> Artificial
<220>
   <223> Antisense oligonucleotide
<400> 29
   gcatgtctat tttgtaaacc 20
<210> 30
   <211> 18
   <212> DNA
   <213> Artificial
<220>
   <223> Antisense oligonucleotide
<400> 30
   cggcatgtct attttgta 18
<210> 31
   <211> 14
   <212> DNA
   <213> Artificial
<220>
   <223> Antisense oligonucleotide
<400> 31
   ggcatcaagg tacc 14
<210> 32
   <211> 14
   <212> DNA
   <213> Artificial
<220>
   <223> Antisense oligonucleotide
<400> 32
   ctgtagaaag tggg 14
<210> 33
   <211> 18
   <212> DNA
   <213> Artificial
<220>
   <223> Antisense oligonucleotide
<400> 33
   acaattctga agtagggt 18
<210> 34
   <211> 18
   <212> DNA
   <213> Artificial
<220>
   <223> Antisense oligonucleotide
<400> 34
   tcaccaaatt ggaagcat 18
<210> 35
   <211> 20
   <212> DNA
   <213> Artificial
<220>
   <223> Antisense oligonucleotide
<400> 35
   gctttcacca aattggaagc 20
<210> 36
   <211> 14
   <212> DNA
   <213> Artificial
<220>
   <223> Antisense oligonucleotide
<400> 36
   ctggcttttg ggtt 14
<210> 37
   <211> 18
   <212> DNA
   <213> Artificial
<220>
   <223> Antisense oligonucleotide
<400> 37
   tctgatatag ctcaatcc 18
<210> 38
   <211> 18
   <212> DNA
   <213> Artificial
<220>
   <223> Antisense oligonucleotide
<400> 38
   tcctagtgga ctttatag 18
<210> 39
   <211> 16
   <212> DNA
   <213> Artificial
<220>
   <223> Antisense oligonucleotide
<400> 39
   tttttcctag tggact 16
<210> 40
   <211> 19
   <212> DNA
   <213> Artificial
<220>
   <223> Antisense oligonucleotide
<400> 40
   caattatcct gcacatttc 19
<210> 41
   <211> 16
   <212> DNA
   <213> Artificial
<220>
   <223> Antisense oligonucleotide
<400> 41
   gcaattatcc tgcaca 16
<210> 42
   <211> 16
   <212> DNA
   <213> Artificial
<220>
   <223> Antisense oligonucleotide
<400> 42
   gcagcaatta tcctgc 16
<210> 43
   <211> 14
   <212> DNA
   <213> Artificial
<220>
   <223> Antisense oligonucleotide
<400> 43
   tggcattgta ccct 14
<210> 44
   <211> 14
   <212> DNA
   <213> Artificial
<220>
   <223> Antisense oligonucleotide
<400> 44
   tgtgctgagt gtct 14
<210> 45
   <211> 16
   <212> DNA
   <213> Artificial
<220>
   <223> Antisense oligonucleotide
<400> 45
   cctgctgtgc tgagtg 16
<210> 46
   <211> 14
   <212> DNA
   <213> Artificial
<220>
   <223> Antisense oligonucleotide
<400> 46
   cttgggtgtt ttgc 14
<210> 47
   <211> 18
   <212> DNA
   <213> Artificial
<220>
   <223> Antisense oligonucleotide
<400> 47
   tttagctgca tttgcaag 18
<210> 48
   <211> 14
   <212> DNA
   <213> Artificial
<220>
   <223> Antisense oligonucleotide
<400> 48
   gccacttttc caag 14
<210> 49
   <211> 18
   <212> DNA
   <213> Artificial
<220>
   <223> Antisense oligonucleotide
<400> 49
   cttcttttgc aagtctgt 18
<210> 50
   <211> 18
   <212> DNA
   <213> Artificial
<220>
   <223> Antisense oligonucleotide
<400> 50
   tgagctgtgc atgccttc 18
<210> 51
   <211> 15
   <212> DNA
   <213> Artificial
<220>
   <223> Antisense oligonucleotide
<400> 51
   agtcaggagg accag 15
<210> 52
   <211> 15
   <212> DNA
   <213> Artificial
<220>
   <223> Antisense oligonucleotide
<400> 52
   tgggtgccct ggcct 15
<210> 53
   <211> 15
   <212> DNA
   <213> Artificial
<220>
   <223> Antisense oligonucleotide
<400> 53
   catgttaggc aggtt 15
<210> 54
   <211> 17
   <212> DNA
   <213> Artificial
<220>
   <223> Antisense oligonucleotide
<400> 54
   aggcatctcg gagatct 17
<210> 55
   <211> 16
   <212> DNA
   <213> Artificial
<220>
   <223> Antisense oligonucleotide
<400> 55
   aaagtcttca ctctgc 16
<210> 56
   <211> 17
   <212> DNA
   <213> Artificial
<220>
   <223> Antisense oligonucleotide
<400> 56
   aacaagttgt ccagctg 17
<210> 57
   <211> 18
   <212> DNA
   <213> Artificial
<220>
   <223> Antisense oligonucleotide
<400> 57
   gtaaaactgg atcatctc 18
<210> 58
   <211> 15
   <212> DNA
   <213> Artificial
<220>
   <223> Antisense oligonucleotide
<400> 58
   catcacctcc tccag 15
<210> 59
   <211> 18
   <212> DNA
   <213> Artificial
<220>
   <223> Antisense oligonucleotide
<400> 59
   gggtcttcag gttctccc 18
<210> 60
   <211> 18
   <212> DNA
   <213> Artificial
<220>
   <223> Antisense oligonucleotide
<400> 60
   cacggccttg ctcttgtt 18
<210> 61
   <211> 18
   <212> DNA
   <213> Artificial
<220>
   <223> Antisense oligonucleotide
<400> 61
   ttattaaagg cattcttc 18
<210> 62
   <211> 18
   <212> DNA
   <213> Artificial
<220>
   <223> Antisense oligonucleotide
<400> 62
   aagatgtcaa actcactc 18
<210> 63
   <211> 18
   <212> DNA
   <213> Artificial
<220>
   <223> Antisense oligonucleotide
<400> 63
   gtagttgatg aagatgtc 18
<210> 64
   <211> 16
   <212> DNA
   <213> Artificial
<220>
   <223> Antisense oligonucleotide
<400> 64
   gattttggag acctct 16
<210> 65
   <211> 16
   <212> DNA
   <213> Artificial
<220>
   <223> Antisense oligonucleotide
<400> 65
   tcagctatcc cagagc 16
<210> 66
   <211> 15
   <212> DNA
   <213> Artificial
<220>
   <223> Antisense oligonucleotide
<400> 66
   ggctgggtca gctat 15
<210> 67
   <211> 18
   <212> DNA
   <213> Artificial
<220>
   <223> Antisense oligonucleotide
<400> 67
   aaatcgttca cagagaag 18
<210> 68
   <211> 18
   <212> DNA
   <213> Artificial
<220>
   <223> Antisense oligonucleotide
<400> 68
   tctttctaaa tcgttcac 18
<210> 69
   <211> 14
   <212> DNA
   <213> Artificial
<220>
   <223> Antisense oligonucleotide
<400> 69
   tcgagcttcc ccga 14
<210> 70
   <211> 14
   <212> DNA
   <213> Artificial
<220>
   <223> Antisense oligonucleotide
<400> 70
   cccggagccg aagg 14
<210> 71
   <211> 13
   <212> DNA
   <213> Artificial
<220>
   <223> Antisense oligonucleotide
<400> 71
   cccgaggagc ggg 13
<210> 72
   <211> 14
   <212> DNA
   <213> Artificial
<220>
   <223> Antisense oligonucleotide
<400> 72
   acgcagcaag gcga 14
<210> 73
   <211> 15
   <212> DNA
   <213> Artificial
<220>
   <223> Antisense oligonucleotide
<400> 73
   cgggttgtcg agccg 15
<210> 74
   <211> 13
   <212> DNA
   <213> Artificial
<220>
   <223> Antisense oligonucleotide
<400> 74
   cggcagtgcc ccg 13
<210> 75
   <211> 14
   <212> DNA
   <213> Artificial
<220>
   <223> Antisense oligonucleotide
<400> 75
   cggaattctg ctcg 14
<210> 76
   <211> 14
   <212> DNA
   <213> Artificial
<220>
   <223> Antisense oligonucleotide
<400> 76
   ttcgttgtgc tccg 14
<210> 77
   <211> 14
   <212> DNA
   <213> Artificial
<220>
   <223> Antisense oligonucleotide
<400> 77
   attccgactc ggtg 14
<210> 78
   <211> 17
   <212> DNA
   <213> Artificial
<220>
   <223> Antisense oligonucleotide
<400> 78
   acgtgggtca tcaccgt 17
<210> 79
   <211> 10
   <212> DNA
   <213> Artificial
<220>
   <223> Antisense oligonucleotide
<400> 79
   cgaagaagcg 10
<210> 80
   <211> 14
   <212> DNA
   <213> Artificial
<220>
   <223> Antisense oligonucleotide
<400> 80
   cctaatggct tcca 14
<210> 81
   <211> 14
   <212> DNA
   <213> Artificial
<220>
   <223> Antisense oligonucleotide
<400> 81
   tcagcagggc cagg 14
<210> 82
   <211> 17
   <212> DNA
   <213> Artificial
<220>
   <223> Antisense oligonucleotide
<400> 82
   gcaaagttca gcagggc 17
<210> 83
   <211> 16
   <212> DNA
   <213> Artificial
<220>
   <223> Antisense oligonucleotide
<400> 83
   ggcaaagttc agcagg 16
<210> 84
   <211> 18
   <212> DNA
   <213> Artificial
<220>
   <223> Antisense oligonucleotide
<400> 84
   gtggcaaagt tcagcagg 18
<210> 85
   <211> 14
   <212> DNA
   <213> Artificial
<220>
   <223> Antisense oligonucleotide
<400> 85
   gtggcaaagt tcag 14
<210> 86
   <211> 18
   <212> DNA
   <213> Artificial
<220>
   <223> Antisense oligonucleotide
<400> 86
   gaccgtggca aagttcag 18
<210> 87
   <211> 15
   <212> DNA
   <213> Artificial
<220>
   <223> Antisense oligonucleotide
<400> 87
   agagaggctg accgt 15
<210> 88
   <211> 17
   <212> DNA
   <213> Artificial
<220>
   <223> Antisense oligonucleotide
<400> 88
   gacagagaga ggctgac 17
<210> 89
   <211> 15
   <212> DNA
   <213> Artificial
<220>
   <223> Antisense oligonucleotide
<400> 89
   acagagagag gctga 15
<210> 90
   <211> 15
   <212> DNA
   <213> Artificial
<220>
   <223> Antisense oligonucleotide
<400> 90
   gtggacagag agagg 15
<210> 91
   <211> 18
   <212> DNA
   <213> Artificial
<220>
   <223> Antisense oligonucleotide
<400> 91
   caagtggaca gagagagg 18
<210> 92
   <211> 16
   <212> DNA
   <213> Artificial
<220>
   <223> Antisense oligonucleotide
<400> 92
   tcttcttgat gtggcc 16
<210> 93
   <211> 17
   <212> DNA
   <213> Artificial
<220>
   <223> Antisense oligonucleotide
<400> 93
   ccctcttctt cttgatg 17
<210> 94
   <211> 14
   <212> DNA
   <213> Artificial
<220>
   <223> Antisense oligonucleotide
<400> 94
   caccctcttc ttct 14
<210> 95
   <211> 17
   <212> DNA
   <213> Artificial
<220>
   <223> Antisense oligonucleotide
<400> 95
   atggatttct ttggcat 17
<210> 96
   <211> 13
   <212> DNA
   <213> Artificial
<220>
   <223> Antisense oligonucleotide
<400> 96
   ggatttcttt ggc 13
<210> 97
   <211> 18
   <212> DNA
   <213> Artificial
<220>
   <223> Antisense oligonucleotide
<400> 97
   aagttggact ctcttctc 18
<210> 98
   <211> 18
   <212> DNA
   <213> Artificial
<220>
   <223> Antisense oligonucleotide
<400> 98
   taagttggac tctcttct 18
<210> 99
   <211> 16
   <212> DNA
   <213> Artificial
<220>
   <223> Antisense oligonucleotide
<400> 99
   gacctaagtt ggactc 16
<210> 100
   <211> 18
   <212> DNA
   <213> Artificial
<220>
   <223> Antisense oligonucleotide
<400> 100
   tttctagacc taagttgg 18
<210> 101
   <211> 18
   <212> DNA
   <213> Artificial
<220>
   <223> Antisense oligonucleotide
<400> 101
   ctgatttcta gacctaag 81
<210> 102
   <211> 17
   <212> DNA
   <213> Artificial
<220>
   <223> Antisense oligonucleotide
<400> 102
   gaagcagtaa ttggtgt 17
<210> 103
   <211> 15
   <212> DNA
   <213> Artificial
<220>
   <223> Antisense oligonucleotide
<400> 103
   ggaatcatca tgagg 15
<210> 104
   <211> 15
   <212> DNA
   <213> Artificial
<220>
   <223> Antisense oligonucleotide
<400> 104
   gggaatcatc atgag 15
<210> 105
   <211> 15
   <212> DNA
   <213> Artificial
<220>
   <223> Antisense oligonucleotide
<400> 105
   ggttgtcgag ccggt 15
<210> 106
   <211> 17
   <212> DNA
   <213> Artificial
<220>
   <223> Antisense oligonucleotide
<400> 106
   gtcctcccaa catagta 17
<210> 107
   <211> 14
   <212> DNA
   <213> Artificial
<220>
   <223> Antisense oligonucleotide
<400> 107
   gggtcctccc aaca 14